# EUROPÄISCHE PATENTSCHRIFT

(11) **EP 1 165 563 B1**
(45) Veröffentlichungstag und Bekanntmachung des Hinweises auf die Patenterteilung: **02.03.2005**
(21) Anmeldenummer: 00916932.7
(22) Anmeldetag: 13.03.2000
(51) Int. Cl.: C07D 487/04, A61K 31/395, A01N 43/713

(54) **BICYCLISCHE HETEROCYCLEN, VERFAHREN ZU IHRER HERSTELLUNG UND IHRE VERWENDUNG ALS HERBIZIDE UND PHARMAZEUTISCHE MITTEL**
BICYCLIC HETEROCYCLES, METHOD FOR PRODUCING THEM AND THEIR USE AS HERBICIDES AND PHARMACEUTICAL AGENTS
HETEROCYCLES BICYCLIQUES, LEUR PROCEDE DE PRODUCTION ET LEUR UTILISATION COMME HERBICIDES ET PRODUITS PHARMACEUTIQUES

(30) Priorität: 20.03.1999 DE 19912636
(43) Veröffentlichungstag der Anmeldung: 02.01.2002
(73) Patentinhaber: Bayer CropScience GmbH, 65929 Frankfurt/Main (DE)
(72) Erfinder: BOJACK, Guido, D-65207 Wiesbaden (DE); LINDELL, Stephen, D-65779 Kelkheim-Fischbach (DE); ROSINGER, Christopher, D-65719 Hofheim (DE); DUDFIELD, Philip, Saffron Walden CB10 2LG (GB); EARNSHAW, Christopher, Chesterton, Cambridge CB4 1RS (GB)
(86) Internationale Anmeldenummer: PCT/EP2000/002206
(87) Internationale Veröffentlichungsnummer: WO 2000/056734

(56) Entgegenhaltungen:
- WO-A-94/18200
- DUDFIELD, PHILIP J. ET AL: "Synthesis of C-ribosyl 1,2,4-triazolo[3,4-f ][1,2,4]triazines as inhibitors of adenosine and AMP deaminases" J. CHEM. SOC., PERKIN TRANS. 1 (20), 2937-2942 , 1999, XP000916905
- DUDFIELD, PHILIP J. ET AL: "Synthesis of C-ribosyl imidazo[2,1-f ][1,2,4]triazines as inhibitors of adenosine and AMP deaminases" J. CHEM. SOC., PERKIN TRANS. 1 , (20), 2929-2936 , 1999, XP000916904
- LINDELL, STEPHEN D. ET AL: "The design and synthesis of inhibitors of adenosine 5'-monophosphate deaminase" BIOORG. MED. CHEM. LETT. , 9(14), 1985-1990 , 1999, XP004171623
- AVILA, JOSE LUIS ET AL: "Action of pyrazolopyrimidine derivatives on american Leishmania species promastigotes" COMP. BIOCHEM. PHYSIOL., C: COMP. PHARMACOL. TOXICOL.. , 83C(2), 285-9 , 1986, XP000916769
- TAKEUCHI, TOMIO ET AL: "Antiviral effect of formycin derivatives" J. ANTIBIOT., SER. A , 20(5), 297-8 , 1967, XP000916768
- CHEMICAL ABSTRACTS, vol. 93, no. 25, 1980 Columbus, Ohio, US; abstract no. 233857, XP002143999 & BIOCHEMISTRY, Bd. 19, Nr. 23, 1980, Seiten 5303-9,

## Beschreibung

Die Erfindung betrifft Wirkstoffe für den Einsatz in Landwirtschaft, Gartenbau und/oder Pharma, z. B. als Wirkstoffe für den Pflanzenschutz oder als Arzneimittel für die Anwendung an Mensch oder Tier. Vorzugsweise betrifft die Erfindung chemische Wirkstoffe für Pflanzenschutzmittel wie Herbizide oder Pflanzenwachsumsregulatoren, beispielsweise Herbizide zur selektiven Bekämpfung von Schadpflanzen in Nutzpflanzenkulturen oder Herbizide für den nichtselektiven Einsatz zur Bekämpfung von unerwünschtem Pflanzenwuchs. Außerdem betrifft die Erfindung vorzugsweise auch Arzneimittel zur Behandlung von Krankheiten, die durch Beinflussung oder Inhibierung des Enzyms Adenosinmonophosphatdeaminase behandelt werden können.

Adenosinmonophosphatdeaminase (AMPDA) ist ein Enzym, das in Zellen die Deaminierung von Adenosinmonophosphat (AMP) zu lnosinmonophosphat (IMP) katalysiert. Die Bedeutung dieses Enzyms, vor allem für den Stoffwechsel höherer biologischer Organismen ist die Grundlage dafür, daß mit der Beeinflussung der Enzymaktivität, z. B. durch Inhibitoren, eine biologische Wirkung sowohl in Pflanzen als auch bei Mensch und Tier entfaltet werden kann. Unterschiede in der Struktur der AMPDA-Enzyme und im biologischen Umfeld von Pflanzen und Tieren können jedoch prinzipiell unterschiedliche Enzymaktivitäten einerseits und unterschiedliche Effekte beim Einsatz derselben Enzyminhibitoren in verschiedenen Organismen ergeben.

Einige Inhibitoren des Enzyms AMPDA sind bereits bekannt. WO-A-96/1326 (US-A-5,786,165) beschreibt Hemmstoffe des Enzyms AMPDA in Pflanzen. Die Hemmstoffe können als Herbizide angewendet werden.

Aus WO-A-94/18200 (US-A-5,731,432) sind Hemmstoffe von AMPDA und deren vielfältige pharmazeutische Anwendungen beschrieben, z. B. als Mittel gegen Krankheiten, die durch Sauerstoffmangel im Gewebe mit verursacht werden, beispielsweise Herz-Kreislauferkrankungen, Entzündungen, Arthritis.

Aus Biochemistry, 19 (1980), 5303 (C.A. 93:233857) sind synthetische AMPDA-Inhibitoren bekannt, z. B. Nebularin-5'-Monophosphat, einem Purinderivat.

Die bekannten Wirkstoffe vom Typ der AMPDA-Inhibitoren weisen jedoch teilweise Nachteile auf, sei es unzureichende Wirkung, unzureichende Stabilität, schlechte Herstellbarkeit, unerwünschte Nebenwirkungen oder mangelnde Abbaubarkeit in biologischen Systemen. Es bestand deshalb ein Bedarf nach alternativen Wirkstoffen, die als AMPDA-Inhibitoren eingesetzt werden können. Vorzugsweise sollen die Verbindungen als Herbizide oder Pflanzenwachstumsregulatoren eingesetzt werden können.

Gegenstand der vorliegenden Erfindung ist die Verwendung von Verbindungen der Formel (I), deren Tautomeren, deren Salzen und deren Wasseradditionsprodukten, 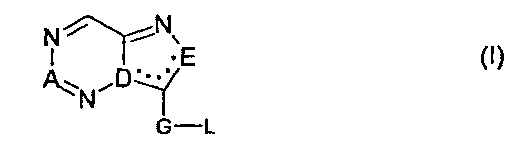 wobei in Formel (I)
- A: ein Stickstoffatom oder eine Gruppe der Formel C-R bedeutet, wobei R weiter unten definiert ist,
- D: ein Kohtenstoffatom oder ein Stickstoffatom bedeutet,
- E: a) im Fall, daß D ein Stickstoffatom ist, ein Stickstoffatom oder eine
Gruppe der Formel C-R^{O} bedeutet, wobei R^{O} weiter unten definiert ist, oder
b) im Fall, daß D ein Kohlenstoffatom ist, eine Gruppe der Formel N-R^{O}, -O-, -S-, -SO- oder -SO₂- bedeutet,
die Reihe von Punkten (•••••) von D über ein benachbartes Ring-C-atom bis E eine Doppelbindung zwischen dem Ring-C-Atom und E, wenn D ein Stickstoffatom ist (Fall a), oder
eine Doppelbindung zwischen dem Ring-C-Atom und D, wenn D ein Kohlenstoffatom ist (Fall b), bedeutet,
- R, R⁰: unabhängig voneinander jeweils ein Wasserstoffatom. Amino, Hydroxy, Mercapto, Cyano, Halogen, Azido, Nitro, SF₅, gegebenenfalls substituiertes Aminosulfonyl, vorzugsweise Aminosulfonyt oder Mono- oder Di(C₁-C₄)alkyl-aminosulfonyl, oder Acyl, Acylamino, vorzugsweise dabei Monoacylamino, Diacylamino oder N-Acyl-N-(C₁-C₄)alkyl-amino, oder Acyloxy, Acylthio, Mono- oder Di-(C₁-C₄)alkylamino, Mono- oder Di-(C₃-C₉)cycloalkylamino, (C₁-C₄)Alkylthio, (C₂-C₄)Alkenylthio, (C₂-C₄)Alkinylthio, (C₃-C₉)Cycloalkylthio, (C₅-C₉)Cycloalkenylthio, (C₁-C₄)Alkylsulfinyl, (C₁-C₄)Alkylsulfonyl, (C₁-C₄)Alkoxy, (C₂-C₄)Alkenyloxy, (C₂-C₄)Alkinyloxy, (C₃-C₉)Cycloalkoxy, (C₅-C₉)Cycloalkenyloxy, (C₁-C₄)Alkyl, (C₂-C₄)Alkenyl, (C₂-C₄)Alkinyl, (C₃-C₉)Cycloalkyl, (C₅-C₉)Cycloalkenyl, (C₁-C₄)Alkylaminosulfonyl oder Di[(C₁-C₄)alkyl]aminosulfonyl, wobei jeder der letztgenannten 23 Reste unsubstituiert oder im Kohlenwasserstoffteil durch einen oder mehrere Reste aus der Gruppe Halogen, Hydroxy, Amino, Nitro, Formyl, Carboxy, Cyano, Thiocyanato, (C₁-C₄)Alkoxy, (C₃-C₉)Cycloalkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, (C₃-C₉)Cycloalkyl-amino, [(C₁-C₄)Alkyl]carbonyl, [(C₁-C₄)Alkoxy]carbonyl, Aminocarbonyl, Mono(C₁-C₄)alkylamino-carbonyl und Di(C₁-C₄)alkylamino-carbonyl substituiert ist, bedeutet,
- G: eine divalente geradkettige gesättigte oder ungesättigte Kohlenwasserstoffbrücke mit 1 bis 24 C-Atomen, vorzugsweise 1 bis 12 C-Atomen, besonders 1 bis 8 C-Atomen, ganz besonders 4 bis 6 C-Atomen, in der Kette bedeutet, in der ein oder mehrere Kettenglieder jeweils unabhängig voneinander durch O, S, NH, (C₁-C₄)Alkyl-N oder Acyl-N, vorzugsweise O, S, NH oder (C₁-C₄)Alkyl-N ausgetauscht sein können oder, im ungesättigten Fall, eine oder mehrere CH-Gruppen jeweils durch ein Stickstoffatom ausgetauscht sein können,
wobei die jeweilige Brücke unsubstituiert ist oder
(a) durch einen oder mehrere gleiche oder verschiedene Reste aus der Gruppe Halogen, Nitro, von Wasserstoff verschiedene Reste der Formel R', Reste der Formel R²R³C= und Reste der Formel L* substituiert ist, wobei R¹, R², R³ und L* weiter unten definiert sind,
(b) zwei oder vier Substituenten trägt, von denen jeweils zwei zusammen mit dem sie verbindenden Brückenteil einen carbocyclischen oder heterocyclischen Ring mit 3 bis 7 Ringatomen bilden, wobei im Fall eines Heterocyclus die Heteroatome, vorzugsweise 1, 2 oder 3 Heteroatome, aus der Gruppe N, O und S, vorzugsweise ein oder zwei Heteroatome aus der Gruppe O und S, insbesondere ein Sauerstoffatom, ausgewählt sind und wobei der jeweilige Ring noch ankondensierte Ringe aufweisen kann und im übrigen unsubstituiert oder durch einen oder mehrere gleiche oder verschiedene Reste aus der Gruppe Halogen, Nitro, von Wasserstoff verschiedene Reste der Formel R¹, Reste der Formel L* und Oxo substituiert ist, wobei R¹ und L* weiter unten definiert sind,
(c) über eine zweite direkte Bindung oder über ein Heteroatom aus der Gruppe N, O und S mit L cyclisch verbunden ist,
(d) zwei oder mehrere Substituenten aus den vorstehenden Gruppen (a) bis (c) gemeinsam aufweist,
und G vorzugsweise inklusive der in Formel (I) nicht durch Symbole angezeigten Substituenten 1 bis 30 C-Atome, insbesondere 1 bis 20 C-Atome, ganz besonders 1 bis 12 C-Atome enthält,
- L, L*: unabhängig voneinander jeweils OR⁴, SR⁴, CN, Tetrazolo, C(OR⁵)(OR⁶)(OR⁷), -Z¹, -O-Z², -S-Z² oder -NH-Z², wobei R⁴, R⁵, R⁶, R⁷, Z¹ und Z² weiter unten definiert ist und wobei L über eine zweite direkte Bindung oder über ein Heteroatom aus der Gruppe N, O und S mit der Brücke G cyclisch verbunden sein kann,
- Z¹, Z²: unabhängig voneinander jeweils den Rest einer anorganischen oder organischen Sauerstoffsäure der Formel Z¹-OH bzw. Z²-OH, wobei der Rest formal durch Abtrennen der Hydroxygruppe an der Säurefunktion entsteht,
- R¹ bis R⁷: unabhängig voneinander jeweils ein Wasserstoffatom, (C₁-C₆)Alkyl, (C₂-C₆)Alkenyl, (C₂-C₆)Alkinyl, (C₃-C₉)Cycloalkyl, (C₅-C₉)Cycloalkenyl, Aryl oder Heterocyclyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosulfonyl, Acyl, Acylamino, Acyloxy, Acylthio, [(C₁-C₄)Alkoxy]carbonyl, Mono(C₁-C₄)alkylamino, Mono(C₃-C₉)cycloalkylamino, Di(C₁-C₄)alkylamino, (C₁-C₄)Alkylthio, (C₂-C₄)Alkenylthio, (C₂-C₄)Alkinylthio, (C₃-C₉)Cycloalkylthio, (C₅-C₉)Cycloalkenylthio, (C₁-C₄)Alkylsulfinyl, (C₁-C₄)Alkylsulfonyl, (C₁-C₄)Alkoxy, (C₂-C₄)Alkenyloxy, (C₂-C₄)Alkinyloxy, (C₃-C₉)Cycloalkoxy, (C₅-C₉)Cycloalkenyloxy, (C₃-C₉)Cycloalkyl, (C₅-C₉)Cycloalkenyl, Aryl, substituiertes Aryl, Heteroaryl, substituiertes Heteroaryl und im Falle cyclischer Reste auch (C₁-C₄)Alkyl, (C₂-C₄)Alkenyl, (C₂-C₄)Alkinyl, (C₁-C₄)Haloalkyl, (C₂-C₄)Haloalkenyl, (C₂-C₄)Haloalkinyl, (C₁-C₄)Hydroxyalkyl und (C₁-C₄)Alkoxy(C₁-C₄)alkyl substituiert ist, bedeuten,
wobei Heteracyclyl ein heterocyclischer gesättigter, ungesättigter oder heteroaromatischer Ring mit vorzugsweise 3 bis 9 Ringatomen und 1 bis 3 Heteroatomen aus der Gruppe N, O und S ist und
wobei Heteroaryl vorzugsweise ein heteroaromatischer Ring mit vorzugsweise 5 bis 6 Ringatomen und 1 bis 3 Heteroatomen aus der Gruppe N, O und S ist und
wobei die Substituenten für substituiertes Aryl oder substituiertes Heteroaryl vorzugsweise ein oder mehrere aus der Gruppe Halogen, Nitro, (C₁-C₄)Alkyl, (C₁-C₄)Haloalkyl, (C₁-C₄)Alkoxy, (C₁-C₄)Haloalkoxy und (C₁-C₄)Alkylthio sind, oder
R², R³ zusammen mit dem C-Atom der Gruppe R²R³C= einen nicht aromatischen carbocyclischen Ring oder einen heterocyclischen Ring mit 3 bis 9 Ringatomen und 1 bis 4 Heteroringatomen aus der Gruppe N, O und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten oder
R⁵, R⁶ zusammen mit dem C-Atom und den benachbarten Sauerstoffatomen der Gruppe C(OR⁵)(OR⁶)(OR⁷) einen gesättigen oder ungesättigten nicht aromatischen heterocyclischen Ring mit 4 bis 9 Ringatomen und 1 bis 4 Heteroringatomen aus der Gruppe N, O, P und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, oder die Gruppe C(OR⁵)(OR⁶)(OR⁷) zusammen einen bicyclischen Rest der Formel 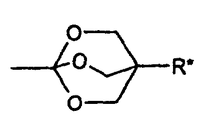 worin
R* (C₁-C₄)Alkyl, (C₁-C₄)Haloalkyl, (C₁-C₄)Alkoxy, (C₁-C₄)Haloalkoxy, (C₁-C₄)Alkylthio und Phenyl, das unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist,
bedeuten, zwecks direkter oder indirekter Hemmung des Enzyms Adenosinmonophosphatdeaminase (AMPDA) oder Adenosindeaminase (ADA), vorzugsweise unter physiologischen Bedingungen oder analogen wäßrigen Bedingungen, insbesondere als Herbizide im Pflanzenschutz oder zur Herstellung von pharmazeutischen Mitteln zur Behandlung von Krankheiten, welche durch Inhibierung des Enzms AMPDA oder ADA behandelt werden können, ausgenommen die Verwendung zur chirurgischen oder therapeutischen Behandlung des menschlichen oder tierischen Körpers oder die Verwendung in Diagnostizierverfahren, die am menschlichen oder tierischen Körper durchgeführt werden.

Gegenstand der Erfindung sind auch alle neuen Verbindungen der Formel (I) und deren Salze. Teilstrukturen der Verbindungen (I) stimmen mit denen der Naturstoffe Formycin A (7-Amino-3-(β-D-ribofuranosyl)-pyrazolo[4,3-d]pyrimidin) und Formycin B (7-Oxo-3-(β-D-ribofuranosyl)-pyrazolo[4,3-d]pyrimidin) überein. Bekannt ist bereits das Deaminoformycin, d. h. die Verbindung der Formel (I), worin A = CH, D = C, E = NH und G-L = β-D-Ribofuranosyl bedeuten; speziell ist dabei G-L ein Rest der Formel (GL1) mit L = Hydroxy,

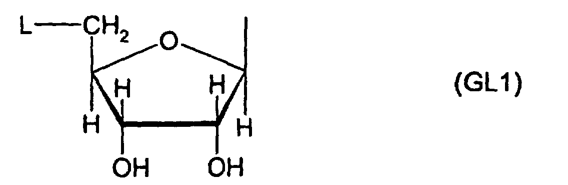

G. H. Milne, L. B. Townsend, J. Chem. Soc. Perkin Trans. I, 1972, 2677, beschreiben die Herstellung von Deaminoformycin aus 7-Chlor-3-(β-Dribofuranosyl)-pyrazolo[4,3-d]pyrimidin. Aus S. Watanabe et al., J. Antibiotic. Ser. A., 19 (1966) 93 sind Derivate des Formycins bekannt, unter anderem auch Deaminoformycin, für die eine fungizide Wirkung gegen Xanthomonas oryzae angegeben wird. Aus J. Antibiot. Ser. A (1967), 20(5), 267-8 und Comp. Biochem. Physiol. Vol. 83C, (2), 258-9 sind Deaminoformycin oder strukturverwandte Verbindungen und deren schwach antibakterielle und antivirale Eigenschaften beschrieben. Die genannten Druckschriften beschreiben weder eine Inhibierung des Enzyms AMPDA oder ADA durch Einwirkung eines der Formycinderivate, noch lehren sie deren Anwendung als Herbizide oder für pharmazeutische Zwecke.

Viele der Verbindungen (I) treten in tautomeren Formen auf, d. h. chemischen Verbindungen, die durch Umlagerung, vorzugsweise durch Prototropie (= Wasserstoffverschiebung) in Verbindung mit einer Verlagerung von Doppelbindungen, entstehen und meist miteinander im Gleichgewicht stehen. Besonders zu beachten sind tautomere Formen, die sich aus Verbindungen mit D = C-Atom und E = NH ergeben, wobei das Wasserstoffatom an das andere Stickstoffatom im Fünfring verlagert wird:

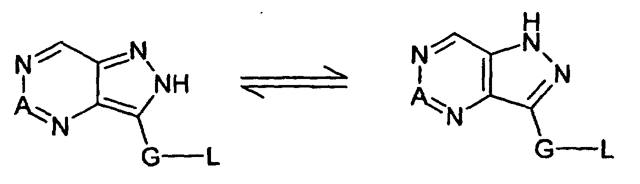

Die Verbindungen (I) reagieren unter sauren bis neutralen wäßrigen Bedingungen leicht unter Addition von Wasser zu Verbindungen der Formel (I'),

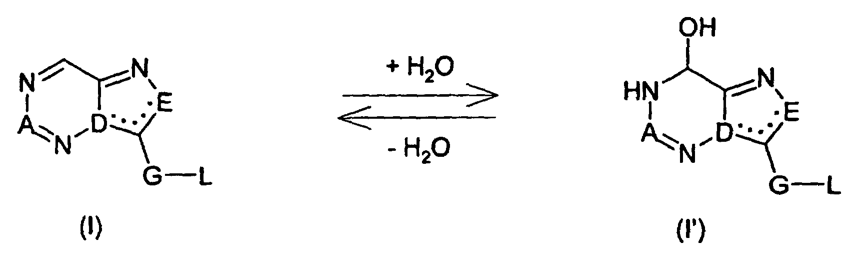

Die Wasseradditionsprodukte (I') sind ebenfalls Gegenstand der Erfindung.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, daß bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natriumund Kaliumsalze, oder auch Ammoniumsalze, Salze mit organischen Aminen oder quaternäre Ammoniumsalze.

Die Verbindungen kommen in der Regel auch in mehreren Stereoisomeren vor. Solche Verbindungen der Formel (I) enthalten ein oder mehrere asymmetrische C-Atome (= asymmetrisch substituierte C-Atome) oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt. Im Prinzip können die Stereoisomere nach üblichen Methoden aus Gemischen der Stereoisomeren erhalten oder auch durch stereoselektive Reaktionen in Kombination mit dem Einsatz von stereochemisch reinen oder angereicherten Ausgangsstoffen hergestellt werden. Weitgehend enantiomerenreine Verbindungen (I) können auch durch Trennung von Racematen nach üblichen Methoden, z. B. durch Kristallisation oder chirale Chromatographie erhalten werden.

Besondere Bedeutung im Rahmen der Erfindung haben die Reste der Formel G-L, soweit sie natürliche Zucker umfassen. Von besonderem Interesse sind die Reste mit den natürlichen Zuckern und Resten mit der Stereochemie, die den natürlichen Zuckern entspricht.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t-oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Entsprechendes gilt für Cycloalkyliden, wie Cyclopentyliden oder Cyclohexyliden.

Cycloalkyl bedeutet ein carbocyclisches, gesättigtes Ringsystem mit vorzugsweise 3-8 C-Atomen, vorzugsweise 3 bis 6 C-Atomen, z.B. Cyclopropyl, Cyclobutyl, Cyclopentyl oder Cyclohexyl. Im Falle von substituiertem Cycloalkyl werden cyclische Systeme mit Substituenten umfaßt, wobei die Substituenten mit einer Doppelbindung am Cycloalkylrest, z. B. eine Alkylidengruppe wie Methyliden, gebunden sind. Im Falle von substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Adamantan-1-yl und Adamantan-2-yl. Cycloalkenyl bedeutet ein carbocyclisches, nicht aromatisches, partiell ungesättigtes Ringsystem mit vorzugsweise 4-8 C-Atomen, insbesondere 5 bis 7 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend.

Halogen bedeutet beispielsweise Fluor, Chlor, Brom oder lod. in Restedefinitionen wird mit "Halogen" ein Halogenrest, d. h. ein Halogenatom bezeichnet. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCI₂; CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein Kohlenwasserstoffrest kann geradkettig, verzweigt oder cyclisch, gesättigt, ungesättigt oder aromatisch sein oder kann eine Kombination gleicher oder verschiedener der genannten Kohlenwasserstoffreste enthalten. Beispielsweise werden von "Kohlenwasserstoffrest" die Reste Alkyl, Alkenyl, Alkinyl, Cycloalkyl, Cycloalkenyl, Cycloalkyl-alkyl, Cycloalkenyl-alkyl, Aryl wie Phenyl oder Naphthyl, Benzyl, Phenethyl etc. umfasst. Ein Kohlenwasserstoffrest enthält vorzugsweise 1 bis 30 C-Atome, insbesondere 1 bis 24 C-Atome.

Ein heterocyclischer Rest oder Ring (Heterocyclyl) kann gesättigt, ungesättigt oder heteroaromatisch sein; wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält. Vorzugsweise ist er ein heteroaromatischer Ring mit einem Heteroatom aus der Gruppe N, O und S, beispielsweise Pyridyl, Pyrrolyl, Thienyl oder Furyl; weiterhin bevorzugt ist er ein entsprechender heteroaromatischer Ring mit 2 oder 3 Heteroatomen, z. B. Pyrimidinyl, Pyridazinyl, Pyrazinyl, Triazinyl, Thiazolyl, Thiadiazotyl, Oxazolyl, Isoxazolyl, Pyrazolyl, Imidazolyl und Triazolyl. Weiterhin bevorzugt ist er ein partiell oder vollständig hydrierter heterocyclischer Rest mit einem Heteroatom aus der Gruppe N, O und S, beispielsweise Oxiranyt, Oxetanyl, Oxolanyl (= Tetrahydrofuryl), Oxanyl, Pyrrolinyl, Pyrrolidyl oder Piperidyl. Weiterhin bevorzugt ist er ein partiell oder vollständig hydrierter heterocyclischer Rest mit 2 Heteroatomen aus der Gruppe N, O und S, beispielsweise Piperazinyl, Dioxolanyl, Oxazolinyl, Isoxazotinyl, Oxazolidinyl, Isoxazolidinyl und Morpholinyl.
Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsutfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.
Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugt Substituenten für die Subtituentenebenen sind beispielsweise Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkylaminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Fluorphenyl, 2-, 3-und 4-Trichlormethylphenyl, 2-, 3- und 4-Trifluorphenyl, 2,4-, 3,5-, 2,5-, 2,6- und 2,3-Dichlorphenyl oder -Difluorphenyl, 2,3,4- oder 2,3,5- oder 2,4,6- oder 2,3,6-Trifluor- und -Trichlorphenyl, o-, m- und p-Methoxyphenyl.

Der Rest Z¹ oder Z² einer anorganischen oder organischen Sauerstoffsäure, der formal durch Abtrennen einer Hydroxygruppe an der Säurefunktion entsteht, ist beispielweise der Sulfo-Rest -SO₃H, der von der Schwefelsäure H₂SO₄ abgeleitet ist, oder der Sulfinorest -SO₂H, der von der Schwefligen Säure H₂SO₃ abgeleitet ist, oder entsprechend die Gruppe SO₂NH₂, der Phosphorest -PO(OH)₂, die Gruppe -PO(NH₂)₂, -PO(OH)(NH₂), -PS(OH)₂, -PS(NH₂)₂ oder -PS(OH)(NH₂), der Carboxy-Rest COOH, der von der Kohlensäure abgeleitet ist, Reste der Formel -CO-SH, -CS-OH, -CS-SH, -CO-NH₂, -CS-NH₂, -C(=NH)-OH oder -C(=NH)-NH₂; außerdem kommen Reste mit Kohlenwasserstoffresten oder substituierten Kohlenwasserstoffresten in Frage, d. h. Acylreste im weiteren Sinne (= "Acyl").

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren, Phosphinsäuren. Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch ein oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.
Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

Vor allem aus den Gründen der höheren biologischen Wirkung, vorzugsweise herbiziden Wirkung, besseren Selektivität und/oder besseren Herstellbarkeit sind erfindungsgemäße Verwendungen von Verbindungen der genannten Formel (I) oder deren Salze von besonderem Interesse, worin in Formel (I) einzelne Reste eine der bereits genannten oder im folgenden genannten bevorzugten Bedeutungen haben, oder insbesondere solche, worin eine oder mehrere der bereits genannten oder im folgenden genannten bevorzugten Bedeutungen kombiniert auftreten. Von besonderem Interesse ist die erfindungsgemäße Verwendung von Verbindungen der Formel (I) und deren Tautomere, deren Salze und deren Wasseradditionsprodukte (nachstehend auch summarisch als "Verbindungen (I)" bezeichnet), 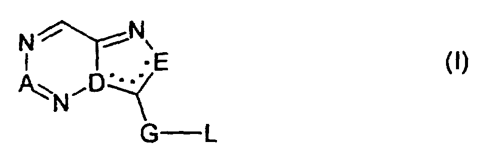 worin
- A: ein Stickstoffatom oder eine Gruppe der Formel C-R bedeutet, in der
R ein Wasserstoffatom, Amino, Hydroxy, Mercapto, Cyano, Halogen, Azido, Nitro, SF₅, Aminosulfonyl, (C₁-C₅)Alkanoylamino, [(C₁-C₄)Alkoxy]-carbonylamino, (C₁-C₅)Alkanoyl, [(C₁-C₄)Alkoxy]-carbonyl, (C₁-C₅)Alkanoyloxy, [(C₁-C₄)Alkoxy]-carbonyloxy, Mono(C₁-C₄)alkylamino, Mono(C₃-C₆)cycloalkylamino, Di(C₁-C₄)alkylamino, (C₁-C₄)Alkylthio, (C₁-C₄)Alkylsulfinyl, (C₁-C₄)Alkylsulfonyl, (C₁-C₄)Alkoxy, (C₃-C₄)Alkenyloxy, (C₃-C₄)Alkinyloxy, (C₃-C₆)Cycloalkoxy, (C₅-C₆)Cycloalkenyloxy, (C₁-C₄)Alkyl, (C₂-C₄)Alkenyl, (C₂-C₄)Alkinyl, (C₃-C₆)Cycloalkyl, (C₅-C₆)Cycloalkenyl, (C₁-C₄)Alkylaminosulfonyl oder Di[(C₁-C₄)alkyl]aminosulfonyl, wobei jeder der letztgenannten 24 Reste unsubstituiert oder im Kohlenwasserstoffteil durch einen oder mehrere Reste aus der Gruppe Halogen, Hydroxy, Amino, Nitro, Formyl, Carboxy, Cyano, Thiocyanato, (C₁-C₄)Alkoxy, (C₃-C₆)Cycloalkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₆)Cycloalkyl, (C₃-C₆)Cycloalkyl-amino, [(C₁-C₄)Alkyl]carbonyl, [(C₁-C₄)Alkoxy]carbonyl, Aminocarbonyl, Mono(C₁-C₄)alkylaminocarbonyl und Di(C₁-C₄)alkylamino-carbonyl substituiert ist, bedeutet.
Vorzugsweise bedeutet A ein Stickstoffatom.
Ebenfalls bevorzugt bedeutet A eine Gruppe der Formel C-R bedeutet, in der R ein Wasserstoffatom, Amino, Hydroxy, Mercapto, Cyano, Halogen, Azido, Nitro, Mono(C₁-C₄)alkylamino, Di(C₁-C₄)alkylamino, (C₁-C₄)Alkylthio, (C₁-C₄)Alkoxy, (C₃-C₄)Alkenyloxy, (C₃-C₄)Alkinyloxy, (C₃-C₆)Cycloalkoxy, (C₁-C₄)Alkyl, wobei jeder der letztgenannten 8 Reste unsubstituiert oder im Kohlenwasserstoffteil durch einen oder mehrere Reste aus der Gruppe Halogen, (C₁-C₄)Alkoxy, (C₁-C₄)Haloalkoxy, und (C₁-C₄)Alkylthio, substituiert ist.
Insbesondere bedeutet R ein Wasserstoffatom, Amino, OH, SH, CN, Halogen, wie F, Cl, Br oder I, N₃, NO₂, Mono(C₁-C₄)alkylamino, wie Methylamino, Di(C₁-C₄)alkylamino, wie Dimethylamino, oder (C₁-C₃)Alkylthio, wie Methylthio, (C₁-C₃)Alkoxy, wie Methoxy, (C₁-C₃)Alkyl, wie Methyl oder Ethyl, Vinyl, Ethinyl, (C₁-C₃)Haloalkyl, wie CF₃. Ganz besonders bevorzugt ist R = H.

In den erfindungsgemäß zu verwendenden Verbindungen (I) bedeuten vorzugsweise
- D: ein Kohlenstoffatom und
- E: eine Gruppe der Formel NH, (C₁-C₄)Alkyl-N, -N-OH, -N-NH₂, -O-, -S-, -SO- oder -SO₂-, vorzugsweise E = NH, wobei die Verbindung dann vorwiegend als Gemisch der Tautomeren folgender beiden Formeln vorliegt: 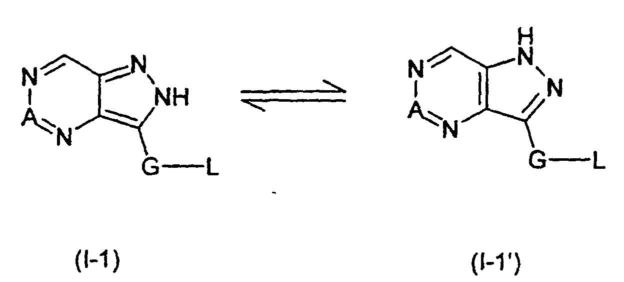 Außerdem bevorzugt sind Verbindungen (I) auf Basis der Formel (I-2), (I-3), (I-4), (I-5) und (I-6): 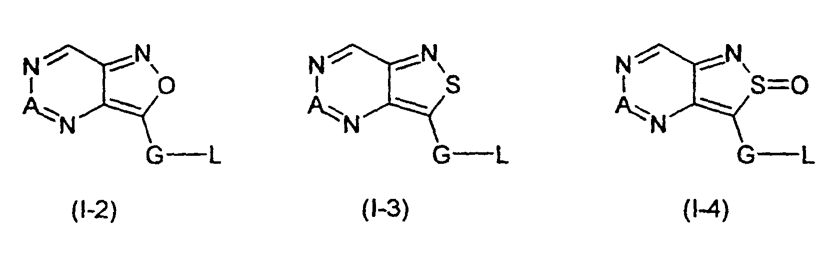 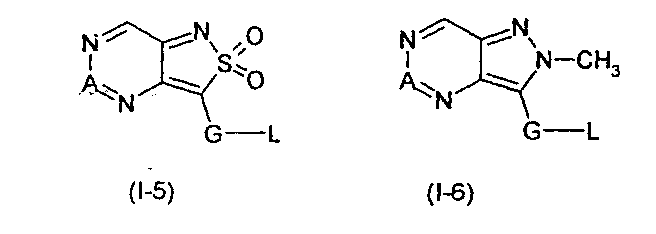 Außerdem bedeuten vorzugsweise
D ein Stickstoffatom und
E ein Stickstoffatom oder eine Gruppe der Formel C-R^{O},
wobei Verbindungen (I) auf Basis der Formeln (I-7) und (I-8) umfaßt sind: 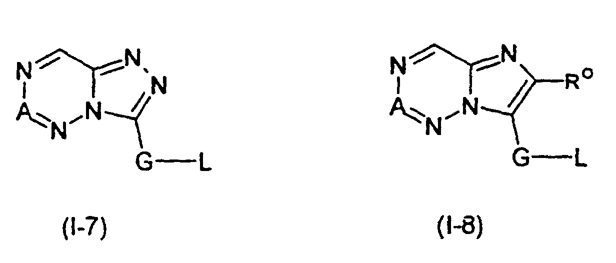
- R^{O}: ist vorzugsweise H, OH, NH₂, Halogen, CH₃ oder CF₃.

In den erfindungsgemäß einzusetzenden Verbindungen (I) ist vorzugsweise
- G: eine divalente geradkettige gesättigte oder ungesättigte Kohlenwasserstoffbrücke mit 1 bis 8 C-Atomen vorzugsweise 4 bis 6 C-Atomen in der Kette, in der ein oder mehrere CH₂-Gruppen jeweils unabhängig voneinander durch O oder S, vorzugsweise O ausgetauscht sind, wobei die jeweilige Brücke unsubstituiert ist oder wie oben genannt substituiert ist, vorzugsweise unsubstituiert oder
(a) durch einen oder mehrere Halogenatome und zusätzlich oder alternativ dazu einen oder mehrere, vorzugsweise 1 bis 4 gleiche oder verschiedene Reste aus der Gruppe Nitro, von Wasserstoff verschiedene Reste der Formel R¹, Reste der Formel R²R³C= und Reste der Formel L* substituiert ist, wobei R¹, R², R³ und L* die genannte Bedeutung haben oder wie weiter unten definiert sind,
(b) zwei oder vier Substituenten trägt, von denen jeweils zwei zusammen mit dem sie verbindenden Brückenteil einen carbocyclischen Ring mit 3 bis 6 C-Atomen, vorzugsweise 1,2-Cyclopentylen, 1,3-Cyclopentylen, 1,2-Cyclohexylen, 1,3-Cyclohexylen, 1,4-Cyclohexylen, 1,2-Phenylen, 1,3-Phenylen oder 1,4-Phenylen, oder einen heterocyclischen gesättigten oder partiell ungesättigten Ring mit 3 bis 6 Ringatomen oder einen heteroaromatischen Ring mit 5 oder 6 Ringatomen bilden, wobei im Fall eines Heterocyclus die Heteroatome, vorzugsweise 1, 2 oder 3 Heteroatome, aus der Gruppe N, 0 und S ausgewählt sind und wobei der jeweilige Ring noch einen ankondensierten carbocyclischen Ring mit 4 bis 6 Ringatomen oder einen ankondensierten heterocyclischen Ring mit 4 bis 6 Ringatomen und 1, 2 oder 3 Heteroatomen aus der Gruppe N, O und S aufweisen kann und im übrigen unsubstituiert oder durch einen oder mehrere Halogenatome und zusätzlich oder alternativ dazu einen oder mehrere, vorzugsweise 1 bis 3 gleiche oder verschiedene Reste aus der Gruppe Nitro, von Wasserstoff verschiedene Reste der Formel R¹, Reste der Formel L* und Oxo substituiert ist, wobei R¹ und L* die genannte Bedeutung haben oder wie weiter unten definiert sind,
(c) Substituenten aus den vorstehenden Gruppen (a) und (b) gemeinsam aufweist.

In den erfindungsgemäß einzusetzenden Verbindungen (I) bedeuten vorzugsweise
L, L* unabhängig voneinander jeweils OR⁴, SR⁴, CN, Tetrazolo, C(OR⁵)(OR⁶)(OR⁷), -Z¹, -O-Z², -S-Z² oder -NH-Z², wobei R⁴, R⁵, R⁶, R⁷, Z¹ und Z² weiter unten definiert ist und wobei L über eine zweite direkte Bindung oder über ein Heteroatom aus der Gruppe N, O und S, vorzugsweise O, mit der Brücke G cyclisch verbunden sein kann,
Z' den Rest der Formel COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)(NR¹⁶R¹⁷), P(=S)(OR¹³)(NR¹⁰R¹¹) oder P(=S)(NR¹⁰R¹¹)(NR¹⁶R¹⁷), vorzugsweise den Rest der Formel COOR⁸, CO-NR⁹-SO₂-R⁸, CO-NR¹⁰R¹¹, CS-NR¹⁰R¹¹, SO₂NR⁹COR¹², SO₂NR⁹COOR¹², CO-R¹², SO-R¹², SO₂R¹², SO₃R⁸, SO₂NR¹⁰R¹¹, P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)(NR¹⁶R¹⁷), P(=S)(OR¹³)(NR¹⁰R¹¹) oder P(=S)(NR¹⁰R¹¹)(NR¹⁶R¹⁷), insbesondere den Rest der Formel COOR⁸, CO-NR⁹-SO₂-R⁸, CO-NR¹⁰R¹¹, SO₂NR⁹COR¹², SO₂NR⁹COOR¹², SO₂NR¹⁰R¹¹, P(=O)(OR¹³)(OR¹⁴), P(=S)(OR¹³)(OR¹⁴) oder P(=O)(OR¹³)(NR¹⁰R¹¹),
Z² den Rest der Formel COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)(NR¹⁶R¹⁷), P(=S)(OR¹³)(NR¹⁰R¹¹) oder P(=S)(NR¹⁰R¹¹)(NR¹⁶R¹⁷), vorzugsweise den Rest der Formel CO-NR⁹-SO₂-R⁸, CO-NR¹⁰R¹¹, CS-NR¹⁰R¹¹, SO₂NR⁹COR¹², SO₂NR⁹COOR¹², CO-R¹², CS-R¹², SO-R¹², SO₂R¹², P(=O)(OR¹³)(OR¹⁴), P(=S)(OR¹³)(OR¹⁴), P(=O)(R¹⁵)(OR¹⁴) oder P(=O)(OR¹³)(NR¹⁰R¹¹), insbesondere den Rest der Formel CO-R¹², CS-R¹², CO-NR¹⁰R¹¹, CS-NR¹⁰R¹¹, P(=O)(OR¹³)(OR¹⁴), P(=S)(OR¹³)(OR¹⁴), P(=O)(R¹⁵)(OR¹⁴) oder P(=O)(OR¹³)(NR¹⁰R¹¹),
wobei R⁸ bis R¹⁷ nachstehend oder weiter unten definiert sind.

Vorzugsweise bedeuten
- R¹ bis R¹⁷: unabhängig voneinander jeweils ein Wasserstoffatom, (C₁-C₆)Alkyl, (C₂-C₆)Alkenyl, (C₂-C₆)Alinyl, (C₃-C₉)Cycloalkyl, (C₅-C₉)Cycloalkenyl, Aryl oder Heterocyclyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosuifonyl, (C₁-C₄)Alkanoyl, Acylamino, Acytoxy, Acylthio, [(C₁-C₄)Alkoxy]carbonyl, Mono(C₁-C₄)alkylamino, Mono(C₃-C₉)cycloalkylamino, Di(C₁-C₄)alkylamino, (C₁-C₄)Alkylthio, (C₂-C₄)Alkenylthio, (C₂-C₄)Alkinylthio, (C₃-C₉)Cycloalkylthio, (C₅-C₉)Cycloalkenylthio, (C₁-C₄)Alkylsulfinyl, (C₁-C₄)Alkylsulfonyl, (C₁-C₄)Alkoxy, (C₂-C₄)Alkenyloxy, (C₂-C₄)Alkinylaxy, (C₃-C₉)Cycloalkoxy, (C₅-C₉)Cycloalkenyloxy, (C₃-C₉)Cycloalkyl, (C₅-C₉)Cycloalkenyl, Phenyl, substituiertes Phenyl, Heteroaryl, substituiertes Heteroaryl und im Falle cyclischer Reste auch (C₁-C₄)Alkyl, (C₂-C₄)Alkenyl, (C₂-C₄)Alkinyl, (C₁-C₄)Haloalkyl, (C₂-C₄)Haloalkenyl, (C₂-C₄)Haloalkinyl, (C₁-C₄)Hydroxyalkyl und (C₁-C₄)Alkoxyl(C₁-C₄)alkyl substituiert ist,
wobei Heterocyclyl ein heterocyclischer gesättigter, ungesättigter oder heteroaromatischer Ring mit vorzugsweise 3 bis 6 Ringatomen und 1 bis 3 Heteroatomen aus der Gruppe N, O und S ist und
wobei Heteroaryl vorzugsweise ein heteroaromatischer Ring mit vorzugsweise 5 bis 6 Ringatomen und 1 bis 3 Heteroatomen aus der Gruppe N, O und S ist und
wobei die Substituenten für substituiertes Phenyl oder substituiertes Heteroaryl vorzugsweise ein oder mehrere Reste aus der Gruppe Halogen, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Hydroxyalkyl und (C₁-C₄)Alkoxy(C₁-C₄)alkyl sind, oder
R², R³ zusammen mit dem C-Atom der Gruppe R²R³C= einen nicht aromatischen carbocyclischen Ring oder einen heterocyclischen Ring mit 3 bis 6 Ringatomen und 1 bis 3 Heteroringatomen aus der Gruppe N, O und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten oder
R⁵, R⁶ zusammen mit dem C-Atom und den benachbarten Sauerstoffatomen der Gruppe C(OR⁵)(OR⁶)(OR⁷) einen gesättigen oder ungesättigten nicht aromatischen heterocyclischen Ring mit 3 bis 6 Ringatomen und 1 bis 3 Heteroringatomen aus der Gruppe N, O, P und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten oder
- R⁸, R⁹: oder R¹⁰, R¹¹ oder R¹³, R¹⁴ oder R¹⁴, R¹⁵ oder R¹⁶, R¹⁷ jeweils paarweise mit den Atomen der jeweils definierten Gruppe einen gesättigen oder ungesättigten nicht aromatischen heterocyclischen Ring mit 3 bis 9 Ringatomen und 1 bis 4 Heteroringatomen aus der Gruppe N, O, P und S, der unsubstituiert oder durch einen oder mehrere Reste aus der Gruppe Halogen, Nitro, (C₁-C₄)Alkyl, (C₁-C₄)Haloalkyl, (C₁-C₄)Alkoxy, (C₁-C₄)Haloalkoacy und (C₁-C₄)Alkylthio substituiert ist, bedeuten.

In den erfindungsgemäß einzusetzenden Verbindungen (I) ist weiter bevorzugt
- G: eine divalente geradkettige gesättigte oder ungesättigte Kohlenwasserstoffbrücke mit 1 bis 8 C-Atomen vorzugsweise 4 bis 6 C-Atomen in der Kette, in der ein oder mehrere CH₂-Gruppen jeweils unabhängig voneinander durch O oder S, vorzugsweise O ausgetauscht sind, oder
eine Brücke der Formel -W¹-Cyclus-W²-, worin
W', W² unabhängig voneinander eine direkte Bindung, CH₂, CH₂CH₂, OCH₂, SCH₂, CH₂CH₂CH₂, CH₂OCH₂, CH₂SCH₂, OCH₂CH₂ oder SCH₂CH₂ und "Cyclus" 1,4-Cyclohexylen, 1,2-Phenylen, 1,3-Phenylen, 1,4-Phenylen, 1,2-Naphthylen, 1,3-Naphthylen, 1,4-Naphthylen, 1,2-Tetrahydronaphthylen, 1,3-Tetrahydronaphthylen, 1,4-Tetrahydronaphthylen, 1,2-Cyclopentylen, 1,3-Cyclopentylen, 1,2-Cyclohexylen, 1,3-Cyclohexylen, 1,4-Cyciohexylen,Tetrahydrofuran-2,5-diy) (Oxolan), Tetrahydrothiophen 2,5-diyl, 2,5-Dihydrofuran-2,5-diyl oder 2,5-Dihydrothiophen-2,5-diyl bedeuten, wobei die jeweilige Brücke unsubstituiert ist oder
durch einen oder mehrere Halogenatome und zusätzlich oder alternativ dazu einen oder mehrere, vorzugsweise 1 bis 4, gleiche oder verschiedene Reste aus der Gruppe von Wasserstoff verschiedene Reste der Formel R¹, Reste der Formel R²R³C= und Reste der Formel L^{*} substituiert ist, wobei R¹, R², R³ und L* wie oben oder weiter unten definiert sind, oder zusätzlich-oder alternativ dazu über eine zweite direkte Bindung oder über ein Heteroatom aus der Gruppe N, O und S mit L cyclisch verbunden ist.
- R¹ bis R¹⁷: bedeuten vorzugsweise jeweils unabhängig voneinander ein Wasserstoffatom, (C₁-C₄)Alkyl, (C₂-C₄)Alkenyl, (C₂-C₄)Alkinyl, (C₃-C₆)Cycloalkyl, (C₅-C₆)Cycloalkenyl, Phenyl oder Heterocyclyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosulfonyl, (C₁-C₄)Alkanoyl, (C₁-C₄)Alkanoylamino, Benzoylamino, (C₁-C₄)Alkanoyloxy, (C₁-C₄)Alkanoylthio, [(C₁-C₄)Alkoxy]carbonyl, Mono(C₁-C₄)alkylamino, Di(C₁-C₄)alkylamino, (C₁-C₄)Alkylthio, (C₃-C₄)Alkenylthio, (C₃-C₄)Alkinylthio, (C₁-C₄)Alkylsulfinyl, (C₁-C₄)Alkylsulfonyl, (C₁-C₄)Alkoxy, (C₃-C₄)Alkenyloxy, (C₃-C₄)Alkinyloxy, (C₃-C₉)Cycloalkoxy, (C₃-C₉)Cycloalkyl, Phenyl, substituiertes Phenyl, Heteroaryl, substituiertes Heteroaryl und im Falle cyclischer Reste auch (C₁-C₄)Alkyl, (C₂-C₄)Alkenyl, (C₂-C₄)Alkinyl, (C₁-C₄)Haloalkyl, (C₂-C₄)Haloalkenyl, (C₂-C₄)Haloalkinyl, (C₁-C₄)Hydroxyalkyl und (C₁-C₄)Alkoxyl(C₁-C₄)alkyl substituiert ist,
wobei Heterocyclyl ein heterocyclischer gesättigter oder ungesättigter Ring mit 3 bis 6 Ringatomen oder ein heteroaromatischer Ring mit 5 oder 6 Ringatomen und jeweils 1 bis 3 Heteroatomen aus der Gruppe N, O und S ist und
wobei Heteroaryl vorzugsweise ein heteroaromatischer Ring mit vorzugsweise 5 bis 6 Ringatomen und 1 bis 3 Heteroatomen aus der Gruppe N, O und S ist und
wobei die Substituenten für substituiertes Phenyl oder substituiertes Heteroaryl vorzugsweise ein oder mehrere Reste aus der Gruppe Halogen, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Hydroxyalkyl und (C₁-C₄)Alkoxy(C₁-C₄)alkyl sind.

Insbesondere bedeuten
- R¹ bis: R⁴, R⁸, R¹², R¹³, R¹⁴ und R¹⁵ jeweils unabhängig voneinander ein Wasserstoffatom, (C₁-C₄)Alkyl, (C₂-C₄)Alkenyl, (C₂-C₄)Alkinyl, (C₃-C₆)Cycloalkyl oder Phenyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosulfonyl, (C₁-C₃)Alkanoylamino, Benzoylamino, (C₁-C₄)Alkanoyloxy, (C₁-C₄)Alkanoylthio, [(C₁-C₄)Alkoxy]carbonyl, Mono(C₁-C₄)alkylamino, Di(C₁-C₄)alkylamino, (C₁-C₄)Alkylthio, (C₃-C₄)Alkenylthio, (C₃-C₄)Alkinylthio, (C₁-C₄)Alkoxy, (C₃-C₄)Alkenyloxy, (C₃-C₄)Alkinyloxy, Phenyl, substituiertes Phenyl, Heteroaryl, substituiertes Heteroaryl und im Falle cyclischer Reste auch (C₁-C₄)Alkyl, (C₁-C₄)Haloalkyl, (C₁-C₄)Hydroxyalkyl und (C₁-C₄)Alkoxyl(C₁-C₄)alkyl substituiert ist,
wobei Heteroaryl vorzugsweise ein heteroaromatischer Ring mit vorzugsweise 5 bis 6 Ringatomen und einem Heteroatom aus der Gruppe N, O und S oder Heteroaryl aus der Gruppe Pyrimidinyl, Pyridazinyl, Pyrazinyl, Triazinyl, Thiazolyl, Thiadiazolyl, Oxazolyl, lsoxazolyl, Pyrazolyl, Imidazolyl und Triazolyl ist und
wobei die Substituenten für substituiertes Phenyl oder substituiertes Heteroaryl vorzugsweise ein oder mehrere aus der Gruppe Halogen, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Hydroxyalkyl und (C₁-C₄)Alkoxy(C₁-C₄)alkyl sind.

Ganz besonders bedeuten
- R¹ bis: R⁴, R⁸, R¹², R¹³, R¹⁴ und R¹⁵ jeweils unabhängig voneinander ein Wasserstoffatom, (C₁-C₄)Alkyl, wie Methyl, Ethyl, n- oder i-Propyl, (C₁-C₄)Haloalkyl, wie CF₃, (C₁-C₄)Hydroxyalkyl, wie CH₂OH, oder CN (C₁-C₄)Alkanoyloxy(C₁-C₄)alkyl , wie Acetyloxymethyl, Di(C₁-C₄)alkylamino(C₁-C₄)alkyl, wie Dimethylaminomethyl, (C₁-C₄)Alkylthio(C₁-C₄)alkyl, wie CH₃SCH₂, (C₁-C₄)Alkoxy(C₁-C₄)alkyl, wie Methoxymethyl, Dimethoxymethyl oder Ethoxymethyl, oder Benzyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Hydroxyalkyl und (C₁-C₄)Alkoxy(C₁-C₄)alkyl substituiert ist.

Insbesondere bedeuten
- R⁵ bis: R⁷, R¹⁰, R¹¹, R¹⁶ und R¹⁷ jeweils unabhängig voneinander ein Wasserstoffatom, (C₁-C₄)Alkyl, insbesondere Methyl oder Ethyl, wobei jeder der letztgenannten Reste unsubstituiert oder durch einen oder mehrere Reste aus der Gruppe Halogen substituiert ist.

Vorzugsweise bedeutet
- L: OR⁴, SR⁴, CN, Tetrazolo, C(OR⁵)(OR⁶)(OR⁷), -Z¹, -O-Z², -S-Z² oder -NH-Z², insbesondere OR⁴, CN, -Z¹, -O-Z² oder -NH-Z², wobei R⁴, R⁵, R⁶, R⁷, Z¹ und Z² eine der genannten bevorzugten Bedeutungen haben.

Insbesondere bedeutet
- L: Hydroxy, Carboxy, [(C₁-C₄)Alkoxy]-carbonyl, CONH₂, [(C₁-C₄)Alkylamino]-carbonyl, [(C₁-C₄)Alkylsulfonylamino]carbonyl, wie CONHSO₂CH₃ oder CONHSO₂C₂H₅, oder [(C₁-C₄)Haloalkylsulfonylamino]carbonyl, [Cyano(C₁-C₄)alkylsulfonylarnino]carbonyl, (C₁-C₄)Alkylsulfonylamino, (C₁-C₄)Haloalkylsulfonylamino, Cyano-(C₁-C₄)alkylsulfonylamino, (C₁-C₅)Alkanoyloxy, wie Acetyloxy, oder Benzoyloxy, [(C₁-C₄)Alkoxy]-carbonyloxy, wie Methoxycarbonyloxy, oder [(C₁-C₄)Alkylamino]carbonyloxy, (C₁-C₄)Alkoxy, (C₁-C₄)Alkylthio, (C₁-C₄)Hydroxyalkoxy, SO₂NHCONH₂, (C₁-C₅)Alkanoylaminosulfonyl, wie SO₂NHCOCH₃ oder SO₂NHCOC₂H₅, oder [(C₁-C₄)Haloalkyl]carbonylaminosulfonyl, [(C₁-C₄)Alkoxy carbonyl]aminosulfonyl, wie SO₂NHCOOCH₃ oder SO₂NHCOOC₂H₅, oder [(C₁-C₅)Haloalkoxy]carbonyl aminosulfonyl, SO₂NH₂, Di[(C₁-C₄)alkyl]aminosulfonyl, P(=O)(OH)₂, P(=S)(OH)₂, P(=O)(OR')₂ oder P(=O)(OH)(OR'), wobei in den letztgenannten beiden Formeln R' jeweils unabhängig von anderen Resten R' (C₁-C₄)Alkyl, (C₁-C₄)Haloalkyl, (C₁-C₄)Hydroxyalkyl, (C₁-C₄)Alkanoyl(C₁-C₄)alkyl, (C₁-C₄)Alkanoyloxy(C₁-C₄)alkyl oder Phenyl ist.

Ganz besonders bedeutet
- L: Hydroxy, Carboxy, [(C₁-C₄)Alkoxy]-carbonyl, wie Methoxycarbonyl oder Ethoxycarbonyl, [(C₁-C₄)Alkylsulfonylamino]carbonyl, wie CONHSO₂CH₃ oder CONHSO₂C₂H₅, oder [(C₁-C₄)Haloalkylsulfonylamino]carbonyl, [(C₁-C₄)Alkylamino]carbonyl, (C₁-C₄)Alkylsulfonylamino, (C₁-C₄)Haloalkylsulfonylamino, Cyanomethylsulfonylamino, (C₁-C₅)Alkanoyloxy, wie Acetyloxy, oder Benzoyloxy, SO₂NH₂, P(=O)(OH)₂, P(=S)(OH)₂, P(=O)(OR')₂ oder P(=O)(OH)(OR'), wobei in den letztgenannten beiden Formeln R' jeweils unabhängig von anderen Resten R' (C₁-C₄)Alkyl, (C₁-C₄)Haloalkyl, (C₁-C₄)Hydroxyalkyl, (C₁-C₄)Alkanoyl(C₁-C₄)alkyl, (C₁-C₄)Alkanoyloxy(C₁-C₄)alkyl oder Phenyl ist.

Weiter bevorzugt ist
- L: Hydroxy, Carboxy, [(C₁-C₄)Alkoxy]-carbonyl, Methoxycarbonyl, Ethoxycarbonyl, CONH₂, CONHSO₂CH₃, CONHSO₂C₂H₅, Acetoxy oder Benzoyloxy, SO₂NH₂, P(=O)(OH)₂, P(=S)(OH)₂, P(=0)(OR')₂, worin R' = Methyl, Ethyl, n-Propyl, i-Propyl, n-Butyl, i-Butyl, sec.-Butyl, tert.-Butyl, Phenyl oder (C₁-C₄)Alkanoyloxy(C₁-C₄)alkyl ist.

Vorzugsweise bedeutet L* einen Rest aus der Gruppe der für L definierten bevorzugten Reste, insbesondere OR⁴, -O-Z² oder -S-Z², insbesondere OR⁴ oder -O-Z², wobei R⁴ und Z² eine der genannten bevorzugten Bedeutungen haben. Besonders bevorzugt bedeutet L* = Hydroxy, (C₁-C₅)Alkanoyloxy, wie Acetyloxy, oder Benzoyioxy, [(C₁-C₄)Alkoxy]-carbonyloxy, wie Methoxycarbonyloxy, oder (C₁-C₄)Alkoxy, (C₁-C₄)Alkylthio oder (C₁-C₄)Hydroxyalkoxy, insbesondere Hydroxy oder Acetoxy.

Besonders bevorzugt stellt die Gruppe G-L inklusive Substituenten einen Rest eines cyclischen Zuckermoleküls, insbesondere den Rest von Ribosefuranosyl dar.

Besonders bevorzugt sind auch die jeweiligen Salze der oben bevorzügt genannten sauren Reste.

Bevorzugt sind Verbindungen (I), in denen Kombinationen von zwei oder mehreren der bevorzugt genannten Resten enthalten sind.
Besonders bevorzugt stellen die allgemein definierten Reste in Formel (I) auch die in den Ausführungsbeispielen und Tabellenbeispielen spezifisch erwähnten Reste bzw. deren homologen Reste oder Reste aus der korrespondierenden generischen Gruppe dar, insbesondere in den in den genannten Beispielen erwähnten Kombinationen von bevorzugten Resten.

Gegenstand der Erfindung sind auch Verfahren zur Herstellung der Verbindungen der allgemeinen Formel (I), deren Salze, Tautomere und Wasseradditionsverbindungen, dadurch gekennzeichnet, daß man
a) eine Verbindung der Formel (II), 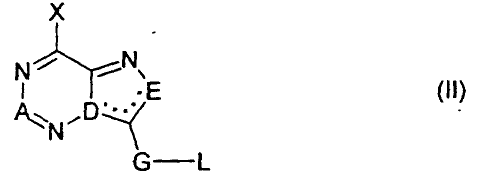 worin X eine Abgangsgruppe darstellt, zur Verbindung der Formel (I) reduziert oder
b) eine Verbindung der Formel (III), 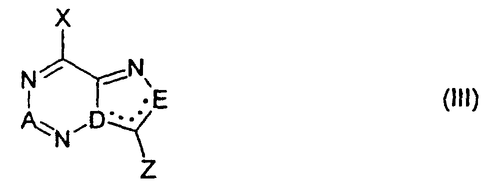 worin X eine Abgangsgruppe darstellt und Z eine Vorstufe zum Rest G-L bedeutet, zur Verbindung der Formel (III'), 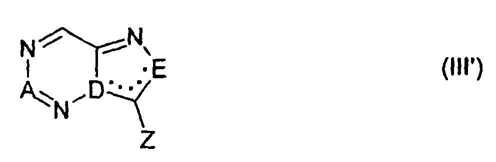 worin Z wie in Formel (III) definiert ist, reduziert und anschließend die Verbindung (III) an der Gruppe Z modifiziert, so daß die Verbindung (I) erhalten wird,
c) eine Verbindung der Formel (III'), worin Z eine Vorstufe zum Rest G-L bedeutet, an der Gruppe Z modifiziert, so daß die Verbindung (I) erhalten wird, oder
d) im Falle, daß A eine Gruppe der Formel C-R ist, eine Verbindung der Formel (III"), 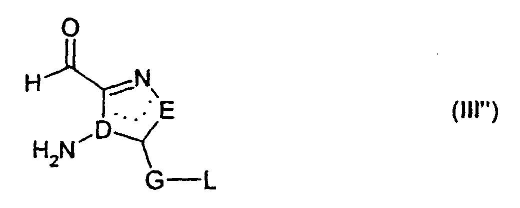 mit einer Verbindung der Formel (III''')

   H₂N - A = NH (III''')

   worin A eine Gruppe C-R darstellt, zur Verbindung der Formel (I) cyclisiert,
wobei in den Formeln (II), (III), (III'), (III") und (III''') die Symbole A, D, E, G, L und R, wenn nicht ausdrücklich anders definiert, wie in Formel (I) definiert sind.

Für die Reduktion der Verbindung (II) zur Verbindung (I) oder der Verbindung (III) zur Verbindung (III') kommen mehrere Methoden in Betracht:

Beispielsweise kann im Falle X = Halogen, wie Chlor, unter Bedingungen einer katalytischen Hydrierung, z. B. mit H₂/Pd, das Chloratom reduktiv gegen ein Wasserstoffatom ausgetauscht werden; vgl. Methode nach G. H. Milne et al., J. Chem. Soc. Perkin Trans. I (1972) 2677. Die Verbindung der genannten Formel (II) mit X = Chlor kann dabei aus der Verbindung (II) mit X = OH, welche in der Ketoform (II-a) vorliegt, 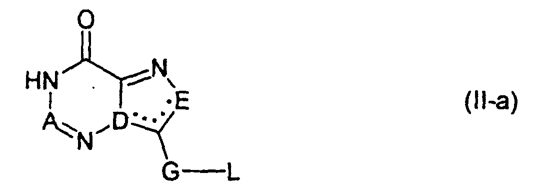 durch Umsetzung mit POCl₃ erhalten werden. Das entsprechende Thioketon [= Verbindung (II-a')] kann aus der Verbindung (II-a) durch Umsetzung mit P₂S₅ oder aus der Verbindung (II), X = Cl, durch Umsetzung mit Thioharnstoff erhalten und anschließend durch Reduktion mit Raney-Nickel zur Verbindung (I) umgesetzt werden; Entsprechendes gilt auch für die Herstellung der Verbindung (III), X=Cl aus dem Keton (III-a) [= Verbindung wie (II-a), doch G-L durch Rest Z ausgetauscht] und dessen Umsetzung zum entsprechenden Thioketon (III-a'); vgl. Methoden nach R. A. Long et al., J. Chem. Soc. (C) (1971) 2443 und R. Kandasamy et al., J. Med. Chem. 29 (1986) 2231, J. J. Fox et al. J. Am. Chem. Soc. 80 (1958) 1669 und K. Poreba et al., Acta Pol. Pharm. Drug Research 51 (1994) 355-358.

Die Verbindungen der Formel (II) und (III) mit X = Alkylthio, beispielsweise Methylthio, können ebenfalls durch Reduktion mit Raney-Nickel zur Verbindung (I) umgesetzt werden. Die Methylthio-Verbindung ist aus dem genannten Thioketon (IIa') durch Deprotonierung, z. B. mit Natriumhydrid, und Alkylierung mit Methyliodid erhältlich;
vgl. Methoden nach R. A. Long et al., J. Chem. Soc. (C) (1971) 2443, R. Kandasamy et al., J. Med. Chem. 29 (1986) 2231, A. Hampton et al., J. Am. Chem. Soc. 78 (1956) 5695 und R. J. Rousseau et al., J. Med. Chem. 15 (1972) 214.

Analog zur Methylthio-Verbindung gelingt die Methode mit Raney-Nickel auch mit der entsprechenden Seien-Verbindung (II) und (III), X = SeCH₃. Letztere Verbindung kann aus der Chlorverbindung (II), X=Cl durch Umsetzung mit Selenharnstoff Se=C(NH₂)₂, Deprotonierung mit Natriummethanolat und Alkylierung mit Methyliodid hergestellt werden; vgl. J. A. Milne et al., J. Chem. Soc. Perkin Trans. I (1972) 2677.

Eine weitere Alternative geht über die Verbindung (II) oder (III) mit X = Amino, wobei die Aminogruppe durch Umsetzung mit Butylnitrit in THF reduktiv entfernt werden kann. Die Aminoverbindung kann aus dem Keton (II-a) oder (III-a) oder aus der Methylthio-verbindung (II) oder (III), jeweils X = SCH₃, durch Umsetzung mit NH₃ erhalten werden; G. H. Milne et al., J. Chem. Soc. (C), 1971, 2443, K Kandasamy et al., J. Med. Chem. 29 (1986) 2231 und V. Nair et. al, Synthesis (1984) 401. Eine weitere Alternative verwendet die Verbindung (II) oder (III) mit jeweils X = NHNH₂, wobei die Hydrazinogruppe durch Umsetzung mit Quecksilberoxid entfernt werden kann. Die Hydrazinoverbindung kann ebenfalls der Chlorverbindung (II) oder (III), jeweils X=Cl, oder aus der Methylthio-verbindung (II) oder (III), jeweils X = SCH₃, durch Umsetzung mit Hydrazin erhalten werden; vgl.
G. H. Milne et al., J. Chem. Soc. (C), 1971, 2443, C. B. Reese et al., Tetrahedron, 30 (1994) 9195 und C. C. Tzeng et al., J. Chem. Soc. Perkin Trans. I, 1994, 2253.

Zur Herstellung der Verbindungen der Formel (I) werden die Verbindungen der Formel (III'), worin Z eine Vorstufe zum Rest G-L bedeutet, an der Gruppe Z so modifiziert, daß die Verbindung (I) mit der gewünschten Gruppe G-L erhalten wird. Für die Derivatisierungsreaktionen stehen dem Fachmann ein breite Palette allgemein bekannter oder üblicher Methoden zur Verfügung.
Von besonderem Interesse sind die Gruppen der Formel Z, aus denen der Rest G-L durch Abspaltung von Schutzgruppen an Hydroxygruppen oder Aminogruppen und/oder durch Acylierung mit einer organischen Säure oder Umsetzung mit einer anorganischen Säure oder einem Säurederivat davon erhalten wird. Ein Beispiel ist die Abspaltung einer oder mehrerer Tri(alkyl/phenyl)silylgruppen aus entsprechenden Verbindungen (III'), in denen Z ein oder mehrere Tri(alkyl/phenyl)silyloxygruppen enthalten, zu Verbindungungen (I), in denen L eine Hydroxygruppe bzw. G weitere Hydroxygruppen enthält. Die Abspaltung gelingt nach üblichen Methoden, z. B. in vielen Fällen mit Tetrabutylammoniumfluorid in einem organischen Lösungsmittel. Die erhaltenen Verbindung (I) kann anschließend weiter modifiziert werden, beispielsweise durch Phosphorylierung oder Acylierung zu Verbindungen (I), in denen L eine Phosphatestergruppe oder eine Acyloxygruppe darstellt.
Weitere Schutzgruppen sind die 1,3-Dioxolane, Benzylether, Acylate, Ether, Tetrahydropyranether, vorzugsweise Schutzgruppen, die in der Zuckerchemie üblich oder bekannt sind; vgl. J. Falbe, M. Regitz (Ed.), Römpp Chemie Lexikon, 9. Auflage, Bd. 5 (1992), Abschnitt "Schutzgruppen" und dort zitierte Literatur. Vorzugsweise stellt Z einen Rest eines natürlichen Zuckers, insbesondere Ribosylrest dar, der an einer oder mehreren Hydroxygruppen noch mit Schutzgruppen modifiziert ist.

Eine weitere Synthesemöglichkeit für Verbindungen (I), in denen A eine Gruppe der Formel C-R ist, besteht im Aufbau des heterocyclischen Sechsrings ausgehend von einer Verbindung der Formel (III"), die mit einer Verbindung der Formel (III''') (H₂N-A=NH mit A gleich CR) unter kondensierenden Bedingungen zum Bicyclus umgesetzt wird. Die Umsetzung erfolgt gegebenenfalls in Gegenwart eines sauren oder basischen Katalysators und Maßnahmen zur Entfernung oder zum Abfangen des Reaktionswassers und eines Moläquivalents Ammoniak.

Wasseradditionsverbindungen auf Basis der Verbindungen der Formel (I) können durch Addition von Wasser unter wässrig sauren bis neutralen Bedinungen erhalten werden.
Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in Wasser oder einem geeigneten organischen Lösungsmittel wie z.B. Methanol, Aceton, Methylenchlorid oder Petrolether oder entsprechenden wäßrig-organischen Lösungsmitteln und Hinzufügen der Säure bei Temperaturen von 0 bis 100 °C erhalten werden. Die Isolierung und Reinigung gelingt in bekannter oder üblicher Weise, z.B. in einfacher Weise durch Abfiltrieren und gegebenfalls Waschen mit einem inerten organischen Lösemittel.

Die Basenadditionssalze der Verbindungen der Formel (I) werden vorzugsweise in inerten polaren Lösungsmitteln wie z.B. Wasser, Methanol oder Aceton bei Temperaturen von 0 bis 100°C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdatkalihydride, z.B. NaH, Alkali- und Erdalkalialkoholate, z.B. Natriummethanolat, Kalium-tert.Butylat, oder Ammoniak oder Ethanolamin.

Vorstufen der Formel (II), (II-a), (III) oder (III-a) mit jeweils D = C und E = N können wie folgt nach Schema 1, Schema 2, Schema 3 bzw. Schema 4 erhalten werden:

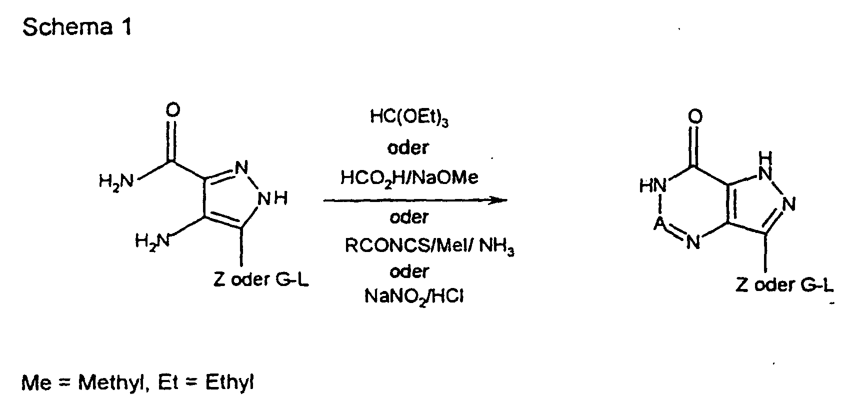

Die im Schema 1 bezeichneten Reaktionen sind bekannt oder können analog der bekannten Reaktionen durchgeführt werden; vgl.J. G. Buchanan et al., J. Chem. Soc. Perkin Trans. I, (1986) 1267; A.F. Lewis et al., J. Am. Chem. Soc. 104 (1982) 1073; J.W. Hennen et al., J. Org. Chem., 50 (1985) 1741; G. A. Ivanovics et al., J. Org. Chem. 39 (1974) 3651 und B. Rayner et al., J. Heterocycl. Chem. 10 (1973) 417 und jeweils dort zitierte Literatur. Mit Orthoformat oder Ameisensäure wird das Produkt erhalten, in dem A = CH bedeutet. Die Variante mit R-CO-N=C=S, Iodmethan und Ammoniak ergibt das Produkt mit A = -C-NH₂, und unter Verwendung von Natriumnitrit resultiert ein Produkt mit A = N.

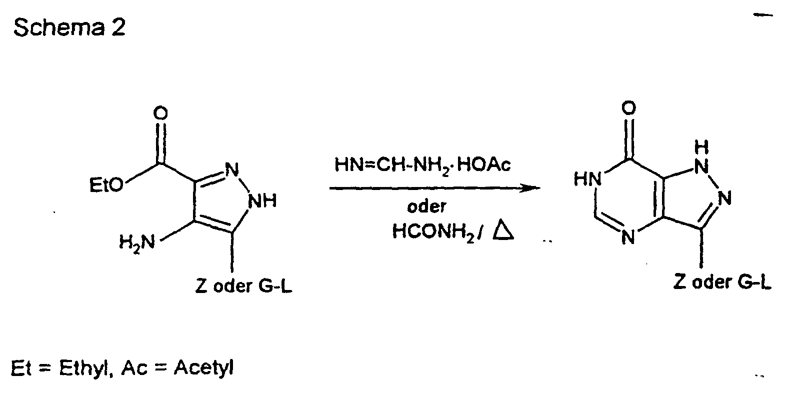

Die im Schema 2 bezeichneten Reaktionen sind bekannt oder können analog bekannter Reaktionen durchgeführt werden; vgl. G. J. Ellames et al., J. Chem. Soc. Perkin Trans. I, (1985) 2087, J. Wierzchowski et al., J. Chem. Acta Biochemica Polonica 27 (1980) 35 und L. Kalvoda, Coll. Czech. Chem. Commun., 43 (1978) 1431 und dortige Zitate. Der Ringschluß zur Ketoverbindung des Typs (11-a) oder (III-a) ist demnach mit Formamid oder Formamidin/Essigsäure möglich.

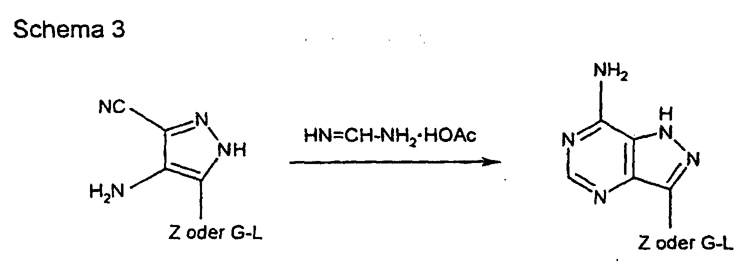

Die Reaktion gemäß Schema 3 zur Aminoverbindung des Typs (II) und (III) ist beispielsweise in J. W. Hennen et al., J. Org. Chem. 50 (1985) 1741 und G. J. Ellames et al., J. Chem. Soc. Perkin Trans. I, (1985) 2087 und dort zitierter Literatur beschrieben.

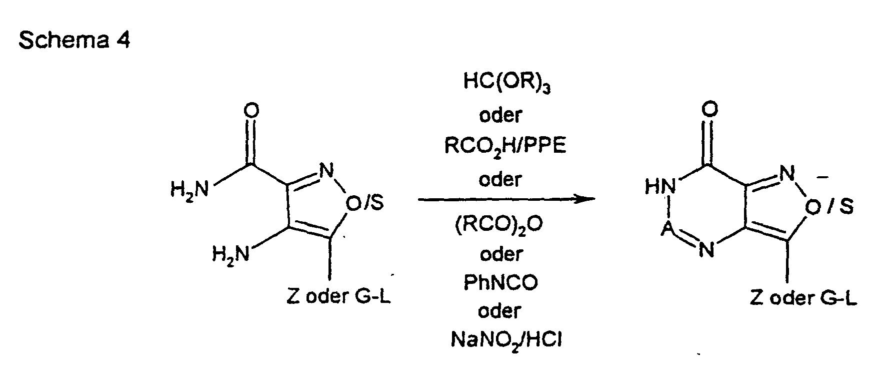

In Schema 4 sind mehrere Alternativen zur Herstellung der Verbindungen des Typs (II-a) und (III-a), worin E = O oder S bedeutet, zusammengefaßt. Zur Herstellung mit Trialkylorthoformiat (A = CH) oder Carbonsäuren RCOOH in Kombination mit Polyphosphorsäureethylester (PPE), wobei im Produkt A = CR ist, siehe beispielsweise K. Poreba et al., II Farmaco 49 (1994) 529.
Verbindungen mit einem Thiazolring werden z.B. nach der Methode mit Trialkylorthoformiat oder mit Phenylisocyanat PhNCO hergestellt,in S. A. EI Maaty et al., Bull. Fac. Pharm. Cairo Univ. 29 (1991) 41 bzw. S. A. EI Maaty et al., Egypt J. Pharm. Sci. 34 (1993) 421 beschrieben. Die Methode mit Natriumnitrit wurde bereits in Schema 1 erwähnt und ergibt Verbindungen mit A = N.

Vorstufen der Formel (II-a), (II), (III) oder (III-a) mit jeweils D = N und E = N oder C-R° können wie folgt nach Schema 5, Schema 6 und Schema 7 erhalten werden:

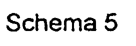

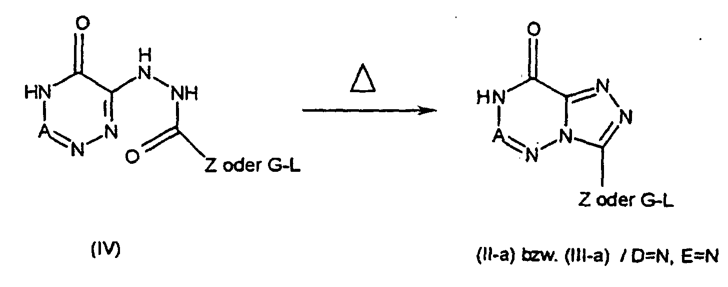

Der Ringschluß gemäß Schema 5 zum ankondensierten Triazolring gelingt durch Erhitzen, beispielsweise auf bis 200 °C in hochsiedenden Lösungsmitteln wie Ethylenglycol; siehe beispielsweise B. K. Bhattacharya et al., J. Heterocycl. Chem. 30 (1993) 1341, K. Ramasamy et al., J. Med. Chem. 29 (1986) 2231 und T. S. Rao et al., Nucleosides Nucleotides, 14 (1995) 1601.

Entsprechende chlorsubstituierte Verbindungen können unter Zusatz von Chlorierungsmitteln bei der thermischen Cyclisierung erhalten werden (siehe Schema 6). Diese Variante und die Chlorverbindungen der Formeln (II) und (III) mit jeweils X gleich Chlor (summarisch mit Formel (V), R*= G-L oder Z bezeichnet) sind neu und ebenfalls Gegenstand der Erfindung.

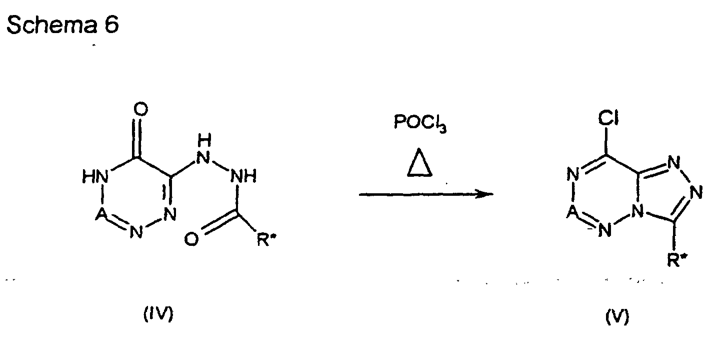

Die Variante nach Schema 6 wird beispielsweise durch Erhitzen einer Lösung der Ausgangsverbindung in einem inerten organischen Lösungsmittel unter Zusatz von Chlorierungsmitteln wie SO₂Cl₂, POCl₃, PCl₃, PCl₅ etc. oder direkt ohne zusätzliches Lösungsmittel in Mischung mit vorzugsweise flüssigen Chlorierungsmitteln wie Phosphoroxychlorid bei geeigneten Temperaturen, beispielsweise 0 bis 200 °C, vorzugsweise 50 bis 160 °C, insbesondere mit POCl₃ bei Reaktionstemperaturen bis zur Rückflußtemperatur durchgeführt.

Zur Herstellung von Verbindungen (II) oder (III) mit jeweils X = Alkylthio und mit einem ankondensierten Imidazolring, d. h. D=N und E=CH, (summarisch mit Formel (VI), R*= G-L oder Z bezeichnet), eignet sich die Reaktion gemäß Schema 7:

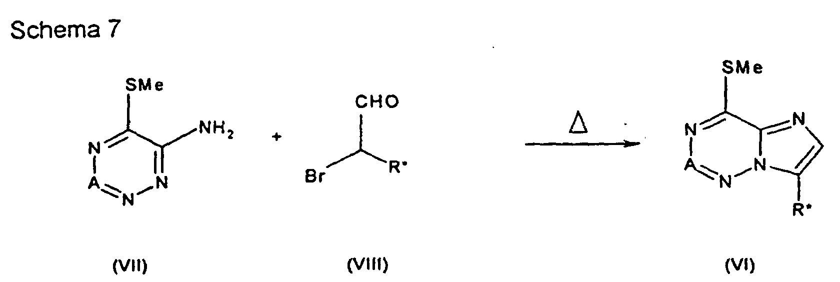

Die Reaktion kann beispielsweise in einem inerten organischen Lösungsmittel, wie einem gegebenenfalls halogenierten aromatischen Kohlenwasserstoff, z. B. Toluol oder Xylol, in Gegenwart einer Base wie Kaliumcarbonat durchgeführt werden. Das Reaktionswasser läßt sich im letztgenannten Fall azeotrop entfernen. Die Ringschlußreaktion gemäß Schema 7 und deren Endprodukte sind neu und ebenfalls Gegenstand der Erfindung.

Die Verbindungen der Formel (III") können aus Verbindungen der Formel 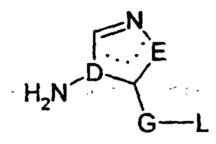 durch Formylierung erhalten werden. Die Ausgangsverbindungen sind durch übliche Ringaufbaureaktionen zugänglich.

Letztgenannte Ringaufbaureaktionen und andere Synthesewege zu den gewünschten heterocyclischen Systemen sind beschrieben in M. A. E. Shaban, Advances in Heterocyclic Chemistry 1998, 70, 163. Methoden zur Herstellung der Reste der Formel G-L und Z sind den bereits genannten Druckschriften, in US-A-5,731,432, in M.D. Erion et al, J. Am. Chem. Soc., 1999, 121, 308 sowie in den Herstellungsbeispielen (siehe weiter unten) angegeben.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.
Die erfindungsgemäßen Verbindungen der Formel (I), deren Tautomeren, Wasseradditionsverbindungen und deren Salze werden im folgenden zusammengefaßt als "Verbindungen (I)" oder "erfindungsgemäße Verbindungen" bezeichnet.

Kollektionen aus Verbindungen (I), die nach den obengenannten Verfahren synthetisiert werden können, können zusätzlich in parallelisierter Weise hergestellt werden , wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.
Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen (I) in Form von Substanzkollektionen oder -bibliotheken. Gegenstand der vorliegenden Erfindung sind daher auch Bibliotheken der Verbindungen (I), die mindestens zwei Verbindungen (I) enthalten, und deren Vorprodukte.

Die Verbindungen (I) hemmen die Aktivität des Enzyms AMPDA, welches in höheren Lebewesen, unter anderem Mensch, Tier und Pflanzen vorkommt, direkt oder zumindest indirekt unter physiologischen Bedingungen, wo sie als Vorstufen von direkten wirkenden Enzyminhibitoren in letztere umgewandelt werden. Viele Verbindungen (I) hemmen nach Applikation unter physiologischen Bedingungen auch das Enzym ADA, das in Mensch und Tier nachweislich vorkommt. Unter physiologischen Bedingungen sind nicht nur Bedingungen in vivo, sondern allgemein solche umfaßt, bei denen Phosphorylierungen und Hydrolysen ablaufen können.
Die Enzyme der unterschiedlichen Organismen haben die Substrate Adenosinmonophosphat bzw. Adenosin gemeinsam. Im Allgemeinen weisen die Enzyme AMPDA oder ADA in Abhängigkeit der Organismen jedoch Unterschiede in der Aminosäuresequenz und damit der Struktur auf. Die Verbindungen (I) hemmen direkt oder indirekt die Enzyme AMPDA und ADA in verschiedenen Arten von Lebewesen. Beispielsweise können Hemmwirkungen nach Standardmethoden für Enzymtests an den Enzymen AMPDA oder ADA beobachtet werden, die aus Geweben von Kaninchen oder Rindern stammen. Ebenso werden Hemmwirkungen an AMPDA beobachtet, die aus Pflanzenarten wie der Erbse gewonnen worden sind. Inhibitionen von 50 Prozent der Enzymaktivität liegen dabei in der Regel bei einer Konzentrationen (IC₅₀) von bis zu 1000 µmol/l, vorzugsweise bis zu 500 µmol/l insbesondere bis zu 50 µmol/l.

Durch die Hemmung des Enzyms und durch gegebenenfalls weitere, im Detail noch unbekannte Eigenschaften der einzelnen Verbindungen (I) werden biologische Wirkungen der Verbindungen (I) in einem breiten Anwendungsfeld beobachtet.

Die direkte oder indirekte Inhibitorwirkung (Enzymhemmung) kann beispielsweise zur Bekämpfung von unerwünschtem Pflanzenwuchs oder zur Bekämpfung von Schadpflanzen in Nutzpflanzenkulturen, die gegenüber dem Inhibitor natürlicherweise tolerant sind oder durch besondere Maßnahmen wie Mutation und Selektion der toleranten Mutanten oder mittels der Gentechnik als tolerante Pflanzen erhalten worden sind, angewendet werden.
Gegenstand der Erfindung ist deshalb auch die Verwendung der Verbindungen (I) als Herbizide für den Einsatz in Landwirtschaft, Gartenbau oder industriellem Bereich im nichtselektiven oder selektiven Einsatz. Dies schließt beispielsweise auch eine Anwendung zur Bekämpfung von unerwünschtem Pflanzenwuchs in Plantagen wie Obst-, Kautschuk oder Ölbaumplantagen oder auf Nichtkulturland wie Wegen, Plätzen, Pflasterzwischenräumen, Bahndämmen usw. ein.

Die erfindungsgemäßen Verbindungen der Formel (I) und deren Salze weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.
Auf der Seite der monokotylen Unkrautarten werden z.B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea,
Matricaria, Abutilon und Sida auf der annuellen Seite sowie Convolvulus, Cirsium, Rumex und Artemisia bei den perennierenden Unkräutern.
Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden bei einzelnen Verbindungen Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Diese Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Verbindungen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Verbindungen (I) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Emtegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung der erfindungsgemäßen Verbindungen (I) in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung
- (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, 'Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind. Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Suffonylhamstoffe, Glufosinate-ammonium oder Glyphosate-isopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen (I) enthalten.

Die Verbindungen (I) können als agrochemische Mittel auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. ÖI-in-Wasser-Emulsionen (EW), lassen sich beispielsweise mittels Rührem, Kolloidmühlen und/oder statischen Mischem unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Verbindung (I) (Wirkstoff) oder ein Gemisch des Wirkstoffs mit weiteren Wirkstoffen.
in Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Als Kombinationspartner für die erfindungsgemäßen Wirkstoffe in Mischungsformulierungen oder im Tank-Mix sind beispielsweise bekannte Wirkstoffe einsetzbar, die auf einer Inhibition des Stoffwechsels in Pflanzen, beispielsweise der Acetolactat-Synthase, Acetyl-Coenzyl-A-Carboxylase, PS l, PS II, HPPDO, Phytoene-Desaturase, Protoporphyrinogen-Oxidase, Glutamine- Synthetase, Cellulosebiosynthese, 5-Enolpyruvylshikimat-3-phosphat-Synthetase in Pflanzen beruhen. Solche Verbindungen und auch andere einsetzbare Verbindungen mit teilweise unbekanntem oder anderem Wirkungsmechanismus sind z.B. in Weed Research 26, 441-445 (1986), oder "The Pesticide Manual", 11. Auflage 1997 (im Folgenden auch kurz "PM") und 12. Auflage 2000, The British Crop Protection Council and the Royal Soc. of Chemistry (Herausgeber), und dort zitierter Literatur beschrieben. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen(-sodium); aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester, alachlor; alloxydim(-sodium); ametryn; amicarbazone, amidochlor, amidosulfuron; amitrol; AMS, d.h. Ammoniumsulfamat; anilofos; asulam; atrazin; azafenidin, azimsulfurone (DPX-A8947); aziprotryn; barban; BAS 516 H, d.h. 5-Fluor-2-phenyl-4H-3,1-benzoxazin-4-on; beflubutamid, benazolin(-ethyl); benfluralin; benfuresate; bensulfuron(-methyl); bensulide; bentazone; benzobicyclon, benzofenap; benzofluor; benzoylprop(-ethyl); benzthiazuron; bialaphos; bifenox; bispyribac(-sodium), bromacil; bromobutide; bromofenoxim; bromoxynil; bromuron; buminafos; busoxinone; butachlor; butafenacil, butamifos; butenachlor; buthidazole; butralin; butroxydim, butylate; cafenstrole (CH-900); carbetamide; carfentrazone(-ethyl) (ICI-A0051); caloxydim, CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlotimuron(-ethyl); chlomitrofen; chlorotoluron; chloroxuron; chlorpropham; chlorsulfuron; chlorthal-dimethyl; chlorthiamid; chlortoluron, cinidon(-methyl und -ethyl), cinmethylin; cinosulfuron; clefoxydim, clethodim; clodinafop und dessen Esterderivate (z.B. clodinafop-propargyl); clomazone; clomeprop; cloproxydim; clopyralid; clopyrasulfuron(-methyl), cloransulam(-methyl), cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-D, 2,4-DB;, dalapon; desmedipham; desmetryn; di-allate; dicamba; dichlobenil; dichlorprop; diclofop und dessen Ester wie dictofop-methyl; dicfosulam, diethatyl(-ethyl); difenoxuron; difenzoquat; diflufenican; diflufenzopyr, dimefuron; dimepiperate, dimethachlor; dimethametryn; dimethenamid (SAN-582H); dimethazone, dimexyflam, dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron-methyl; ethidimuron; ethiozin; ethofumesate; ethoxyfen und dessen Ester (z.B. Ethylester, HN-252); ethoxysulfuron, etobenzanid (HW 52); F5231, d.h. N-[2-Chlor-4-fluor-5-[4-(3-fluorpropyl)-4,5-dihydro-5-oxo-1H-tetrazot-1-yl]-phenyl]-ethansulfonamid; fenoprop; fenoxan, fenoxaprop und fenoxaprop-P sowie deren Ester, z.B. fenoxaprop-P-ethyl und fenoxaprop-ethyl; fenoxydim; fentrazamide, fenuron; flamprop(-methyl oder -isopropyl oder -isopropyl-L); flazasulfuron; floazulate, florasulam, fluazifop und fluazifop-P und deren Ester, z.B. fluazifop-butyl und fluazifop-P-butyl; flucarbazone(sodium), fluchloralin; flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupyrsulfuron(-methyl oder -sodium), flurenol(-butyl), fturidone; flurochloridone; fluroxypyr(-meptyl); flurprimidol, flurtamone; fluthiacet(-methyl), fluthiamide, fomesafen; foramsulfuron, fosamine; furyloxyfen; glufosinate(-ammonium); glyphosate(-isopropylammonium); halosafen; halosulfuron(-methyl) und dessen Ester (z.B. Methylester, NC-319); haloxyfop und dessen Ester; haloxyfop-P (= R-haloxyfop) und dessen Ester; hexazinone; imazamethabenz(-methyl); imazapyr; imazaquin und Salze wie das Ammoniumsalz; imazamethapyr, imazamox, imazapic, imazethamethapyr; imazethapyr; imazosulfuron; indanofan, ioxynil; isocarbamid; isopropalin; isoproturon; isouron; isoxaben; isoxachlortole, isoxaflutole, isoxapyrifop; karbutilate; lactofen; lenacil; linuron; MCPA; MCPB; mecoprop; mefenacet; mefluidid; mesosulfuron, mesotrione, metamitron; metazachlor; methabenzthiazuron; metham; methazole; methoxyphenone; methyldymron; metabenzuron, methobenzuron; metobromuron; (alpha-)metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxasulfuron, oxaziclomefone, oxyfluorfen; paraquat; pebulate; pelargonic acid, pendimethalin; pentoxazone, perfluidone; phenisopham; phenmedipham; picloram; picolinafen, piperophos; piributicarb; pirifenop-butyl; pretilachlor; primisulfuron(-methyl); procarbazone-(sodium), procyazine; prodiamine; profluralin; proglinazine(-ethyl); prometon; prometryn; propachlor; propanil; propaquizafop und dessen Ester; propazine; propham; propisochlor; propyzamide; prosulfalin; prosulfocarb; prosulfuron (CGA-152005); prynachlor; pyraflufen(-ethyl), pyrazolinate; pyrazon; pyrazosulfuron(-ethyl); pyrazoxyfen; pyribenzoxim, pyributicarb, pyridafol, pyridate; pyrimidobac(-methyl), pyrithiobac(-sodium) (KIH-2031); pyroxofop und dessen Ester (z.B. Propargylester); quinclorac; quinmerac; quinoclamine, quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und - methylester; sulcotrione, sulfentrazon (FMC-97285, F-6285); sulfazuron; sulfometuron(-methyl); sutfosate (ICI-A0224); sulfosulfuron, TCA; tebutam (GCP-5544); tebuthiuron; tepraloxydim, terbacil; terbucarb; terbuchlor; terbumeton; terbuthylazine; terbutryn; TFH 450, d.h. N,N-Diethyl-3-[(2-ethyl-6-methylphenyl)-sulfonyl]-1 H-1,2,4-triazol-1-carboxamid; thenylchlor (NSK-850); thiafluamide, thiazafluron; thiazopyr (Mon-13200); thidiazimin (SN-24085); thifensulfuron(-methyl); thiobencarb; tiocarbazil; tralkoxydim; tri-allate; triasulfuron; triaziflarn, triazofenamide; tribenuron(-methyl); triclopyr; tridiphane; trietazine; trifluralin; triflusulfuron und Ester (z.B. Methylester, DPX-66037); trimeturon; tritosulfuron, tsitodef; vernolate; WL 110547, d.h. 5-Phenoxy-1-[3-(trifluormethyl)-phenyl]-1H-tetrazol; BAY MKH 6561, UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", PM, S. 781-782), und verwandte Verbindungen, wie sie in der WO 91/07874 beschrieben sind,
b) Derivate der Dichlorphenylpyrazolcarbonsäure, vorzugsweise Verbindungen wie 1-(2,4-Dichlorphenyt)-5-methyl-pyrazol-3-carbonsäureethylester (S1-2), 1-(2,4-Dichiorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind.
c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-6), und verwandte Verbindungen EP-A-174 562 und EP-A-346 620);
d) Verbindungen vom Typ der 5-Benzyl- oder 5-Phenyl-2-isoxazolin-3-carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethytester (S1-8) und verwandte Verbindungen, wie sie in WO 91/08202 beschrieben sind, bzw. der 5,5-Diphenyl-2-isoxazolincarbonsäureethylester (S1-9) ("lsoxadifen-ethyl") oder -n-propylester (S1-10) oder der 5-(4-Fluorphenyl)-5-phenyl-2-isoxazolin-3-carbonsäureethylester (S1-11), wie sie in der deutschen Patentanmeldung (WO-A-95/07897) beschrieben sind.
e) Verbindungen vom Typ der 8-Chinolinoxyessigsäure (S2), vorzugsweise (5-Chlor-8-chinolinoxy)-essigsäure-(1-methyl-hex-1-yl)-ester (Common name "Cloquintocet-mexyl" (S2-1) (siehe PM, S. 263-264) (5-Chlor-8-chinolinoxy)-essigsäure-(1,3-dimethyl-but-1-yl)-ester (S2-2), (5-Chlor-8-chinolinoxy)-essigsäure-4-allyl-oxy-butylester (S2-3), (5-Chlor-8-chinolinoxy)-essigsäure-1-allyloxy-prop-2-ylester (S2-4), (5-Chlor-8-chinolinoxy)-essigsäureethylester (S2-5), (5-Chlor-8-chinolinoxy)-essigsäuremethylester (S2-6), (5-Chlor-8-chinolinoxy)-essigsäureallylester (S2-7), (5-Chlor-8-chinolinoxy)-essigsäure-2-(2-propyliden-iminoxy)-1-ethylester (S2-8), (5-Chlor-8-chinolinoxy)-essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind.
f) Verbindungen vom Typ der (5-Chlor-8-chinolinoxy)-malonsäure, vorzugsweise Verbindungen wie (5-Chlor-8-chinolinoxy)-malonsäurediethylester, (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.
g) Wirkstoffe vom Typ der Phenoxyessig- bzw. -propionsäurederivate bzw. der aromatischen Carbonsäuren, wie z.B. 2,4-Dichlorphenoxyessigsäure(ester) (2,4-D), 4-Chlor-2-methyl-phenoxy-propionester (Mecoprop), MCPA oder 3,6-Dichlor-2-methoxy-benzoesäure(ester) (Dicamba).
h) Wirkstoffe vom Typ der Pyrimidine, die als bodenwirksame Safener in Reis angewendet werden, wie z. B. "Fenclorim" (PM, S. 512-511) (= 4,6-Dichlor-2-phenylpyrimidin), das als Safener für Pretilachlor in gesätem Reis bekannt ist,
i) Wirkstoffe vom Typ der Dichloracetamide, die häufig als Vorauflaufsafener (bodenwirksame Safener) angewendet werden, wie z. B. "Dichlormid" (PM, S. 363-364) (= N,N-Diallyl-2,2-dichloracetamid), "R-29148" (= 3-Dichloracetyl-2,2,5-trimethyl-1,3-oxazolidin von der Firma Stauffer),
   "Benoxacor" (PM, S. 102-103) (= 4-Dichloracetyl-3,4-dihydro-3-methyl-2H-1,4-benzoxazin).
   "PPG-1292" (= N-Allyl-N-[(1,3-dioxolan-2-yl)-methyl]-dichloracetamid von der Firma PPG Industries),
   "DK-24" (= N-Allyl-N-[(allylaminocarbonyl)-methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "Diclonon" oder "BAS145138" oder "LAB145138" (= 3-Dichloracetyl-2,5,5-trimethyl-1,3-diazabicyclo[4.3.0]nonan von der Firma BASF) und
   "Furilazol" oder "MON 13900" (siehe PM, 637-638) (= (RS)-3-Dichloracetyl-5-(2-furyl)-2,2-dimethyloxazolidin)
j) Wirkstoffe vom Typ der Dichloracetonderivate, wie z. B.
   "MG 191" (CAS-Reg. Nr. 96420-72-3) (= 2-Dichlormethyl-2-methyl-1,3-dioxolan von der Firma Nitrokemia), das als Safener für Mais bekannt ist,
k) Wirkstoffe vom Typ der Oxyimino-Verbindungen, die als Saatbeizmittel bekannt sind, wie z. B.
   "Oxabetrinil" (PM, S. 902-903) (= (Z)-1,3-Dioxolan-2-ylmethoxyimino(phenyl)acetonitril), das als Saatbeiz-Safener für Hirse gegen Schäden von Metolachlor bekannt ist,
   "Fluxofenim" (PM, S. 613-614) (= 1-(4-Chlorphenyl)-2,2,2-trifluor-1-ethanon-O-(1,3-dioxolan-2-ylmethyl)-oxim, das als Saatbeiz-Safener für Hirse gegen Schäden von Metolachlor bekannt ist, und
   "Cyometrinil" oder "-CGA-43089" (PM, S. 1304) (= (Z)-Cyanomethoxyimino(phenyl)acetonitril), das als Saatbeiz-Safener für Hirse gegen Schäden von Metolachlor bekannt ist,
l) Wirkstoffe vom Typ der Thiazolcarbonsäureester, die als Saatbeizmittel bekannt sind, wie z. B.
   "Flurazol" (PM, S. 590-591) (= 2-Chlor-4-trifluormethyl-1,3-thiazol-5-carbonsäurebenzylester), das als Saatbeiz-Safener für Hirse gegen Schäden von Alachlor und Metolachlor bekannt ist,
m) Wirkstoffe vom Typ der Naphthalindicarbonsäurederivate, die als Saatbeizmittel bekannt sind, wie z. B.
   "Naphthalic anhydrid" (PM, S. 1342) (= 1,8-Naphthalindicarbonsäureanhydrid), das als Saatbeiz-Safener für Mais gegen Schäden von Thiocarbamatherbiziden bekannt ist,
n) Wirkstoffe vom Typ Chromanessigsäurederivate, wie z. B. "CL 304415" (CAS-Reg. Nr. 31541-57-8) (= 2-(4-Carboxy-chroman-4-yl)-essigsäure von der Firma American Cyanamid), das als Safener für Mais gegen Schäden von lmidazolinonen bekannt ist,
o) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (PM, S. 404-405) (= Piperidin-1-thiocarbonsäure-S-1-methyl-1-phenylethylester), das als Safener für Reis gegen Schäden des Herbizids Molinate bekannt ist,
   "Daimuron" oder "SK 23" (PM, S. 330) (= 1-(1-Methyl-1-phenylethyl)-3-p-tolylharnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist,
   "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
p) N-Acylsulfonamide der Formel (S3) und ihre Salze, 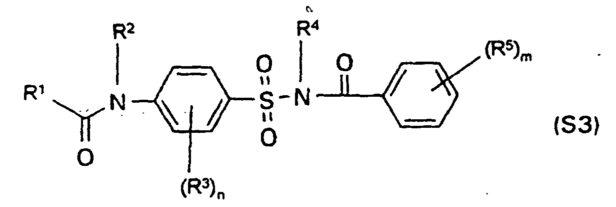 wie sie in WO-A-97/45016 beschrieben sind,
q) Acylsulfamoylbenzoesäureamide der allgemeinen Formel (S4), gegebenenfalls auch in Salzform, 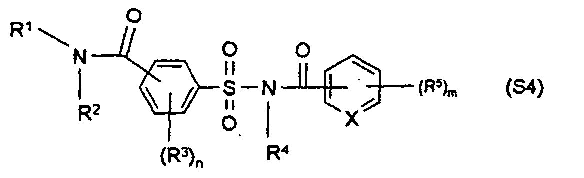 wie sie in der Internationalen Anmeldung Nr. PCT/EP98/06097 beschrieben sind, und
r) Verbindungen der Formel (S5), 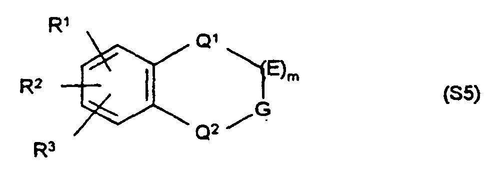 wie sie in der WO-A 98/13 361 beschrieben sind,
   einschließlich der Stereoisomeren und den in der Landwirtschaft gebräuchlichen Salzen.

Von besonderem Interesse sind unter den genannten Safenern (S1-1) und (S1-9) und (S2-1), insbesondere (S1-1) und (S1-9).
Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen (I). Sie kann innerhalb weiter Grenzen schwanken, z.B. zwischen 0,0005 und 10,0 kg/ha oder mehr Aktivsubstanz, vorzugsweise zwischen 0,001 und 3 kg/ha, insbesondere von 0,005 bis 1 kg/ha.

Die erfindungsgemäßen Verbindungen weisen darüberhinaus auch wertvolle pharmazeutische Wirkungen auf. Es ist bekannt, daß Adenosin und Adenosinmonophosphat (AMP) bei Erkrankungen des Formenkreises der Durchblutungsstörungen oder der Sauerstoffmangelversorgung (tschämie) durch Abbau von Adenosintriphosphat in den ischämischen Geweben gebildet wird. Der weitere Metabolismus von AMP durch AMPDA zu Inosinmonophosphat oder von Adenosin durch Adenosindeaminase (ADA) zu Inosin führt zu einer verringerten Adenosinkonzentration im Gewebe, die mit weiteren Krankheitsbildern ursächlich in Verbindung gebracht wird (vgl. z. B. WO-A-94/18200 und dort zitierte Literatur). Inhibitoren der AMPDA oder der ADA können deshalb dazu beitragen, den übermäßigen Abbau von Adenosin zu verringern und damit das Gewebe vor Schädigung zu schützen.

Die erfindungsgemäßen AMPDA Inhibitoren können zur Behandlung einer breiten Palette von klinischen Erscheinungsformen eingesetzt werden, bei denen eine lokale Erhöhung der Adenosinkonzentration im Gewebe hilfreich ist. Beispielsweise sind sie geeignet zur Behandlung kardiovaskulärer Störungen, beispielsweise Herzinfarkt, Angina pectoris und andere Herzkreislauferkrankungen. Weiter können sie als Analgetika zur Behandlung akuter oder permanenter Schmerzen durch Arthritis, Krebs, Neuralgien, Multiple Sklerose und allgemein Neuropathien eingesetzt werden.
Außerdem eignen sich die Inhibitoren zur Behandlung von Infektionen, beispielweise solchen, die durch Protozoen oder Würmer hervorgerufen werden.
Die Anwendung ist auch sinnvoll in Kombination mit Therapien, welche dem Metabolismus von Purinen und/oder Pyrimidinen betreffen. Solche Therapien schließen Behandlungen mit Antivirusmittel wie Acyclovir, Azidothymidin, Dideoxyinosin, Adenosinarabinosid, Dideoxyadenosin und Ribovirin oder Krebsbehandlungsmitteln wie 5-Fluoruracil, Azathiopyrin, Dacarbazin, Cytosinarabinosid, Methotrexat, Brendinin, Tiazafurin, 2'-Deoxycoformycin und 2'-Deoxy-2-chloradenosin ein.
Ein weiterer Indikationsbereich ist die Behandlung von Alzheimer-Erkrankungen, die mit einem pathologisch erhöhten Gehalt an AMPDA auftreten; vgl. B. Sims et al., Neurobiol. Aging, 9 (1998) 385.

Die direkt oder indirekt wirkenden Inhibitoren können in einem weiten Dosis- und Konzentrationsbereich eingesetzt werden.

Sind die Verbindungen (I) Salze von Verbindungen der Formel (I) eignen sich insbesondere für den Einsatz als pharmazeutische Mittel vor allem physiologisch oder toxikologisch verträglichen Salze. Pharmazeutisch verwendbaren Salze, die sich von Verbindungen der Formel (I) mit sauren Gruppen ableiten, sind beispielsweise Alkalimetallsalze, wie Natriumsalze oder Kaliumsalze, Erdalkalimetallsalze, wie Calciumsalze oder Magnesiumsalze, oder Ammoniumsalze auf Basis von Ammoniak oder organischen Aminen wie beispielsweise Ethylamin, Ethanolamin, Triethanolamin oder Aminosäuren. Pharmazeutisch verwendbaren Salze, die sich von Verbindungen der Formel (I) mit basischen (protonierbaren) Gruppen ableiten, sind beispielsweise Säureadditionssalze mit physiologisch verträglichen anorganischen oder organischen Säuren wie Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bemsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw.
Enthalten die Verbindungen der Formel (I) gleichzeitig saure und basische Gruppen im Molekül, so gehören neben den geschilderten Salzformen auch innere Salze oder Betaine (Zwitterionen) zu der Erfindung. Die Salze können aus den Verbindungen der Formel (I) nach den bereits oben erwähnten Verfahren hergestellt werden. Unter physiologisch verträglichen Salzen von Verbindungen der Formel (I) werden beispielsweise deren organische als auch anorganische Salze, wie sie in Remington's Pharmaceutical Sciences (17. Auflage, Seite 1418 (1985)) beschrieben sind, verstanden. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Verbindungen (I) können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen (I) zur Anwendung als Arzneimittel, ihre Verwendung in der Therapie und Prophylaxe der genannten Krankheitsbilder und ihre Herstellung von Medikamenten dafür. Weiterhin sind Gegenstand der vorliegenden Erfindung pharmazeutische Zubereitungen, die als aktiven Bestandteil eine wirksame Dosis mindestens einer Verbindung (I) neben üblichen pharmazeutisch einwandfreien Träger- und Hilfsstoffen enthalten. Die pharmazeutischen Zubereitungen enthalten normalerweise 0,1 bis 99 Gewichtsprozent, bevorzugt 0,5 bis 95 Gewichtsprozent der Verbindungen (I). Die Herstellung der pharmazeutischen Zubereitungen kann in an sich bekannter Weise erfolgen. Dazu werden die Verbindungen (I) zusammen mit einem oder mehreren festen oder flüssigen galenischen Trägerstoffen und/oder Hilfsstoffen und, wenn gewünscht, in Kombination mit anderen Arzneimittefwirkstoffen in eine geeignete Darreichungsform bzw. Dosierungsform gebracht, die dann als Arzneimittel in der Humanmedizin oder Veterinärmedizin verwendet werden kann.

Arzneimittel, die eine Verbindung (I) enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen (I) können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgem können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucöse oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes (I) in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs (I) und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung (I) bei einem etwa 75 kg schweren Patienten vorzugsweise 0,001 mg/kg bis 50 mg/kg, insbesondere 0,01 bis 10 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden. Eine akute Behandlung von Koronarverschlüssen kann beispielsweise durch Infusion einer sterilen wässrigen Lösung des Wirkstoffs oder einer Lösung des Wirkstoffs in isotonischer Kochsalzlösung in die Halsschlagader oder in Herzkranzgefäße bei gelegtem Herzkatheder durchgeführt werden. Die Applikationsrate bei der Infusion ist beispielsweise im Bereich von 1 bis 20 nmol/min/kg Wirkstoff bei einem Infusionsvolumen von 30 ml/h über mehrere Tage.

In den folgenden Beispielen beziehen sich Mengenangaben (auch Prozentangaben) auf das Gewicht, sofern nichts anderes speziell angegeben ist.

### A. Chemische Beispiele

Häufig verwendete Abkürzungen in Text, Schemata und Tabellen:
- Ac =: COCH₃ = Acetyl
- Bu =: Butyl
- t-Bu =: tertiär-Butyl
- Bz =: Benzoyl = -CO-C₆H₅
- Et =: Ethyl
- Me =: Methyl
- Ph =: Phenyl
- Pr =: Propyl
- i-Pr =: Isopropyl
- c-Pr =: Cyclopropyl

- DCC =: Dicyclohexylcarbodiimid
- DCM =: Dichlormethan
- DMAP =: Dimethylaminopyridin
- DMF =: Dimethylformamid
- NBA =: Nitrobenzylalkohol
- THF =: Tetrahydrofuran
- FAB =: "Fast atom bombardment" (Ionisierungstechnik f. Massenspektrum)

### Beispiel 1

7-Chlor-3-(2',3',5'-tri-O-acetyl-β-D-ribofuranosyl)-1H-pyrazolo[4,3-d]pyrimidin (II-1)

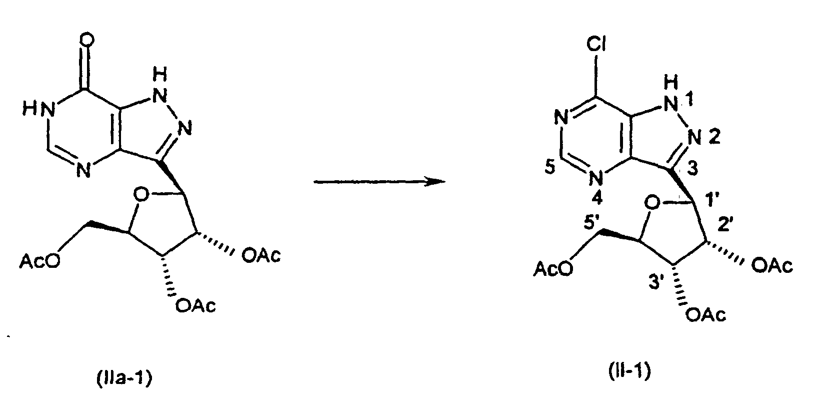

Eine Mischung aus der Ketoverbindung (IIa-1) (J. Chem. Soc. (C), 1971, 2443) (210 mg, 0,52 mmol) und POCl₃ (3 ml) wurde langsam auf Rückflußtemperatur erhitzt und 30 min bei dieser Temperatur gehalten. Nach Abkühlen auf Raumtemperatur wurde das Lösungsmittel unter reduziertem Druck entfernt und ein Eis/Wasser-Gemisch (2ml) zum Rückstand gegeben. Nach Extrahieren mit Essigester (3 x 2 ml), Trocknen über MgSO₄ und Entfernen des Lösungsmittels unter reduziertem Druck erhielt man einen Rückstand, der nach Chromatographie über Kieselgel (Laufmittel 5-10 %iger Essigester in Petrolether) als farbloser Schaum erhalten wurde. Ausbeute: 130 mg, 60 %;

IR-Spektrum: νₘₐₓ (NaCl/Film/cm⁻¹) 3235 bw (NH), 3073w, 1747s (C=O, ester), 1607w, 1540s, 1475w, 1447w, 1375s, 1240s, 1173w, 1146w, 1094s, 1049s, 936s, 918s, 866w, 817w, 733s; ¹H-NMR: δ_{H} (270 MHz, CDCl₃) 8.86 (1H, s, H-5), 5.98 (1H, t, J 5.5 Hz, H-2'), 5.70 (1H, t, J 5.4 Hz, H-3'), 5.57 (1H, d, J 5.6 Hz, H-1'), 4.53-4,48 (1H, m, H-5a'), 4.47 - 4.42 (1H, m, H-4'), 4.33 - 4.28 (1H, m, H-5b'), 2.15 (3H, s, CH₃), 2.09 (3H, s, CH₃), 2,08 (3H, CH₃)

Massenspektrum (FAB, NBA): Gefunden MH⁺, 413.0867, errechnet C₁₆H₁₈ClN₄O₇, MH, 413.0864

### Beispiel 2

3-(2',3',5'-Tri-O-acetyl-β-D-ribofuranosyl)-1H-pyrazolo[4,3-d]pyrimidin (la)

### Methode A

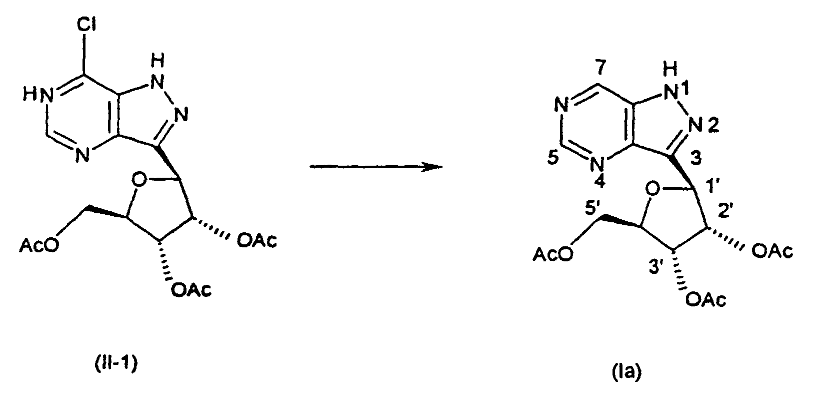

Eine Lösung des Chlorids (II-1 ) (72 mg, 0,18 mmol) in trockenem Ethylacetat wurde mit 5 % Pd/C (20 mg) und MgO (18 mg, 0,45 mmol) versetzt und unter Rühren mit Wasserstoffgas überlagert. Nach Umsetzung des Ausgangsstoffs (II-1) wurde über eine kurze Säule mit Celite filtriert und mit Essigester eluiert, das Lösungsmittel abgezogen und der Rückstand über Kieselgel chromatographiert (Laufmittel 3-5 % Essigester in Petrolether). Ausbeute an (la): 46 mg, 70 % d. Th. als farbloser Schaum.

IR-Spektrum: νₘₐₓ (NaCl/Film)/cm⁻¹ 3307 (NH), 3078w, 3035w, 1747s, (C=O, Ester), 1660w, 1644w, 1602w. 1557w, 1479w, 1435w, 1376w, 1240s, 1089s, 1049s, 917w, 778w, 733w; NMR-Spektrum: δ_{H} (270 MHz, CDCl₃): 12.10.(1H, bs, NH), 9.20 (1H, s, H-7), 9.09 (1H, s, H-5), 6.00 (1H, t, J=5.3 Hz, H-2'), 5.70 (1H, t, J 5.5Hz, H-3'), 5.60 (1H, d, J 5.5 Hz. H-1'), 4.47-4.53 (1H, m, H-5a'), 4.42-4.45 (1H, m, H-4'), 4.24-4.30 (1H, m, H-5b'), 2.13 (3H, s. CH₃), 2.07 (3H, s, CH₃), 2,03 (3H, s CH₃); Massenspektrum (FAB, NBA): Gefunden MH⁺, 379.1267, errechnet: C₁₆H₁₉N₄O₇, MH, 379.1254

### Methode B

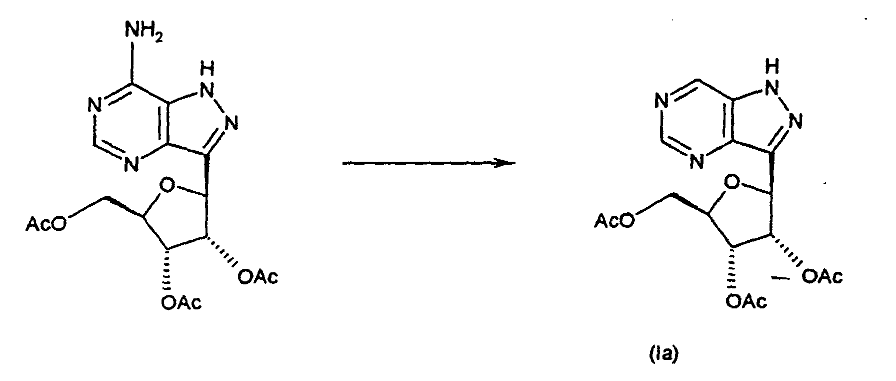

Eine Mischung aus 2',3',5'-Tri-O-acetyl-formycin A (hergestellt aus Formycin A durch Modifikation der Methode aus Synthesis (1989) 401) (1,1 g, 2,8 mmol) und n-Butylnitrit (2,6 ml, 22 mmol) in THF (30 ml) wurde 25 h bei 50°C erhitzt. Nach dem Abkühlen auf Raumtemperatur wurde das Lösungsmittel unter reduziertem Druck entfernt. Die Mischung wurde in EtOH gelöst und nochmals eingeengt (je 2 mal). Nach Chromatographie an Kieselgel mit 2 % MeOH in DCM erhielt man Verbindung (Ia) (0,49 g, 46 %), deren Analyse die chemische Identität mit dem Produkt aus Methode A bestätigte.

### Beispiel 3

3-β-D-Ribofuranosyl-1H-pyrazolo[4,3-d]pyrimidin (Ib)

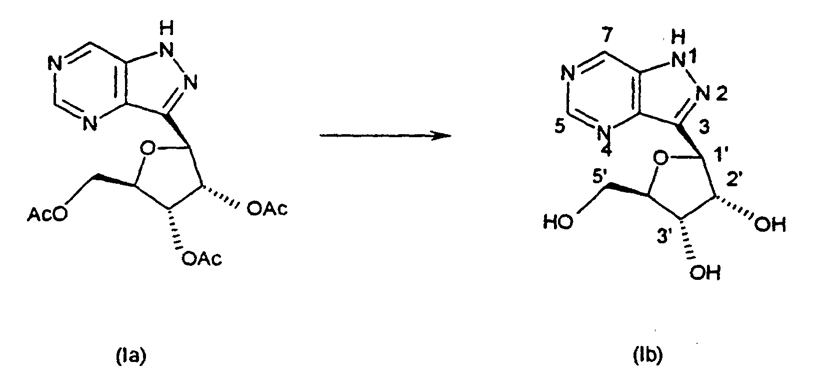

Eine Lösung des Triacetats (Ia) (46 mg, 0.12 mmol) in EtOH/NH₃ (4 ml, gesättigt bei 0°C) wurde 4 Tage bei Raumtemperatur gerührt. Nach Einengen und Chromatographie über Kieselgel (Gradienteneluierung mit 5 bis 10 %igem Methanol in DCM) ergab die Titelverbindung (Ib) als farblose Kristalle (Ausbeute: 30 mg, 98% d. Th.), Schmelzpunkt 227-228°C aus Ethanol; IR-Spektrum: νₘₐₓ (NaCl/Nujol/cm⁻¹) 3380bw, 3325bw, 3115bw (NH und OH), 1695w, 1617w, 1551w, 1532w, 1287w, 1266w, 1244w, 1242w, 1125w, 1112w, 1099s, 1049s, 1026s, 984w, 930s, 868w, 826w, 795w; NMR-Spektrum: δ_{H} (270 MHz, DMSO-d₆ + D₂O) 9.34 (1H, s, H-7), 9.01 (1H, s, H-5), 5.08 (1H, d, J 7.1 Hz, H-1'), 4.58 (1H, dd, J 7.0, 5.3Hz, H-2'), 4.13 (1H, dd, J 5.0, 3.7Hz, H-3'), 3.95 (1H, q, J 3.7Hz, H-4'), 3.65 (1H, AB, J 12.2, 4.1 Hz, H-5a'), 3.52 (1H, AB, J 12.0, 4.4Hz, H-5b'); Massenspektrum: (FAB, NBA): Gefunden MH⁺ 253.0941. Errechnet: C₁₀H₁₃N₄O₄ MH, 253.0937.

### Beispiel 4

3-(5'-O-Phosphoryl-β-D-ribofuranosyl)-pyrazolo[4,3-d]pyrimidin-di-natriumsalz (lc)

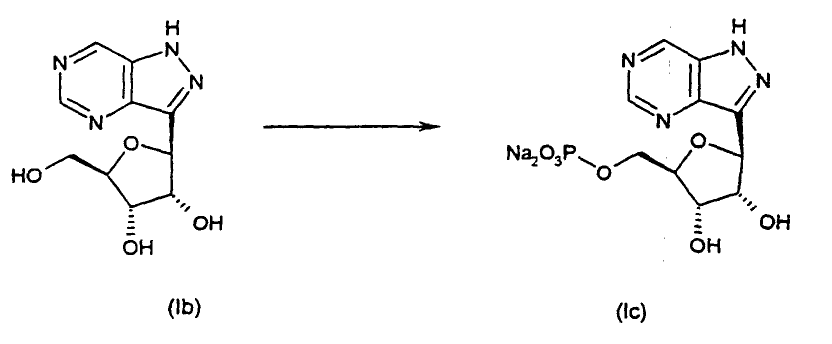

POCl₃ (60 µl, 0,66 mmol) ließ man bei 0°C unter N₂-Gas in eine Suspension von Deaminoformycin A (15 mg, 0,06 mmol) (Ib) in trockenem Phosphorsäuretriethylester (1,5 ml) zutropfen. Nach 1,5 h ergab die Reaktionsmischung eine klare Lösung; nach 15 h wurde Eiswasser (5 ml) zugegeben, die Mischung mit DCM extrahiert und mit gesättigter Natriumhydrogencarbonat-Lösung neutralisiert. Nach Chromatographie der Lösung über eine "Reverse-Phase"-Säule (Elution mit Wasser) und Abziehen des Lösungsmittels erhielt man das Titelprodukt (Ic) als weißen Feststoff mit einem Schmelzpunkt von mehr als 220 °C;
Massenspektrum (Elektrospray, Positivionen) 377 (MH⁺).
IR-Spektrum: νₘₐₓ [cm⁻¹] ("Golden-Gate"-Technik) 3370 (bw), 3230 (bw), 1677 (s), 1201 (s), 1110 (s), 975 (m).
¹H-NMR (D₂O, 270 MHz): δ [ppm] = 9,30 (s, 1H, H-7), 8,95 (s, 1H, H-5); 5,36 (d, 1H, J = 7,5 Hz, H-1'), 4,76 (m, 1H, H-2'), 4,38 (t, 1H, J = 5 Hz, H-3'), 4,12 (q, 1H, J = 5 Hz, H-4'), 3,91 (m, 2H, H-5').

### Beispiel 5

6,8-Di(methylthio)-3-(5'-O-tert-butyldiphenylsilyl-2',3'-O-isopropylidene-β-ribofuranosyl)imidazo[2,1-f]-1,2,4-triazin (IIIa)

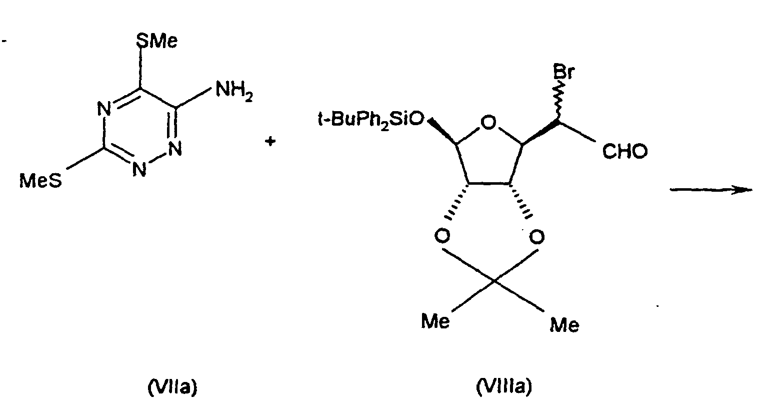

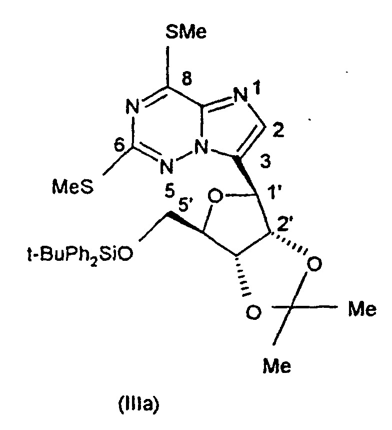

### Methode A

Eine Mischung des Bromaldehyds (VIIIa) (hergestellt analog J. Org. Chem. 48 (1983) 3141, jedoch mit der Si-haltigen Schutzgruppe) (664 mg, 1,25 mmol), des Amins (VIIa) (siehe J. Org. Chem. 48 (1983) 1271) (234 mg, 1,25 mmol) und wasserfreiem Kaliumcarbonat (208 mg, 1,50 mol) in trockenem Toluol (80 ml) wurde zum Rückfluß erhitzt, wobei das entstehende Wasser azeotrop entfernt wurde.

Nach 24 h ließ man auf Raumtemperatur abkühlen und entfernte das Lösungsmittel unter reduziertem Druck. Nach Chromatographie an Kieselgel mit 10 bis 15 % Ether in Petrolether erhielt man 434 mg, 56 %.
IR-Spektrum: νₘₐₓ (NaCl/Film/cm⁻¹) 3071w, 1694w, 1631w, 1574w, 1516w, 1441s, 1372w, 1352w, 1214w, 1154s, 1114s. ¹H-NMR-Spektrum: δ_{H} (270 MHz, CDCl₃) 7.63-7.69 (4H, m, Ar H), 7.60 (1H, s, H-2), 7.32-7.43 (6H, m, Ar H), 5.39 (1H, d, J 4.9 Hz, H-1'), 4.97 (1H, dd, J 6.6, 4.8Hz, H-2'), 4.82 (1H, dd, J 6.5, 3.7Hz, H-3"), 4.24-4.27 (1H, m H-4'), 3.84 - 3.86 (2H, m, H-5'), 2.67 (3H, s, SCH₃), 2.52 (3H, s, SCH₃), 1,37 (3H, s, CH₃), 1.37 (3H, s, CH₃), 1.05 (9H, s, tert-Bu); Massenspektrum: Gefunden MH⁺, 623.2182, C₃₁H₃₉N₄O₄S₂Si errechnet MH, 623.2111

### Methode B

Zur Lösung des Amins (VIIa) (1,20g, 6,38 mmol) in HMPA (6 ml) wurde eine Lösung des Aldehyds (VIIIa) (3,40g, 6,38 mmol) in trockenem Toluol gegeben, die Mischung bei 100°C für 18 Stunden unter Stickstoffgas gerührt und auf 25°C abgekühlt. Nach Entfernen des Lösungsmittels unter reduziertem Druck wurde der Rückstand dreimal mit Essigester extrahiert, die vereinigten organischen Phasen wurden mit Wasser und Kochsalzlösung gewaschen, über MgSO₄ getrocknet und im Vakuum eingeengt. Nach Chromatographie über Kieselgel mit 10 bis 15 % Ether in Petrolether erhielt man Verbindung (IIIa) (2,46g, 62 % d.Th.); Analyse wie unter Methode A.

### Beispiel 6

8-Hydrazino-6-methylthio-3-(5-O-tert-butyldiphenylsilyl-2',3'-O-isopropyliden-β-D-ribofuranosyl)imidazo[2,1-f]-1,2,4-triazin (IIIb)

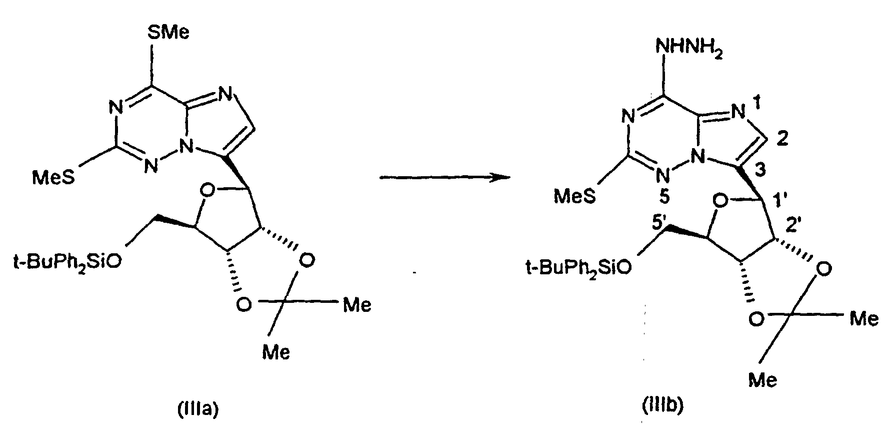

Zur Lösung des Dithioethers (IIIa) (50,9 mg, 0,08 mmol) in Ethanol (2ml) wurde Hydrazinmonohydrat (0,02 ml, 0,70 mmol) gegeben und die Lösung unter Rühren für 1 Stunde zum Rückfluß erhitzt. Nach Abkühlen auf Raumtemperatur wurde im Vakuum eingeengt, der Rückstand in DCM (6 ml) gelöst, die organische Phase mit Wasser gewaschen, über MgSO₄ getrocknet und nach Einengen im Vakuum über Kieselgel chromatographiert (Ether). (Ausbeute: 43 mg, 87 %). IR-Spektrum: νₘₐₓ (NaCl/Film/cm⁻¹) 3442bs (NH), 2931w, 2858w, 1694s, 1631s, 1603s, 1443s, 1428s, 1353s, 1265s, 1113s, 1079s, 863w, 703s;
¹H-NMR: δ_{H} (270 MHz, CDCl₃) 7.63 - 7.69 (4H, m, Ar H), 7.54 (1H, s, H-2), 7.31-7.40 (6H, m, Ar H), 5,35 (1H, d, J 4.85 Hz, H-1'), 5.02 (1H, dd, J 6.5, 4.8 Hz, H-2'), 4.82 (1H, dd, J 6.5, 3.7, H-3'), 4.24-4.25 (1H, m, H-4'), 3.84-3,86 (2H, m,
H-5'), 2.48 (3H, s, SCH₃), 1.62 (3H, s, CH₃), 1.37 (3H,s CH₃), 1.05 (9H, s, tert.-Bu); Massenspektrum (FAB, NBA): Gefunden MH⁺, 607.2537 C₃₀H₃₉N₆O₄SSi; errechnet: MH, 607.2523.

### Beispiel 7

6-Methylthiosulfanyl-3-(5'-O-tert-butyldiphenylsilyl-2',3'-O-isopropyliden-β-Dribofuranosyl)imidazo[2,1-f]1,2,4-triazin (IIIc)

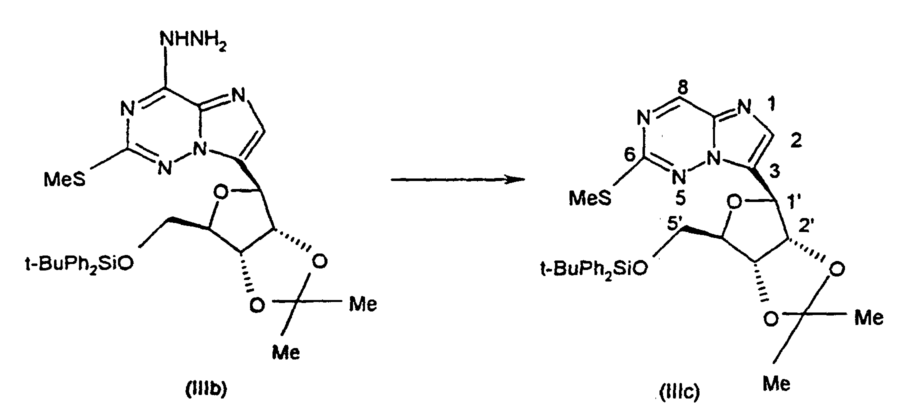

Eine Lösung des Hydrazinderivats (IIIb) (1,04 g, 1,72 mmol) in Ethanol (60 ml) wurde bei Raumtemperatur mit gelbem HgO (1,12 g, 5,15 mmol) versetzt. Nach Erhitzen zum Rückfluß (2 h) wurde auf 25°C abgekühlt, anorganische Bestandteile wurden durch Filtration über Celite entfernt, mit Ethanol nachgewaschen und die organische Phase wurde im Vakuum eingeengt. Der erhaltene gelbe Schaum wurde mit 5 bis 10 %igem Ether in Petrolether an Kieselgel chromatographiert, wonach die Titelverbindung (712 mg, 72 %), als farbloser Schaum erhalten wurde;
NMR-Spektrum δ_{H} (300 MHz, CDCl₃) 9.00 (1H, s, H-8), 7.80 (1H, s, H-2), 7.64-7.70 (4H, m, Ar H), 7.33-7.44 (6H, m, Ar H), 5.46 (1H, d, J 5.0 Hz, H-1'), 4.98 (1H, dd, J 6.4, 5.1 Hz, H-2'), 4.86 (1H, dd, J 6.1. 3.6 Hz, H-3'), 4.30-4.31 (1H, m, H-4'), 3.87 - 3.89 (2H, m, H-5'), 2.56 (3H, s, SCH₃), 1.65 (3H, s, CH₃), 1.39 (3H, s, CH₃), 1.07 (9H, s, tert.-Bu); Massenspektrum (FAB, NBA): Gefunden MH⁺, 577.2321 C₃₀H₃₇N₄O₄SSi, errechnet: MH, 577.2305

### Beispiel 8

6-Methylthio-3-(2',3'-O-isopropyliden-β-D-ribofuranosyl-imidazo[2,1-f]-1,2,4-triazin (Id)

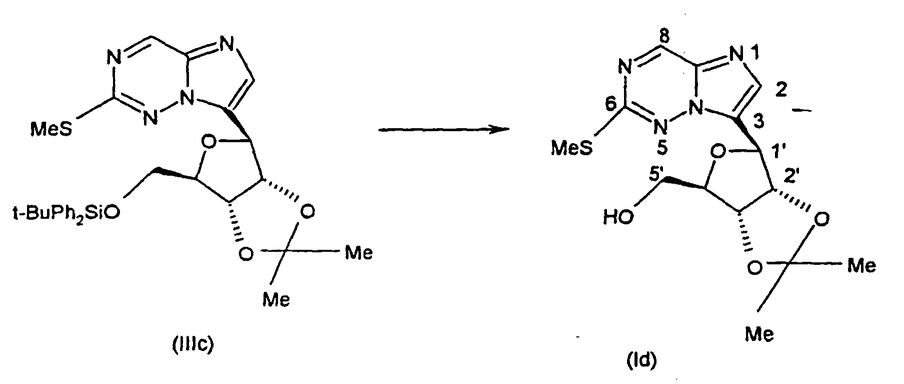

Zur Lösung des Silylethers (IIIc) (211 mg, 0,37 mmol) in trockenem Tetrahydrofuran (THF) (12 ml) wurde n-Bu₄NF (TBAF, 0,73 ml, 1M in THF, 0,73 mmol) gegeben und das Gemisch 30 min bei 25°C gerührt. Nach Verdünnen mit Ether wurde Wasser (20 ml) zugegeben, die organische Phase abgetrennt, mit Kochsalzlösung (10 ml) gewaschen, über MgSO₄ getrocknet und im Vakuum eingeengt. Nach Chromatographie an Kieselgel mit 60 bis 70 % Ether in Petrolether erhielt man den Alkohol (Id) als weißen Feststoff (111 mg, 90 %) mit Schmelzpunkt 92 bis 93°C (aus DCM/Petrolether). IR-Spektrum: νₘₐₓ ((NaCl-Film/cm⁻¹) 3441w (OH), 2935w, 2876w, 1694w, 1628w, 1591s, 1531s, 1471s, 1422s, 1382s, 1342s, 1304s, 1274w, 1214s, 1157w, 1122s, 1078s, 921w 862w, 763w, 735w, 662w; ¹H-NMR-Spektrum: δ_{H}(270 MHz, CDCl₃) 8.99 (1H, s, H-8), 7.82 (1H, s, H-2), 5.31 (1H, d J 5.5 Hz, H-1'), 5.16 (1H, dd, J 6.6, 5.5 Hz, H-2'), 4.96 (1H, dd, J 6.6, 3.7 Hz, H-3'), 4.25-4.27 (1H, m, H-4'), 3.89 - 3.90 (2H, m, H-5'), 2.91 - 3.22 (1H, m, OH), 2.62 (3H, s, SCH₃), 1.62 (3H, s, CH₃), 1.37 (3H, s, CH₃), Massenspektrum (FAB, NBA): Gefunden MH⁺, 339.1144, C₁₄H₁₉N₄O₄S, errechnet: MH, 339.1127

### Beispiel 9

6-Methylthio-3-β-D-ribofuranosylimidazo[2,1-f]-1,2,4-triazin (Ie)

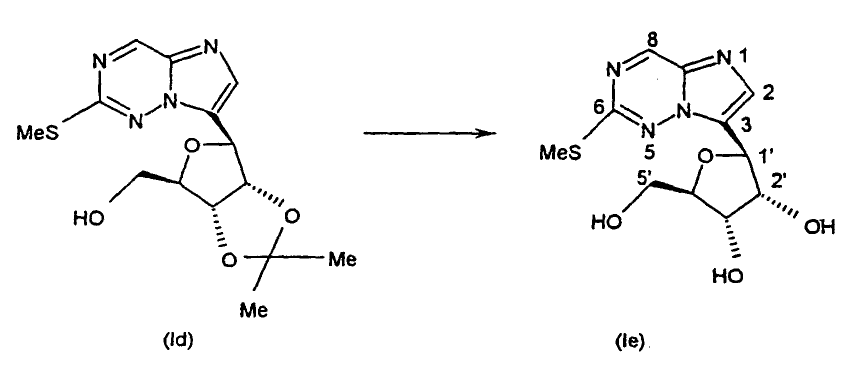

Eine Lösung aus 1,3-Dioxolan (Id) (50,9 mg, 0,15 mmol), Eisessig (2ml) und Wasser (1 ml) wurde bei 25°C 18 Stunden gerührt. Anschließend wurde das Lösungsmittel im Vakuum entfernt. Nach Umkristallisation in Ethanol wurde (le) (27 mg, 60 %) als weiße Kristalle erhalten; Schmelzpunkt (227-228°C) (EtOH); IR-Spektrum: νₘₐₓ (NaCl/Nujol/cm⁻¹) 3441 (OH), 3202 (OH), 1693w, 1630w, 1595s, 1529w, 1311s, 1225s, 1172s, 1116s, 1069w, 1040w, 985w, 957w, 931w, 774w; NMR-Spektrum: δ_{H} (270 MHz, DMSO-d₆+D₂O) 9.14 (1H, s, H-8), 7.97 (1H; s, H-2), 5.16 (1H, d, J 6.2 Hz, H-1'), 4.42 (1H, dd, J 6.0, 5.5 Hz, H-2'), 4.00-4.08 (1H, m, H-3'), 3.90-3.91 (1H, m, H-4'), 3.52-3.58 (2H, m, H-5'), 2.60 (3H, s, SCH₃); Massenspektrum (FAB, NBA): Gefunden MH⁺, 299.0809 C₁₁H₁₅N₄O₄S, errechnet MH, 299.0814.

### Beispiel 10

6-Brom-3-dimethylamino-1,2,4-triazin-5(4H)-on (X)

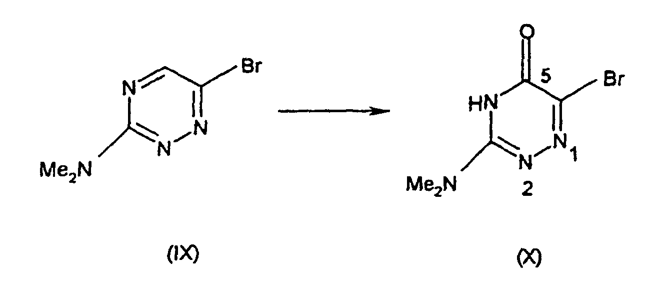

Wasserstoffperoxid (27 %ige Lösung in Wasser, 0,53 ml, 4,56 mmol) wurde der Lösung von 6-Brom-3-dimethylamino-1,2,4-triazin (IX) (J. Org. Chem. 43 (1978) 2514) (500 mg, 2,46 mmol) in Eisessig (4 ml) bei 5°C hinzugefügt und die Reaktionsmischung bei 25°C 12 Stunden gerührt. Der entstandene Niederschlag wurde gesammelt, mit Wasser gewaschen, an der Luft getrocknet und aus Ethanol umkristallisiert. Man erhielt das Keton (X) (368 mg, 68 %) als weiße Kristalle mit Schmelzpunkt 261-262°C (Ethanol).
IR-Spektrum: νₘₐₓ (NaCl/Nujol/cm⁻¹) 3114w, 1608s, 1567s, 1504s, 1435s, 1403s, 1254w, 1211 w, 1156w, 1133w, 1076s, 1016s, 908s, 865w, 769s 701w, 646w; ¹H-NMR-Spektrum: δ_{H} (270 MHz, DMSO-d₆), 3.72 (6H, s, NCH₃); Massenspektrum: Gefunden M⁺, 220.9866. C₅H₇⁸¹BrN₄O, berechnet M, 220.9861;
Gefunden M⁺, 218.9885. C₅H₇⁷⁹BrN₄O berechnet M, 218.9881

### Beispiel 11

3-Dimethylamino-6-hydrazino-1,2,4-triazin-5(4H)-on (XI)

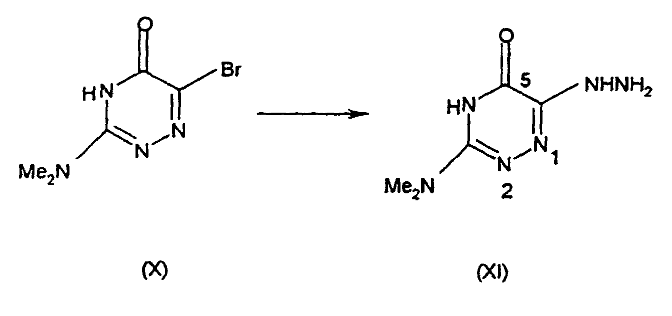

Hydrazin-monohydrat (1,44 ml, 30 mmol) wurde unter Rühren zur Lösung des Bromids (X) (2,20 g, 10 mmol) in Wasser (80 ml) bei 25°C hinzugefügt. Die Reaktionsmischung wurde zum Rückfluß erhitzt, auf Raumtemperatur abgekühlt und zur Kristallisation mehrere Stunden stehengelassen. Nach Abfiltrieren der Kristalle, Waschen mit Wasser, Trocknen an der Luft erhielt man das Hydrazin (XI) (1,08 g, 63 %) als weißen Feststoff mit Schmelzpunkt 264-266°C (aus Ethanol): IR-Spektrum νₘₐₓ (NaCl/Nujol/cm⁻¹) 3324s, 3301s, (NH); 1641s (C=O), 1575s, 1516s, 1397s, 1304w, 1260w, 1204w, 1165w, 1133w, 1068w, 1050s, 1005w, 923s, 833w, 786s, 709s, 683s; NMR-Spektrum: δ_{H} (270 MHz, DMSO-d₆): 11.25 (2H, bs, NH₂), 7.18 (1H, bs, NH), 2.96 (6H, s, NCH₃); Massenspektrum: Gefunden M⁺, 170,0933 C₅H₁₀N₆O, berechnet M, 170.0916

### Beispiel 12

N-[(2',3',5'-Tri-O-benzoyl-β-D-ribofuranosyl)-carbonyloxy]bernsteinsäureimid (XIII)

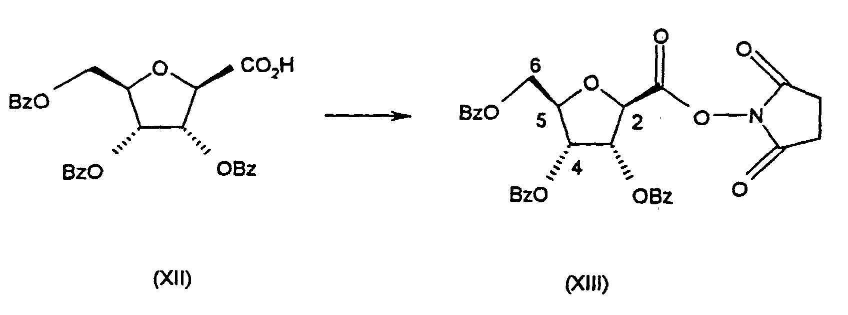

Zur Lösung von 2,3,5-Tri-O-benzoyl-β-D-ribofuranosylcarbonsäure (XII) (Collect. Czech. Chem. Comm. 43 (1978) 1431) (1,85 g,
3,77 mmol) in trockenem 1,2-Dichlorethan (32 ml) fügte man 1,3-Dicyclohexylcarbodiimid (57 mg, 4,15 mmol) und N-Hydroxybernsteinsäure (478 mg, 4,15 mmol) zu. Die Reaktionsmischung wurde 24 Stunden bei Raumtemperatur unter N₂-Gas gerührt. Der enstandene Niederschlag wurde abfiltriert und das Filtrat im Vakuum eingeengt. Das Rohprodukt der Verbindung (XIII) (99 %) konnte direkt für die Folgeumsetzung verwendet werden (siehe Beispiel 13).

### Beispiel 13

Kondensation des Hydrazins (XI) mit der aktivierten Säure (XIII) zum Hydrazid (IVa)

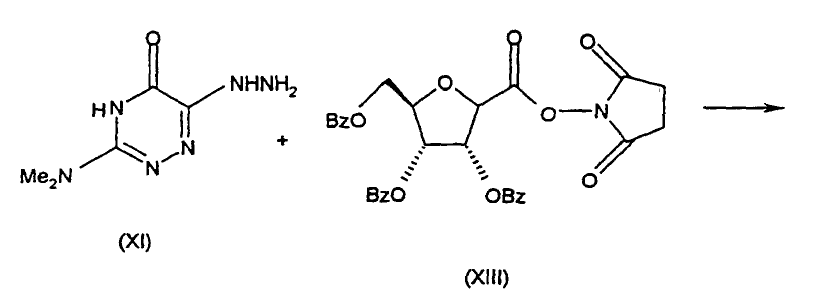

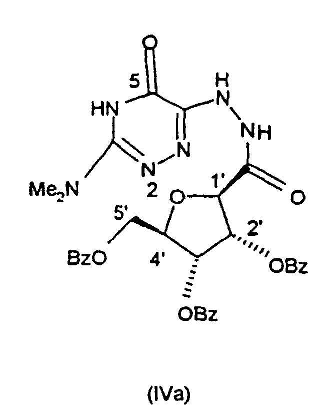

Zur Lösung des Hydrazins (XI) (706 mg, 4,15 mmol) in trockenem DMF (120 ml) wurde eine Lösung der aktivierten Säure (XIII) (2,2 g, 3,78 mmol) in trockenem DMF (20 ml) bei Raumtemperatur hinzugefügt. Nach 24 Stunden Rühren bei 60°C unter Stickstoffatmosphäre wurde überschüssiges Lösungsmittel im Vakuum entfernt und der Rückstand in DCM gelöst. Die organische Phase wurde mit Wasser und Kochsalzlösung gewaschen und über MgSO₄ getrocknet. Nach Abziehen des Lösungsmittels im Vakuum erhielt man einen gelben Schaum, der über Kieselgel chromatographiert wurde (Laufmittel 10 %iges Aceton in DCM). Ausbeute: 1,88 g = 78 % d.Th. Verbindung (IVa) als farblose Kristalle; Schmelzpunkt 126-128°C. IR-Spektrum: νₘₐₓ (NaCl/Film/cm⁻¹) 3253bs (NH), 3064w, 3010w, 2978w, 1730s (C=O, Ester), 1715s (C=O, Amid), 1651s, 1644s, 1634s, 1602s, 1587s, 1557w, 1538w, 1515s, 1505s, 1471w, 1464w, 1454s, 1397w, 1316w, 1271s, 1179s, 1179w, 1097w, 1026w, 931s, 756s, 711s, 687w;
NMR-Spektrum: δ_{H} (270 MHz, CDCl₃) 9.98 (1H, bs, NH), 8.96 (1H, d, J 3.5, NH), 7.83 - 7.96 (4H, m Ar H), 7.80 (2H, d, J 1.4 Hz, Ar H), 7.28 - 7.58 (9H, m, Ar H), 6.16 (1H, dd, J 8.2, 4.6Hz, H-2'), 6.00 (1H, dd, J 4.8, 2.1 Hz, H-3'), 4.94 (1H, d, J 2.1 Hz, H-1'), 4.81 - 4.86 (1H, m, H-4'), 4.67 (1H, s, NH), 4.62 - 4.71 (2H, m, H-5'), 3.04 (6H, s, NCH₃); Massenspektrum (FAB, NBA): Gefunden MH⁺, 643.2222 C₃₂H₃₁N₆O₉, errechnet MH, 643.2153.

### Beispiel 14

6-Dimethylamino-3-(2',3',5'-tri-O-benzoyl-β-ribofuranosyl)-1,2,4-triazolo[3,4-f]-1,2,4-triazin-8(7H)-on (IIa-2)

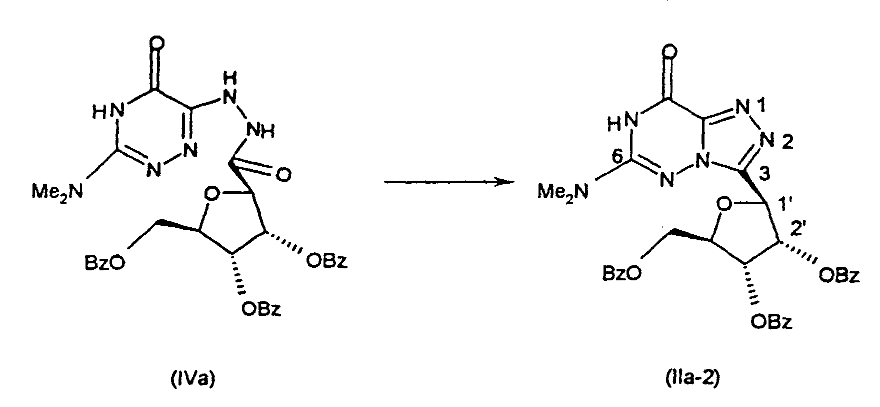

### Methode A

Die Lösung des Hydrazids (IVa) (200 mg, 0,31 mmol) in trockenem DMF (40 ml) wurde für 24 Stunden unter Stickstoffgas auf Rückfluß erhitzt. Nach Abkühlen auf Raumtemperatur und Entfernen überschüssigen Lösungsmittels im Vakuum wurde der Rückstand in Dichlormethan (DCM) gelöst. Nach Waschen der organischen Phase mit Wasser, Trocknen über MgSO₄ und Einengen im Vakuum erhielt man einen gelblichen Feststoff, der nach Chromatographie über Kieselgel mit 2 bis 5 % Aceton/DCM die Verbindung (IIa-2) (113 mg, 58 %) mit dem Schmelzpunkt 125-126°C (Petrolether/EtOAc) ergab: IR-Spektrum: vₘₐₓ (NaCl/Film/cm⁻¹) 3200w (NH), 1728w (C=O, Ester), 1611s, 1493s, 1452s, 1381s, 1316s, 1270s, 1178w, 1123s, 1071s, 1026w, 912w, 786w, 711s, 648w; NMR-Spektrum: δ_{H}(270 MHz, CDCl₃) 7.92-8.11 (6H, m, Ar H), 7.31 - 7.57 (9H, m, Ar H), 6.44 (1H, dd, J 5.8, 4.4 Hz, H-2'), 6.12 (1H, t, J 6.2Hz, H-3'). 5.82 (1H, d, J 4.4 Hz, H-1'), 4.64 - 4.80 (3H, m, H-4' und H-5'), 3.19 (6H, s, NCH₃); Massenspektrum (FAB, NBA): Gefunden MH⁺, 625.2090 C₃₂H₂₉N₆O₆, berechnet MH, 625.247.

### Methode B

Eine Lösung des Hydrazids (XI) (100 mg, 0,59 mmol) (s. Beispiel 13), der Säure (XIII) (318 mg, 0,65mmol) (s. Beispiel 13), DCC (134 mg, 0,65 mmol) und N-Hydroxysuccinimid (74,5 mg, 0,65 mmol) in trockenem DMF wurde für 24 Stunden im Rückfluß erhitzt. Nach Abkühlen auf Raumtemperatur wurde der Rückstand in Essigester gelöst, die organische Phase mit Wasser und Kochsalzlösung gewaschen, über MgSO₄ getrocknet und im Vakuum eingeengt. Der erhaltene gelbliche Feststoff wurde mit 2 bis 5 % Aceton/DCM an Kieselgel chromatographiert und ergab die Verbindung (IIa-2) (56,5 mg, 58 % d.Th.) als weißen Feststoff der Zusammensetzung wie nach Methode A erhalten.

### Beispiel 15

8-Chlor-6-dimethylamino-3-(2',3',5'-tri-O-benzoyl-β-D-ribofuranosyl)-1,2,4-triazolo[3,4-f]-1,2,4-triazin (II-2)

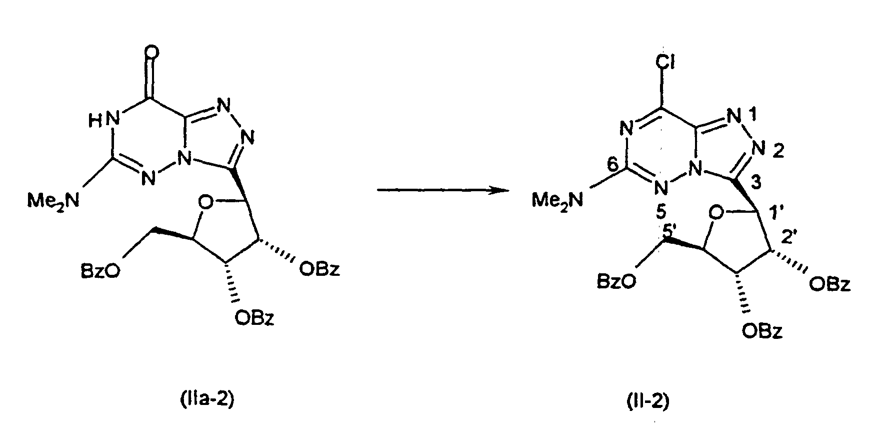

### Methode A

Eine Mischung des geschützen C-Nukleosids (IIa-2) (150 mg, 0,24 mmol), N,N-Dimethylanilin (1ml) und Phosphoroxychlorid (4 ml) wurde 40 min unter Rückfluß erhitzt. Nach Abkühlen auf Raumtemperatur und Entfernen des überschüssigen Lösungsmittels im Vakuum wurde mit einem Eis/Wasser-Gemisch versetzt. Nach Extraktion mit Essigester (3x) wurde die kombinierte organische Phase über MgSO₄ getrocknet und im Vakuum eingeengt. Chromatographie des Rückstands mit 30 bis 40 %igem Ether in Petrolether ergab das Chlorid (II-2) (93 mg, 60 % d.Th.) als gelbe Kristalle mit dem Schmelzpunkt 88-90°C (DCM/Petrolether). IR-Spektrum: νₘₐₓ (NaCl/Film/cm⁻¹) 3065w, 3034w, 3010w, 1729s (C=O, Ester), 1596s, 1574s, 1505s, 1486s, 1452s, 1417s, 1382w, 1344w, 1316s, 1270s, 1179w, 1155, 1123s, 1096s, 1071s, 1027w, 958w, 912w, 805w, 785w, 712s, 688w;
NMR-Spektrum: δ_{H} (270 MHz, CDCl₃) 7.91-8.00 (6H, m, Ar H), 7.49-7.56 (3H, m, Ar H), 7.31 - 7.38 (6H, m, Ar H), 6.48 (1H, dd, J 5.7, 4.39 Hz, H-2'), 6.18 (1H, t, J 6.2 Hz, H-3'), 5.88 (1H, d, J 4.4 Hz, H-1'), 4.63-4.82 (2H, m, H-4' und H5'), 3.21 (6H, s, NCH₃);
Massenspektrum (FBA, NBA): Gefunden MH+, 643.1738, C₃₂H₂₈ClN₆O₇, berechnet: MH, 643.1708

### Methode B

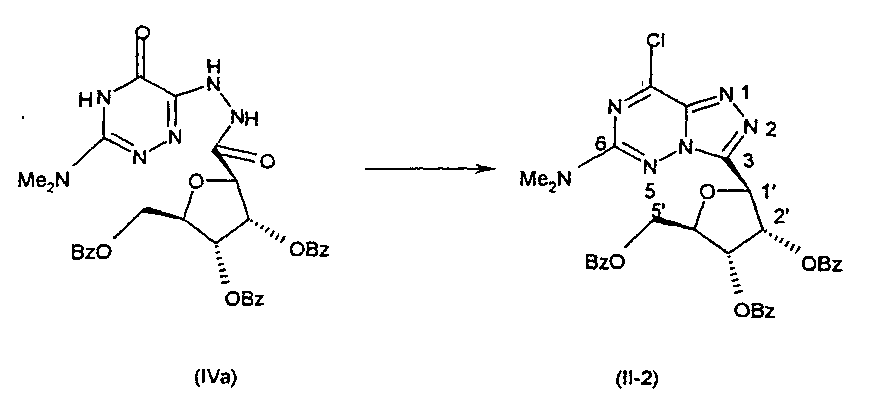

Eine Mischung des Hydrazids (IVa) (100 mg, 0,16 mmol) und Phosphoroxychlorid (POCl₃) (4 ml) wurde für 40 min unter Rückfluß erhitzt. Nach Abkühlen auf Raumtemperatur und Einengen im Vakuum wurde der Rückstand mit einem Eis-Wasser-Gemisch versetzt. Nach Extraktion mit Essigester (3x), Trocknen über MgSO₄ und Einengen im Vakuum wurde der Rückstand mit 30 bis 40 % Ether in Petrolether an Kieselgel chromatographiert. Man erhielt das Chlorid (II-2) als gelbe Kristalle (78 mg, 62 %) deren Analyse die chemische Identität mit dem Produkt aus Methode A bestätigte.

### Beispiel 16

6-Dimethylamino-8-hydrazino-3-(2',3',5'-tri-O-benzoyl-β-D-ribofuranosyl)-1,2,4-triazolo[3,4-f]-1,2,4-triazin (11-3)

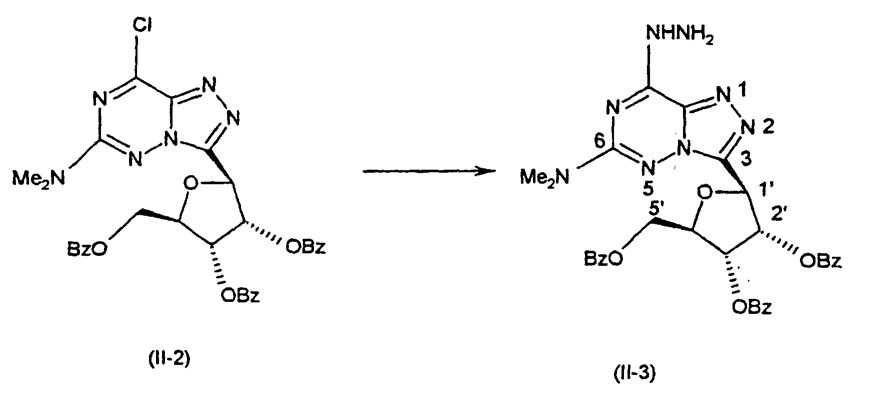

Zur Lösung des Chlorids (11-2) (80 mg, 0,13 mmol) in 2 ml Essigester wurde Hydrazinmonohydrat (0,007 ml, 0,14 mmol) hinzugefügt und bei 25°C 5 min gerührt. Dabei entstand ein gelber Niederschlag, der mit Essigester gewaschen und aus Essigester/Petrolether umkristalisiert wurde. Man erhielt Verbindung (11-3) (70 mg, 88 %) als gelbliche Kristalle vom Schmelzpunkt 78 bis 79°C.
IR-Spektrum: νₘₐₓ (NaCl/Film/cm⁻¹) 3320w, 3217w (NH), 3061w, 1725s (C=O, Ester), 1692w, 1680w, 1659w, 1602s, 1584s, 1574w, 1548s, 1537s, 1514w, 1485w, 1452s, 1409w, 1316s, 1270s, 1124s, 1098s, 1026w, 962w, 711w.
NMR-Spektrum: δ_{H} (270 MHz, CDCl₃) 7.92 - 8.00 (6H, m, Ar H), 7.80 (1H, d, J 1.6 Hz, NH), 7.31 - 7.57 (9H, m, Ar H), 6.51 (1H, dd, J 5.7, 3.9Hz, H-2'), 6.27 (1H, dd, J 6.9, 5.8Hz, H-3'), 5.85 (1H, d, J 3.9 Hz, H-1'), 4.71-4.80 (2H, m, H-4' und H-5a'), 4.64-4.68 (1H, M, H-5b'), 3.18 (6H, s, NCH₃),
Massenspektrum (FAB, NBA): Gefunden MH⁺, 639.2366 C₃₂H₃₁N₈O₇; berechnet MH, 639.2316.

### Beispiel 17

6-Dimethylamino-3-(2',3',5'-tri-O-benzoyl-β-ribofuranosyl)-1,2,4-triazolo[3,4-f]-1,2,4-triazin (If)

### Methode A

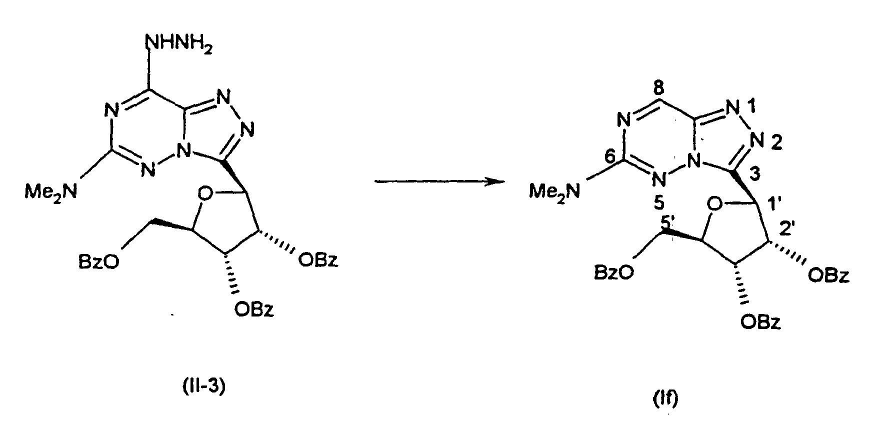

Zur Lösung der Hydrazinverbindung (II-3) (60 mg, 0,09 mmol) in Ethanol (4 ml) wurde gelbes HgO (61 mg, 0,28 mmol) 25°C hinzugefügt und die Reaktionsmischung für 1 Stunde unter Rückfluß erhitzt. Nach Abkühlen, Abfiltrieren über Celite, Waschen mit Ethanol und Abziehen des Lösungsmittels im Vakuum erhielt man einen gelblichen Feststoff, der mit 10 bis 20 % Ether im Petrolether an Kieselgel chromatographiert wurde. Ausbeute: 31 mg = 55 % d.Th. an Verbindung (If) als schwach gelbe Kristalle vom Schmelzpunkt 79 bis 80°C (aus DCM/Petrolether).
IR-Spektrum: νₘₐₓ (NaCl/Film/cm⁻¹) 3065w, 3035w, 2929w, 1726s, (C=O, ester), 1682w, 1601s, 1565w, 1515w, 1493w, 1452w, 1416w, 1387w, 1342w, 1316w, 1269s, 1178w, 1122s, 1097s, 1071s, 1026s, 993w, 921w, 875w, 805w, 789w, 761w, 711s, 687w; NMR-Spektrum: δ_{H} (270 MHz, CDCl₃) 9.19 (1H, s, H-8), 7.93-8.02 (6H, m, Ar H), 7.50-7.56 (3H, m, Ar H), 7.34-7.40 (6H, m, Ar H), 6.48 (1H, dd, J 5.8, 4.4 Hz, H-2'), 6.21 (1H, t, J 6.2Hz, H-3'), 5.90 (1H, d, J 4.2Hz, H-1'),
4.66-4.80 (3H, m, H-4' und H-5'), 3.24 (6H, s, NCH₃);
Massenspektrum: Gefunden MH⁺, 609.2113 C₃₂H₂₉N₆O₇, berechnet MH, 609.2098.

### Methode B

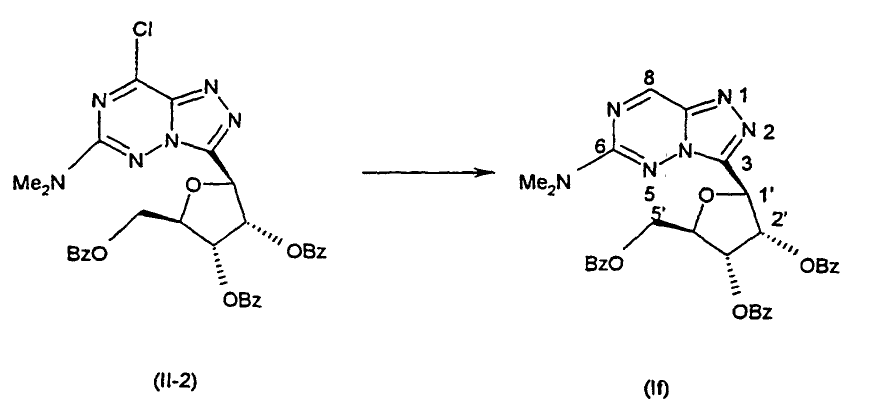

Zur Lösung der Chlorverbindung (II-2) (100 mg, 0,16 mmol) in trockenem Essigester (6 ml) wurden 5 % Palladium/Kohle (17 mg) und Magnesiumoxid (16 mg, 0,41 mmol) zugegeben und die Lösung bei Raumtemperatur mehrere Tage mit Wasserstoffgas überlagert. Nach Abfiltrieren über Celite, Waschen mit Essigester und Abziehen des Lösungsmittels im Vakuum wurde der Rückstand mit 10 bis 20 %igem Ether in Petrolether an Kieselgel chromatographiert. Man erhielt Verbindung (If) als gelbliche Kristalle (91 mg, 96 %) mit identischen Analysedaten, verglichen mit dem Produkt nach Methode A.

### Beispiel 18

6-Dimethylamino-3-β-ribofuranosyl-1,2,4-triazolo[3,4-f]-1,2,4-triazin (Ig)

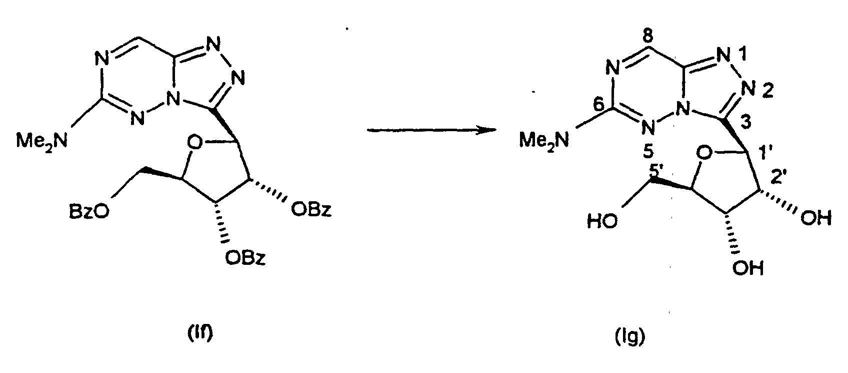

Eine Lösung des geschützten C-Nukleosids (I-f) (90 mg, 0,15 mmol) in MeOH/NH₃ (5 ml, gesättigt bei 0°C) wurde 2 Tage bei Raumtemperatur gerührt. Nach Abziehen des Lösungsmittels im Vakuum und Chromatographie an Kieselgel mit 10 bis 15 %igem MeOH in DCM ergab die Verbindung (I-g) (38 mg, 86 %) als gelbliche Kristalle vom Schmelzpunkt 171-173°C (Aceton);
IR-Spektrum: νₘₐₓ (NaCl/Film/cm⁻¹) 3387bw, 3230bw (OH), 3021w, 1614s, 1573s, 1512w, 1481s, 1466w, 1440s, 1423s, 1384w, 1366w, 1344s, 1285s, 1216s, 1130s, 1100s, 1055s, 1032s, 1004s, 990w, 960s, 853s, 759s;
NMR-Spektrum: δ_{H} (270 MHz, DMSO-d₆+D₂O) 9.35 (1H, s, H-8), 5.16 (1H, d, J 6.24 H-1'), 4.68 (1H, t, J 5.3 Hz, H-2'), 4.15 (1H, t, J 5.1 Hz, H-3'), 3.88-3.93 (1H, m, H-4'), 3.44-3.62 (2H, m, H-5'), 3.12 (6H, s, NCH₃);
Massenspektrum (FAB, NBA): Gefunden MH⁺, 297.1330 C₁₁H₁₇N₆O₄, errechnet MH, 297.1311.

### Methode B

Zur Lösung des geschützten C-Nukleosids (I-f) (50 mg, 0,08 mmol) in trockenem Methanol (4 ml) wurde Natriummethanolat (14 mg, 0,26 mmol) zugegeben. Nach Rühren bei Raumtemperatur für 2 h wurde mit 0,1 ml Wasser die Reaktion beendet, das Lösungsmittel im Vakuum abgezogen und der Rückstand über Kieselgel chromatographiert, Laufmittel 10 bis 15 % Methanol in DCM (Gradientenelution). Man erhielt die Verbindung (I-g) (21,9 mg, 90 %) als gelbliche Kristalle, deren Analyse die chemische Identität mit dem Produkt aus Methode A bestätigte.

### Beispiel 19

Verbindung (Ib) (siehe Beispiel 3 oben) (8 mg, 0,03 mmol) wurde in 6M wässriger salzsaurer Lösung (2 ml) bei Raumtemperatur gelöst. Nach 10 min wurde die Lösung eingeengt und das Wasseradditionsprodukt (I'b) erhalten;
NMR-Spektrum: δ_{H} (300 MHz, D₂O) 8.22 (1H, s, H-5), 6.62 (1H, s, H-7'), 5.05 (1H, d, J = 7 Hz, H-1'), 4.71 (1H, m, H-2'), 4.25 (1H, m, H-3'), 4.18 (1H, m, H-4'), 3.48 (2H, m, H-5').

### Beispiel 20

Verbindung (Ig) (siehe Beispiel 18 oben) wurde in Deuteriumoxid bei Raumtemperatur gelöst, wobei eine Mischung aus Edukt (Ig) und dem entsprechenden Wasseradditionsprodukt (I'g) erhalten wurde.
¹H-NMR (D₂O, 300 MHz) für das Wasseradditionsprodukt (I'g):
δ [ppm] = 6,33 (d, 1H, J = 2,8 Hz, H-8), 5,23 (dd, 1H, J = 6,2 und 2,8 Hz, H-1'); 4,71 (t, 1H, J = 6,0 Hz, H-2'), 4,34 (dt, 1H, J = 5,8 und 9,4 Hz, H-3'), 4,17 (m, 1H, H-4'), 3,68-3,92 (m, 2H, H-5'), 3,04 (s, 3H, NCH₃).

In den folgenden Tabellen sind weitere Beispiele der Formel (I) aufgeführt, welche entsprechend den obengenannten Herstellungsbeispielen oder analog zu diesen Beispielen und den in der Beschreibung genannten Verfahren erhalten werden.

Erläuterungen zu den nachstehenden Tabellen und Tabellenbeispielen:

In der jeweiligen Tabelle gehören die Definitionen der Verbindung mit einer Nummer gemäß Schema **N-1** ("N Bindestrich eins") zur Formel (1), wobei mit der ganzen Zahl "N" die einzelnen Verbindungen laufend nummeriert sind, d. h. die ersten vier Verbindungen der Formel (1) haben die Nummern 1-1, 2-1, 3-1, 4-1. Entsprechend sind Verbindungen der Formel (2) nach dem Schema **N-2** und Verbindungen der Formel (3) nach dem Schema **N-3** numeriert.

Mit den stereochemischen Beziehungen α und β sind die Stellungen der Bindungen an den cyclischen Resten relativ zueinander ablesbar, d. h. eine Bezeichnung 1-β, 4-β oder 1,4-β an einem Dihydrofuranrest der Formel 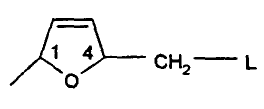 bedeutet, daß die Bindungen an Position 1 und 4 auf derselben Seite stehen, bezogen auf die Ringebene (cis-Orientierung). Hinsichtlich der absoluten Konfiguration sagt diese Bezeichnung nichts aus; die Formel umfaßt deshalb beide enantiomorphe Formen des Restes.
Zur Bezeichnung einer enantiomorphen Form des Restes wird die in der Zuckerchemie üblich D,L-Nomenklatur verwendet. Im Falle eines Fünfringes, der vom Furanosylrest abgeleitet ist, bezieht sich die Bezeichnung "D" auf die absolute Konfiguration am asymmetrischen C-Atom in Ringposition 4 des Restes. Ein Rest der Formel 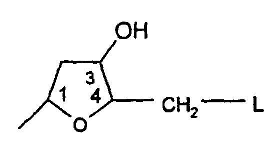 mit der Zusatzbezeichnung D-1,4-β-3-α (oder ausführlicher 4D-1,4-β-3-α) ist gleichbedeutend mit der Stereoformel 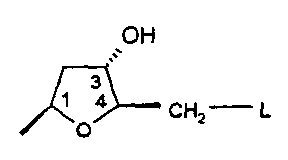 Unter Anwendung der R,S-Nomenklatur entspricht dies der Konfiguration 4R an Ringposition 4, wenn als Prioritätenfolge gilt: 1. Sauerstoffatom, 2. C-Atom an Position 3, 3. Gruppe CH₂-L, 4. Wasserstoffatom.

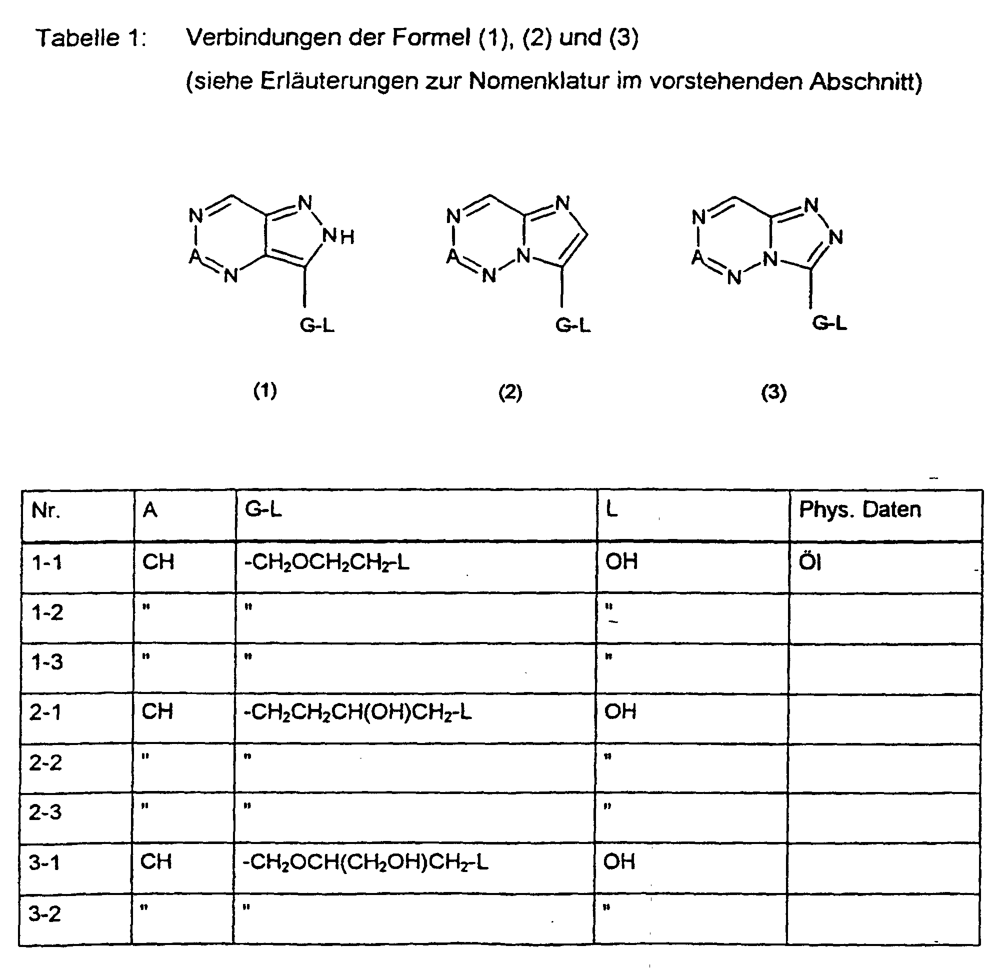

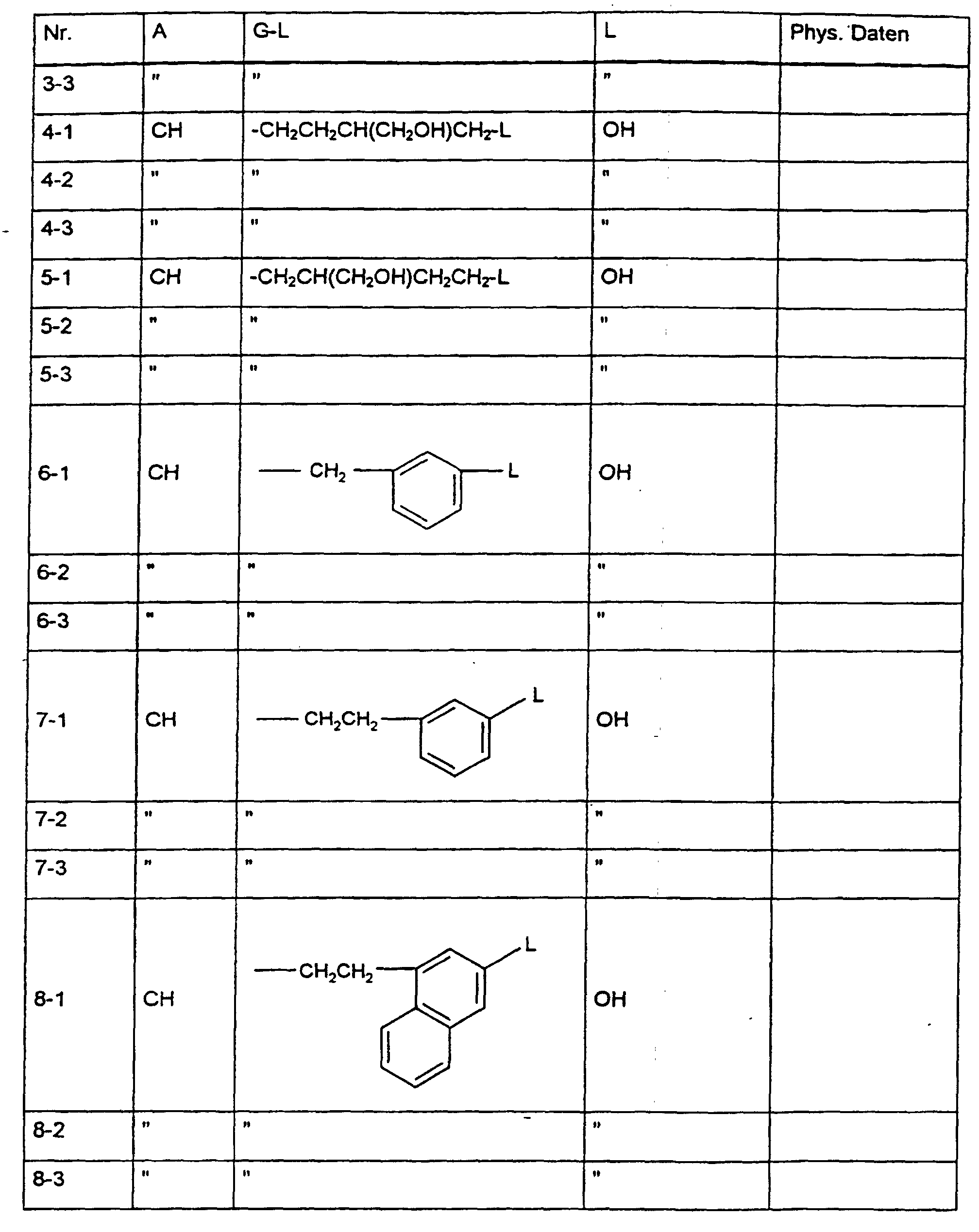

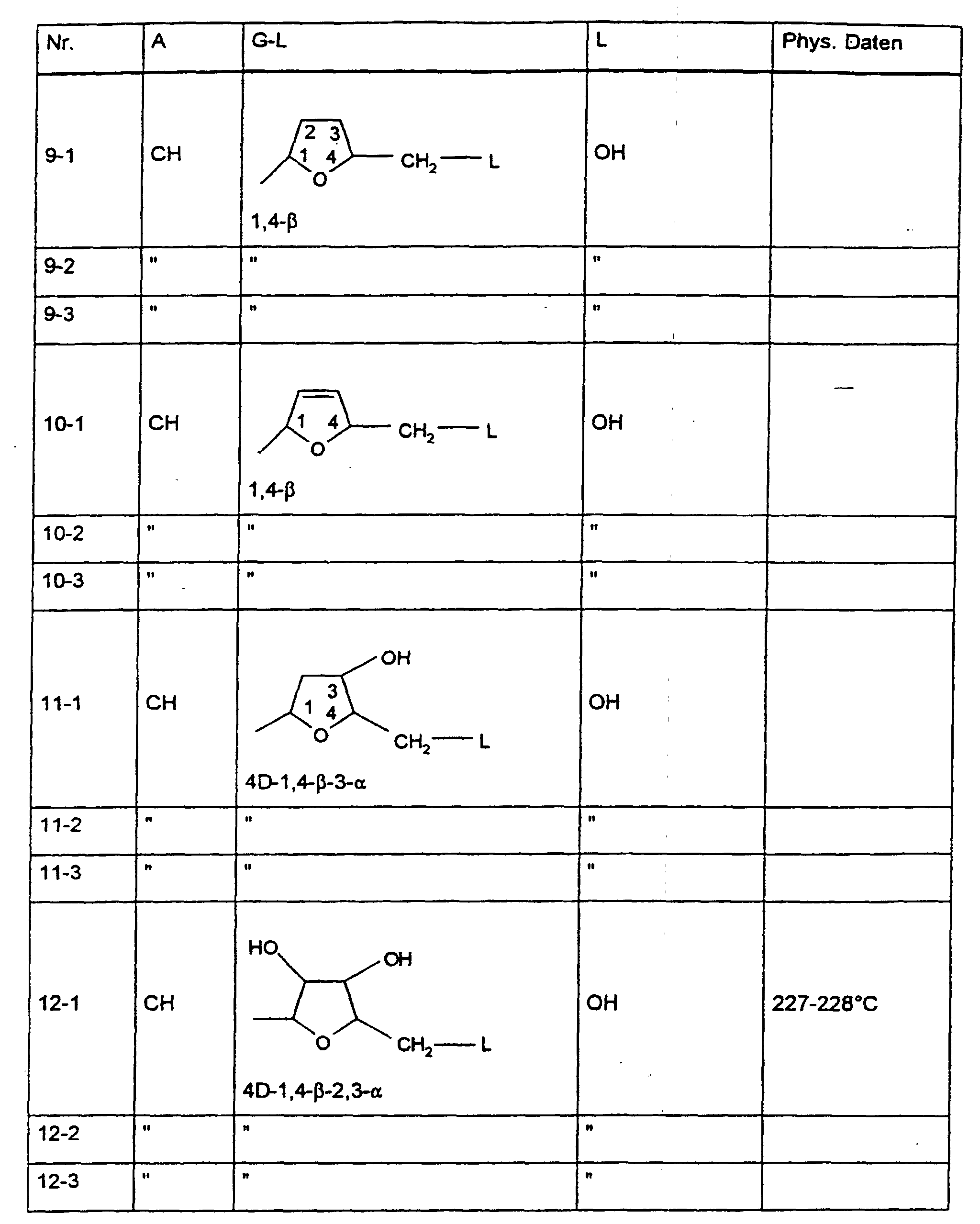

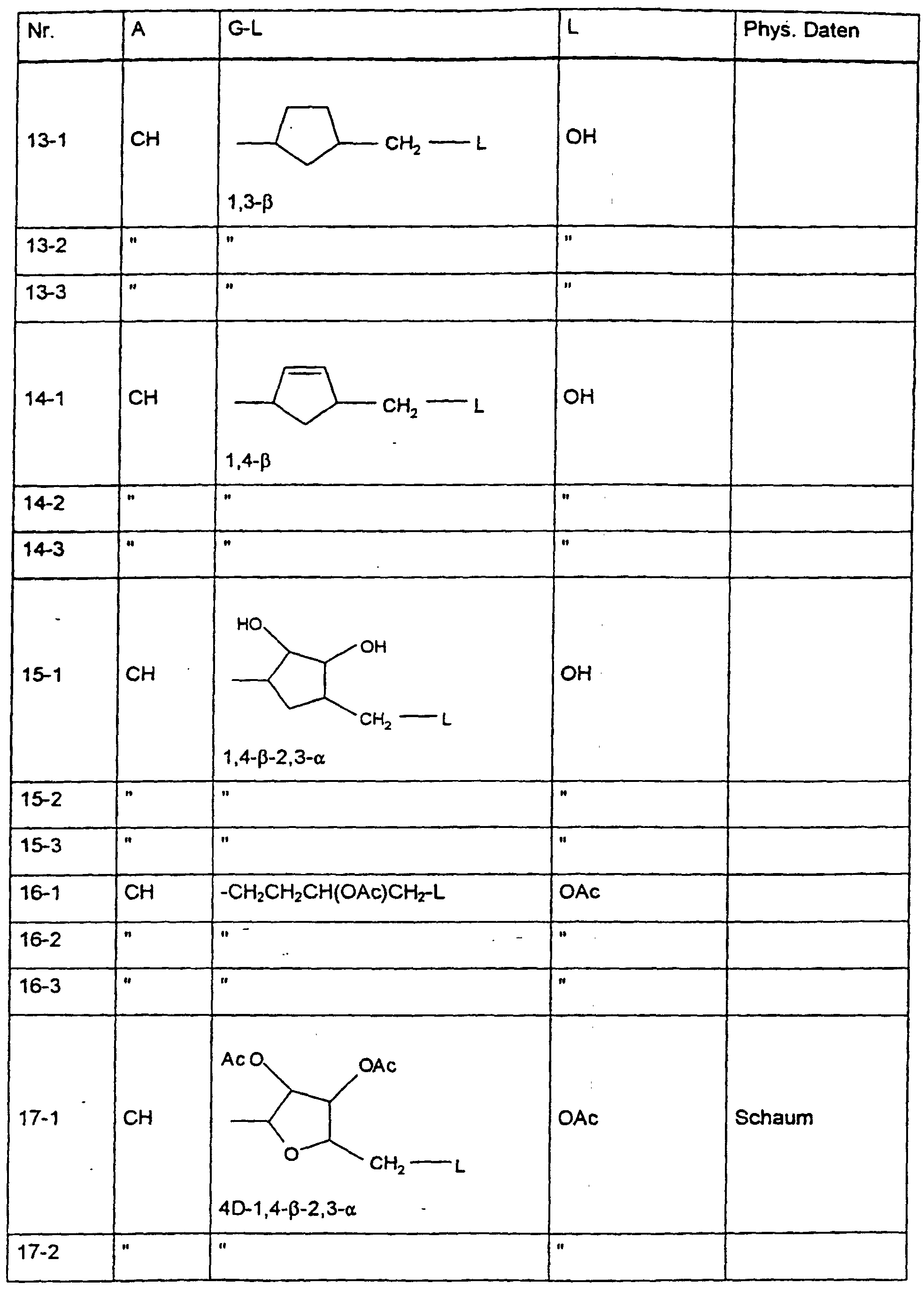

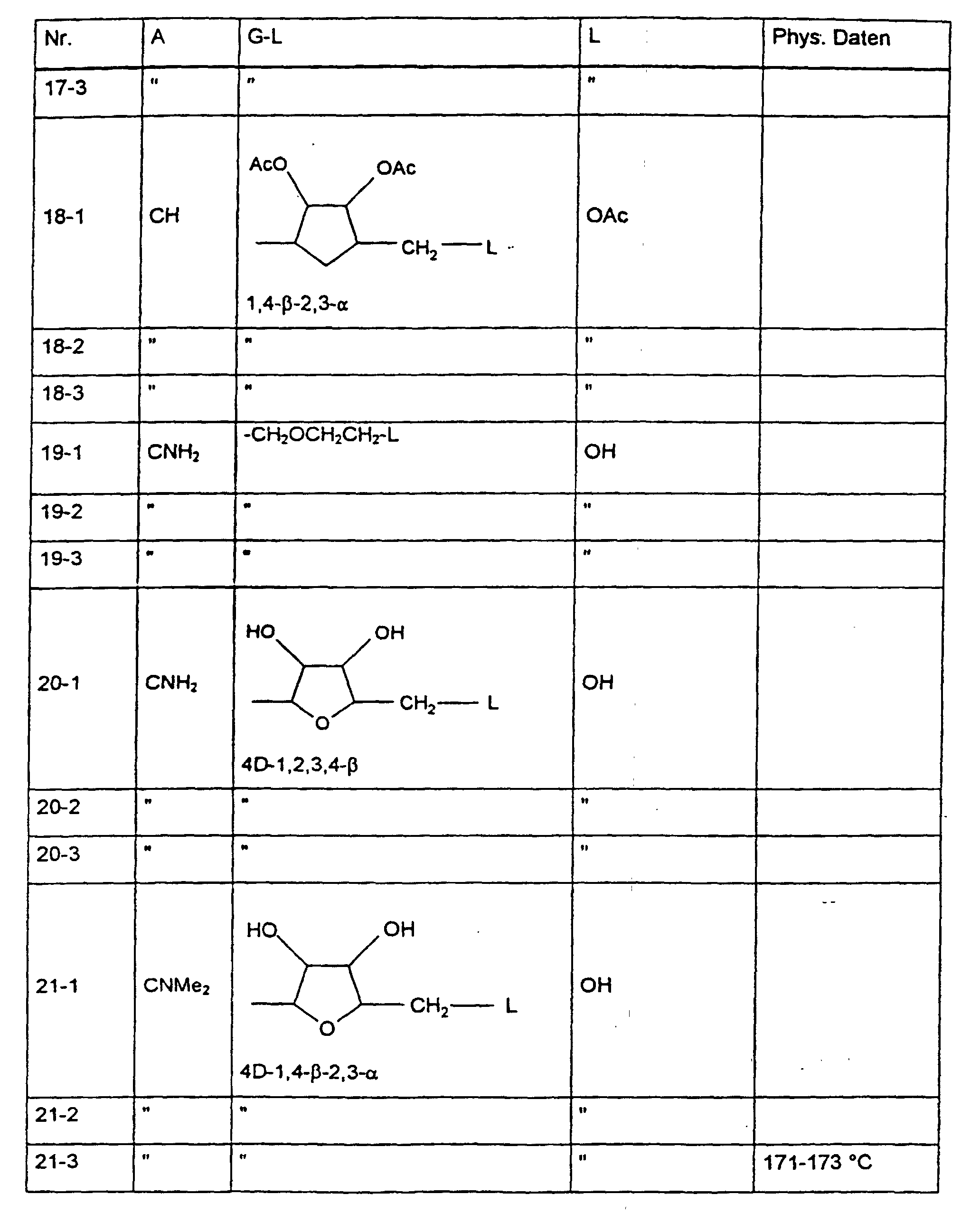

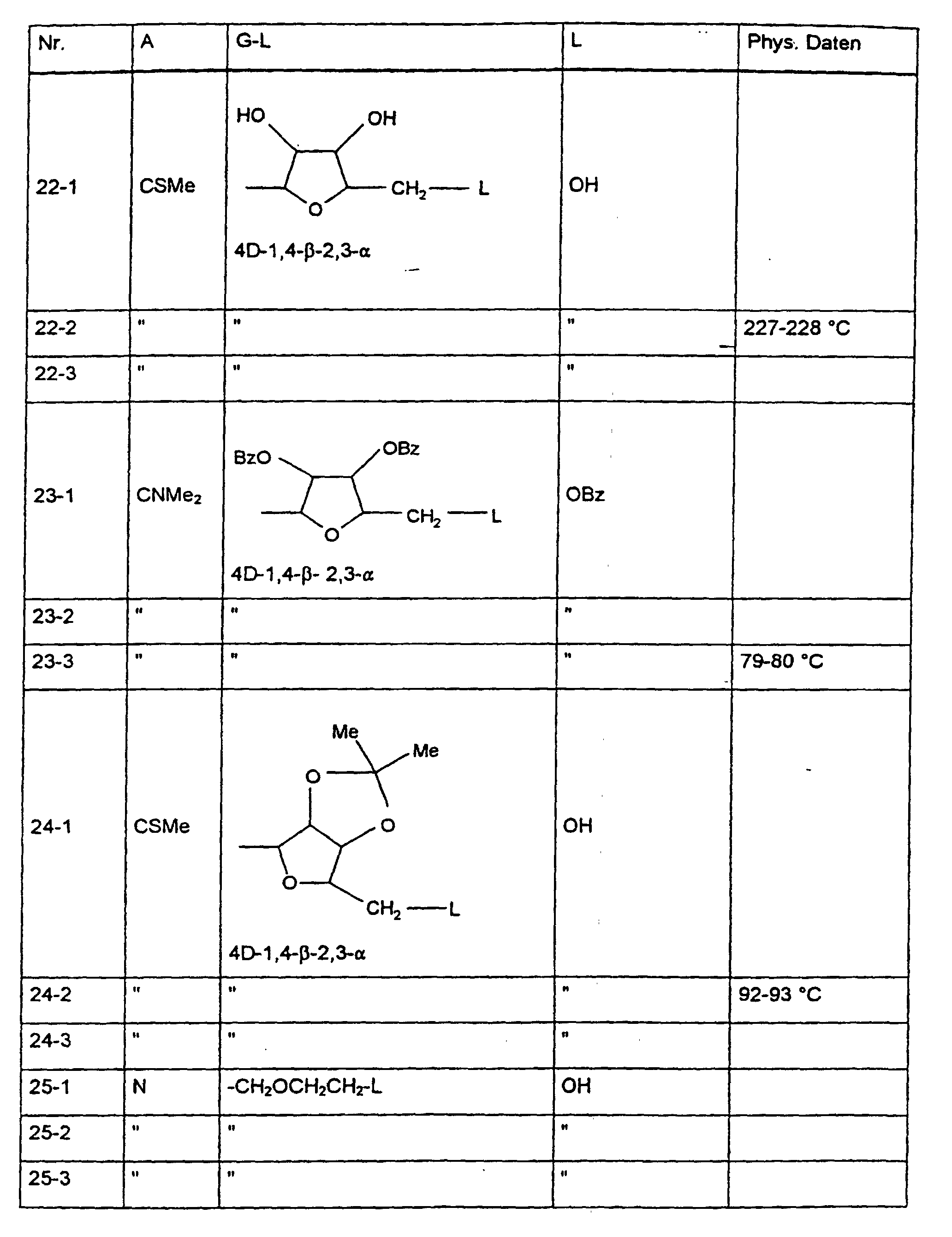

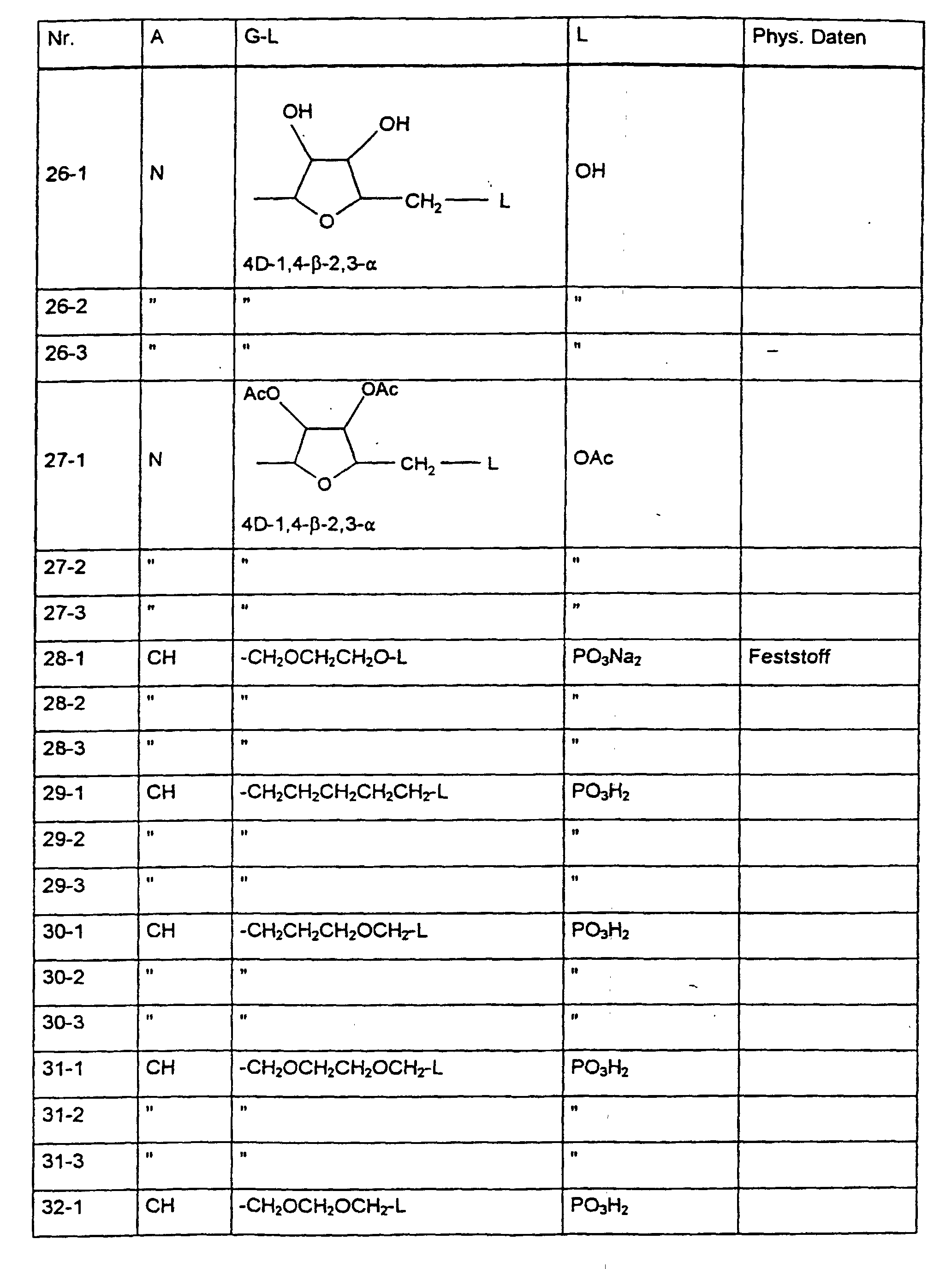

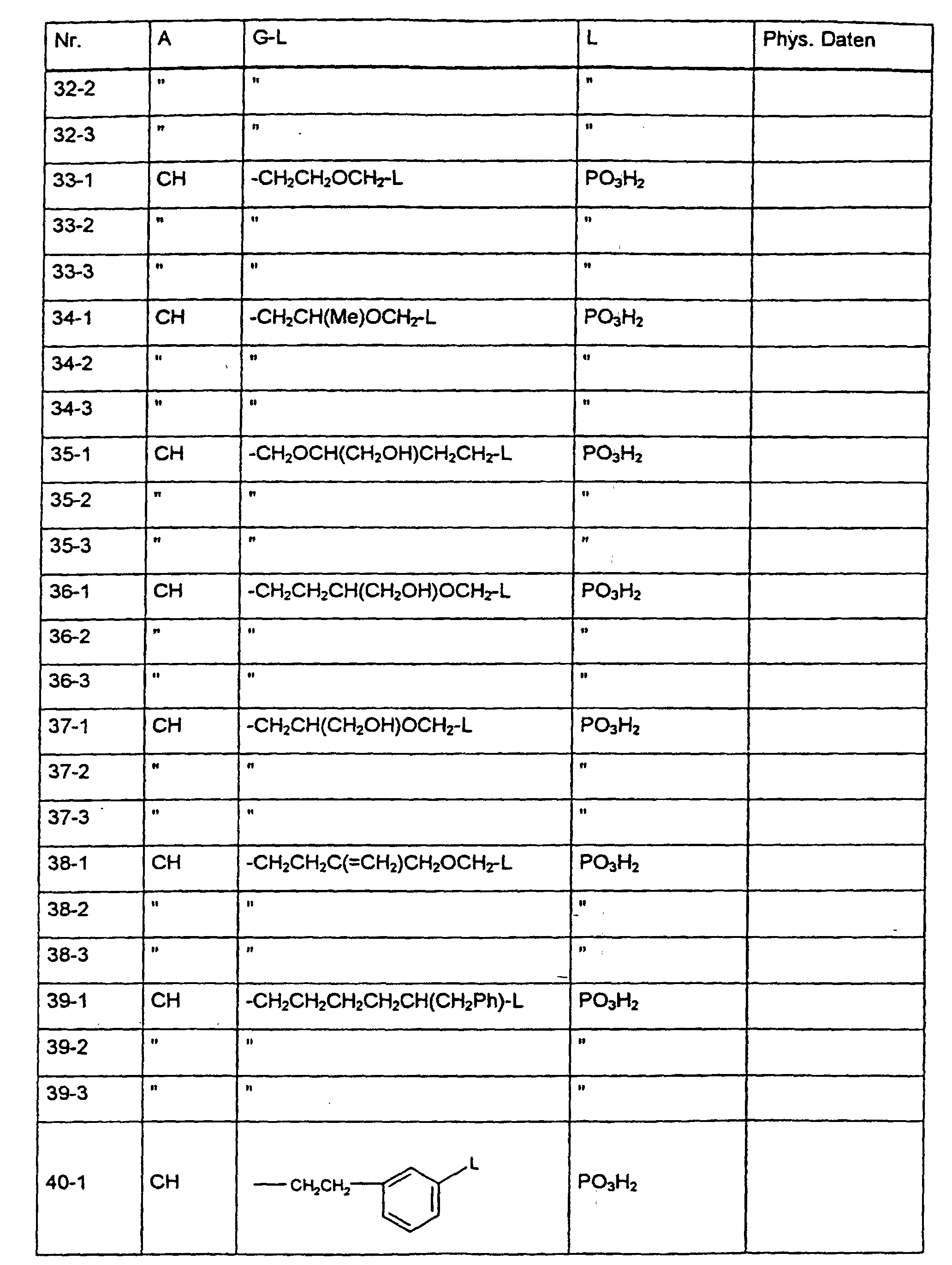

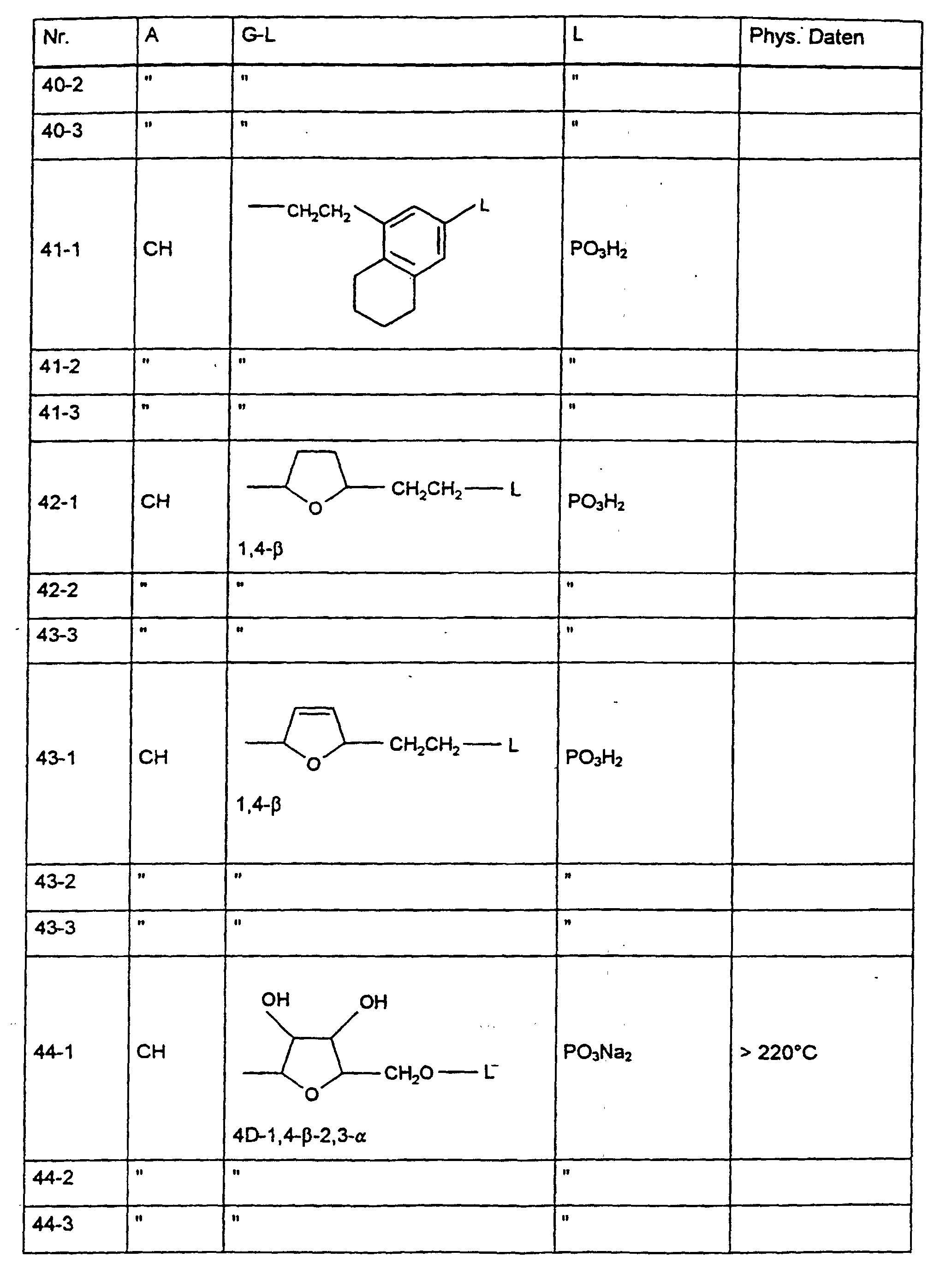

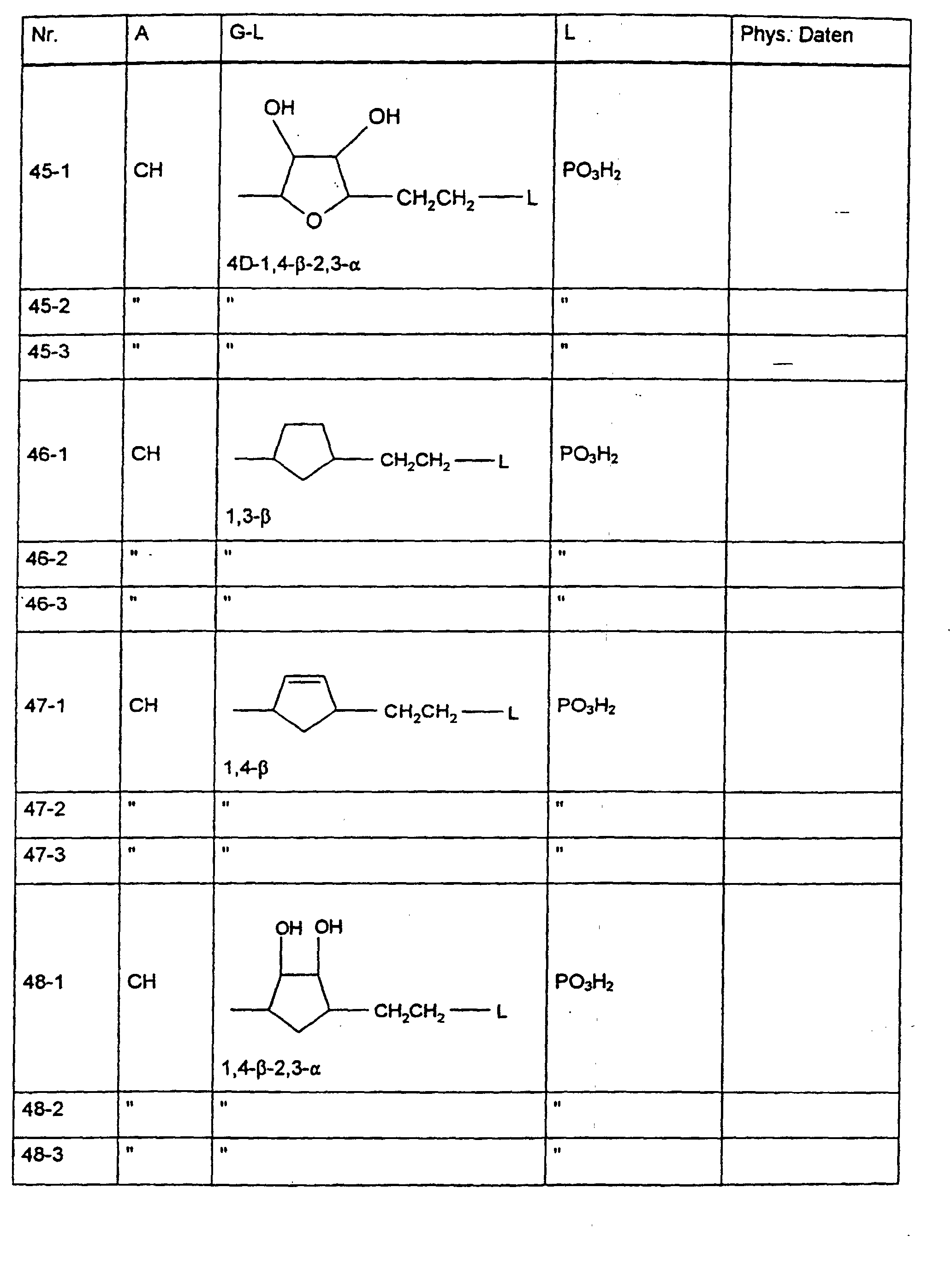

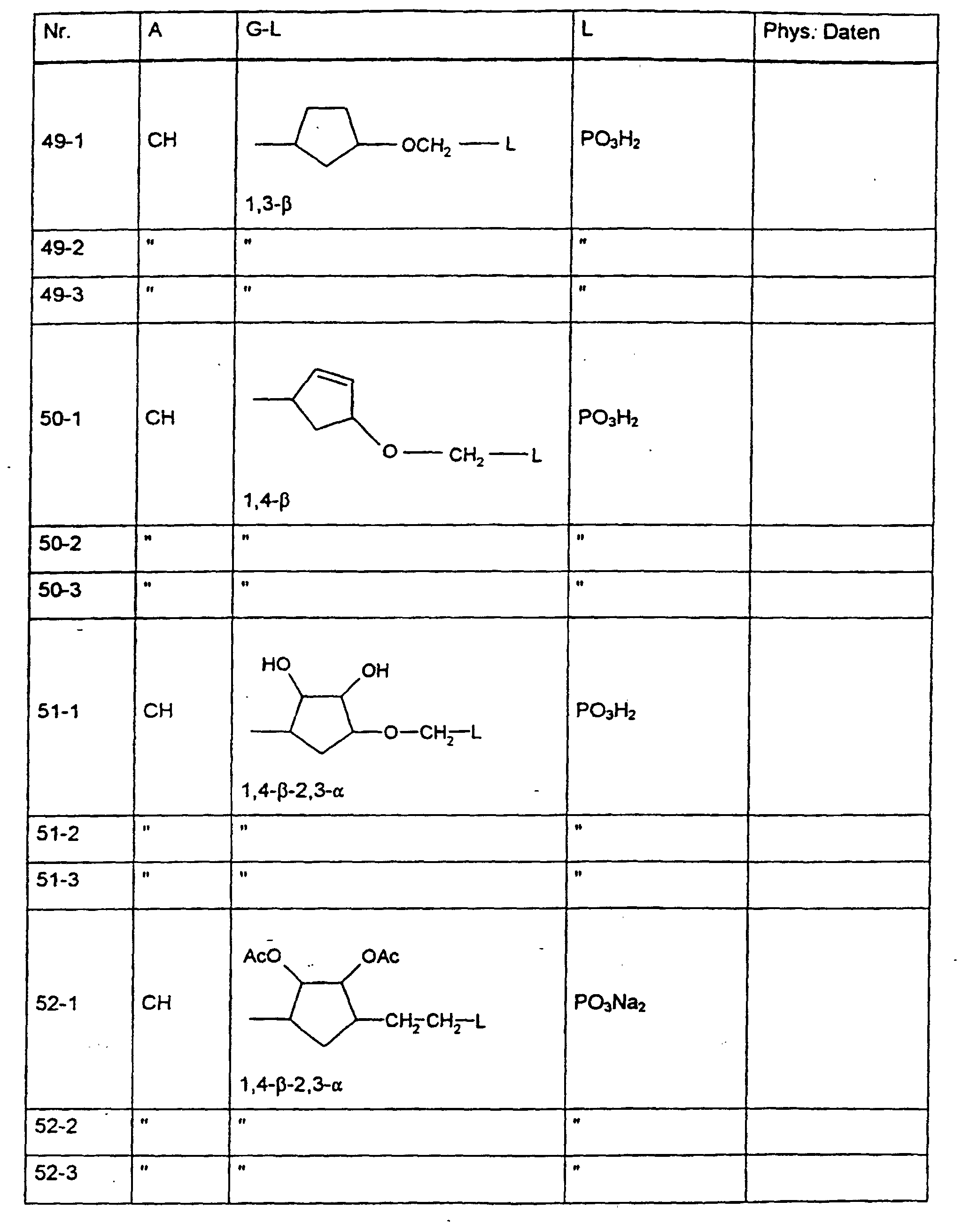

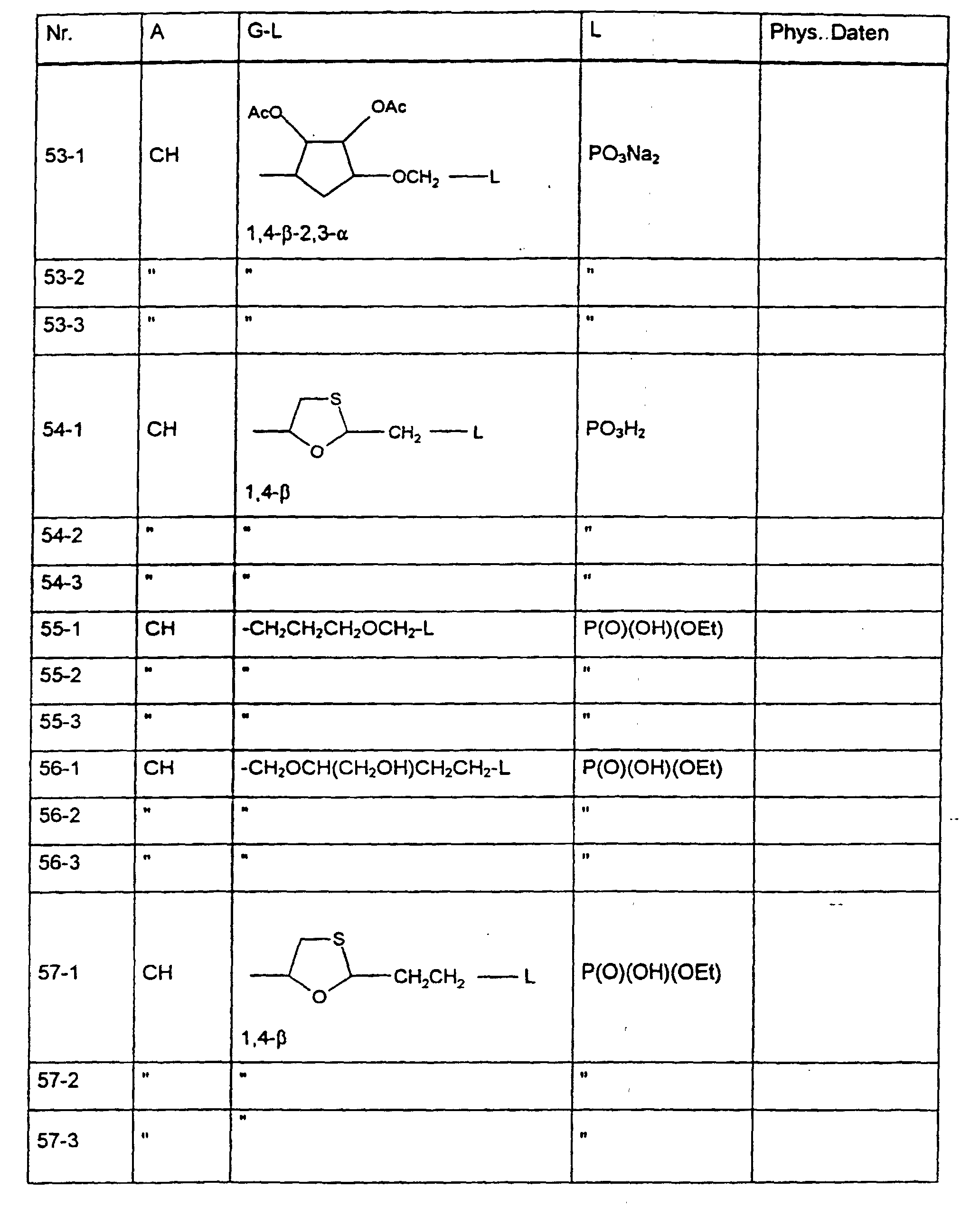

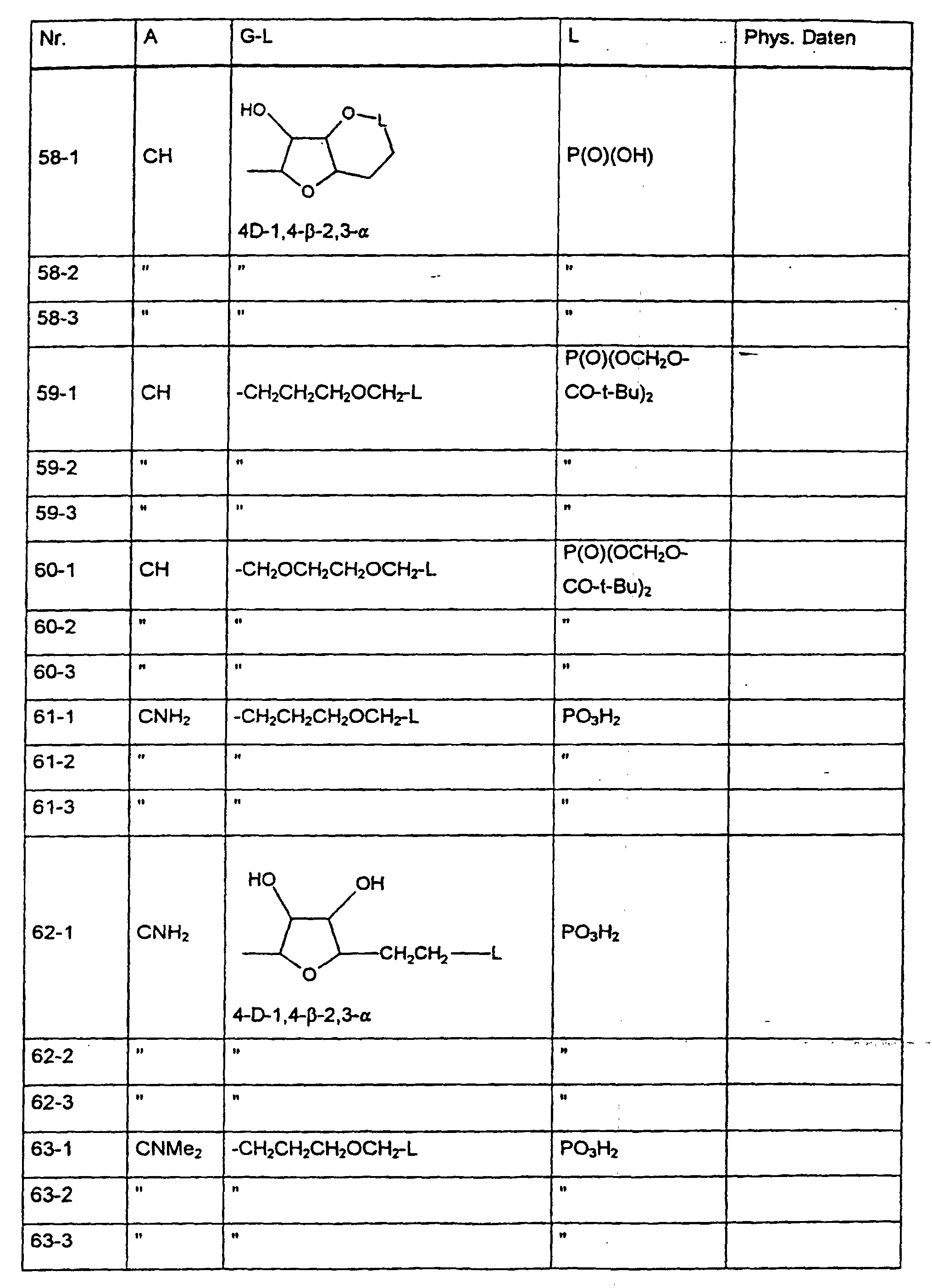

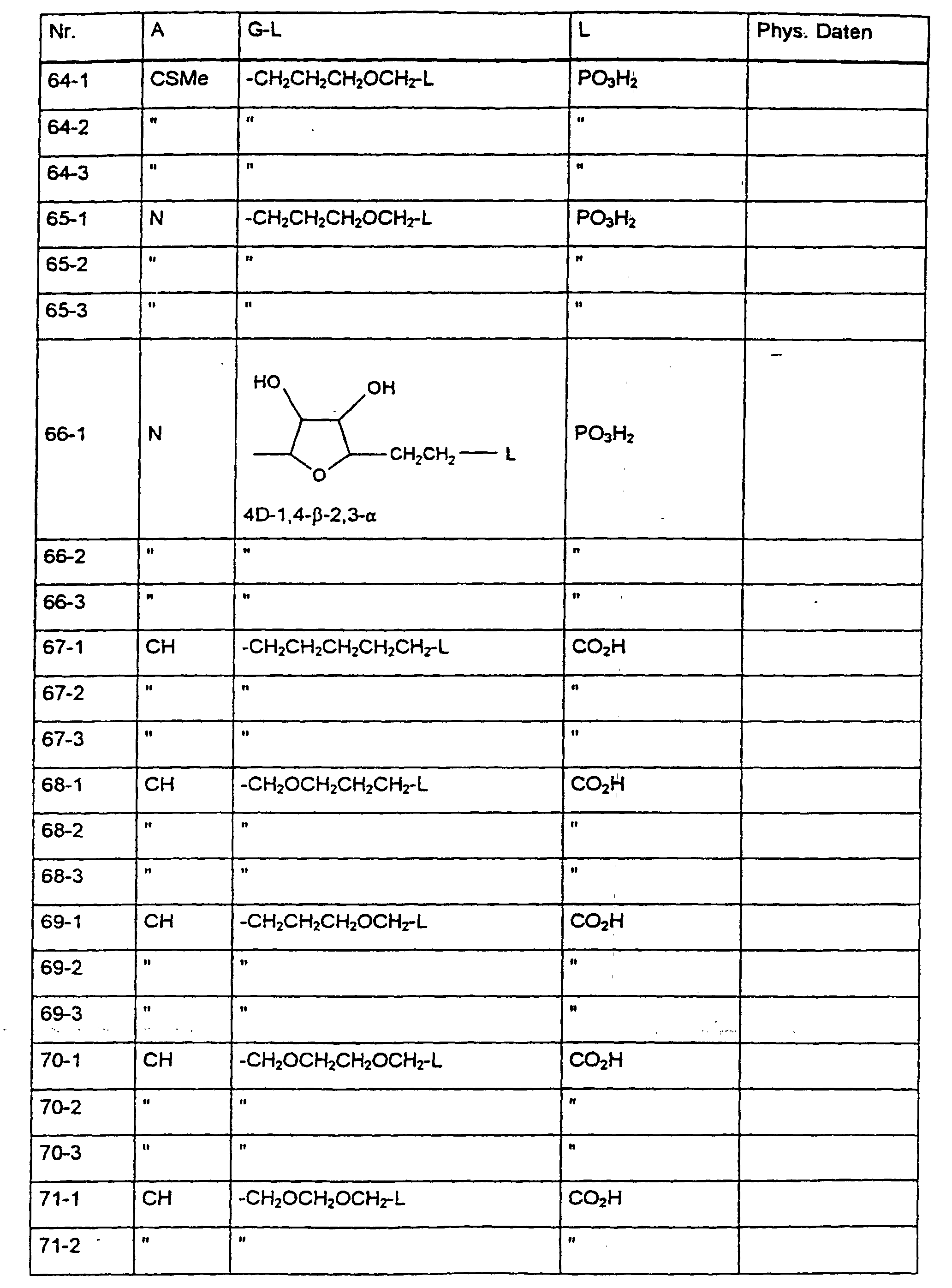

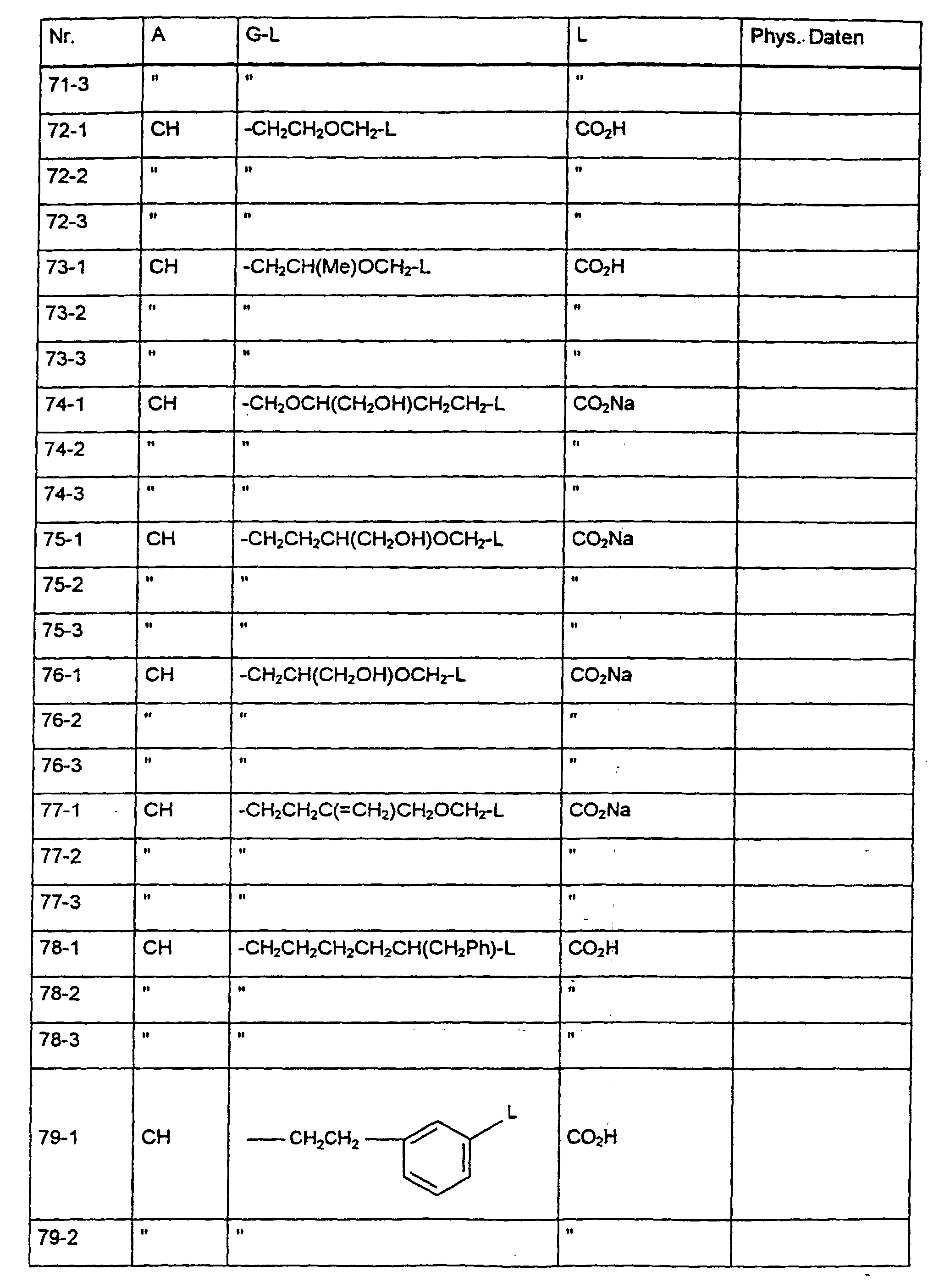

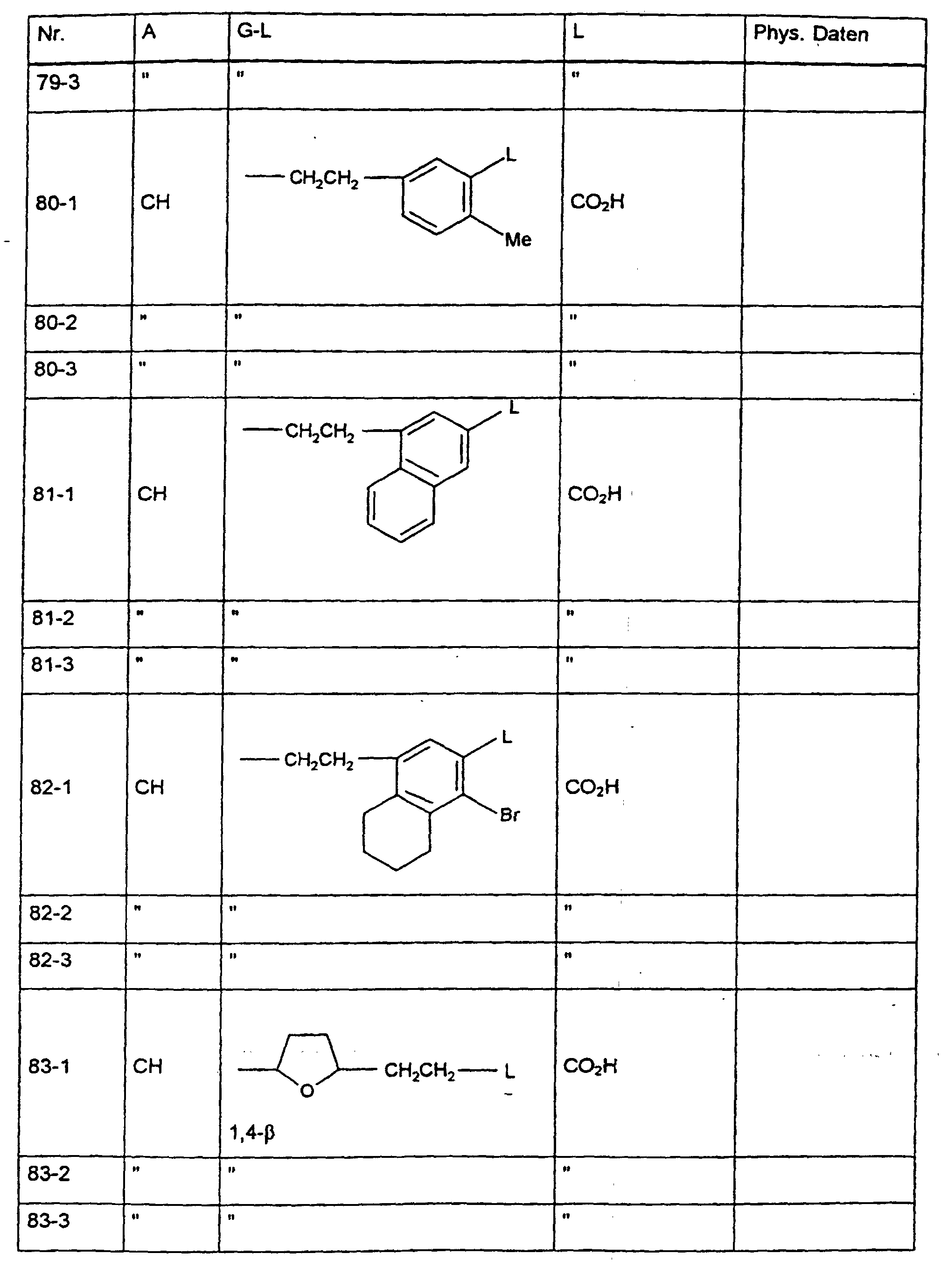

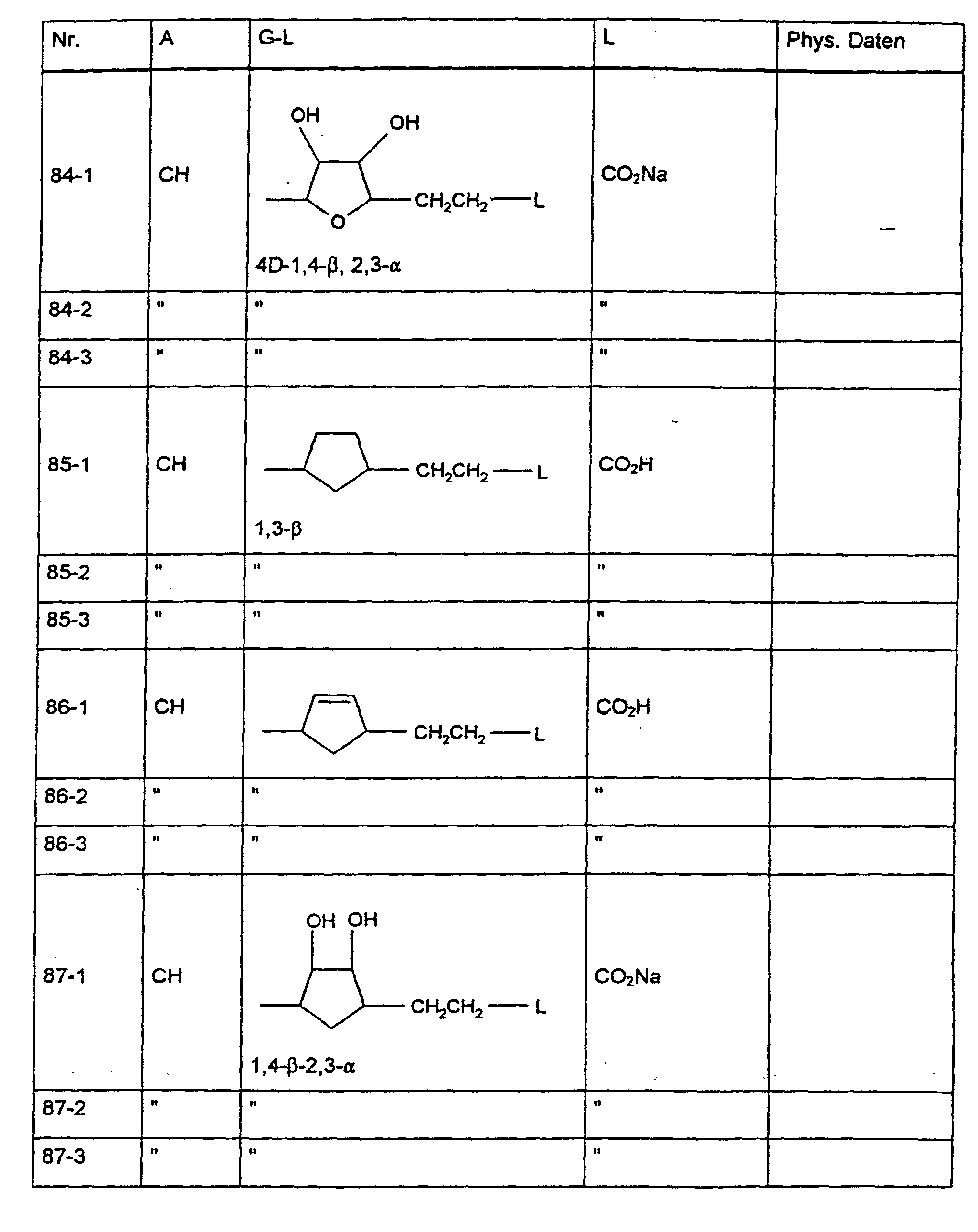

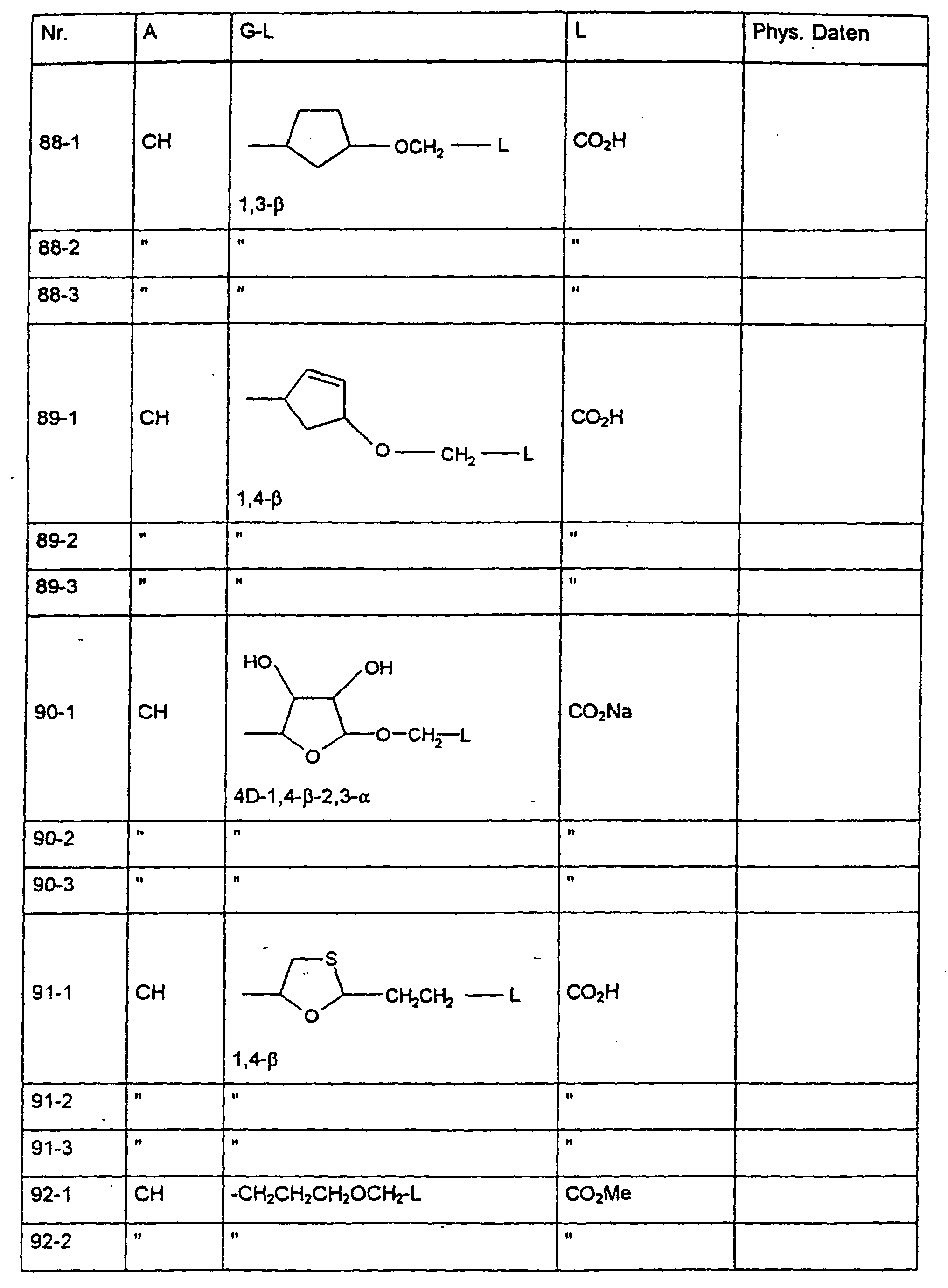

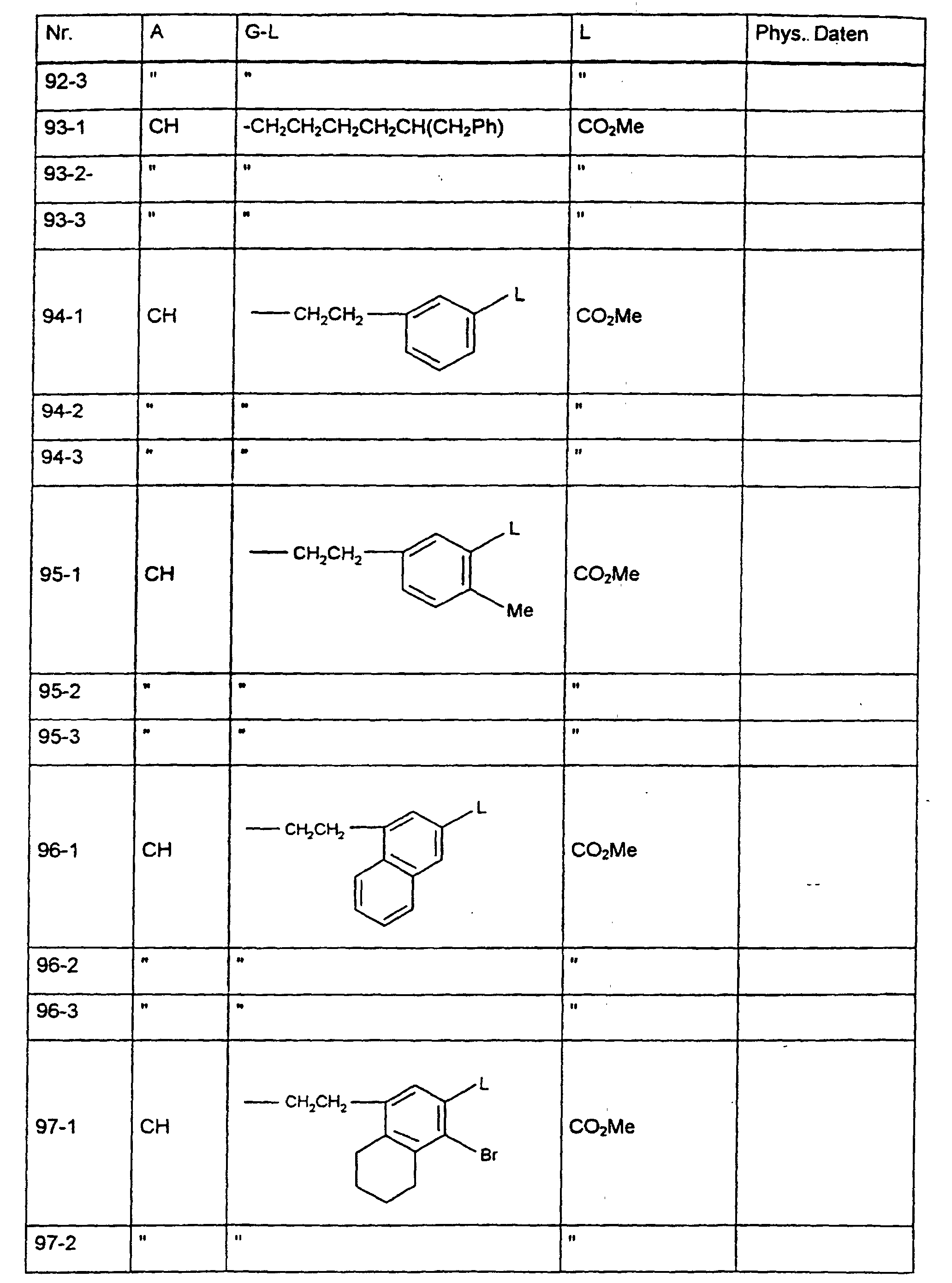

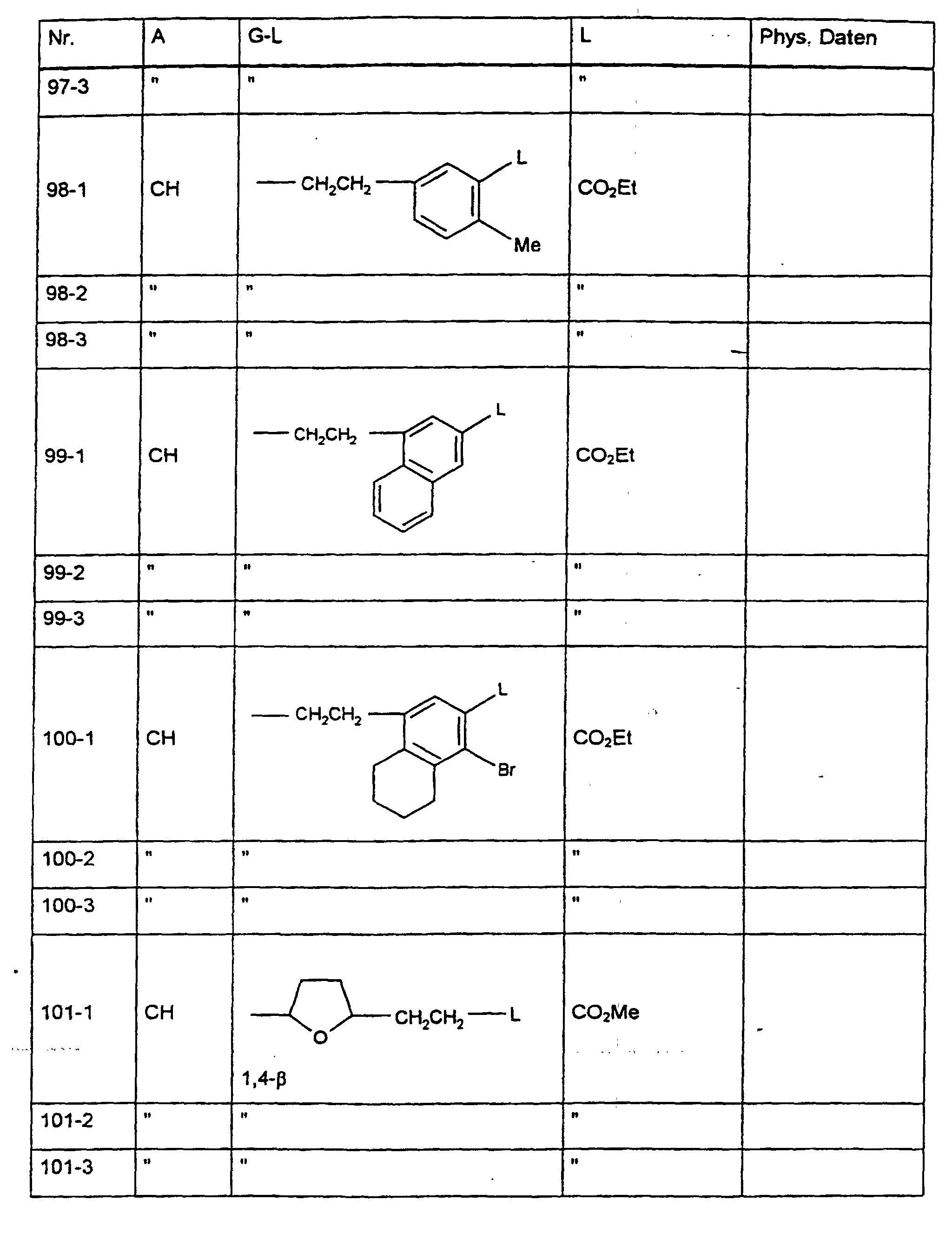

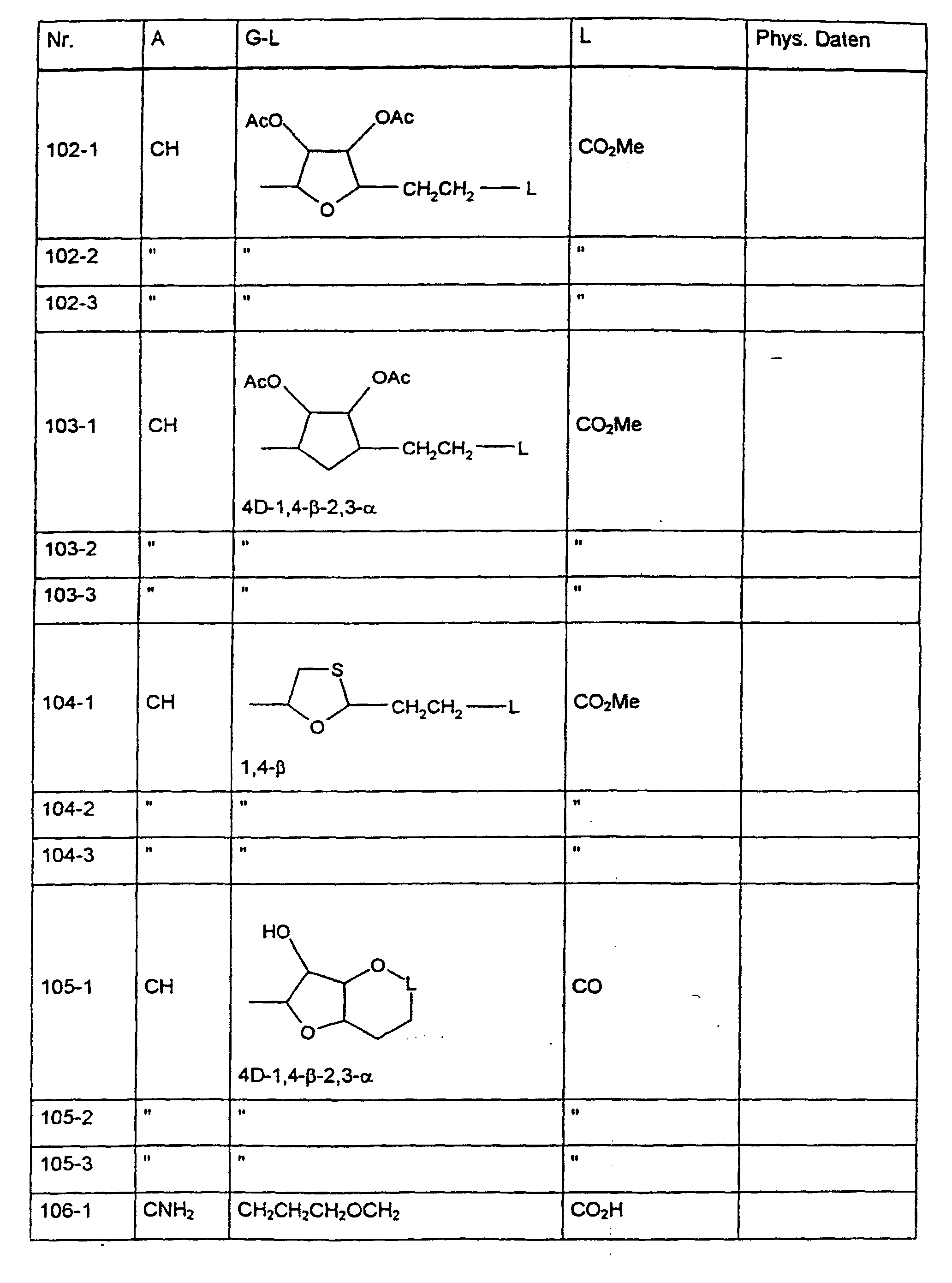

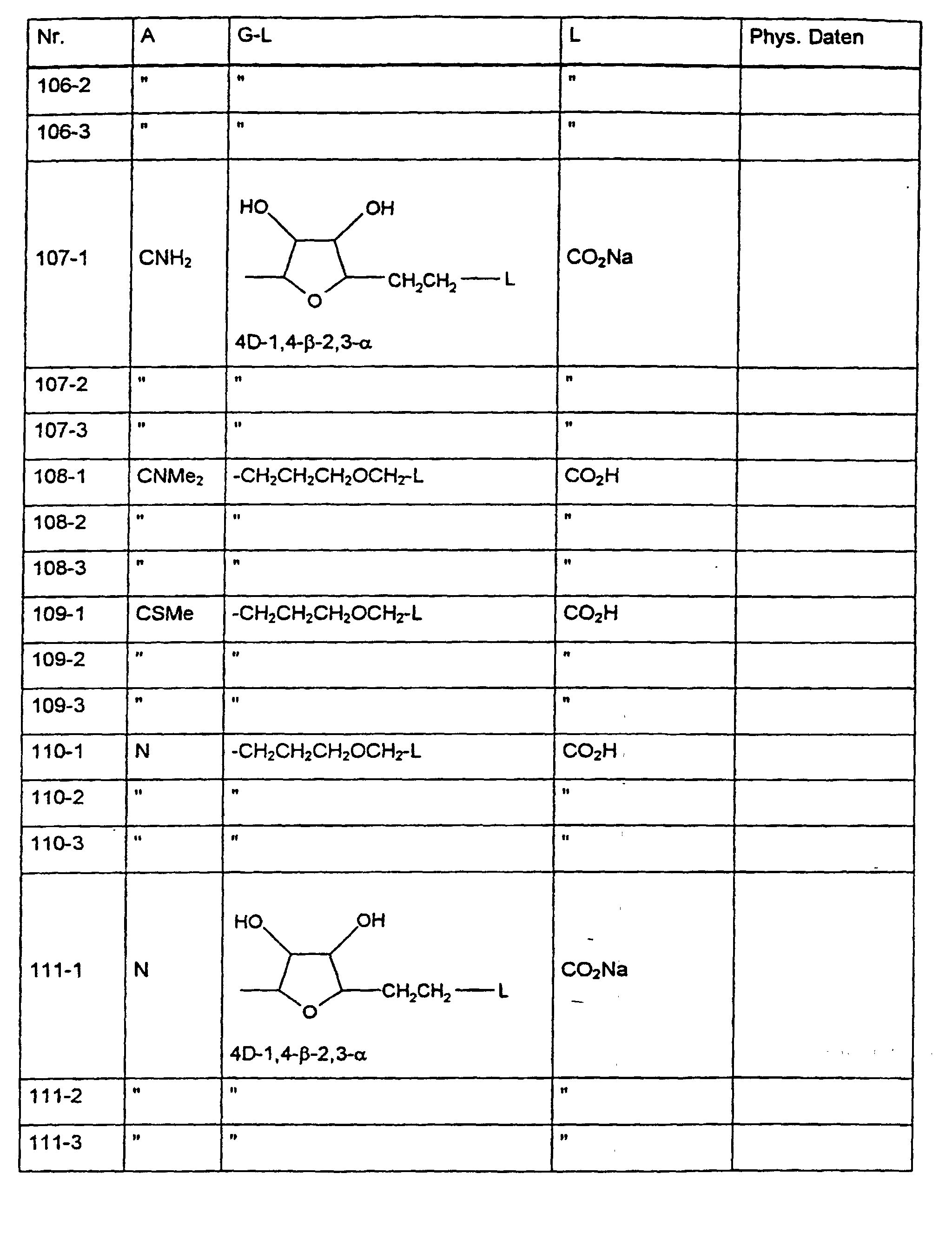

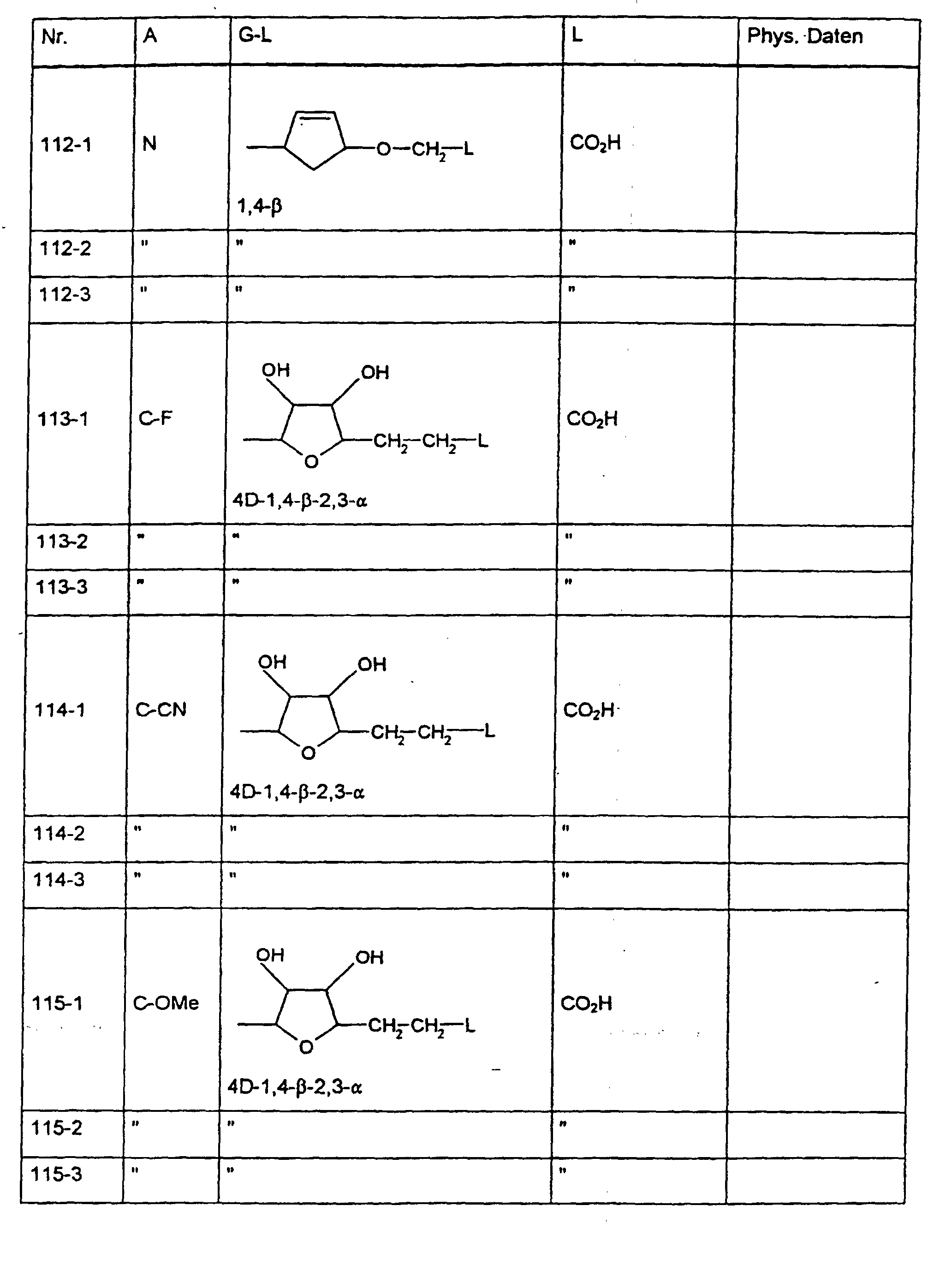

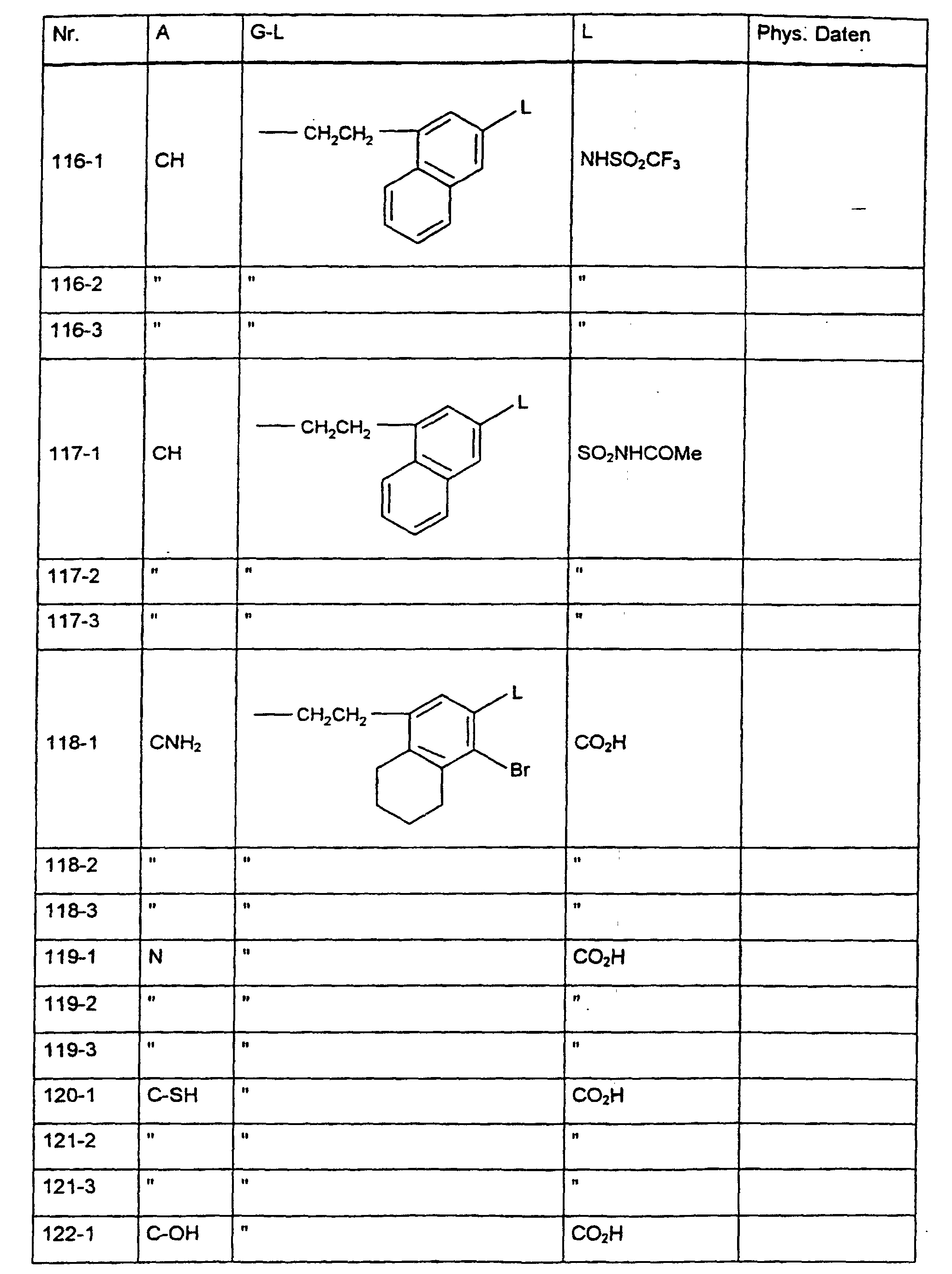

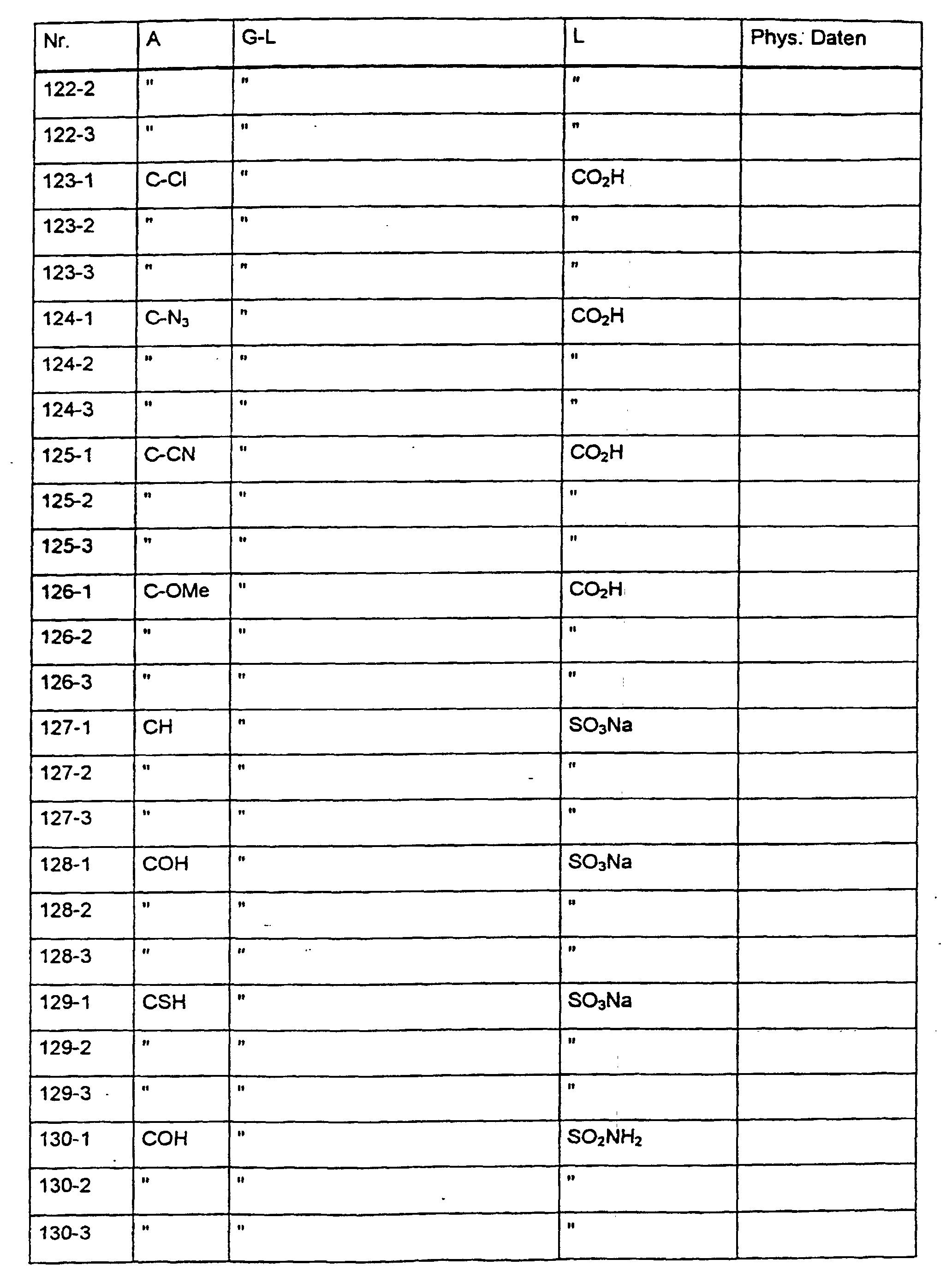

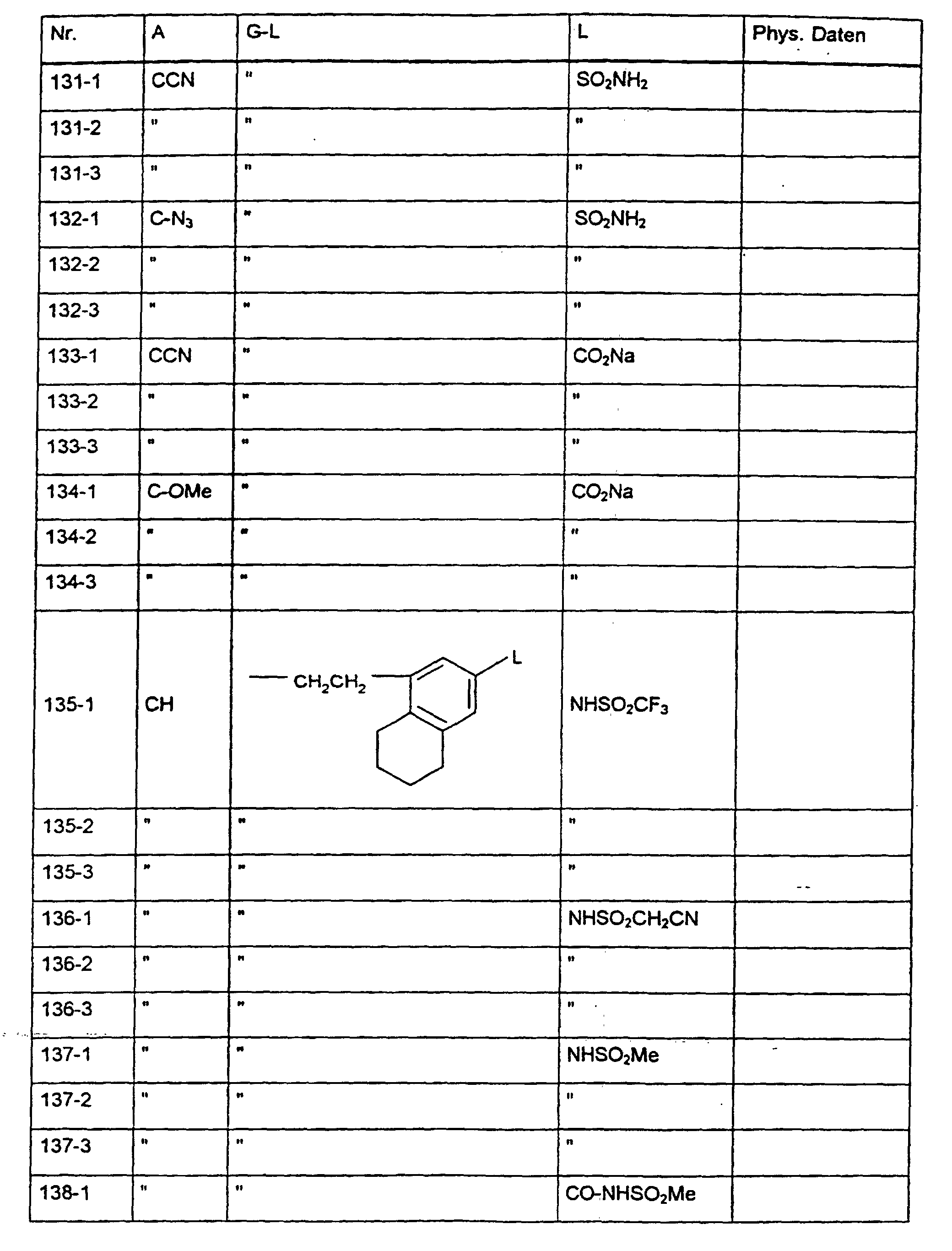

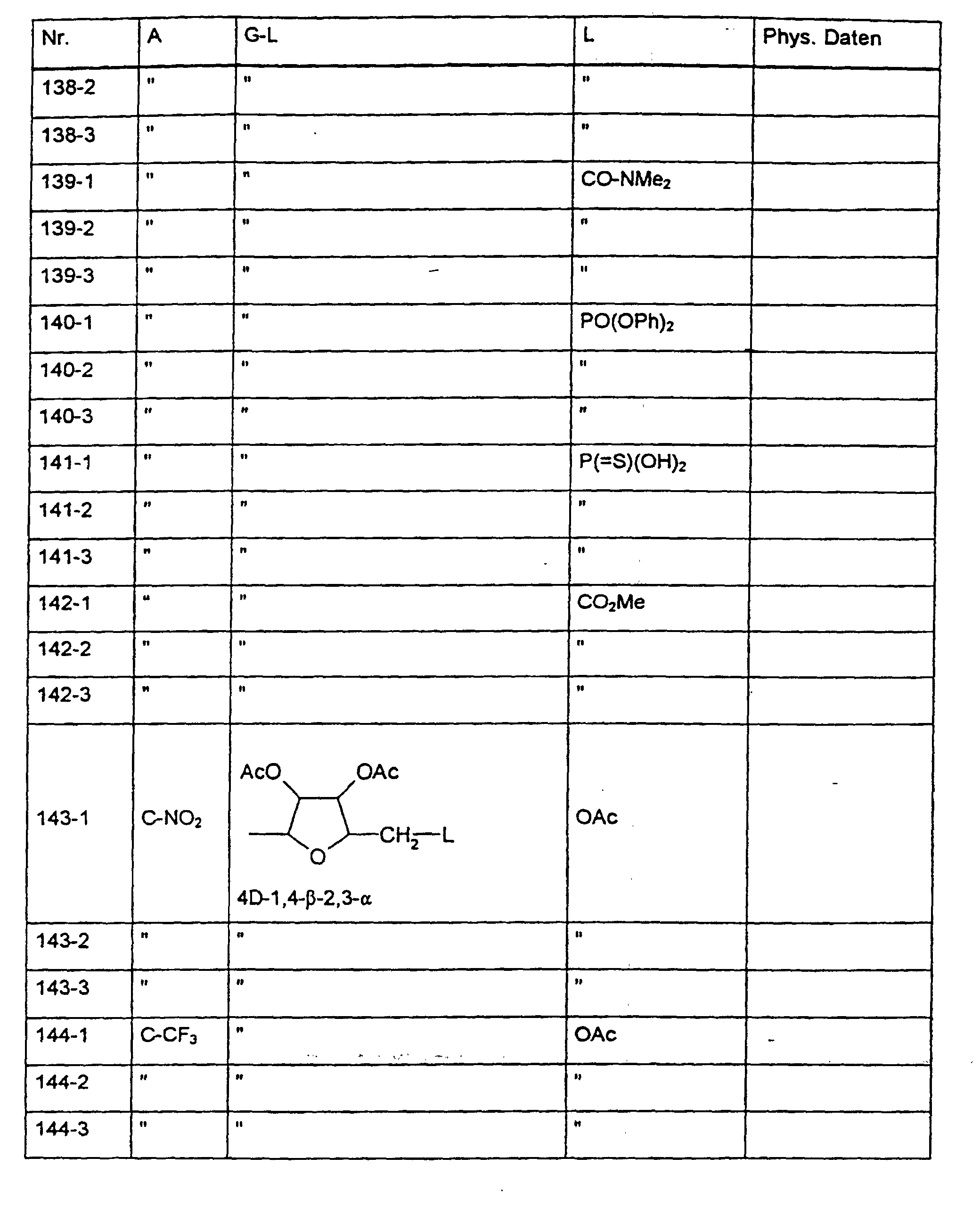

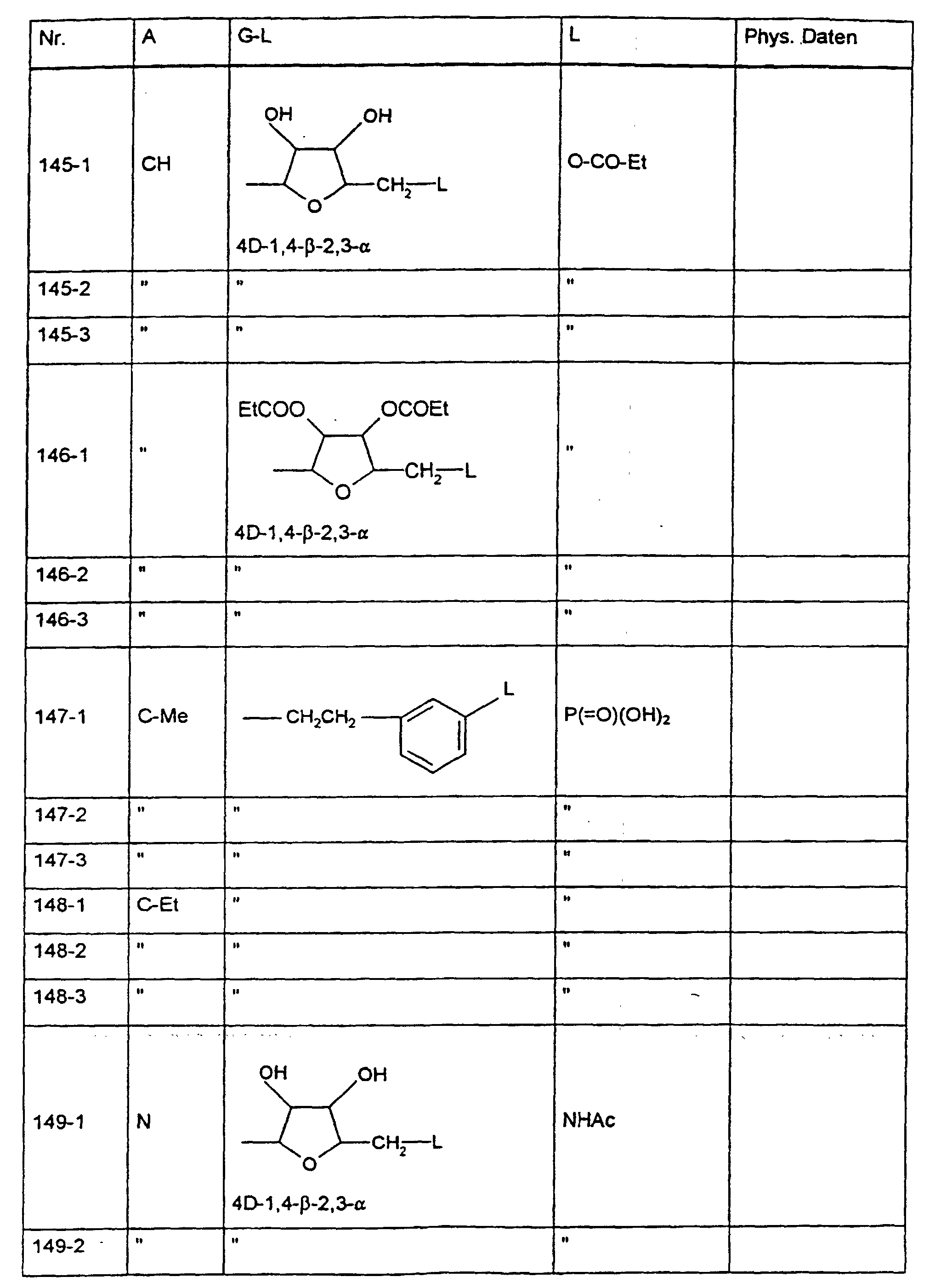

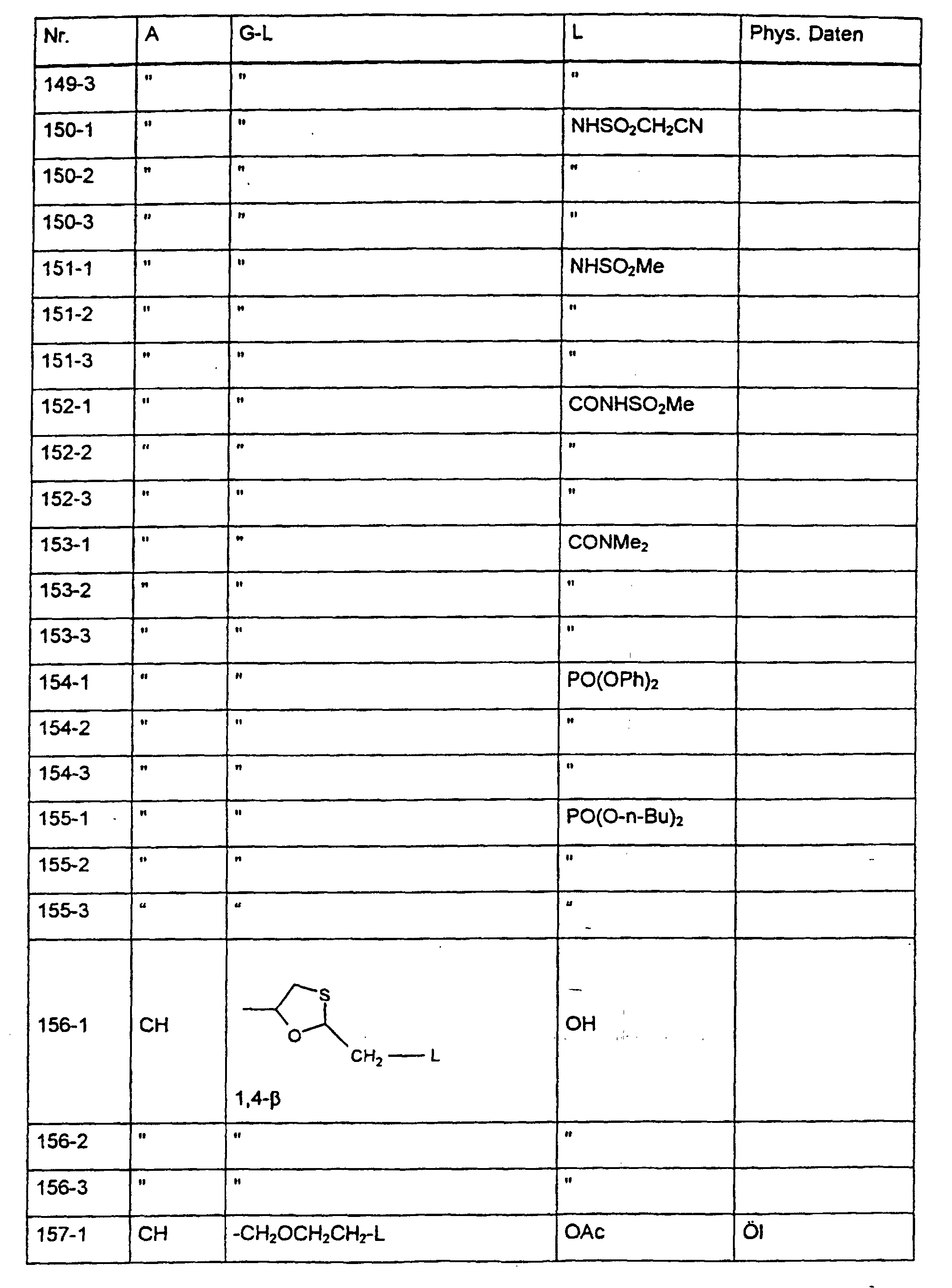

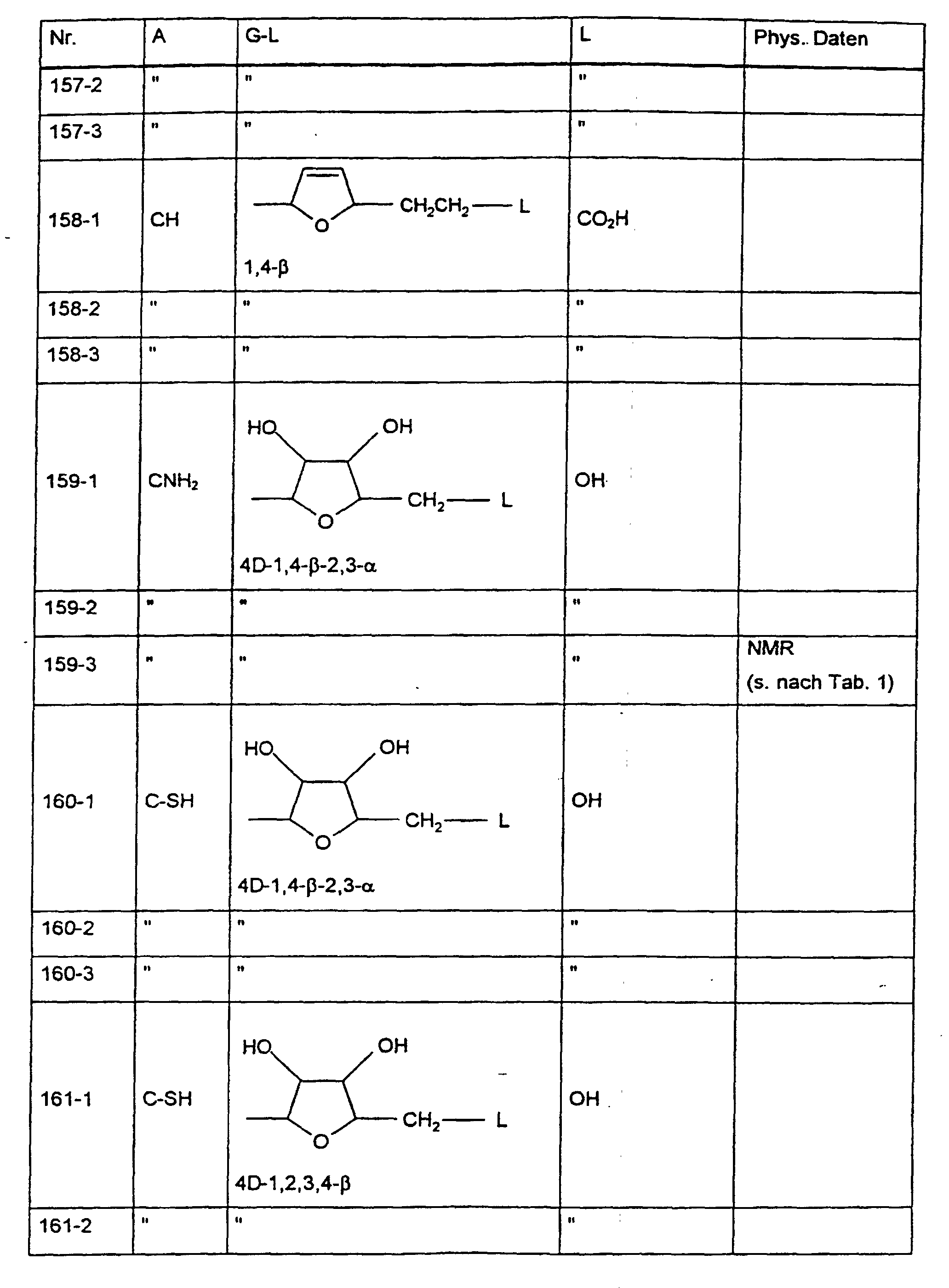

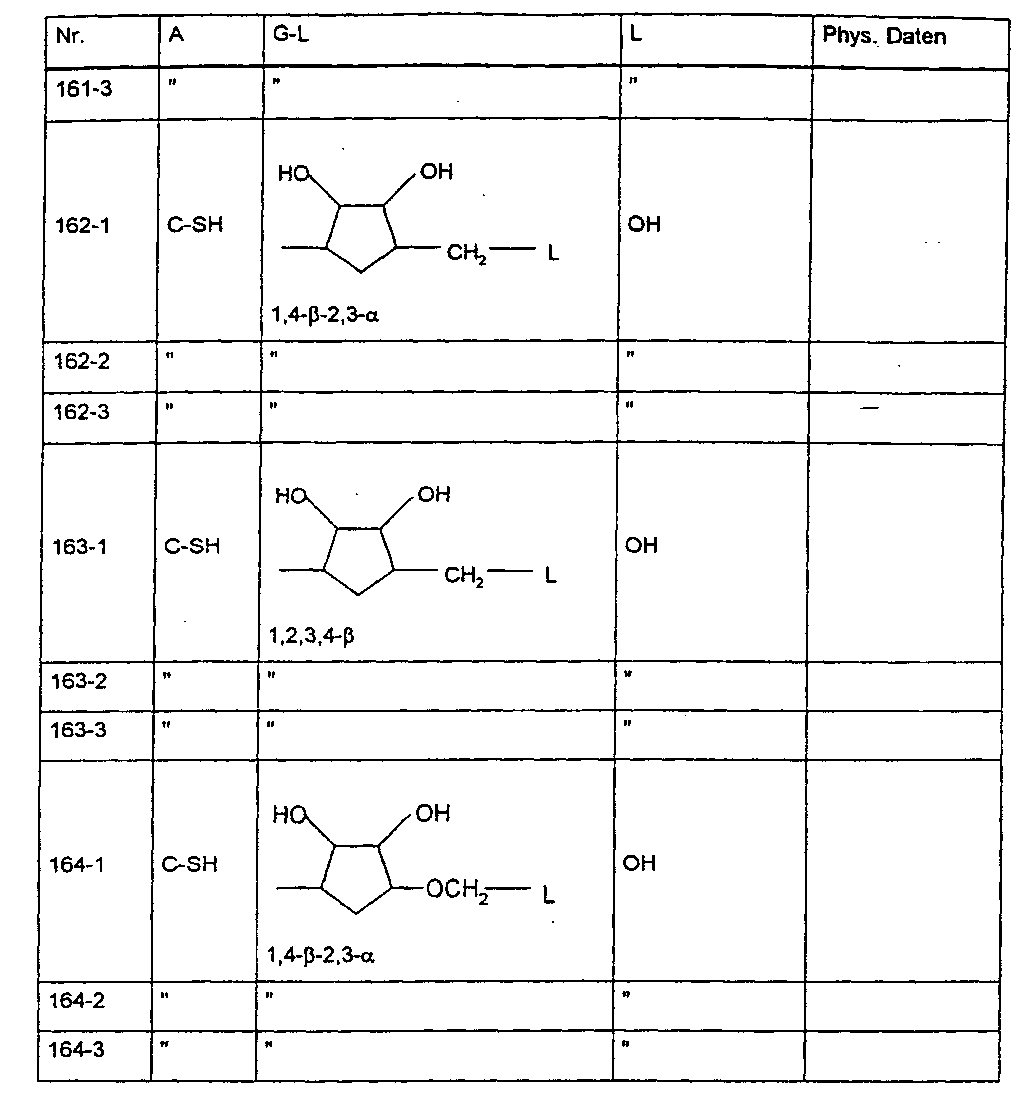

Zu Beispiel 159-3:
Die Verbindung wurde in Deuteriumoxid bei Raumtemperatur gelöst, wobei eine Lösung des Gemischs aus Verbindung 159-3 und dessen Wasseradditionsprodukt (I') im Verhältnis 5:95 erhalten wurde. ¹H-NMR (D₂O, 300 MHz): δ [ppm] = 6,35 (s, 1H, H-6), 5,15 (d, 1H, H-1'); 4,70 (dd, 1H, H-2'), 4,35 (dd, 1H, H-3'), 4,20 (ddd, 1H, H-4'), 3,90-3,60 (dd, 2H, H-5',5").

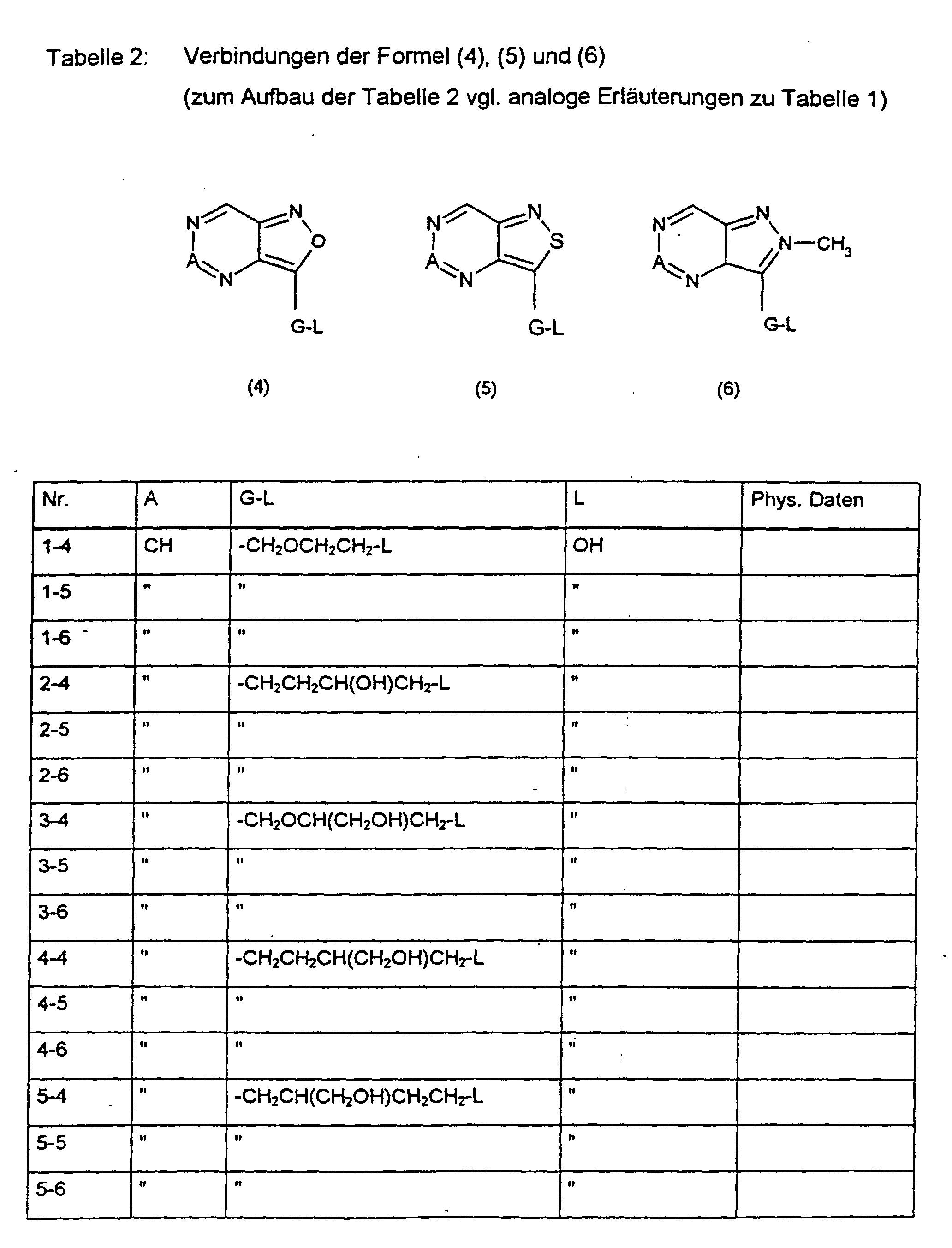

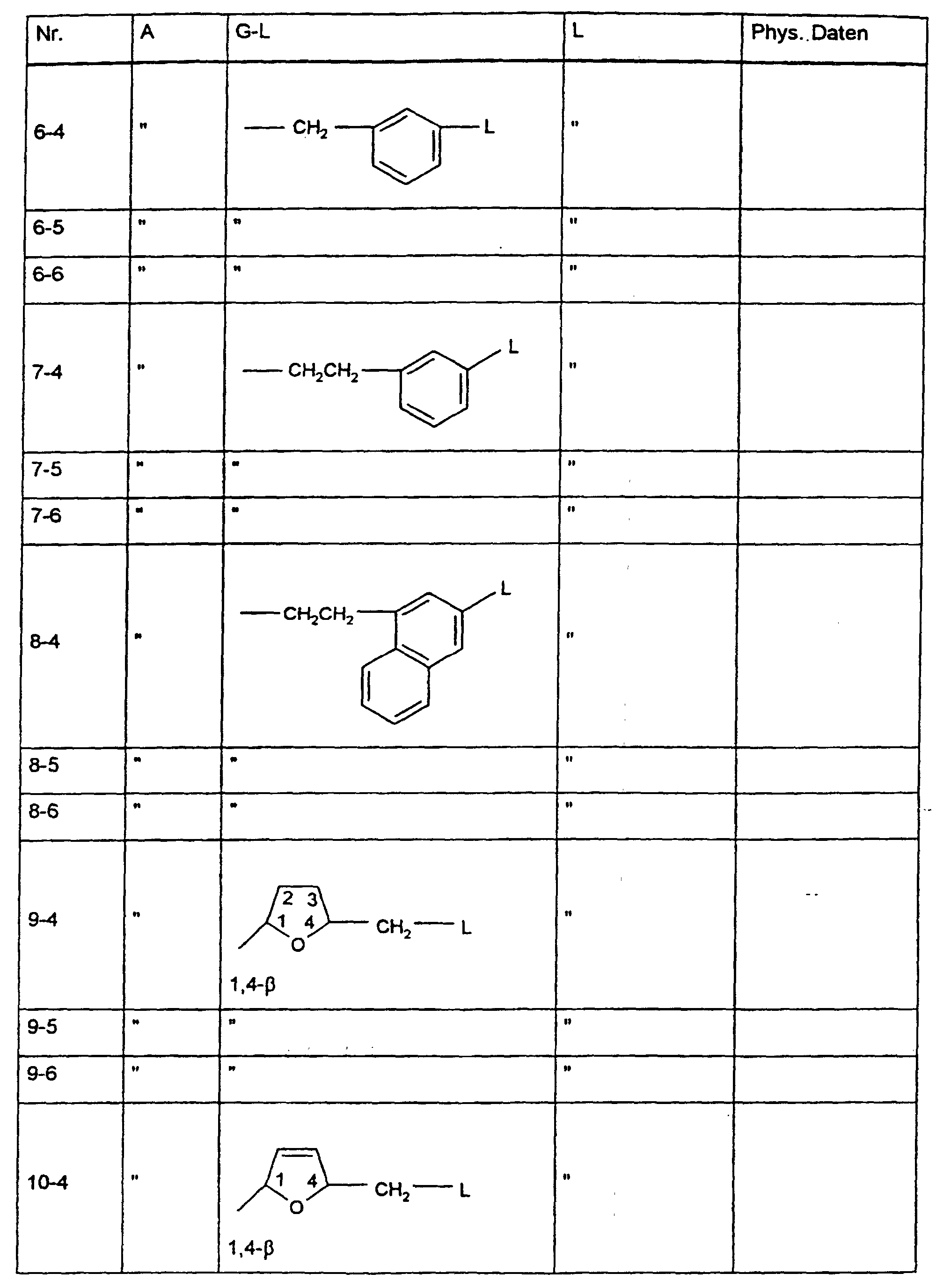

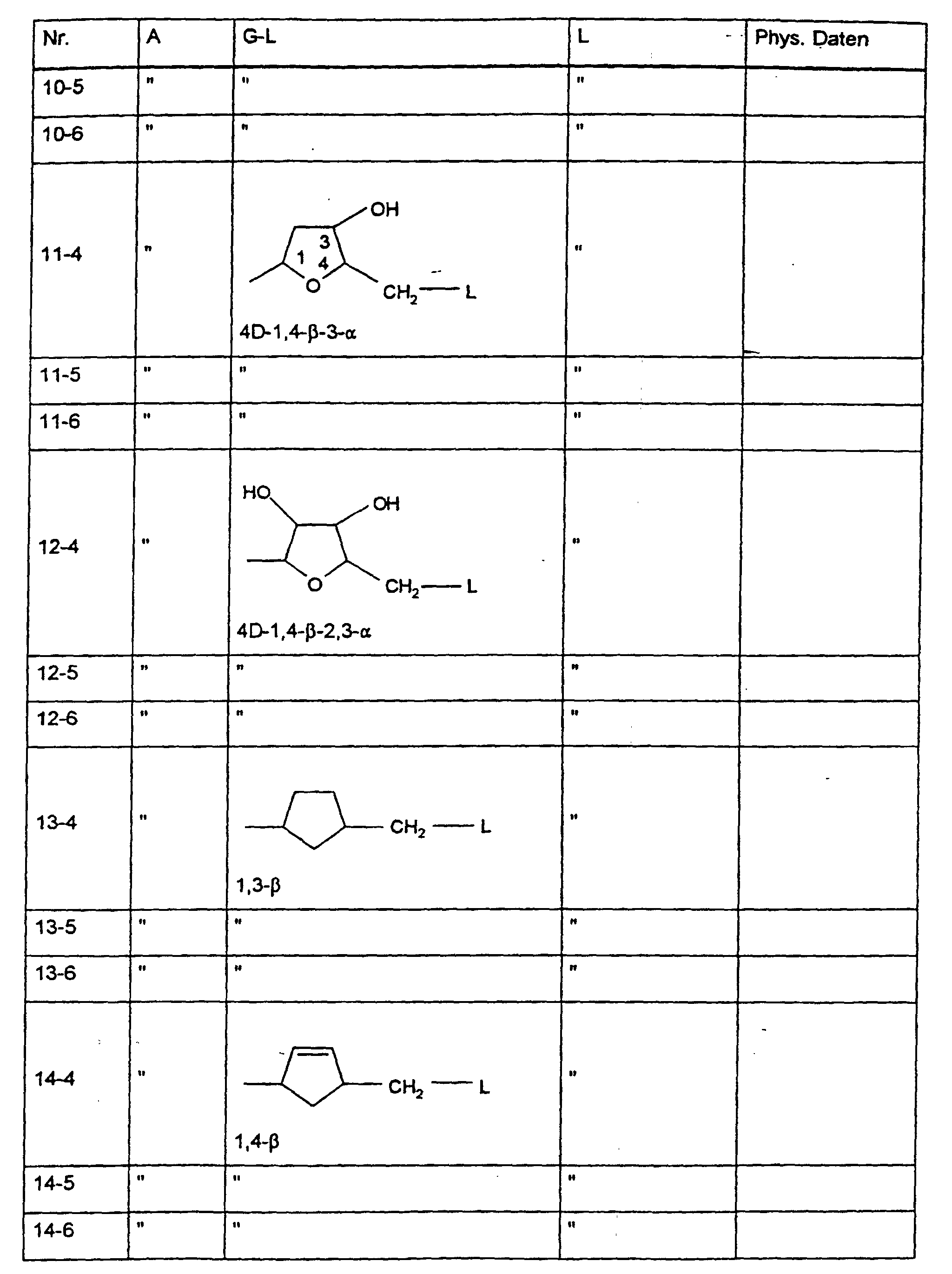

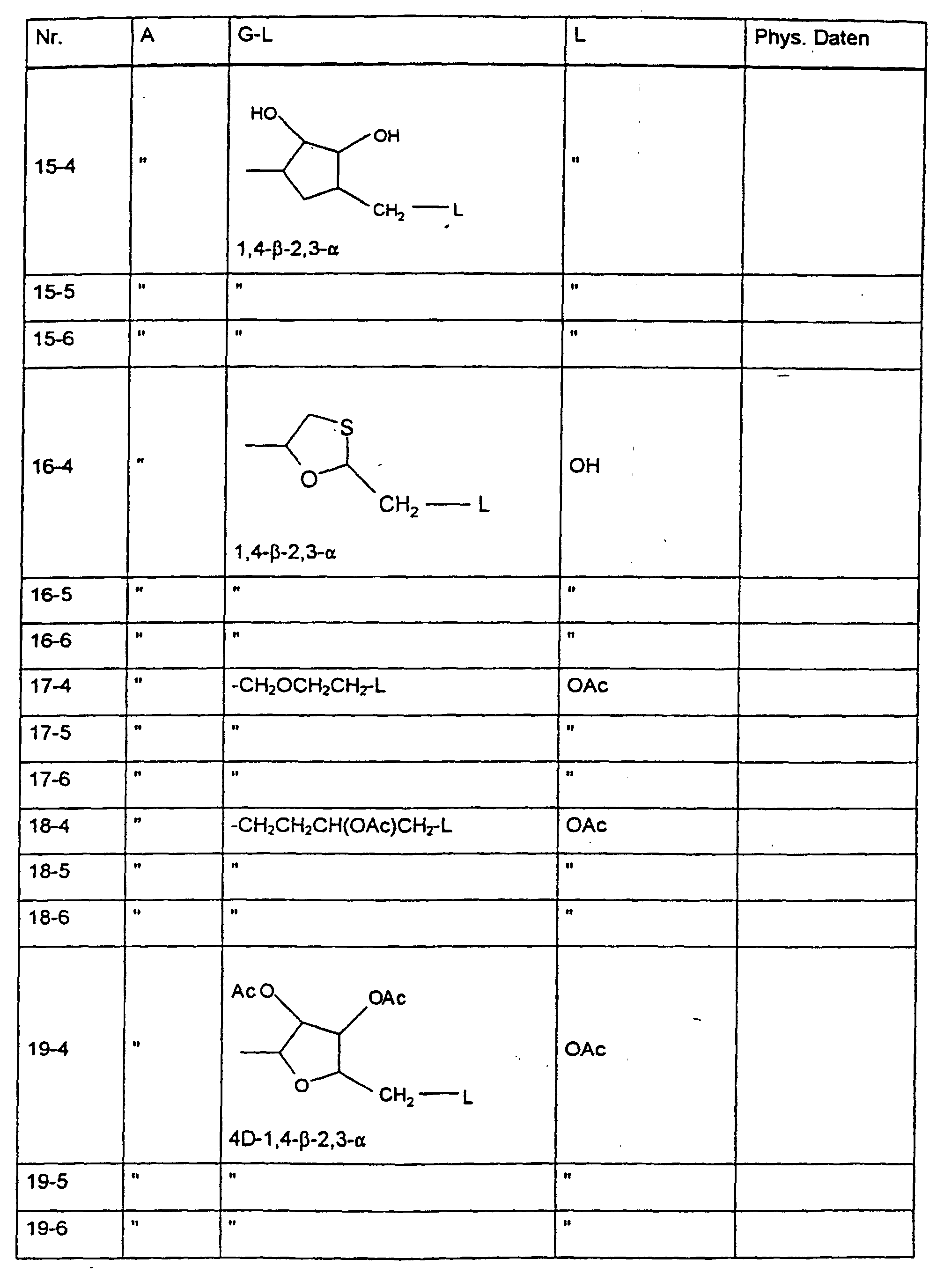

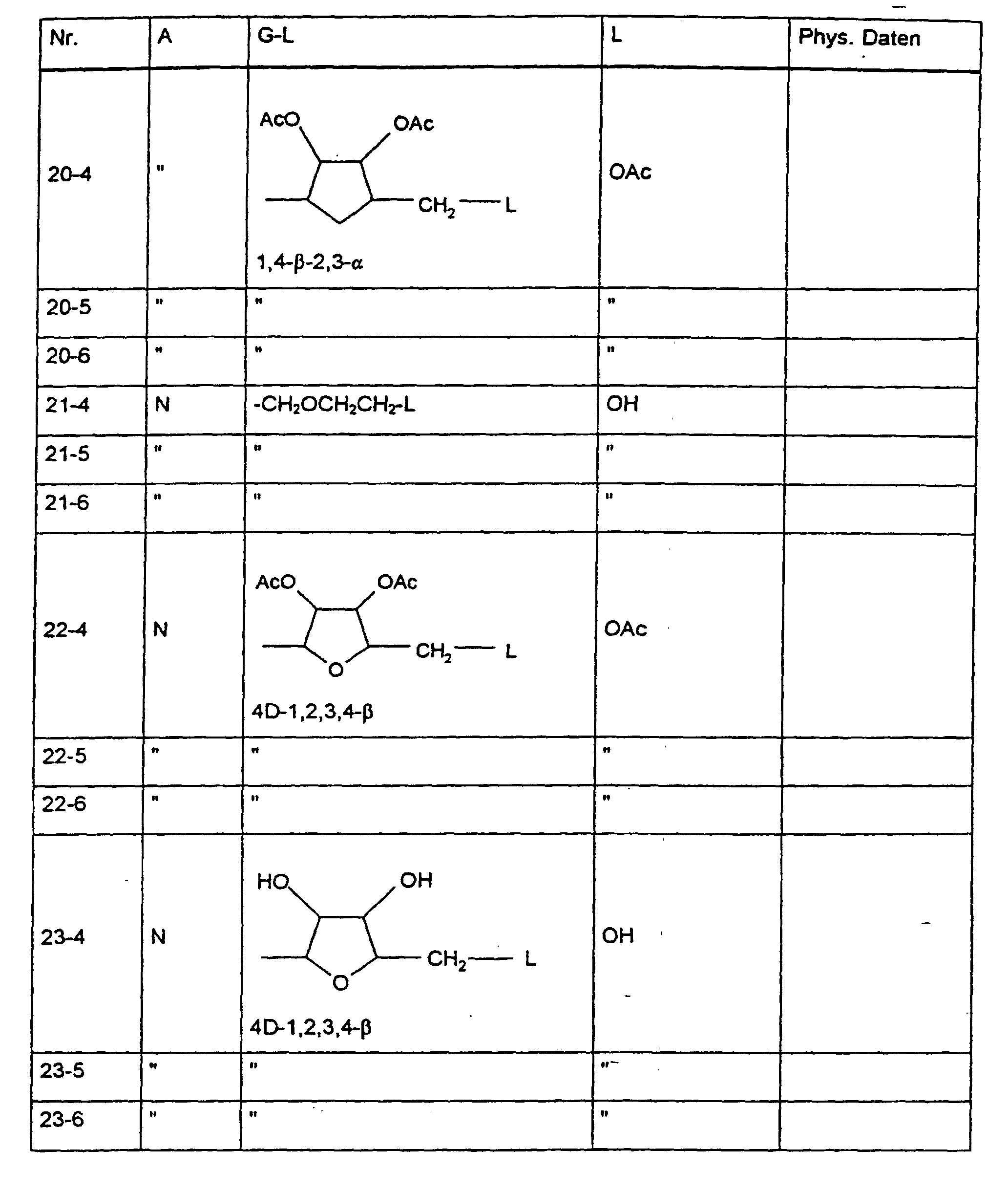

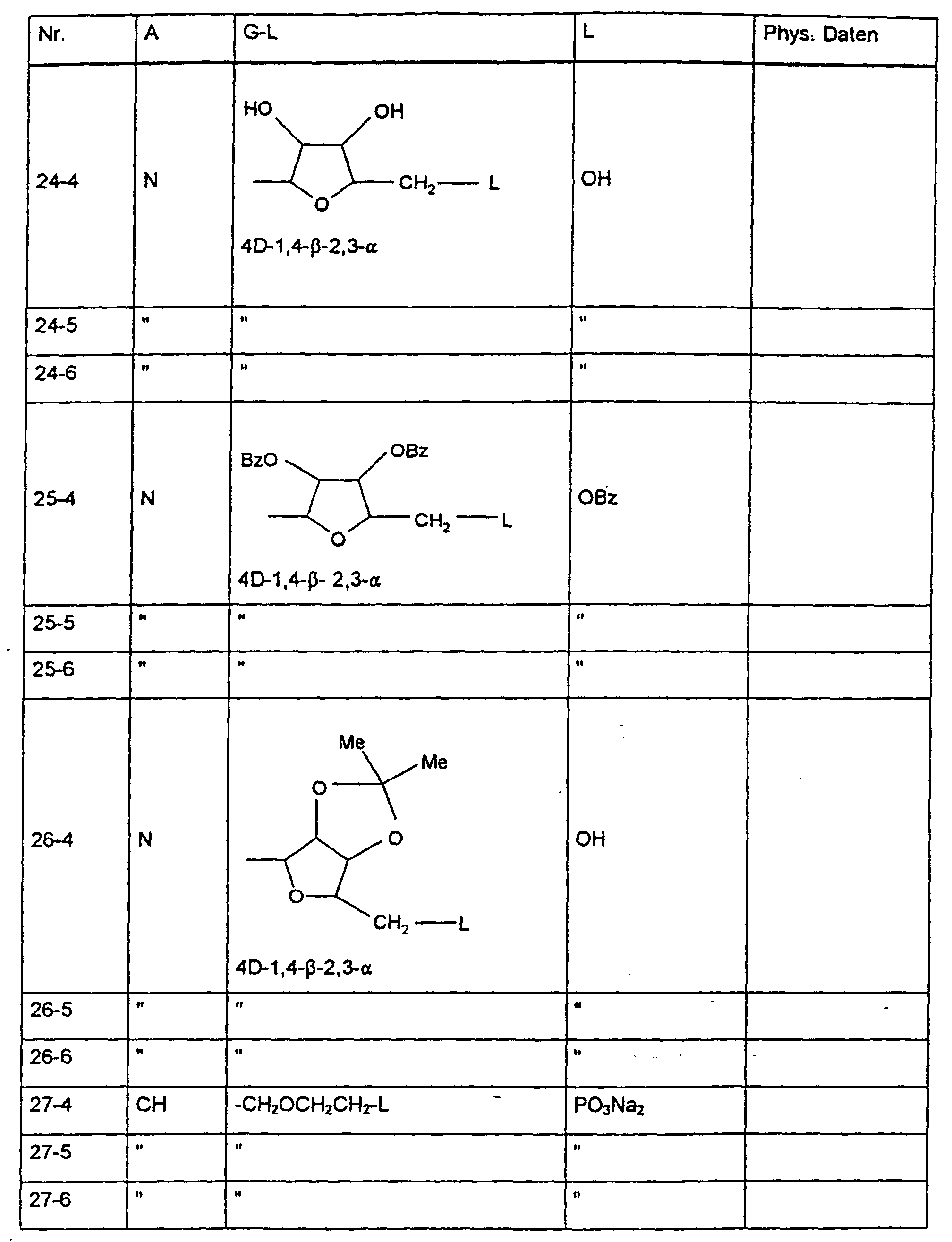

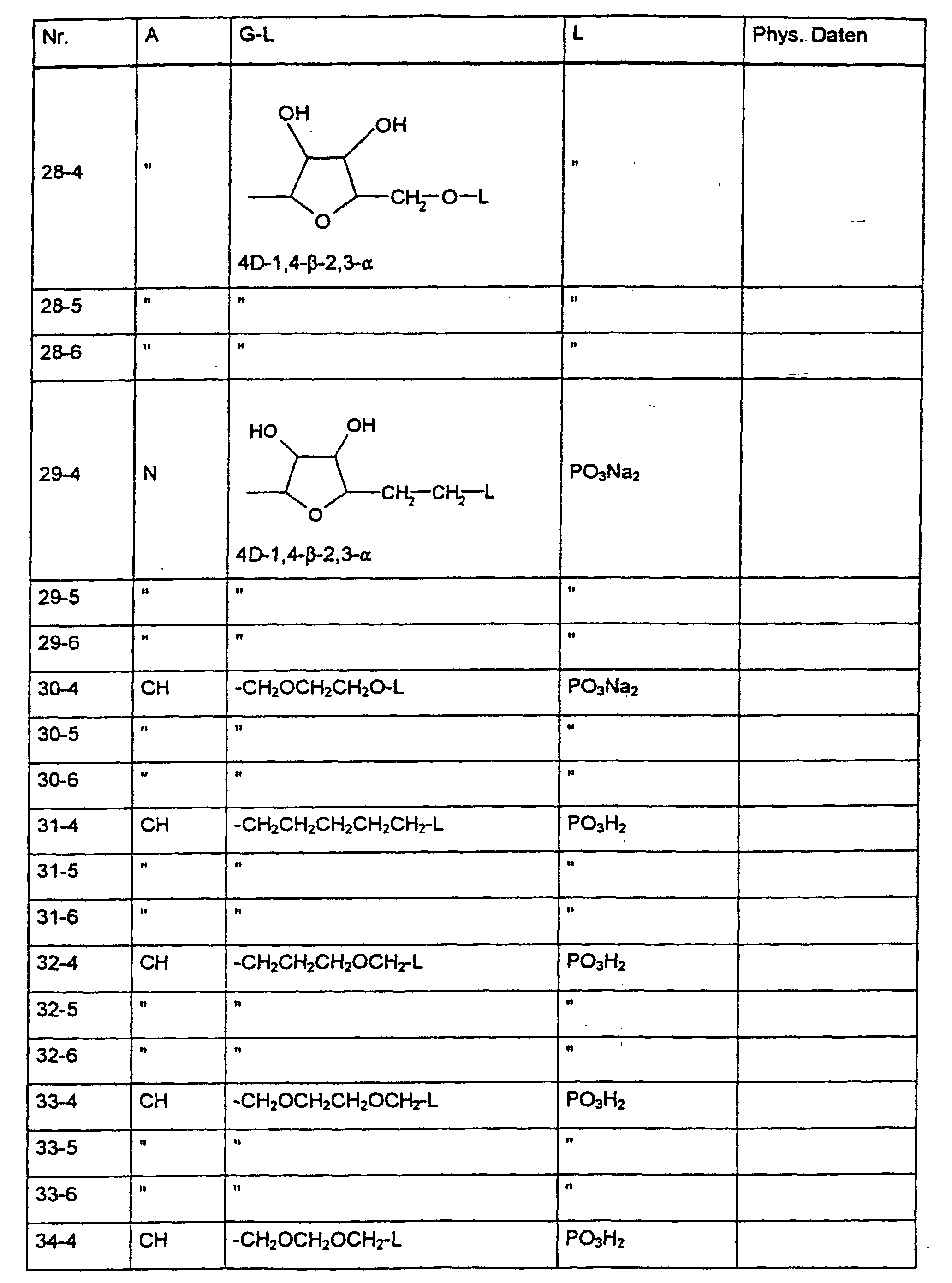

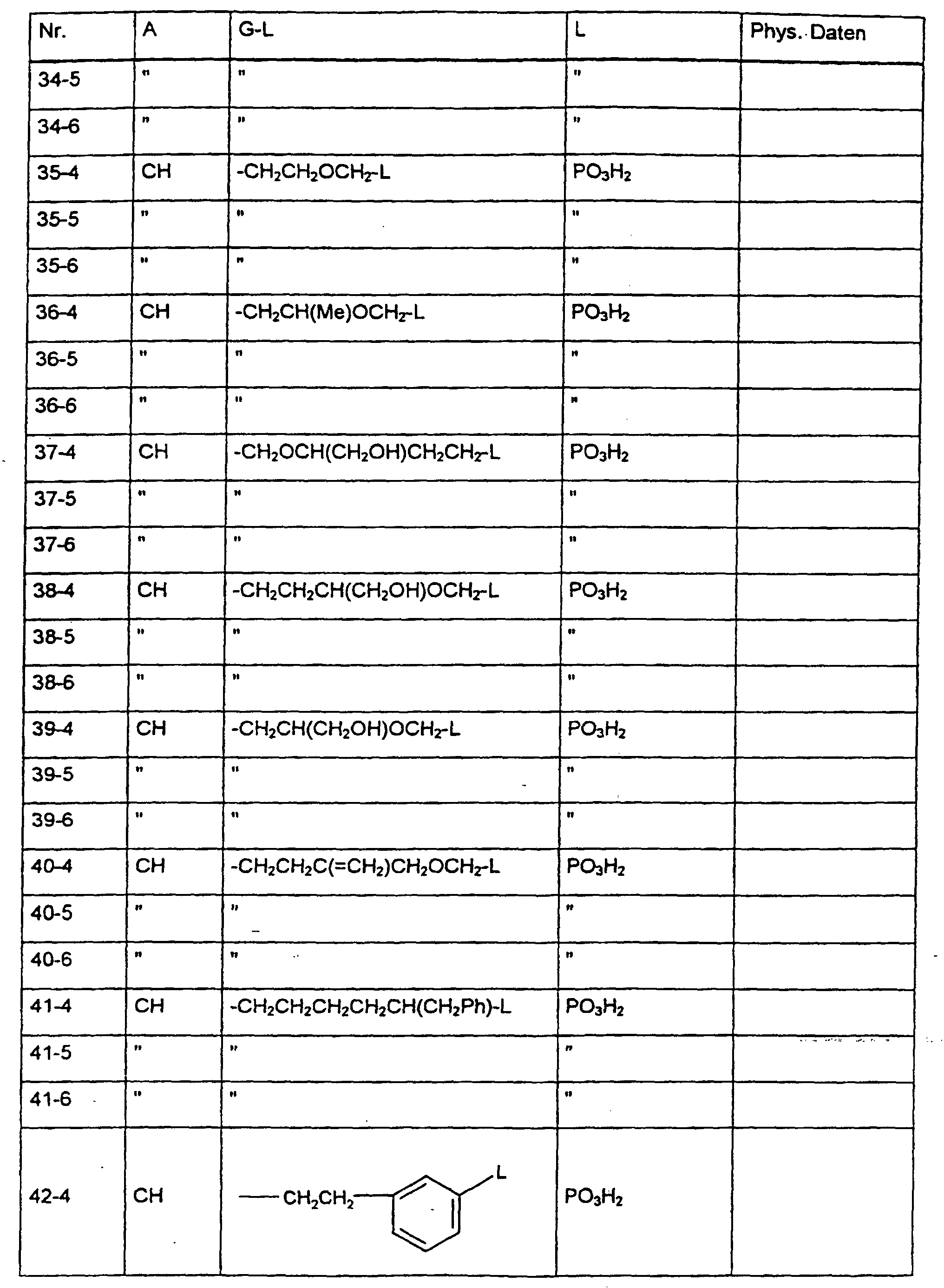

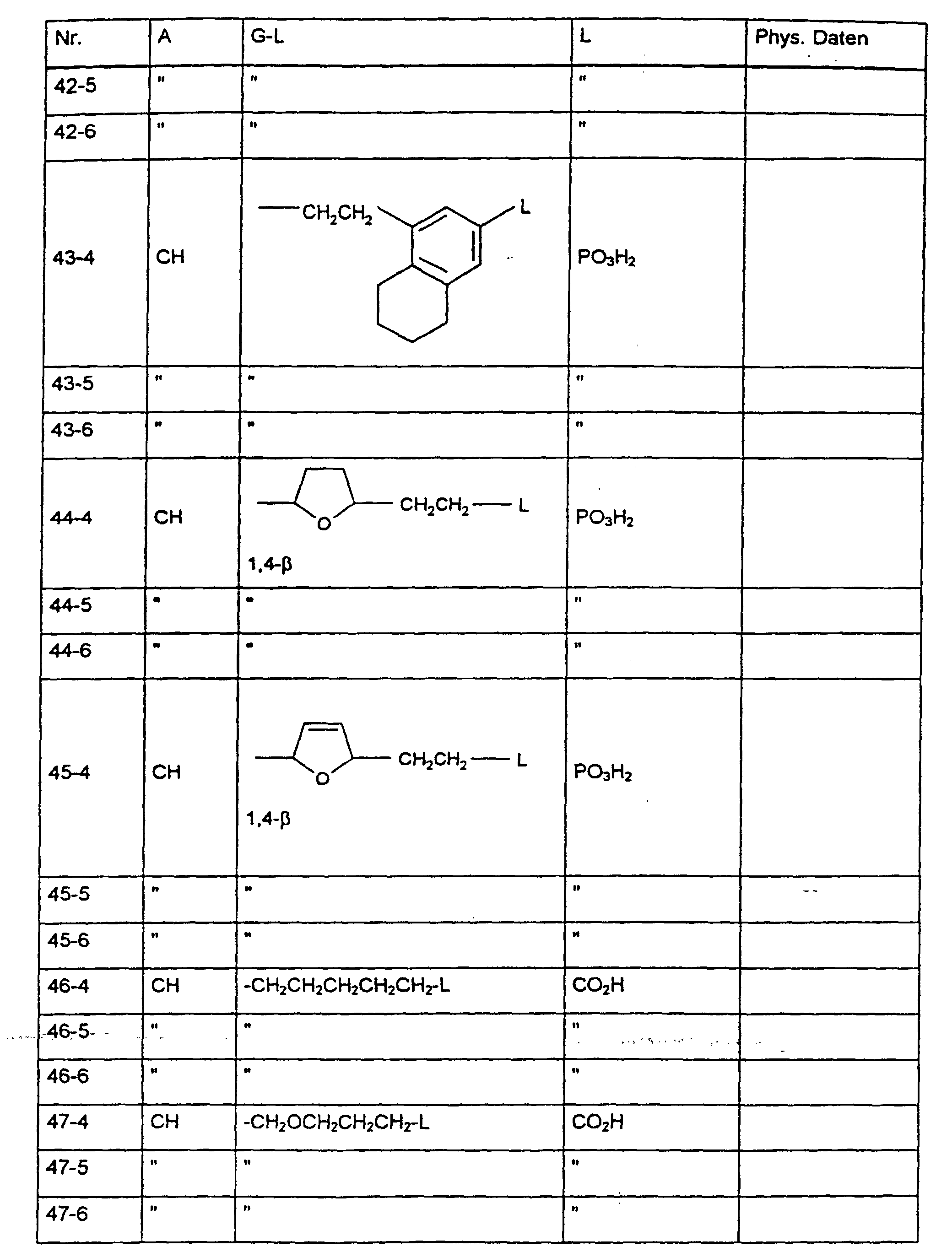

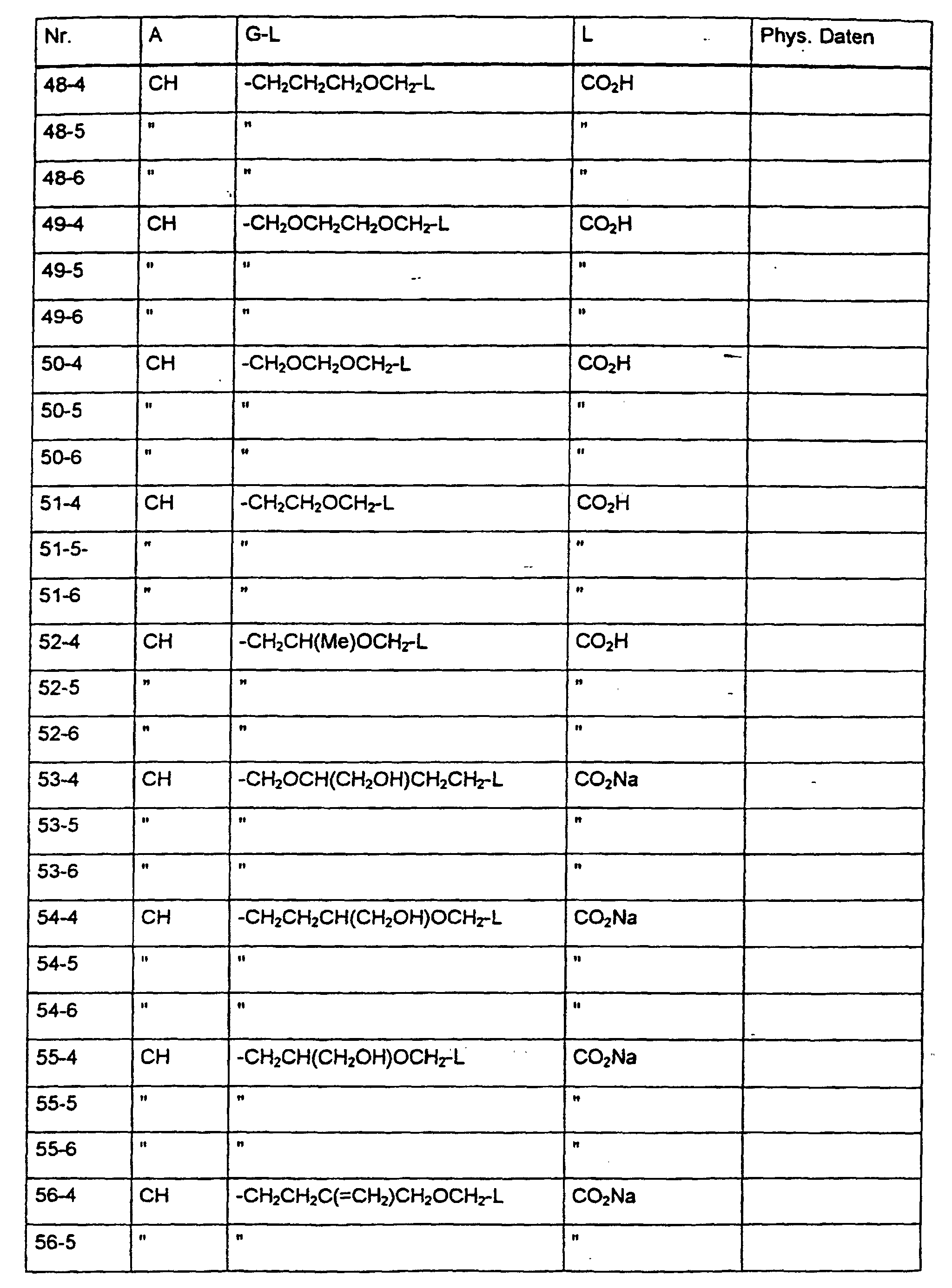

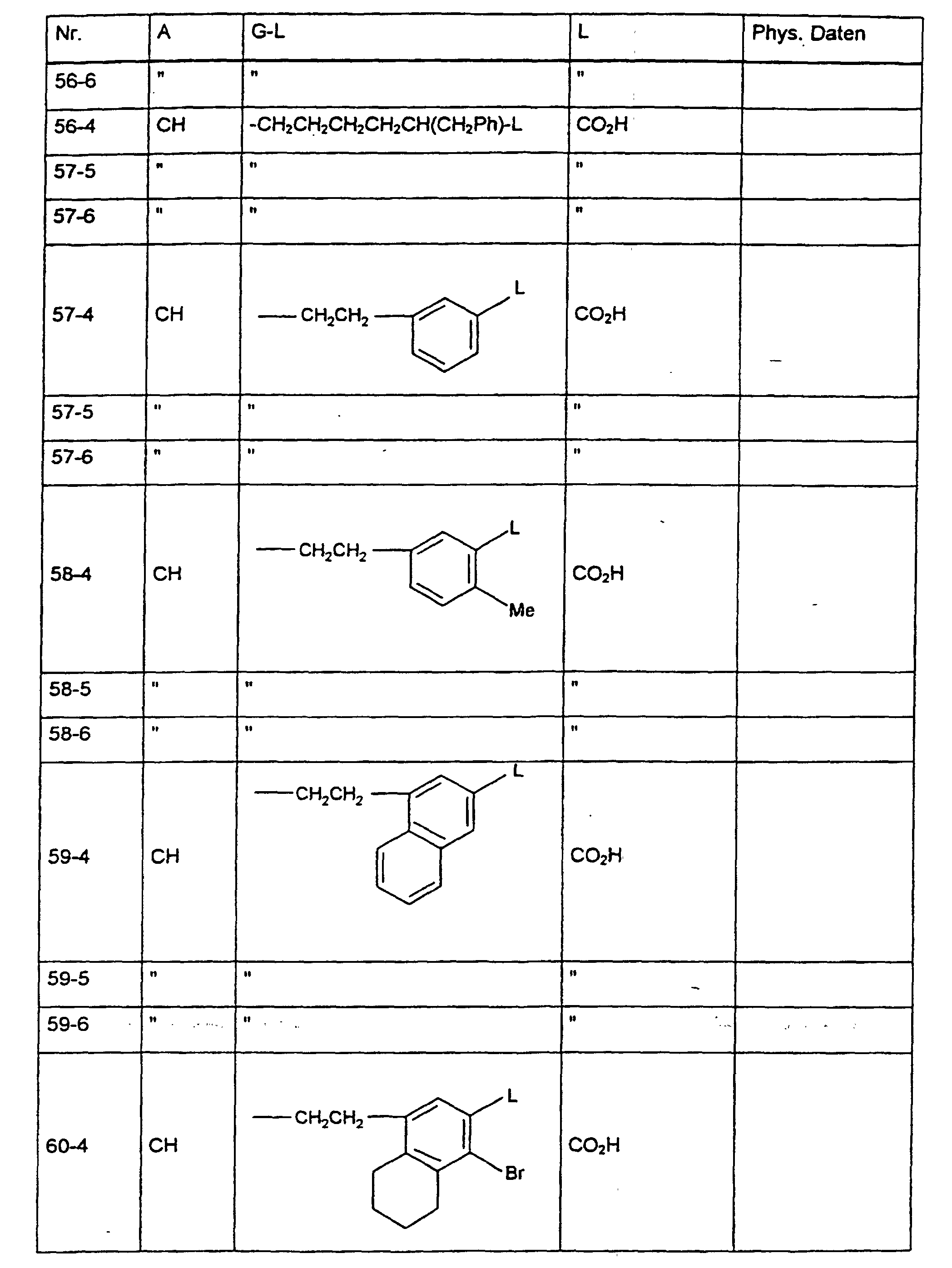

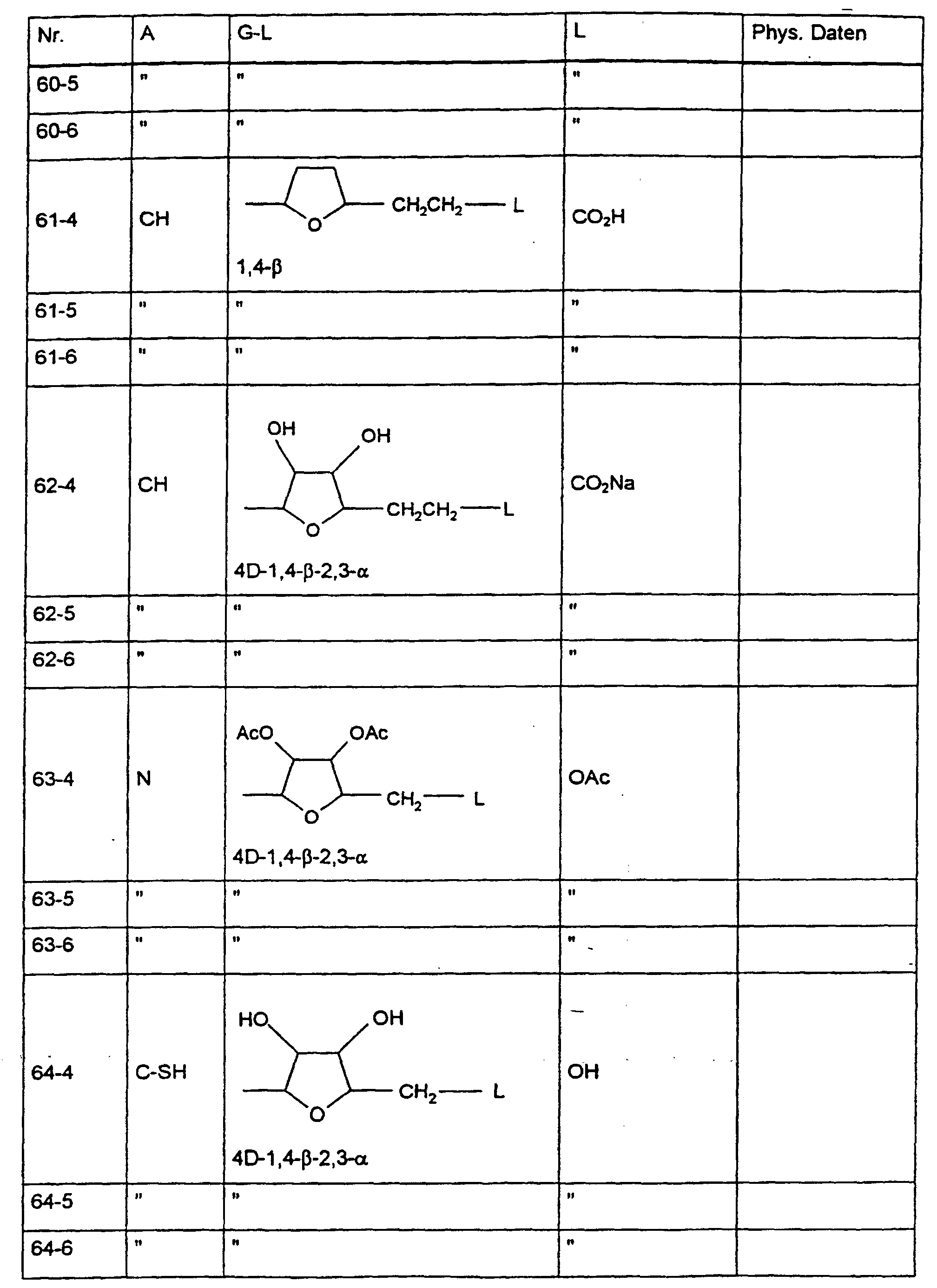

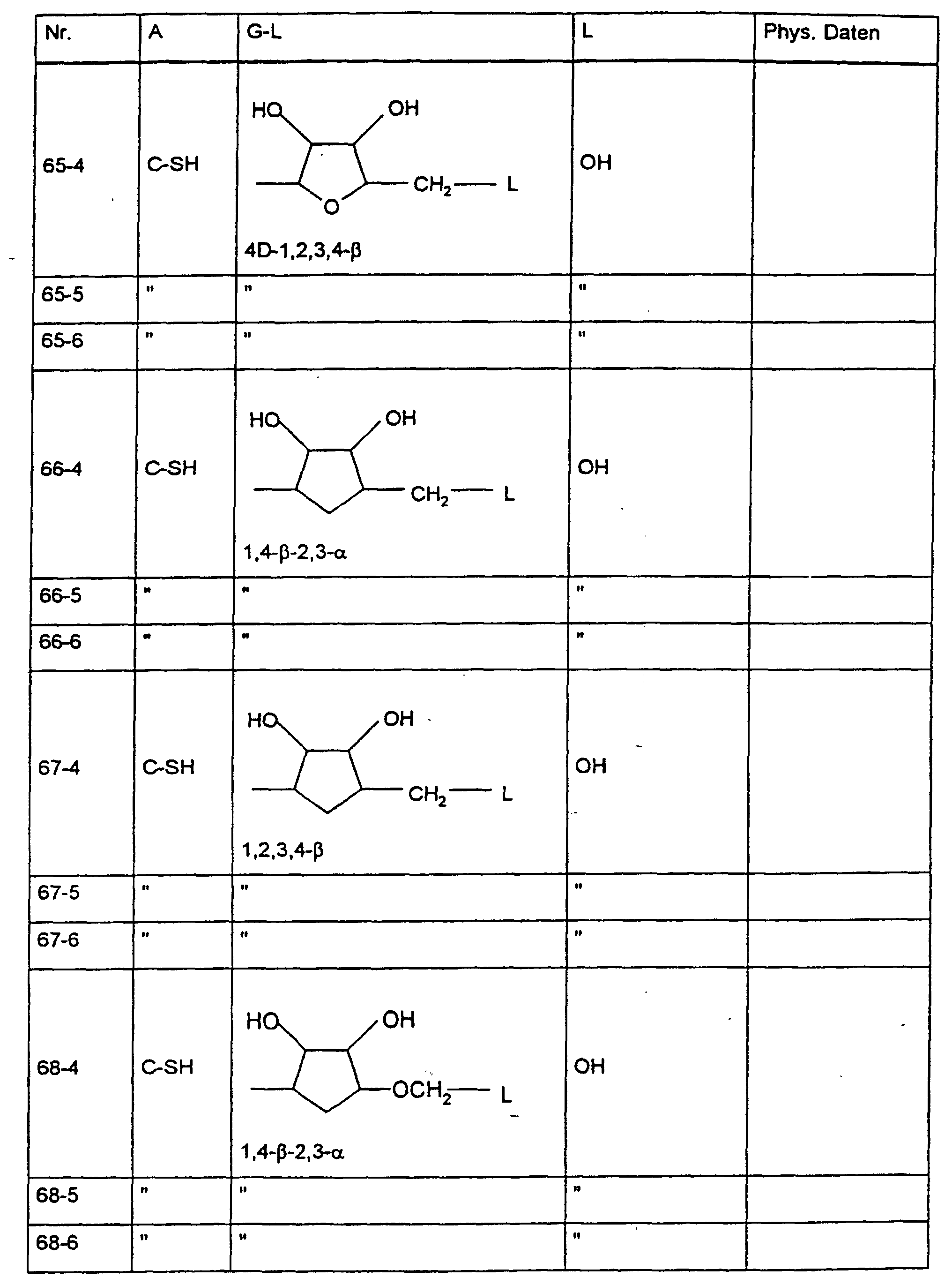

### B. Formulierungsbeispiele

a) Ein Stäubemittel wird erhalten, indem man 10 Gew.-Teile einer Verbindung (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiennittel mischt und in einer Stiftmühle mahlt.
c) Ein in Wasser leicht dispergierbares Dispersionskonzentrat wird erhalten, indem man 20 Gewichtsteile einer Verbindung (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer Verbindung (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | |
|---|---|
| 75 Gewichtsteile einer | Verbindung (I), |
| 10 " | ligninsulfonsaures Calcium, |
| 5 " | Natriumlaurylsulfat, |
| 3 " | Polyvinylalkohol und |
| 7 " | Kaolin |
| | |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | |
|---|---|
| 25 Gewichtsteile einer | Verbindung (I), |
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Spektralphotometrischer Enzymtest

### A) Test mit AMPDA aus Erbsen

Die Testsubstanz wurde in mehreren Ansätzen bei unterschiedlichen Konzentrationen jeweils mit 0,01 Enzymeinheiten (U = Units) Adenosinmonophosphatdeaminase (aus Erbsen) (J. Dancer et al., Plant Physiol., 114 (1997) 119) in 0,15 ml einer wäßrigen Citratpufferlösung (0,06 M, pH 7,1 mit 5 M NaOH), 0,01 g/ml BSA (Bovine Serum Albumin, Albumin aus Rinderserum), 0,01 M KCl und 1 µM Diadenosinpentaphosphat vorinkubiert. Mit Zugabe einer wäßrigen Lösung von 0,6 mM Adenosinmonophosphat und 1 mM Adenosintriphosphat zu der vorinkubierten Lösung wurde die Enzymreaktion gestartet. Nach 60 min bei 25 °C wurde die Reaktion durch Zugabe von 100 µl Reagenz 1 (= 0,1 M Phenol und 0,17 mM Natrium-nitrosyl-prussiat = Na₂[Fe(CN)₅NO]) und 100 µl Reagenz 2 (= 0,125 M NaOH, 0,38 M und Na₂HPO₄ und 5 ml HOCI in 500 ml Wasser) gestoppt. Nach 60 min bei 55 °C wurde die Absorption bei 625 nm gemessen.

Der Vergleich zum Messwert, der im Test ohne Zusatz der Testsubstanz erhatten wurde, stellt ein Maß für die Enzymhemmung dar. Beispielsweise weisen die Verbindungen 28-1 und 44-1 im Test eine mindestens 50 %ige Hemmung der Enzymaktivität bei einer Konzentration von 500 µM auf.

### B) Test mit AMPDA aus Kälberdarm

Die Testsubstanz wurde in mehreren Ansätzen bei unterschiedlichen Konzentrationen jeweils mit 0,04 Enzymeinheiten (U) Adenosinmonophosphatdeaminase (aus Kälberdarm) in 2,1 ml einer wäßrigen Citratpufferlösung (0,01 M, pH 6,5 mit 2 M NaOH), 0,05 g/ml BSA und 0,033 M KCl 10 min bei 25 °C vorinkubiert. Mit Zugabe von 100 µl einer wäßrigen Lösung von 0,8 mM Adenosinmonophosphat zu 700 µl der vorinkubierten Lösung wurde die Enzymreaktion gestartet. Durch spektralphotometrische Messung der Absorption bei 265 nm innerhalb von 2 min im Vergleich zu einer Referenzküvette mit 800 µl der vorinkubierten Lösung erhielt man einen Messwert, der im Vergleich zum Messwert, er im Test ohne Zusatz der Testsubstanz erhalten wurde, ein Maß für die Enzymhemmung darstellt. Bespielsweise weisen die Verbindungen 28-1 und 44-1 im Test eine mindestens 50 %ige Hemmung der Enzymaktivität bei einer Konzentration von 500 µM auf.

### C) Test mit ADA aus Kanninchenmuskel

Die Testsubstanz wurde in mehreren Ansätzen bei unterschiedlichen Konzentrationen jeweils mit 0,04 Enzymeinheiten (U) Adenosindeaminase (aus Kanninchenmuskel) in 2,1 ml einer wäßrigen Phosphatpufferlösung (0,1 M, pH 7,5) für 10 min bei 25 °C vorinkubiert. Mit Zugabe von 100 µl einer wäßrigen Lösung von 0,8 mM Adenosin zu 700 µl der vorinkubierten Lösung wurde die Enzymreaktion gestartet. Durch spektralphotometrische Messung der Absorption bei 265 nm innerhalb von 2 min im Vergleich zu einer Referenzküvette mit 800 µl der vorinkubierten Lösung erhielt man einen Messwert, der im Vergleich zum Messwert, er im Test ohne Zusatz der Testsubstanz erhalten wurde, ein Maß für die Enzymhemmung darstellt. Beispielsweise weisen die Verbindungen 1-1, 12-1, 21-3, 22-2 und 159-3 im Test eine mindestens 50 %ige Hemmung der Enzymaktivität bei einer Konzentration von 500 µM auf.

### 2. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten Verbindungen (I) werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die Verbindungen (I) eine gute herbizide Vorauflaufwirksamkeit gegen ein breites Spektrum von Ungräsern und Unkräutern auf. Beispielsweise zeigt die Verbindung Nr. 17-1 (s. Tabelle 1) im Test gute herbizide Wirkung gegen Schadpflanzen wie Galium aparine, Matricaria inodora und Elymus repens (= Agropyron repens) im Vorauflaufverfahren bei einer Aufwandmenge von 3 kg oder weniger Aktivsubstanz pro Hektar. Vergleichbare Ergebnisse werden mit anderen Verbindungen aus den Tabellen 1 und 2 erhalten, z. B. mit Verbindungen Nr. 1-1, 12-1, 21-3, 22-2, 28-1, 44-1 und 159-3.

### 3. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten Verbindungen (I) werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die Verbindungen (I) weisen auch im Nachauflauf eine gute herbizide Wirksamkeit gegen ein breites Spektrum wirtschaftlich wichtiger Ungräser und Unkräuter auf. Beispielsweise zeigt die Verbindung Nr. 17-1 (s. Tabelle 1) im Test gute herbizide Wirkung gegen Schadpflanzen wie Echinochloa crus-galli, Stellaria media, Amaranthus retroflexus, Xanium orientale, Setaria viridis, Avena fatua, Matricaria inodora und Pharbitis purpurea im Nachauflaufverfahren bei einer Aufwandmenge von 3 kg oder weniger Aktivsubstanz pro Hektar. Vergleichbare Ergebnisse werden mit anderen Verbindungen aus den Tabellen 1 und 2 erhalten, z. B. mit Verbindungen Nr. 1-1, 12-1, 21-3, 22-2, 28-1, 44-1 und 159-3.

### 4. Wirkung auf Schadpflanzen in Reis

Verpflanzter und gesäter Reis sowie typische Reisunkräuter und -ungräser werden im Gewächshaus bis zum Dreiblattstadium (Echinochloa crus- galli 1,5-Blatt) unter Paddyreis-Bedingungen (Anstauhöhe des Wassers: 2 - 3 cm) in geschlossenen Plastiktöpfen angezogen. Danach erfolgt die Behandlung mit den Verbindungen (I). Hierzu werden die formulierten Wirkstoffe in Wasser suspendiert, gelöst bzw. emulgiert und mittels Gießapplikation in das Anstauwasser der Test-pflanzen in unterschiedlichen Dosierungen ausgebracht.

Nach der so durchgeführten Behandlung werden die Versuchspflanzen im Gewächshaus unter optimalen Wachstumsbedingungen aufgestellt und während der gesamten Versuchszeit so gehalten.

Etwa drei Wochen nach der Applikation erfolgt die Auswertung mittels optischer Bonitur der Pflanzenschäden im Vergleich zu unbehandelten Kontrollen. Die Verbindungen (I) weisen zeigen sehr gute herbizide Wirkung gegen Schadpflanzen auf.

### 5. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den Verbindungen (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum, Mais oder Reis. Die Verbindungen (I) zeigen teilweise eine hohe Selektivität und eignen sich deshalb zur Bekämpfung von unerwünschten Pflanzenwuchs in landwirtschaftlichen Kulturen.

## Patentansprüche

1. Verwendung von Verbindungen der Formel (I), deren Tautomeren, deren Salzen und deren Wasseradditionsprodukten, 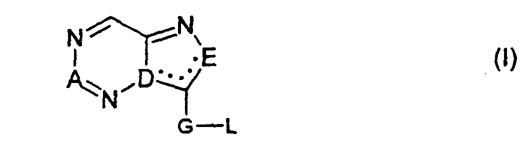 wobei in Formel (I)
A ein Stickstoffatom oder eine Gruppe der Formel C-R bedeutet, wobei R weiter unten definiert ist,
D ein Kohlenstoffatom oder ein Stickstoffatom bedeutet,
E a) im Fall, daß D ein Stickstoffatom ist, ein Stickstoffatom oder eine
Gruppe der Formel C-R^{o} bedeutet, wobei R^{o} weiter unten definiert ist, oder
b) im Fall, daß D ein Kohlenstoffatom ist, eine Gruppe der Formel N-R^{o}, -O-, -S-, -SO- oder -SO₂- bedeutet,
die Reihe von Punkten (•••••) von D über ein benachbartes Ring-C-atom bis E
eine Doppelbindung zwischen dem Ring-C-Atom und E, wenn D ein Stickstoffatom ist (Fall a), oder
eine Doppelbindung zwischen dem Ring-C-Atom und D, wenn D ein Kohlenstoffatom ist (Fall b), bedeutet,
R, R^{O} unabhängig voneinander jeweils ein Wasserstoffatom, Amino, Hydroxy, Mercapto, Cyano, Halogen, Azido, Nitro, SF₅, gegebenenfalls substituiertes Aminosulfonyl, Acyl, Acylamino, Acyloxy, Acylthio, Mono- oder Di-(C₁-C₄)alkylamino, Mono- oder Di-(C₃-C₉)cycloalkylamino, (C₁-C₄)Alkylthio, (C₂-C₄)Alkenylthio, (C₂-C₄)Alkinylthio, (C₃-C₉)Cycloalkylthio, (C₅-C₉)Cycloalkenylthio, (C₁-C₄)Alkylsulfinyl, (C₁-C₄)Alkylsulfonyl, (C₁-C₄)Alkoxy, (C₂-C₄)Alkenyloxy, (C₂-C₄)Alkinyloxy, (C₃-C₉)Cycloalkoxy, (C₅-C₉)Cycloalkenyloxy, (C₁-C₄)Alkyl, (C₂-C₄)Alkenyl, (C₂-C₄)Alkinyl, (C₃-C₉)Cycloalkyl, (C₅-C₉)Cycloalkenyl, (C₁-C₄)Alkylaminosulfonyl oder Di[(C₁-C₄)alkyl]aminosulfonyl, wobei jeder der letztgenannten 23 Reste unsubstituiert oder im Kohlenwasserstoffteil durch einen oder mehrere Reste aus der Gruppe Halogen, Hydroxy, Amino, Nitro, Formyl, Carboxy, Cyano, Thiocyanato, (C₁-C₄)Alkoxy, (C₃-C₉)Cycloalkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, (C₃-C₉)Cycloalkyl-amino, [(C₁-C₄)Alkyl]carbonyl, [(C₁-C₄)Alkoxy]carbonyl, Aminocarbonyl, Mono(C₁-C₄)alkylamino-carbonyl und Di(C₁-C₄)alkylamino-carbonyl substituiert ist, bedeutet,
G eine divalente geradkettige gesättigte oder ungesättigte Kohlenwasserstoffbrücke mit 1 bis 24 C-Atomen in der Kette bedeutet, in der ein oder mehrere Kettenglieder jeweils unabhängig voneinander durch O, S, NH, (C₁-C₄)Alkyl-N oder Acyl-N ausgetauscht sein können oder, im ungesättigten Fall, eine oder mehrere CH-Gruppen jeweils durch ein Stickstoffatom ausgetauscht sein können,
wobei die jeweilige Brücke unsubstituiert ist oder
(a) durch einen oder mehrere gleiche oder verschiedene Reste aus der Gruppe Halogen, Nitro, von Wasserstoff verschiedene Reste der Formel R¹, Reste der Formel R²R³C= und Reste der Formel L* substituiert ist, wobei R¹, R², R³ und L* weiter unten definiert sind,
(b) zwei oder vier Substituenten trägt, von denen jeweils zwei zusammen mit dem sie verbindenden Brückenteil einen carbocyclischen oder heterocyclischen Ring mit 3 bis 7 Ringatomen bilden, wobei im Fall eines Heterocyclus die Heteroatome, vorzugsweise 1, 2 oder 3 Heteroatome, aus der Gruppe N, O und S ausgewählt sind und wobei der jeweilige Ring noch ankondensierte Ringe aufweisen kann und im übrigen unsubstituiert oder durch einen oder mehrere gleiche oder verschiedene Reste aus der Gruppe Halogen, Nitro, von Wasserstoff verschiedene Reste der Formel R¹, Reste der Formel L* und Oxo substituiert ist, wobei R¹ und L* weiter unten definiert sind,
(c) über eine zweite direkte Bindung oder über ein Heteroatom aus der Gruppe N, O und S mit L cyclisch verbunden ist,
(d) zwei oder mehrere Substituenten aus den vorstehenden Gruppen (a) bis (c) gemeinsam aufweist,
L, L* unabhängig voneinander jeweils OR⁴, SR⁴, CN, Tetrazolo, C(OR⁵)(OR⁶)(OR⁷), -Z¹, -O-Z², -S-Z² oder -NH-Z², wobei R⁴, R⁵, R⁶, R⁷, Z¹ und Z² weiter unten definiert ist und wobei L über eine zweite direkte Bindung oder über ein Heteroatom aus der Gruppe N, O und S mit der Brücke G cyclisch verbunden sein kann,
Z¹, Z² unabhängig voneinander jeweils den Rest einer anorganischen oder organischen Sauerstoffsäure der Formel Z¹-OH bzw. Z²-OH, wobei der Rest formal durch Abtrennen der Hydroxygruppe an der Säurefunktion entsteht,
R¹ bis R⁷ unabhängig voneinander jeweils ein Wasserstoffatom, (C₁-C₆)Alkyl, (C₂-C₆)Alkenyl, (C₂-C₆)Alkinyl, (C₃-C₉)Cycloalkyl, (C₅-C₉)Cycloalkenyl, Aryl oder Heterocyclyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosulfonyl, Acyl, Acylamino, Acyloxy, Acylthio, [(C₁-C₄)Alkoxy]carbonyl, Mono(C₁-C₄)alkylamino, Mono(C₃-C₉)cycloalkylamino, Di(C₁-C₄)alkylamino, (C₁-C₄)Alkylthio, (C₂-C₄)Alkenylthio, (C₂-C₄)Alkinylthio, (C₃-C₉)Cycloalkylthio, (C₅-C₉)Cycloalkenylthio, (C₁-C₄)Alkylsulfinyl, (C₁-C₄)Alkylsulfonyl, (C₁-C₄)Alkoxy, (C₂-C₄)Alkenyloxy, (C₂-C₄)Alkinyloxy, (C₃-C₉)Cycloalkoxy, (C₅-C₉)Cycloalkenyloxy, (C₃-C₉)Cycloalkyl, (C₅-C₉)Cycloalkenyl, Aryl, substituiertes Aryl, Heteroaryl, substituiertes Heteroaryl und im Falle cyclischer Reste auch (C₁-C₄)Alkyl, (C₂-C₄)Alkenyl, (C₂-C₄)Alkinyl, (C₁-C₄)Haloalkyl, (C₂-C₄)Haloalkenyl, (C₂-C₄)Haloalkinyl, (C₁-C₄)Hydroxyalkyl und (C₁-C₄)Alkoxy(C₁-C₄)alkyl substituiert ist, bedeuten,
wobei Heterocyclyl ein heterocyclischer gesättigter, ungesättigter oder heteroaromatischer Ring und
wobei Heteroaryl ein heteroaromatischer Ring ist
oder
R², R³ zusammen mit dem C-Atom der Gruppe R²R³C= einen nicht aromatischen carbocyclischen Ring oder einen heterocyclischen Ring mit 3
bis 9 Ringatomen und 1 bis 4 Heteroringatomen aus der Gruppe N, O und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten oder
R⁵, R⁶ zusammen mit dem C-Atom und den benachbarten Sauerstoffatomen der Gruppe C(OR⁵)(OR⁶)(OR⁷) einen gesättigen oder ungesättigten nicht aromatischen heterocyclischen Ring mit 4 bis 9 Ringatomen und 1 bis 4 Heteroringatomen aus der Gruppe N, O, P und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, oder
die Gruppe C(OR⁵)(OR⁶)(OR⁷) zusammen einen bicyclischen Rest der Formel 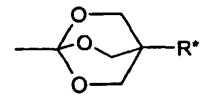 worin
R* (C₁-C₄)Alkyl, (C₁-C₄)Haloalkyl, (C₁-C₄)Alkoxy, (C₁-C₄)Haloalkoxy, (C₁-C₄)Alkylthio und Phenyl, das unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist,
bedeuten, zwecks direkter oder indirekter Hemmung des Enzyms Adenosinmonophosphatdeaminase (AMPDA) oder Adenosindeaminase (ADA), ausgenommen die Verwendung zur chirurgischen oder therapeutischen Behandlung des menschlichen oder tierischen Körpers oder die Verwendung in Diagnostizierverfahren, die am menschlichen oder tierischen Körper durchgeführt werden.

2. Verwendung nach Anspruch 1, **dadurch gekennzeichnet, daß**
A ein Stickstoffatom oder
eine Gruppe der Formel C-R bedeutet, in der
R ein Wasserstoffatom, Amino, Hydroxy, Mercapto, Cyano, Halogen, Azido, Nitro, SF₅, Aminosulfonyl, (C₁-C₅)Alkanoylamino, [(C₁-C₄)Alkoxy]-carbonylamino, (C₁-C₅)Alkanoyl, [(C₁-C₄)Alkoxy]-carbonyl, (C₁-C₅)Alkanoyloxy, [(C₁-C₄)Alkoxy]-carbonyloxy,. Mono(C₁-C₄)alkylamino, Mono(C₃-C₆)cycloalkylamino, Di(C₁-C₄)alkylamino, (C₁-C₄)Alkylthio, (C₁-C₄)Alkylsulfinyl, (C₁-C₄)Alkylsulfonyl, (C₁-C₄)Alkoxy, (C₃-C₄)Alkenyloxy, (C₃-C₄)Alkinyloxy, (C₃-C₆)Cycloalkoxy, (C₅-C₆)Cycloalkenyloxy, (C₁-C₄)Alkyl, (C₂-C₄)Alkenyl, (C₂-C₄)Alkinyl, (C₃-C₆)Cycloalkyl, (C₅-C₆)Cycloalkenyl, (C₁-C₄)Alkylaminosulfonyl oder Di[(C₁-C₄)alkyl]aminosulfonyl, wobei jeder der letztgenannten 24 Reste unsubstituiert oder im Kohlenwasserstoffteil durch einen oder mehrere Reste aus der Gruppe Halogen, Hydroxy, Amino, Nitro, Formyl, Carboxy, Cyano, Thiocyanato, (C₁-C₄)Alkoxy, (C₃-C₆)Cycloalkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₆)Cycloalkyl, (C₃-C₆)Cycloalkyl-amino, [(C₁-C₄)Alkyl]carbonyl, [(C₁-C₄)Alkoxy]carbonyl, Aminocarbonyl, Mono(C₁-C₄)alkylamino-carbonyl und Di(C₁-C₄)alkylamino-carbonyl substituiert ist, bedeutet.

3. Verwendung nach Anspruch 1 oder 2, **dadurch gekennzeichnet, daß**
G eine divalente geradkettige gesättigte oder ungesättigte Kohlenwasserstoffbrücke mit 1 bis 8 C-Atomen in der Kette, in der ein oder mehrere CH₂-Gruppen jeweils unabhängig voneinander durch O oder S ausgetauscht sind,
wobei die jeweilige Brücke unsubstituiert ist oder
(a) durch einen oder mehrere Halogenatome und zusätzlich oder alternativ dazu einen oder mehrere gleiche oder verschiedene Reste aus der Gruppe Nitro, von Wasserstoff verschiedene Reste der Formel R¹, Reste der Formel R²R³C= und Reste der Formel L* substituiert ist, wobei R¹, R², R³ und L* die definierte Bedeutung haben,
(b) zwei oder vier Substituenten trägt, von denen jeweils zwei zusammen mit dem sie verbindenden Brückenteil einen carbocyclischen Ring mit 3 bis 6 C-Atomen oder einen heterocyclischen gesättigten oder partiell ungesättigten Ring mit 3 bis 6 Ringatomen oder einen heteroaromatischen Ring mit 5 oder 6 Ringatomen bilden, wobei im Fall eines Heterocyclus die 1, 2 oder 3 Heteroatome aus der Gruppe N, O und S ausgewählt sind und wobei der jeweilige Ring noch einen ankondensierten carbocyclischen Ring mit 4 bis 6 Ringatomen oder einen ankondensierten heterocyclischen Ring mit 4 bis 6 Ringatomen und 1, 2 oder 3 Heteroatomen aus der Gruppe N, O und S ausweisen kann und im übrigen unsubstituiert oder durch einen oder mehrere Halogenatome und zusätzlich oder alternativ dazu einen oder mehrere gleiche oder verschiedene Reste aus der Gruppe Nitro, von Wasserstoff verschiedene Reste der Formel R¹, Reste der Formel L* und Oxo substituiert ist, wobei R¹ und L* die definierte Bedeutung haben,
(c) Substituenten aus den vorstehenden Gruppen (a) und (b) gemeinsam aufweist.

4. Verwendung nach einem der Ansprüche 1 bis 3, **dadurch gekennzeichnet, daß**
L, L* unabhängig voneinander jeweils OR⁴, SR⁴, CN, Tetrazolo, C(OR⁵)(OR⁶)(OR⁷), -Z¹, -O-Z², -S-Z² oder -NH-Z², wobei R⁴, R⁵, R⁶, R⁷, Z¹ und Z² weiter unten definiert sind und wobei L über eine zweite direkte Bindung oder über ein Heteroatom aus der Gruppe N, O und S mit G cyclisch verbunden sein kann,
Z¹ den Rest der Formel COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)(NR¹⁶R¹⁷), P(=S)(OR¹³)(NR¹⁰R¹¹) oder P(=S)(NR¹⁰R¹¹)(NR¹⁶R¹⁷),
Z² den Rest der Formel COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)(NR¹⁶R¹⁷), P(=S)(OR¹³)(NR¹⁰R¹¹) oder P(=S)(NR¹⁰R¹¹)(NR¹⁶R¹⁷),
R¹ bis R¹⁷ unabhängig voneinander jeweils ein Wasserstoffatom, (C₁-C₆)Alkyl, (C₂-C₆)Alkenyl, (C₂-C₆)Alkinyl, (C₃-C₉)Cycloalkyl, (C₅-C₉)Cycloalkenyl, Aryl oder Heterocyclyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosulfonyl, (C₁-C₄)Alkanoyl, Acylamino, Acyloxy, Acylthio, [(C₁-C₄)Alkoxy]carbonyl, Mono(C₁-C₄)alkylamino, Mono(C₃-C₉)cycloalkylamino, Di(C₁-C₄)alkylamino, (C₁-C₄)Alkylthio, (C₂-C₄)Alkenylthio, (C₂-C₄)Alkinylthio, (C₃-C₉)Cycloalkylthio, (C₅-C₉)Cycloalkenylthio, (C₁-C₄)Alkylsulfinyl, (C₁-C₄)Alkylsulfonyl, (C₁-C₄)Alkoxy, (C₂-C₄)Alkenyloxy, (C₂-C₄)Alkinyloxy, (C₃-C₉)Cycloalkoxy, (C₅-C₉)Cycloalkenyloxy, (C₃-C₉)Cycloalkyl, (C₅-C₉)Cycloalkenyl, Phenyl, substituiertes Phenyl, Heteroaryl, substituiertes Heteroaryl und im Falle cyclischer Reste auch (C₁-C₄)Alkyl, (C₂-C₄)Alkenyl; (C₂-C₄)Alkinyl, (C₁-C₄)Haloalkyl, (C₂-C₄)Haloalkenyl, (C₂-C₄)Hatoalkinyl, (C₁-C₄)Hydroxyalkyl und (C₁-C₄)Alkoxyl(C₁-C₄)alkyl substituiert ist,
wobei Heterocyclyl ein heterocyclischer gesättigter, ungesättigter oder heteroaromatischer Ring mit 3 bis 6 Ringatomen und 1 bis 3 Heteroatomen aus der Gruppe N, O und S ist und
wobei Heteroaryl ein heteroaromatischer Ring mit 5 bis 6 Ringatomen und 1 bis 3 Heteroatomen aus der Gruppe N, O und S ist und
wobei die Substituenten für substituiertes Phenyl oder substituiertes Heteroaryl ein oder mehrere aus der Gruppe Halogen, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Hydroxyalkyl und (C₁-C₄)Alkoxy(C₁-C₄)alkyl sind,
oder
R², R³ zusammen mit dem C-Atom der Gruppe R²R³C= einen nicht aromatischen carbocyclischen Ring oder einen heterocyclischen Ring mit 3 bis 6 Ringatomen und 1 bis 3 Heteroringatomen aus der Gruppe N, O und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten oder
R⁵, R⁶ zusammen mit dem C-Atom und den benachbarten Sauerstoffatomen der Gruppe C(OR⁵)(OR⁶)(OR⁷) einen gesättigen oder ungesättigten nicht aromatischen heterocyclischen Ring mit 3 bis 6 Ringatomen und 1 bis 3 Heteroringatomen aus der Gruppe N, O, P und S, der unsubstituiert oder durch einen oder mehrere Reste aus der Gruppe Halogen, Nitro, Hydroxy, Oxo, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten oder
R⁸, R⁹ oder R¹⁰, R¹¹ oder R¹³, R¹⁴ oder R¹⁴, R¹⁵ oder R¹⁶, R¹⁷ jeweils paarweise mit den Atomen der jeweils definierten Gruppe einen gesättigen oder ungesättigten nicht aromatischen heterocyclischen Ring mit 3 bis 9 Ringatomen und 1 bis 4 Heteroringatomen aus der Gruppe N, O, P und S, der unsubstituiert oder durch einen oder mehrere Reste aus der Gruppe Halogen, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeuten.

5. Verwendung nach einem der Ansprüche 1 bis 4, **dadurch gekennzeichnet, daß**
G eine divalente geradkettige gesättigte oder ungesättigte Kohlenwasserstoffbrücke mit 1 bis 8 C-Atomen in der Kette, in der ein oder mehrere CH₂-Gruppen jeweils unabhängig voneinander durch O oder S ausgetauscht sind,
oder
eine Brücke der Formel -W¹-Cyclus-W²-, worin
W¹, W² unabhängig voneinander eine direkte Bindung, CH₂, CH₂CH₂, OCH₂, SCH₂, CH₂CH₂CH₂, CH₂OCH₂, CH₂SCH₂, OCH₂CH₂ oder SCH₂CH₂ und "Cyclus" 1,4-Cyclohexylen, 1,2-Phenylen, 1,3-Phenylen, 1,4-Phenylen, 1,2-Naphthylen, 1,3-Naphthylen, 1,4-Naphthylen, 1,2-Tetrahydronaphthylen, 1,3-Tetrahydronaphthylen, 1,4-Tetrahydronaphthylen, 1,2-Cyclopentylen, 1,3-Cyclopentylen, 1,2-Cyclohexylen, 1,3-Cyclohexylen, 1,4-Cyctohexylen,Tetrahydrofuran-2,5-diyl (Oxolan), Tetrahydrothiophen-2,5-diyl, 2,5-Dihydrofuran-2,5-diyl oder 2,5-Dihydrothiophen-2,5-diyl bedeuten, wobei die jeweilige Brücke unsubstituiert ist oder
durch einen oder mehrere Halogenatome und zusätzlich oder alternativ dazu einen oder mehrere gleiche oder verschiedene Reste aus der Gruppe von Wasserstoff, verschiedene Reste der Formel R¹, Reste der Formel R²R³C= und Reste der Formel L* substituiert ist, wobei R¹, R², R³ und L* wie oben oder weiter unten definiert sind, oder
zusätzlich oder alternativ dazu über eine zweite direkte Bindung oder über ein Heteroatom aus der Gruppe N, O und S mit L cyclisch verbunden ist, und
R¹ bis R¹⁷ jeweils unabhängig voneinander ein Wasserstoffatom, (C₁-C₄)Alkyl, (C₂-C₄)Alkenyl, (C₂-C₄)Alkinyl, (C₃-C₆)Cycloalkyl, (C₅-C₆)Cycloalkenyl, Phenyl oder Heterocyclyl, wobei jeder der letztgenannten Reste unsubstituiert oder durch einen oder mehrere Reste aus der Gruppe Amino, Hydroxy, Mercapto, Cyano, Halogen, Azido, Nitro, SF₅, Aminosulfonyl, (C₁-C₄)Alkanoyl, (C₁-C₄)Alkanoylamino, Benzoylamino, (C₁-C₄)Alkanoyloxy, (C₁-C₄)Alkanoylthio, [(C₁-C₄)Alkoxy]carbonyl, Mono(C₁-C₄)alkylamino, Di(C₁-C₄)alkylamino, (C₁-C₄)Alkylthio, (C₃-C₄)Alkenylthio, (C₃-C₄)Alkinylthio, (C₁-C₄)Alkylsulfinyl, (C₁-C₄)Alkylsulfonyl, (C₁-C₄)Alkoxy, (C₃-C₄)Alkenyloxy, (C₃-C₄)Alkinyloxy, (C₃-C₉)Cycloalkoxy, (C₃-C₉)Cycloalkyl, Phenyl, substituiertes Phenyl, Heteroaryl, substituiertes Heteroaryl und im Falle cyclischer Reste auch (C₁-C₄)Alkyl, (C₂-C₄)Alkenyl, (C₂-C₄)Alkinyl, (C₁-C₄)Haloalkyl, (C₂-C₄)Haloalkenyl, (C₂-C₄)Haloalkinyl, (C₁-C₄)Hydroxyalkyl und (C₁-C₄)Alkoxyl(C₁-C₄)alkyl substituiert ist,
wobei Heterocyclyl ein heterocyclischer gesättigter oder ungesättigter Ring mit 3 bis 6 Ringatomen oder ein heteroaromatischer Ring mit 5 oder 6 Ringatomen und jeweils 1 bis 3 Heteroatomen aus der Gruppe N, O und S ist und
wobei Heteroaryl ein heteroaromatischer Ring mit 5 bis 6 Ringatomen und 1 bis 3 Heteroatomen aus der Gruppe N, O und S ist und
wobei die Substituenten für substituiertes Phenyl oder substituiertes Heteroaryl ein oder mehrere aus der Gruppe Halogen, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Hydroxyalkyl und (C₁-C₄)Alkoxy(C₁-C₄)alkyl sind,
bedeuten.

6. Verwendung nach einem der Ansprüche 1 bis 5, **dadurch gekennzeichnet, daß**
L Hydroxy, Carboxy, [(C₁-C₄)Alkoxy]-carbonyl, CONH₂, [(C₁-C₄)Alkylamino]-carbonyl, [(C₁-C₄)Alkylsulfonylamino]carbonyl, [(C₁-C₄)Haloalkylsulfonylamino]carbonyl, [Cyano(C₁-C₄)alkylsulfonylaminojcarbonyl, (C₁-C₄)Alkylsulfonylamino, (C₁-C₄)Haloalkylsulfonylamino, Cyano-(C₁-C₄)alkylsulfonylamino, (C₁-C₅)Alkanoyloxy, Benzoyloxy, [(C₁-C₄)Alkoxy]-carbonyloxy, [(C₁- C₄)Alkylamino]carbonyloxy, (C₁-C₄)Alkoxy, (C₁-C₄)Alkylthio, (C₁-C₄)Hydroxyalkoxy, SO₂NHCONH₂, (C₁-C₅)Alkanoylaminosulfonyl, [(C₁-C₄)Haloalkyl]carbonylaminosulfonyl, [(C₁-C₄)Alkoxy]carbonylaminosulfonyl, [(C₁-C₅)Haloalkoxy]carbonylaminosulfonyl, SO₂NH₂, Di[(C₁-C₄)alkyl]aminosulfonyl, P(=O)(OH)₂, P(=S)(OH)₂, P(=O)(OR')₂ oder P(=O)(OH)(OR'), wobei in den letztgenannten beiden Formeln R' jeweils unabhängig von anderen Resten R' (C₁-C₄)Alkyl, (C₁-C₄)Haloalkyl, (C₁-C₄)Hydroxyalkyl, (C₁-C₄)Alkanoyl(C₁-C₄)alkyl, (C₁-C₄)Alkanoyloxy(C₁-C₄)alkyl oder Phenyl ist,
bedeutet.

7. Verbindungen der Formel (I), deren Tautomeren, deren Salzen und deren Wasseradditionsprodukten, wie sie nach einem der Ansprüche 1 bis 6 definiert sind, ausgenommen die Verbindung der Formel (I), worin A = CH, D = C, E = NH und G-L = β-D-Ribofuranosyl bedeuten.

8. Verfahren zur Herstellung der Verbindungen der allgemeinen Formel (I) oder deren Salze gemäß einem der Ansprüche 1 bis 6, **dadurch gekennzeichnet, daß** man
a) eine Verbindung der Formel (II), 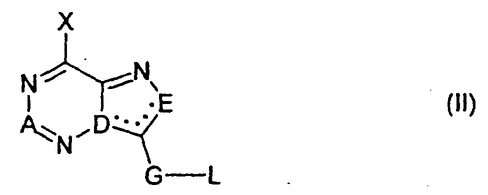 worin X eine Abgangsgruppe darstellt, zur Verbindung der Formel (I) reduziert oder
b) eine Verbindung der Formel (III), 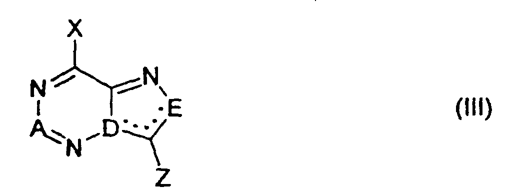 worin X eine Abgangsgruppe darstellt und Z eine Vorstufe zum Rest G-L bedeutet, zur Verbindung der Formel (III'), 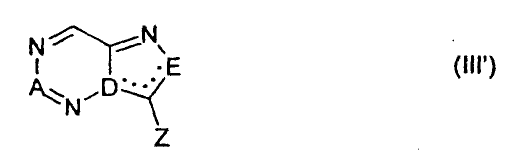 worin Z wie in Formel (III) definiert ist, reduziert und anschließend die Verbindung (III) an der Gruppe Z modifiziert, so daß die Verbindung (I) erhalten wird,
c) eine Verbindung der Formel (III'), worin Z eine Vorstufe zum Rest G-L bedeutet, an der Gruppe Z modifiziert, so daß die Verbindung (I) erhalten wird, oder
d) im Falle, daß A eine Gruppe der Formel C-R ist, eine Verbindung der Formel (III"), 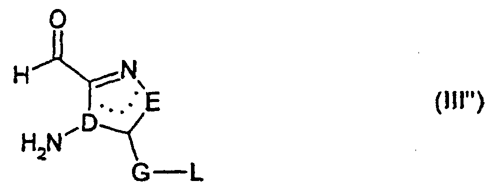 mit einer Verbindung der Formel (III''')
H₂N - A = NH (III''')
worin A eine Gruppe C-R darstellt, zur Verbindung der Formel (I) cyclisiert,
wobei in den Formeln (II), (III), (III'), (III") und (III''') die Symbole A, D, E, G, L und R, wenn nicht speziell anders definiert, wie in Formel (I) definiert sind.

9. Verfahren zur Herstellung einer Verbindung der Formel (V), 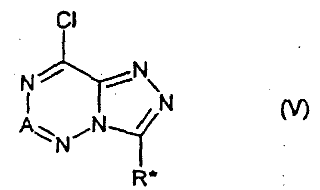 worin R* = Z oder G-L darstellt und A, G und L wie in Formel (I) gemäß Anspruch 1 definiert ist und Z eine Vorstufe zum Rest G-L bedeutet, **dadurch gekennzeichnet, daß** man eine Verbindung der Formel (IV) 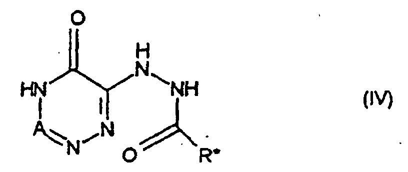 worin A und R* wie in Formel (V) definiert sind,
mit einem Chlorierungsmittel umsetzt und zur Verbindung der Formel (V) cyclisiert.

10. Verbindungen der Formel (V) gemäß Anspruch 9.

11. Verfahren zur Herstellung einer Verbindung der Formel (VI), SMe 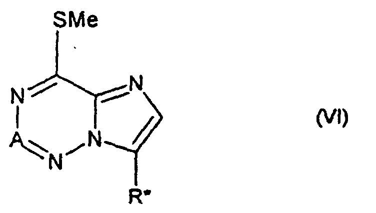 worin R* = Z oder G-L darstellt und A, G und L wie in Formel (I) gemäß Anspruch 1 definiert ist und Z eine Vorstufe zum Rest G-L bedeutet,
**dadurch gekennzeichnet, daß** man eine Verbindung der Formel (VII) mit einer Verbindung der Formel (VIII), 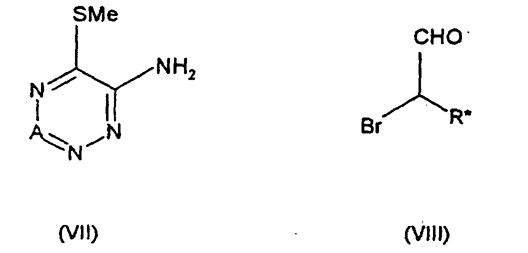 wobei in den Formeln (VII) und (VIII) A und R* wie in Formel (VI) definiert sind,
kondensiert und cyclisiert.

12. Verbindungen der Formel (VI) gemäß Anspruch 11.

13. Herbizides oder pflanzenwachstumsregulierendes Mittel, **dadurch gekennzeichnet, daß** es eine oder mehrere Verbindungen der Formel (I), deren Salze, deren Tautomere oder deren Wasseradditionsprodukte nach einem der Ansprüche 1 bis 7 und im Pflanzenschutz übliche Formulierungshilfsmittel enthält.

14. Verfahren zur Bekämpfung von Schadpflanzen oder zur Wachstumsregulierung von Pflanzen, **dadurch gekennzeichnet, daß** man eine wirksame Menge von einer oder mehreren Verbindungen der Formel (I), deren Salzen, deren Tautomeren oder deren Wasseradditionsprodukten nach einem der Ansprüche 1 bis 7 auf die Pflanzen, Pflanzenteile, Pflanzensamen oder die Anbaufläche appliziert.

15. Verwendung von Verbindungen der Formel (I), deren Salzen, deren Tautomeren oder deren Wasseradditionsprodukten nach einem der Ansprüche 1 bis 7 als Herbizide und Pflanzenwachstumsregulatoren.

16. Verwendung nach Anspruch 15. **dadurch gekennzeichnet, daß** die Verbindungen der Formel (I), deren Salze, deren Tautomere oder deren Wasseradditionsprodukte zur Bekämpfung von Schadpflanzen oder zur Wachstumsregulierung in Kulturen von Nutz- oder Zierpflanzen eingesetzt werden.

17. Verwendung nach Anspruch 16, **dadurch gekennzeichnet, daß** die Kulturpflanzen transgene Kulturpflanzen sind.

18. Arzneimittel, **gekennzeichnet durch** einen Gehalt an einer Verbindung der Formel (I), deren Salzen, deren Tautomeren oder deren Wasseradditionsprodukten gemäß Anspruch 7.

19. Arzneimittel gemäß Anspruch 18 zur Behandlung von Krankheiten, welche durch Inhibierung des Enzms AMPDA oder ADA behandelt werden können.

20. Arzneimittel gemäß Anspruch 19 zur Behandlung von Krankheiten des Formenkreises der Durchblutungsstörungen oder der Sauerstoffmangelversorgung.

21. Verfahren zur Herstellung eines pharmazeutischen Mittels zur Behandlung von Krankheiten, welche durch Inhibierung des Enzyms AMPDA oder ADA behandelt werden können, **dadurch gekennzeichnet, dass** man eine Verbindung der Formel (I) deren Salzen, deren Tautomeren oder deren Wasseradditionsprodukten gemäß Anspruch 7 als Inhibitor zusammen mit Formulierungshilfsmitteln zu dem Mittel formuliert.

22. Verfahren zur Herstellung eines pharmazeutischen Mittels zur Behandlung von Krankheiten aus der Gruppe der
- Durchblutungsstörungen,
- Sauerstoffmangelversorgungen,
- Herzinfarkt,
- Angina pectoris,
- Herzkreislauferkrankungen,
- Schmerzerkrankungen und
- Alzheimer-Erkrankungen,
**dadurch gekennzeichnet, dass** man eine Verbindung der Formel (I), worin A = CH, D = C, E = NH und G-L = β-D-Ribofuranosyl bedeuten, deren Salzen, deren Tautomeren oder deren Wasseradditionsprodukten zusammen mit Formulierungshilfsmitteln zu dem Mittel formuliert.

23. Verfahren nach Anspruch 22, **dadurch gekennzeichnet, dass** die Krankheiten aus der Gruppe der Durchblutungsstörungen oder Sauerstoffmangelversorgungen sind.

## Claims

1. The use of compounds of the formula (I), their tautomers, their salts and their water addition products, 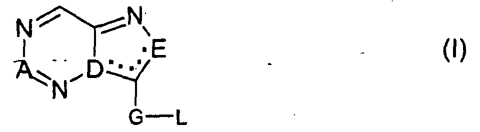 where in formula (I)
A is a nitrogen atom or a group of the formula C-R, where R is as defined further below,
D is a carbon atom or a nitrogen atom,
E
a) in the case that D is a nitrogen atom, is a nitrogen atom or a group of the formula C-R^{O}, where R^{O} is as defined further below, or
b) in the case that D is a carbon atom, is a group of the formula N-R^{O}, -O-, -S-, -SO- or -SO₂-,
the line of dots (•••••) from D via an adjacent ring carbon atom to E
is a double bond between the ring carbon atom and E if D is a nitrogen atom (case a), or
is a double bond between the ring carbon atom and D if D is a carbon atom (case b),
R, R^{O} independently of one another are each a hydrogen atom, amino, hydroxyl, mercapto, cyano, halogen, azido, nitro, SF₅, unsubstituted or substituted aminosulfonyl, acyl, acylamino, acyloxy, acylthio, mono- or di(C₁-C₄)alkylamino, mono- or di(C₃-C₉)cycloalkylamino, (C₁-C₄)alkylthio, (C₂-C₄)alkenylthio, (C₂-C₄)alkynylthio, (C₃-C₉)cycloalkylthio, (C₅-C₉)cycloalkenylthio, (C₁-C₄)alkylsulfinyl, (C₁-C₄)alkylsulfonyl, (C₁-C₄)alkoxy, (C₂-C₄)alkenyloxy, (C₂-C₄)alkynyloxy, (C₃-C₉)cycloalkoxy, (C₅-C₉)cycloalkenyloxy, (C₁-C₄)alkyl, (C₂-C₄)alkenyl, (C₂-C₄)alkynyl, (C₃-C₉)cycloalkyl, (C₅-C₉)cycloalkenyl, (C₁-C₄)alkylaminosulfonyl or di[(C₁-C₄)alkyl]-aminosulfonyl, where each of the 23 last-mentioned radicals is unsubstituted or substituted in the hydrocarbon moiety by one or more radicals selected from the group consisting of halogen, hydroxyl, amino, nitro, formyl, carboxy, cyano, thiocyanato, (C₁-C₄)alkoxy, (C₃-C₉)cycloalkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, (C₃-C₉)cycloalkylamino, [(C₁-C₄)alkyl]carbonyl, [(C₁-C₄)alkoxy]carbonyl, aminocarbonyl, mono(C₁-C₄)alkylaminocarbonyl and di(C₁-C₄)alkylaminocarbonyl,
G is a divalent straight-chain saturated or unsaturated hydrocarbon bridge having 1 to 24 carbon atoms in the chain, in which one or more chain members, in each case independently of one another, can be replaced by O, S, NH, (C₁-C₄)alkyl-N or acyl-N or, in the unsaturated case, one or more CH groups can in each case be replaced by a nitrogen atom,
where the bridge in question is unsubstituted or
(a) substituted by one or more identical or different radicals selected from the group consisting of halogen, nitro, radicals of the formula R which are different from hydrogen, radicals of the formula R²R³C= and radicals of the formula L*, where R¹, R², R³ and L* are as defined further below,
(b) carries two or four substituents, of which in each case two together with the linking bridge moiety form a carbocyclic or heterocyclic ring having 3 to 7 ring atoms, where in the case of a heterocycle the heteroatoms, preferably 1, 2 or 3 heteroatoms, are selected from the group consisting of N, O and S and where the ring in question may also have fused-on rings and is otherwise unsubstituted or substituted by one or more identical or different radicals selected from the group consisting of halogen, nitro, radicals of the formula R¹ which are different from hydrogen, radicals of the formula L* and oxo, where R and L* are as defined further below,
(c) is linked cyclically with L via a second direct bond or via a heteroatom selected from the group consisting of N, O and S,
(d) has two or more substituents from the above groups (a) to (c) together,
L, L* independently of one another are each OR⁴, SR⁴, CN, tetrazolo, C(OR⁵)(OR⁶)(OR⁷), -Z¹, -O-Z², -S-Z² or -NH-Z², where R⁴, R⁵, R⁶, R⁷, Z¹ and Z² are as defined further below and where L may be attached cyclically to the bridge G via a second direct bond or via a heteroatom selected from the group consisting of N, O and S,
Z¹, Z² independently of one another are each the radical of an inorganic or organic oxygen acid of the formula Z¹-OH or Z²-OH, where the radical is formally formed by removing the hydroxyl group from the acid function,
R¹ to R⁷ independently of one another are each a hydrogen atom, (C₁-C₆)alkyl, (C₂-C₆)alkenyl, (C₂-C₆)alkynyl, (C₃-C₉)cycloalkyl, (C₅-C₉)cycloalkenyl, aryl or heterocyclyl, where each of the last-mentioned radicals is unsubstituted or substituted by one or more radicals selected from the group consisting of amino, hydroxyl, mercapto, cyano, halogen, azido, nitro, SF₅, aminosulfonyl, acyl, acylamino, acyloxy, acylthio, [(C₁-C₄)alkoxy]carbonyl, mono(C₁-C₄)alkylamino, mono(C₃-C₉)cycloalkylamino, di(C₁-C₄)-alkylamino, (C₁-C₄)alkylthio, (C₂-C₄)alkenylthio, (C₂-C₄)alkynylthio, (C₃-C₉)cycloalkylthio, (C₅-C₉)cycloalkenylthio, (C₁-C₄)alkylsulfinyl, (C₁-C₄)alkylsulfonyl, (C₁-C₄)alkoxy, (C₂-C₄)alkenyloxy, (C₂-C₄)alkynyloxy, (C₃-C₉)cycloalkoxy, (C₅-C₉)cycloalkenyloxy, (C₃-C₉)cycloalkyl, (C₅-C₉)cycloalkenyl, aryl, substituted aryl, heteroaryl, substituted heteroaryl and, in the case of cyclic radicals, also by (C₁-C₄)alkyl, (C₂-C₄)alkenyl, (C₂-C₄)alkynyl, (C₁-C₄)haloalkyl, (C₂-C₄)haloalkenyl, (C₂-C₄)haloalkynyl, (C₁-C₄)hydroxyalkyl and (C₁-C₄)alkoxy(C₁-C₄)alkyl,
where heterocyclyl is a heterocyclic saturated, unsaturated or heteroaromatic ring and
where heteroaryl is a heteroaromatic ring
or
R², R³ together with the carbon atom of the group R²R³C= are a non-aromatic carbocyclic ring or a heterocyclic ring having 3 to 9 ring atoms and 1 to 4 heteroring atoms selected from the group consisting of N, O and S, which ring is unsubstituted or substituted by one or more radicals selected from the group consisting of halogen, nitro, hydroxyl, oxo, (C₁-C₄)alkyl, (C₁-C₄)haloalkyl, (C₁-C₄)alkoxy, (C₁-C₄)haloalkoxy and (C₁-C₄)alkylthio, or
R⁵, R⁶ together with the carbon atom and the adjacent oxygen atoms of the group C(OR⁵)(OR⁶)(OR⁷) are a saturated or unsaturated non-aromatic heterocyclic ring having 4 to 9 ring atoms and 1 to 4 heteroring atoms selected from the group consisting of N, O, P and S, which ring is unsubstituted or substituted by one or more radicals selected from the group consisting of halogen, nitro, hydroxyl, oxo, (C₁-C₄)alkyl, (C₁-C₄)haloalkyl, (C₁-C₄)alkoxy, (C₁-C₄)haloalkoxy and (C₁-C₄)alkylthio, or
the group C(OR⁵)(OR⁶)(OR⁷) together is a bicyclic radical of the formula 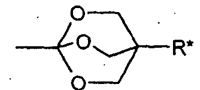 in which
R* is (C₁-C₄)alkyl, (C₁-C₄)haloalkyl, (C₁-C₄)alkoxy, (C₁-C₄)haloalkoxy, (C₁-C₄)alkylthio or phenyl which is unsubstituted or substituted by one or more radicals selected from the group consisting of halogen, nitro, hydroxyl, oxo, (C₁-C₄)alkyl, (C₁-C₄)haloalkyl, (C₁-C₄)alkoxy, (C₁-C₄)haloalkoxy and (C₁-C₄)alkylthio,
for the direct or indirect inhibition of the enzyme adenosine monophosphate deaminase (AMPDA) or adenosine deaminase (ADA), except for the use in a surgical or therapeutic treatment of the human or animal body or the use in diagnostic procedures carried out on the human or animal body.

2. The use as claimed in claim 1, wherein
A is a nitrogen atom or a group of the formula C-R in which
R is a hydrogen atom, amino, hydroxyl, mercapto, cyano, halogen, azido, nitro, SF₅, aminosulfonyl, (C₁-C₅)alkanoylamino, [(C₁-C₄)alkoxylcarbonylamino, (C₁-C₅)alkanoyl, [(C₁-C₄)-alkoxy]carbonyl, (C₁-C₅)alkanoyloxy, [(C₁-C₄)alkoxy]-carbonyloxy, mono(C₁-C₄)alkylamino, mono(C₃-C₆)cyclo-alkylamino, di(C₁-C₄)alkylamino, (C₁-C₄)alkylthio, (C₁-C₄)alkylsulfinyl, (C₁-C₄)alkylsulfonyl, (C₁-C₄)alkoxy, (C₃-C₄)alkenyloxy, (C₃-C₄)alkynyloxy, (C₃-C₆)cycloalkoxy, (C₅-C₆)cycloalkenyloxy, (C₁-C₄)alkyl, (C₂-C₄)alkenyl, (C₂-C₄)alkynyl, (C₃-C₆)cycloalkyl, (C₅-C₆)cycloalkenyl, (C₁-C₄)alkylaminosulfonyl or di[(C₁-C₄)alkyl]aminosulfonyl, where each of the 24 last-mentioned radicals is unsubstituted or substituted in the hydrocarbon moiety by one or more radicals selected from the group consisting of halogen, hydroxyl, amino, nitro, formyl, carboxyl, cyano, thiocyanato, (C₁-C₄)alkoxy, (C₃-C₆)cycloalkoxy, (C₁-C₄)haloalkoxy, (C₁-C₄)alkylthio, (C₁-C₄)haloalkylthio, mono(C₁-C₄)alkyl-amino, di(C₁-C₄)alkylamino, (C₃-C₆)cycloalkyl, (C₃-C₆)cyclo-alkylamino, [(C₁-C₄)alkyl]carbonyl, [(C₁-C₄)alkoxy]carbonyl, aminocarbonyl, mono(C₁-C₄)alkylaminocarbonyl and di(C₁-C₄)alkylaminocarbonyl.

3. The use as claimed in claim 1 or 2, wherein
G is a divalent straight-chain saturated or unsaturated hydrocarbon bridge having 1 to 8 carbon atoms in the chain, in which one or more CH₂ groups, in each case independently of one another, are replaced by O or S,
where the bridge in question is unsubstituted or
(a) substituted by one or more halogen atoms and additionally or alternatively by one or more identical or different radicals selected from the group consisting of nitro, radicals of the formula R¹ which are different from hydrogen, radicals of the formula R²R³C= and radicals of the formula L*, where R¹, R², R³ and L* are as defined above,
(b) carries two or four substituents, in each case two of which together with the linking bridge moiety form a carbocyclic ring having 3 to 6 carbon atoms or a heterocyclic saturated or partially unsaturated ring having 3 to 6 ring atoms or a heteroaromatic ring having 5 or 6 ring atoms, where in the case of a heterocycle, the 1, 2 or 3 heteroatoms are selected from the group consisting of N, O and S and where the ring in question may also have a fused-on carbocyclic ring having 4 to 6 ring atoms or a fused-on heterocyclic ring having 4 to 6 ring atoms and 1, 2 or 3 heteroatoms selected from the group consisting of N, O and S, the ring being otherwise unsubstituted or substituted by one or more halogen atoms and additionally or alternatively by one or more identical or different radicals selected from the group consisting of nitro, radicals of the formula R¹ which are different from hydrogen, radicals of the formula L* and oxo, where R¹ and L* are as defined above,
(c) has substituents from the above groups (a) and (b) together.

4. The use as claimed in any of claims 1 to 3, wherein
L, L* independently of one another are OR⁴, SR⁴, CN, tetrazolo, C(OR⁵)(OR⁶)(OR⁷), -Z¹, -O-Z², -S-Z² or -NH-Z², where R⁴, R⁵, R⁶, R⁷, Z¹ and Z² are as defined further below and where L may be attached cyclically to G via a second direct bond or via a heteroatom selected from the group consisting of N, O and S,
Z¹ is a radical of the formula COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)-(NR¹⁶R¹⁷), P(=S)(OR¹³)(NR¹⁰R¹¹) or P(=S)(NR¹⁰R¹¹)(NR¹⁶R¹⁷),
Z² is a radical of the formula COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(R¹⁵)(OR¹⁴), P(=O)(OR¹³)(NR¹⁰R¹¹), P(=O)(NR¹⁰R¹¹)-(NR¹⁶R¹⁷), P(=S)(OR¹³)(NR¹⁰R¹¹) or P(=S)(NR¹⁰R¹¹)(NR¹⁶R¹⁷),
R¹ to R¹⁷ independently of one another are each a hydrogen atom, (C₁-C₆)alkyl, (C₂-C₆)alkenyl, (C₂-C₆)alkynyl, (C₃-C₉)cycloalkyl, (C₅-C₉)cycloalkenyl, aryl or heterocyclyl, where each of the last-mentioned radicals is unsubstituted or substituted by one or more radicals selected from the group consisting of amino, hydroxyl, mercapto, cyano, halogen, azido, nitro, SF₅, aminosulfonyl, (C₁-C₄)afkanoyl, acylamino, acyloxy, acylthio, [(C₁-C₄)alkoxy]-carbonyl, mono(C₁-C₄)alkylamino, mono(C₃-C₉)cycloalkylamino, di(C₁-C₄)alkylamino, (C₁-C₄)alkylthio, (C₂-C₄)alkenylthio, (C₂-C₄)alkynylthio, (C₃-C₉)cycloalkylthio, (C₅-C₉)cycloalkenylthio, (C₁-C₄)alkylsulfinyl, (C₁-C₄)alkylsulfonyl, (C₁-C₄)alkoxy, (C₂-C₄)alkenyloxy, (C₂-C₄)alkynyloxy, (C₃-C₉)cycloalkoxy, (C₅-C₉)cycloalkenyloxy, (C₃-C₉)cycloalkyl, (C₅-C₉)cycloalkenyl, phenyl, substituted phenyl, heteroaryl, substituted heteroaryl and, in the case of cyclic radicals, also by (C₁-C₄)alkyl, (C₂-C₄)alkenyl, (C₂-C₄)alkynyl, (C₁-C₄)haloalkyl, (C₂-C₄)haloalkenyl, (C₂-C₄)halo-alkynyl, (C₁-C₄)hydroxyalkyl and (C₁-C₄)alkoxyl(C₁-C₄)alkyl,
where heterocyclyl is a heterocyclic saturated, unsaturated or heteroaromatic ring having 3 to 6 ring atoms and 1 to 3 heteroatoms selected from the group consisting of N, O and S and
where heteroaryl is a heteroaromatic ring having 5 to 6 ring atoms and 1 to 3 heteroatoms selected from the group consisting of N, O and S and
where the substituents for substituted phenyl or substituted heteroaryl are one or more radicals selected from the group consisting of halogen, nitro, (C₁-C₄)alkyl, (C₁-C₄)haloalkyl, (C₁-C₄)alkoxy, (C₁-C₄)haloalkoxy, (C₁-C₄)alkylthio, (C₁-C₄)hydroxyalkyl and (C₁-C₄)alkoxy(C₁-C₄)alkyl, or
R², R³ together with the carbon atom of the group R²R³C= are a non-aromatic carbocyclic ring or a heterocyclic ring having 3 to 6 ring atoms and 1 to 3 heteroring atoms selected from the group consisting of N, O and S, which ring is unsubstituted or substituted by one or more radicals selected from the group consisting of halogen, nitro, hydroxyl, oxo, (C₁-C₄)alkyl, (C₁-C₄)haloalkyl, (C₁-C₄)alkoxy, (C₁-C₄)haloalkoxy and (C₁-C₄)alkylthio, or
R⁵, R⁶ together with the carbon atom and the adjacent oxygen atoms of the group C(OR⁵)(OR⁶)(OR⁷) are a saturated or unsaturated non-aromatic heterocyclic ring having 3 to 6 ring atoms and 1 to 3 heteroring atoms selected from the group consisting of N, O, P and S, which ring is unsubstituted or substituted by one or more radicals selected from the group consisting of halogen, nitro, hydroxyl, oxo, (C₁-C₄)alkyl, (C₁-C₄)haloalkyl, (C₁-C₄)alkoxy, (C₁-C₄)haloalkoxy and (C₁-C₄)alkylthio, or
R⁸, R⁹ or R¹⁰, R¹¹ or R¹³, R¹⁴ or R¹⁴, R¹⁵ or R¹⁶, R¹⁷ in each case as a pair and with the atoms of the group defined in each case are a saturated or unsaturated non-aromatic heterocyclic ring having 3 to 9 ring atoms and 1 to 4 heteroring atoms selected from the group consisting of N, O, P and S, which ring is unsubstituted or substituted by one or more radicals selected from the group consisting of halogen, nitro, (C₁-C₄)alkyl, (C₁-C₄)haloalkyl, (C₁-C₄)alkoxy, (C₁-C₄)haloalkoxy and (C₁-C₄)alkylthio.

5. The use as claimed in any of claims 1 to 4, wherein
G is a divalent straight-chain saturated or unsaturated hydrocarbon bridge having 1 to 8 carbon atoms in the chain in which one or more CH₂ groups, in each case independently of one another, are replaced by O or S,
or
is a bridge of the formula -W¹-cycle-W²-, in which
W¹, W² independently of one another are a direct bond, CH₂, CH₂CH₂, OCH₂, SCH₂, CH₂CH₂CH₂, CH₂OCH₂, CH₂SCH₂, OCH₂CH₂ or SCH₂CH₂ and
"cycle" is 1,4-cyclohexylene, 1,2-phenylene, 1,3-phenylene, 1,4-phenylene, 1,2-naphthylene, 1,3-naphthylene, 1,4-naphthylene, 1,2-tetrahydronaphthylene, 1,3-tetrahydronaphthylene, 1,4-tetra-hydronaphthylene, 1,2-cyclopentylene, 1,3-cyclopentylene, 1,2-cyclohexylene, 1,3-cyclohexylene, 1,4-cyclohexylene, tetrahydrofuran-2,5-diyl (oxolane), tetrahydrothiophene-2,5-diyl, 2,5-dihydrofuran-2,5-diyl or 2,5-dihydrothiophene-2,5-diyl,
where the bridge in question is unsubstituted or
substituted by one or more halogen atoms and additionally or alternatively by one or more identical or different radicals selected from the group consisting of hydrogen, various radicals of the formula R¹, radicals of the formula R²R³C= and radicals of the formula L*, where R¹, R², R³ and L* are as defined above or further below, or
is additionally or alternatively attached cyclically to L via a second direct bond or via a heteroatom selected from the group consisting of N, O and S, and
R¹ to R¹⁷ indendently of one another are each a hydrogen atom, (C₁-C₄)alkyl, (C₂-C₄)alkenyl, (C₂-C₄)alkynyl, (C₃-C₆)cycloalkyl, (C₅-C₆)cycloalkenyl, phenyl or heterocyclyl, where each of the last-mentioned radicals is unsubstituted or substituted by one or more radicals selected from the group consisting of amino, hydroxyl, mercapto, cyano, halogen, azido, nitro, SF₅, aminosulfonyl, (C₁-C₄)alkanoyl, (C₁-C₄)alkanoylamino, benzoylamino, (C₁-C₄)alkanoyloxy, (C₁-C₄)alkanoylthio, [(C₁-C₄)alkoxy]carbonyl, mono(C₁-C₄)alkylamino, di(C₁-C₄)alkylamino, (C₁-C₄)alkylthio, (C₃-C₄)alkenylthio, (C₃-C₄)alkynylthio, (C₁-C₄)alkylsulfinyl, (C₁-C₄)alkylsulfonyl, (C₁-C₄)alkoxy, (C₃-C₄)alkenyloxy, (C₃-C₄)alkynyloxy, (C₃-C₉)cycloalkoxy, (C₃-C₉)cycloalkyl, phenyl, substituted phenyl, heteroaryl, substituted heteroaryl and, in the case of cyclic radicals, also by (C₁-C₄)alkyl, (C₂-C₄)alkenyl, (C₂-C₄)alkynyl, (C₁-C₄)haloalkyl, (C₂-C₄)haloalkenyl, (C₂-C₄)halo-alkynyl, (C₁-C₄)hydroxyalkyl and (C₁-C₄)alkoxyl(C₁-C₄)alkyl,
where heterocyclyl is a heterocyclic saturated or unsaturated ring having 3 to 6 ring atoms or a heteroaromatic ring having 5 or 6 ring atoms and in each case 1 to 3 heteroatoms selected from the group consisting of N, O and S and
where heteroaryl is a heteroaromatic ring having 5 to 6 ring atoms and 1 to 3 heteroatoms selected from the group consisting of N, O and S and
where the substituents for substituted phenyl or substituted heteroaryl are one or more substituents selected from the group consisting of halogen, nitro, (C₁-C₄)alkyl, (C₁-C₄)haloalkyl, (C₁-C₄)alkoxy, (C₁-C₄)haloalkoxy, (C₁-C₄)alkylthio, (C₁-C₄)hydroxy-alkyl and (C₁-C₄)alkoxy(C₁-C₄)alkyl.

6. The use as claimed in any of claims 1 to 5, wherein
L is hydroxyl, carboxyl, [(C₁-C₄)alkoxy]carbonyl, CONH₂, [(C₁-C₄)alkylamino]carbonyl, [(C₁-C₄)alkylsulfonylamino]carbonyl, [(C₁-C₄)haloalkylsulfonylamino]carbonyl, [cyano(C₁-C₄)alkylsulfonyl-amino]carbonyl, (C₁-C₄)alkylsulfonylamino, (C₁-C₄)haloalkyl-sulfonylamino, cyano-(C₁-C₄)alkylsulfonylamino, (C₁-C₅)alkanoyloxy, benzoyloxy, [(C₁-C₄)alkoxy]carbonyloxy, [(C₁-C₄)alkylamino]carbonyloxy, (C₁-C₄)alkoxy, (C₁-C₄)alkylthio, (C₁-C₄)hydroxyalkoxy, SO₂NHCONH₂, (C₁-C₅)alkanoylamino-sulfonyl, [(C₁-C₄)haloalkyl]carbonylaminosulfonyl, [(C₁-C₄)alkoxy]-carbonylaminosulfonyl, [(C₁-C₅)haloalkoxy]carbonylaminosulfonyl, SO₂NH₂, di[(C₁-C₄)alkyl]aminosulfonyl, P(=O)(OH)₂, P(=S)(OH)₂, P(=O)(OR')₂ or P(=O)(OH)(OR'), where in the two last-mentioned formulae R', in each case independently of any other radicals R', is (C₁-C₄)alkyl, (C₁-C₄)haloalkyl, (C₁-C₄)hydroxyalkyl, (C₁-C₄)alkanoyl(C₁-C₄)alkyl, (C₁-C₄)alkanoyloxy(C₁-C₄)alkyl or phenyl.

7. A compound of the formula (I), its tautomers, its salts and its water addition products as defined in any of claims 1 to 6, except for the compound of the formula (I) in which A = CH, D = C, E = NH and G-L = β-D-ribofuranosyl.

8. A process for preparing the compounds of the formula (I) or salts thereof as claimed in any of claims 1 to 6, which comprises
a) reducing a compound of the formula (II) 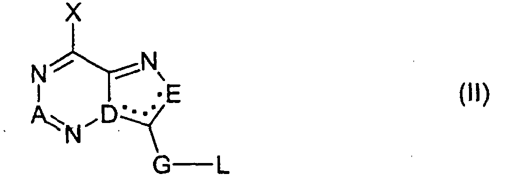 in which Xis a leaving group to the compound of the formula (I) or
b) reducing a compound of the formula (III) 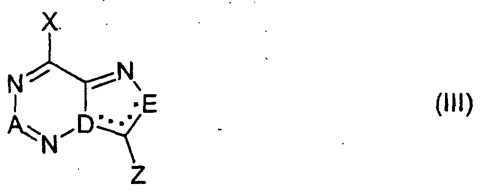 in which X is a leaving group and Z is a precursor of the radical G-L to the compound of the formula (III') 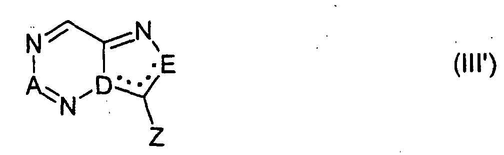 in which Z is as defined in formula (III), and then modifying the compound (III) at the group Z such that the compound (I) is obtained,
c) modifying a compound of the formula (III') in which Z is a precursor of the radical G-L at the group Z such that the compound (I) is obtained, or
d) if A is a group of the formula C-R, cyclizing a compound of the formula (III") 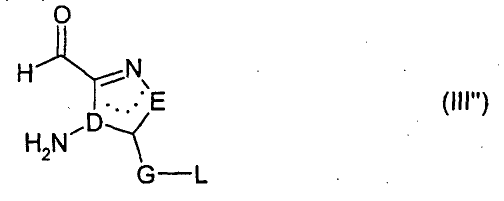 with a compound of the formula (III''')
H₂N - A = NH (III''')
in which A is a group C-R to give the compound of the formula (I),
where the symbols A, D, E, G, L and R in the formulae (II), (III), (III'), (III") and (III''') are as defined in formula (I), unless specifically defined otherwise.

9. A process for preparing a compound of the formula (V) 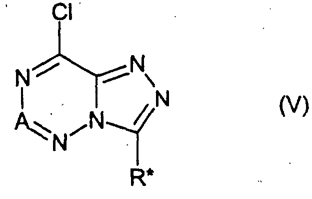 in which R* = Z or G-L and A, G and L are as defined in formula (I) according to claim 1 and Z is a precursor of the radical G-L, which comprises reacting a compound of the formula (IV) 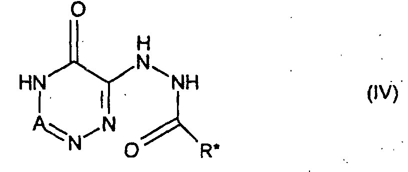 in which A and R* are as defined in formula (V)
with a chlorinating agent and cyclizing it to give the compound of the formula (V).

10. A compound of the formula (V) as set forth in claim 9.

11. A process for preparing a compound of the formula (VI) 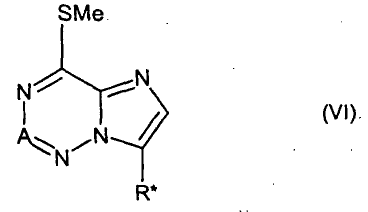 in which R* = Z or G-L and A, G and L are as defined in formula (I) according to claim 1 and Z is a precursor of the radical G-L,
which comprises condensing and cyclizing a compound of the formula (VII) with a compound of the formula (VIII) 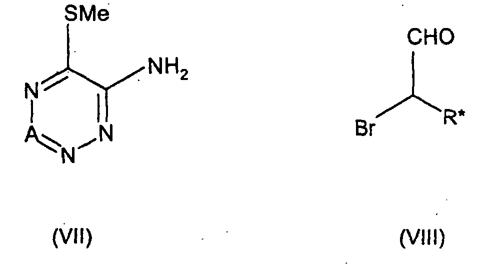 where A and R* in the formulae (VII) and (VIII) are as defined in formula (VI).

12. A compound of the formula (VI) as set forth in claim 11.

13. A herbicidal or plant-growth-regulating composition, comprising one or more compounds of the formula (I), their salts, their tautomers or their water addition products as set forth in any of claims 1 to 7 and formulation auxiliaries which are customary in crop protection.

14. A method for controlling harmful plants or for regulating the growth of plants, which comprises applying an effective amount of one or more compounds of the formula (I), their salts, their tautomers or their water addition products as set forth in any of claims 1 to 7 onto the plants, parts of plants, plant seeds or the area under cultivation.

15. The use of compounds of the formula (I), their salts, their tautomers or their water addition products as set forth in any of claims 1 to 7 as herbicides and plant growth regulators.

16. The use as claimed in claim 15, wherein the compounds of the formula (I), their salts, their tautomers or their water addition products are employed for controlling harmful plants or for regulating the growth in crops of useful or ornamental plants.

17. The use as claimed in claim 16, wherein the crop plants are transgenic crop plants.

18. A pharmaceutical, comprising a compound of the formula (I), its salts, its tautomers or its water addition products as set forth in claim 7.

19. The pharmaceutical as claimed in claim 18 for treating diseases which can be treated by inhibiting the enzyme AMPDA or ADA.

20. The pharmaceutical as claimed in claim 19 for treating diseases of the type circulatory disorders or oxygen deficiencies.

21. A process for preparing a pharmaceutical composition for treating diseases which can be treated by inhibiting the enzyme AMPDA or ADA, which comprises formulating a compound of the formula (I), its salts, its tautomers or its water addition products as set forth in claim 7 as inhibitor together with formulation auxiliaries to give the composition.

22. A process for preparing a pharmaceutical composition for treating disorders from the group of the
- blood flow disturbances,
- oxygen deficits,
- myocardial infarction,
- angina pectoris,
- cardiovascular diseases,
- diseases associated with pain and
- Alzheimer's diseases,
which comprises formulating a compound of the formula (I) in which A = CH, D = C, E = NH and G-L = β-D-ribofuranosyl, its salts, its tautomers or its water addition products together with formulation auxiliaries to give the composition.

23. The process as claimed in claim 22, wherein the diseases are from the group of the blood flow disturbances or the oxygen deficits.

## Revendications

1. Utilisation de composés de formule (I), de leurs tautomères, de leurs sels et de leurs produits d'addition d'eau 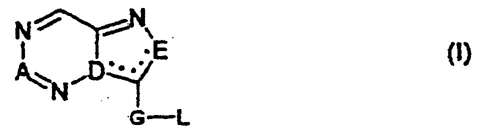 dans la formule (I)
A représente un atome d'azote ou un groupe de formule C-R, R étant défini ci-après,
D représente un atome de carbone ou un atome d'azote,
E
a) dans le cas où D est un atome d'azote, représente un atome d'azote ou un groupe de formule C-R^{o}, R^{o} étant défini ci-dessous, ou
b) dans le cas où D est un atome de carbone, représente un groupe de formule N-R^{o}, -O-, -S-, -SO- ou -SO₂,
la succession des points (•••••) allant de D en passant par un atome de carbone cyclique adjacent à E, représente une double liaison entre l'atome de carbone cyclique et E, lorsque D représente un atome d'azote (cas a), ou une double liaison entre l'atome de carbone cyclique et D, lorsque D représente un atome de carbone (cas b),
R, R° indépendamment l'un de l'autre représentent chacun un atome d'hydrogène, un groupe amino, hydroxy, mercapto, cyano, halogène, azido, nitro, SF₅, aminosulfonyle, acyle, acylamino, acyloxy, acylthio, mono- ou di-(alkyl en C₁-C₄)-amino, mono- ou di-cyclo(alkyl en C₃-C₉)-amino, (alkyl en C₁-C₄) thio, (alcényl en C₂-C₄)thio, (alcynyl en C₂-C₄)thio, cyclo (alkyl en C₃-C₉)thio, cyclo(alcényl en C₅-C₉)thio, (alkyl en C₁-C₄)-sulfinyle, (alkyl en C₁-C₄)sulfonyle, alkoxy en C₁-C₄, (alcényl en C₂-C₄)oxy, (alcynyl en C₂-C₄)oxy, cycloalkoxy en C₃-C₉, cyclo(alcényl en C₅-C₉)oxy, alkyle en C₁-C₄, alcényle en C₂-C₄, alcynyle en C₂-C₄, cycloalkyle en C₃-C₉, cycloalcényle en C₅-C₉, (alkyl en C₁-C₄)-amino-sulfonyle ou di(alkyl en C₁-C₄)-aminosulfonyle éventuellement substitué, chacun des 23 derniers restes cités est non substitué ou substitué dans le fragment hydrocarboné par un ou plusieurs restes pris dans le groupe comprenant halogène, hydroxy, amino, nitro, formyle, carboxy, cyano, thiocyanato, alkoxy en C₁-C₄, cycloalkoxy en C₃-C₉, haloalkoxy en C₁-C₄, (alkyl en C₁-C₄)thio, halo(alkyl en C₁-C₄)thio, mono(alkyl en C₁-C₄)amino, di(alkyl en C₁-C₄)-amino, cycloalkyle en C₃-C₉, cyclo(alkyl en C₃-C₉) -amino, (alkyl en C₁-C₄)carbonyle, (alkoxy en C₁-C₄)carbonyle, aminocarbonyle, mono(alkyl en C₁-C₄)amino-carbonyle et di(alkyl en C₁-C₄)-amino-carbonyle,
G représente une liaison hydrocarbonée bivalente à chaîne droite, saturée ou insaturée présentant 1 à 24 atomes de carbone dans la chaîne, dans laquelle un ou plusieurs chaînons à chaque fois indépendamment peuvent être échangés contre O, S, NH, (alkyl en C₁-C₄)-N ou acyl-N ou, dans le cas insaturé, un ou plusieurs groupes CH pouvant à chaque fois être échangés contre un atome d'azote,
la liaison concernée étant non substituée ou
(a) substituée par un ou plusieurs restes identiques ou différents pris dans le groupe comprenant halogène, nitro, des restes de formule R¹ différents de l'hydrogène, des restes de formule R²R³C= et des restes de formule L*, R¹, R², R³ et L* étant définis ci-dessous,
(b) porte deux ou quatre substituants, dont à chaque fois deux conjointement forment avec le fragment de liaison qui les relie un cycle carbocyclique ou hétérocyclique présentant 3 à 7 chaînons, dans le cas d'un hétérocycle les hétéroatomes, de préférence 1, 2 ou 3 hétéroatomes, sont pris dans le groupe comprenant N, O et S et le cycle concerné peut présenter encore des cycles condensés et par ailleurs est non substitué ou substitué par un ou plusieurs restes identiques ou différents pris dans le groupe comprenant halogène, nitro, des restes de formule R¹ différents de l'hydrogène, des restes de formule L* et oxo, R¹ et L* sont définis ci-dessus,
(c) est lié à L de manière cyclique par une seconde liaison directe ou par un hétéroatome pris dans le groupe comprenant N, O et S,
(d) présente conjointement deux ou plusieurs substituants pris dans les groupes précédents (a) à (c)
L, L* indépendamment l'un de l'autre représentent chacun un groupe OR⁴, SR⁴, CN, tétrazolo, C(OR⁵)(OR⁶)(OR⁷), -Z¹, -O-Z², -S-Z² ou -NH-Z², où R⁴, R⁵, R⁶, R⁷, Z¹ et Z² sont définis ci-dessous et L peut être lié de manière cyclique par une seconde liaison directe ou par un hétéroatome pris dans le groupe comprenant N, O et S à la liaison G,
Z¹, Z² indépendamment l'un de l'autre représentent chacun le reste d'un acide oxygéné organique ou inorganique de formule Z¹-OH ou Z²-OH, le reste résulte selon formule par séparation du groupe hydroxy sur la fonction acide,
R¹ à R⁷ indépendamment les uns des autres représentent chacun un atome d'hydrogène, un groupe alkyle en C₁-C₆, alcényle en C₂-C₆, alcynyle en C₂-C₆, cycloalkyle en C₃-C₉, cycloalcényle en C₅-C₉, aryle ou hétérocyclyle, chacun des derniers restes cités est non substitué ou substitué par un ou plusieurs restes pris dans le groupe comprenant amino, hydroxy, mercapto, cyano, halogène, azido, nitro, SF₅, aminosulfonyle, acyle, acylamino, acyloxy, acylthio, (alkoxy en C₁-C₄)carbonyle, mono(alkyl en C₁-C₄)-amino, monocyclo(alkyl en C₃-C₉)-amino, di(alkyl en C₁-C₄) amino, (alkyl en C₁-C₄)thio, (alcényl en C₂-C₄)thio, (alcynyl en C₂-C₄)thio, cyclo(alkyl en C₃-C₉)thio, cyclo(alcényl en C₅-C₉)thio, (alkyl en C₁-C₄)-sulfinyle, (alkyl en C₁-C₄)sulfonyle, alkoxy en C₁-C₄, (alcényl en C₂-C₄)oxy, (alcynyl en C₂-C₄)-oxy, cycloalkoxy en C₃-C₉, cyclo(alcényl en C₅-C₉)-oxy, cycloalkyle en C₃-C₉, cycloalcényle en C₅-C₉, aryle, aryle substitué, hétéroaryle, hétéroaryle substitué et dans le cas de restes cycliques également alkyle en C₁-C₄, alcényle en C₂-C₄, alcynyle en C₂-C₄, haloalkyle en C₁-C₄, haloalcényle en C₂-C₄, haloalcynyle en C₂-C₄, hydroxyalkyle en C₁-C₄ et (alkoxy en C₁-C₄)-(alkyle en C₁-C₄),
hétérocyclyle est un cycle hétérocyclique saturé, insaturé ou hétéroaromatique et hétéroaryle est un cycle hétéroaromatique
ou
R², R³ conjointement avec l'atome de carbone du groupe R²R³C= forment un cycle carbocyclique non aromatique ou un cycle hétérocyclique présentant 3 à 9 chaînons et 1 à 4 hétéroatomes cycliques pris dans le groupe comprenant N, O et S, qui est non substitué ou substitué par un ou plusieurs restes pris dans le groupe comprenant halogène, nitro, hydroxy, oxo, alkyle en C₁-C₄, haloalkyle en C₁-C₄, alkoxy en C₁-C₄, haloalkoxy en C₁-C₄ et (alkyl en C₁-C₄)-thio, ou
R⁵, R⁶ conjointement avec l'atome de carbone et les atomes d'oxygène adjacents du groupe C(OR⁵)(OR⁶)(OR⁷) forment un cycle hétérocyclique non aromatique saturé ou insaturé comprenant 4 à 9 chaînons et 1 à 4 hétéroatomes cycliques pris dans le groupe comprenant N, O, P et S, qui est non substitué ou substitué par un ou plusieurs restes pris dans le groupe comprenant halogène, nitro, hydroxy, oxo, alkyle en C₁-C₄, haloalkyle en C₁-C₄, alkoxy en C₁-C₄, haloalkoxy en C₁-C₄ et (alkyl en C₁-C₄)thio, ou le groupe C(OR⁵)(OR⁶)(OR⁷) conjointement un reste bicyclique de formule 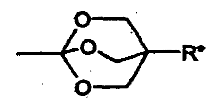
où
R* représente un groupe alkyle en C₁-C₄, haloalkyle en C₁-C₄, alkoxy en C₁-C₄, haloalkoxy en C₁-C₄, (alkyl en C₁-C₄)thio et phényle, qui est non substitué ou substitué par un ou plusieurs restes pris dans le groupe comprenant halogène, nitro, hydroxy, oxo, alkyle en C₁-C₄, haloalkyle en C₁-C₄, alkoxy en C₁-C₄, haloalkoxy en C₁-C₄ et (alkyl en C₁-C₄)thio,
dans le but d'inhiber directement ou indirectement les enzymes adénosine monophosphate déaminase (AMPDA) ou adénosine déaminase (ADA), à l'exception de l'utilisation dans des traitements en chirurgie ou des traitements thérapeutiques du corps humain ou animal, ou pour l'utilisation dans des procédés diagnostiques effectués sur le corps humain ou animal.

2. Utilisation selon la revendication 1, **caractérisé en ce que**
A représente un atome d'azote ou un groupe de formule C-R, dans laquelle
R représente un atome d'hydrogène, un groupe amino, hydroxy, mercapto, cyano, halogène, azido, nitro, SF₅, aminosulfonyle, (alcanoyl en C₁-C₅)-amino, (alkoxy en C₁-C₄)-carbonylamino, alcanoyle en C₁-C₅, (alkoxy en C₁-C₄)carbonyle, (alcanoyl en C₁-C₅)oxy, (alkoxy en C₁-C₄)carbonyloxy, mono-(alkyl en C₁-C₄)-amino, mono-cyclo(alkyl en C₃-C₆)-amino, di(alkyl en C₁-C₄)-amino, (alkyl en C₁-C₄)thio, (alkyl en C₁-C₄)sulfinyle, (alkyl en C₁-C₄)-sulfonyle, alkoxy en C₁-C₄, (alcényl en C₃-C₄)-oxy, (alcynyl en C₃-C₄)oxy, cycloalkoxy en C₃-C₆, cyclo(alcényl en C₅-C₆)-oxy, alkyle en C₁-C₄, alcényle en C₂-C₄, alcynyle en C₂-C₄, cycloalkyle en C₃-C₆, cycloalcényle en C₅-C₆, (alkyl en C₁-C₄)-aminosulfonyle ou di(alkyl en C₁-C₄)-aminosulfonyle, chacun des derniers 24 restes cités pouvant être non substitué ou substitué dans le fragment hydrocarboné par un ou plusieurs restes pris dans le groupe comprenant halogène, hydroxy, amino, nitro, formyle, carboxy, cyano, thiocyanato, alkoxy en C₁-C₄, cycloalkoxy en C₃-C₆, haloalkoxy en C₁-C₄, (alkyl en C₁-C₄)thio, halo(alkyl en C₁-C₄)thio, mono (alkyl en C₁-C₄) amino, di(alkyl en C₁-C₄)-amino, cycloalkyle en C₃-C₆, cyclo (alkyl en C₃-C₆) amino, (alkyl en C₁-C₄)-carbonyle, (alkoxy en C₁-C₄)-carbonyle, aminocarbonyle, mono(alkyl en C₁-C₄) -aminocarbonyle et di (alkyl en C₁-C₄)-aminocarbonyle.

3. Utilisation selon la revendication 1 ou 2, **caractérisée en ce que**
G représente une liaison hydrocarbonée bivalente à chaîne droite, saturée ou insaturée, présentant 1 à 8 atomes de carbone dans la chaîne, dans laquelle un ou plusieurs groupes -CH₂- à chaque fois indépendamment les uns des autres sont échangés contre O ou S, la liaison concernée étant non substituée ou
(a) substituée par un ou plusieurs atomes d'halogène ou, de plus ou alternativement, un ou plusieurs restes identiques ou différents pris dans le groupe comprenant nitro, des restes de formule R¹ différents de l'hydrogène, des restes de formule R²R³C= et des restes de formule L*, R¹, R², R³ et L* ayant les significations définies,
(b) porte deux ou quatre substituants, dont à chaque fois deux conjointement forment avec le fragment de liaison qui les relie un cycle carbocyclique de 3 à 6 atomes de carbone ou un cycle hétérocyclique saturé ou partiellement insaturé présentant 3 à 6 chaînons ou forment un cycle hétéro-aromatique présentant 5 à 6 chaînons, dans le cas d'un hétérocycle les 1, 2 ou 3 hétéroatomes sont pris dans le groupe comprenant N, O et S, et le cycle concerné peut porter.encore un cycle carbocyclique condensé présentant 4 à 6 chaînons ou un cycle hétérocyclique condensé présentant 1, 2 ou 3 hétéroatomes pris dans le groupe comprenant N, O et S, et par ailleurs est non substitué ou substitué par un ou plusieurs atomes d'halogène et de plus ou alternativement par un ou plusieurs restes identiques ou différents pris dans le groupe comprenant nitro, des restes de formule R¹ différents de l'hydrogène, des restes de formule L* et oxo, R¹ et L* possédant les significations définies,
(c) présente conjointement des substituants pris dans les groupes précédents (a) et (b).

4. Utilisation selon l'une quelconque des revendications 1 à 3, **caractérisée en ce que**
L, L* indépendamment l'un de l'autre représentent chacun un groupe OR⁴, SR⁴, CN, tétrazolo, C(OR⁵)(OR⁶)(OR⁷), -Z¹, -O-Z², -S-Z² ou -NH-Z², où R⁴, R⁵, R⁶, R⁷, Z¹ et Z² sont définis ci-dessous et L peut être lié à la liaison G de manière cyclique par une seconde liaison directe ou par un hétéroatome pris dans le groupe comprenant N, O et S,
Z¹ représente le reste de formule COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(OR¹⁵)(OR¹⁴), P(=O)(OR¹³)(NR¹⁰R¹¹), P(=O)(NR¹⁰R¹¹)(NR¹⁶R¹⁷), P(=S)(OR¹³)(NR¹⁰R¹¹) ou P(=S)(NR¹⁰R¹¹)(NR¹⁶R¹⁷),
Z² représente le reste de formule COOR⁸, CS-OR⁸, CO-SR⁸, CS-SR⁸, CO-NR⁹-SO₂-R⁸, CO-NR¹⁰R¹¹, CS-NR¹⁰R¹¹, CO-R¹², CS-R¹², SO-R¹², SO₂R¹², SO₃R⁸, SO₂NR¹⁰R¹¹, SO₂NR⁹COR¹², SO₂NR⁹COOR¹², P(=O)(OR¹³)(OR¹⁴), P(=S)(OR¹³)(OR¹⁴), P(=O)(OR¹⁵)(OR¹⁴), P(=O)(OR¹³)(NR¹⁰R¹¹), P(=O)(NR¹⁰R¹¹)(NR¹⁶R¹⁷), P(=S)(OR¹³)(NP¹⁰R¹¹) ou P (=S)(NR¹⁰R¹¹)(NR¹⁶R¹⁷),
R¹ à R¹⁷ indépendamment les uns des autres représentent chacun un atome d'hydrogène, un groupe alkyle en C₁-C₆, alcényle en C₂-C₆, alcynyle en C₂-C₆, cycloalkyle en C₃-C₉, cycloalcényle en C₅-C₉, aryle ou hétérocyclyle, chacun des derniers restes cités est non substitué ou substitué par un ou plusieurs restes pris dans le groupe comprenant amino, hydroxy, mercapto, cyano, halogène, azido, nitro, SF₅, aminosulfonyle, alcanoyle en C₁-C₄, acylamino, acyloxy, acylthio, (alkoxy en C₁-C₄)-carbonyle, mono(alkyl en C₁-C₄)-amino, mono-cyclo (alkyl en C₃-C₉)-amino, di (alkyl en C₁-C₄)-amino, (alkyl en C₁-C₄)thio, (alcényl en C₂-C₄)-thio, (alcynyl en C₂-C₄)thio, cyclo(alkyl en C₃-C₉) thio, cyclo (alcényl en C₅-C₉)thio, (alkyl en C₁-C₄)-sulfinyle, (alkyl en C₁-C₄)sulfonyle, alkoxy en C₁-C₄, (alcényl en C₂-C₄)oxy, (alcynyl en C₂-C₄)oxy, cycloalkoxy en C₃-C₉, cyclo(alcényl en C₅-C₉)oxy, cycloalkyle en C₃-C₉, cycloalcényle en C₅-C₉, phényle, phényle substitué, hétéroaryle, hétéroaryle substitué et dans le cas de restes cycliques également alkyle en C₁-C₄, alcényle en C₂-C₄, alcynyle en C₂-C₄, haloalkyle en C₁-C₄, haloalcényle en C₂-C₄, haloalcynyle en C₂-C₄, hydroxyalkyle en C₁-C₄ et (alkoxy en C₁-C₄)-(alkyle en C₁-C₄), hétérocyclyle étant un cycle hétérocyclique saturé, insaturé ou hétéroaromatique présentant 3 à 6 chaînons et 1 à 3 hétéroatomes pris dans le groupe comprenant N, O et S, et hétéroaryle étant un cycle hétéroaromatique présentant 5 à 6 chaînons et 1 à 3 hétéroatomes pris dans le groupe comprenant N, O et S, et les substituants de phényle substitué ou d'hétéroaryle substitué sont un ou plusieurs pris dans le groupe comprenant halogène, nitro, alkyle en C₁-C₄, haloalkyle en C₁-C₄, alkoxy en C₁-C₄, haloalkoxy en C₁-C₄, (alkyl en C₁-C₄)thio, hydroxyalkyle en C₁-C₄ et (alkoxy en C₁-C₉) - (alkyle en C₁-C₄),
ou
R², R³ conjointement avec l'atome de carbone du groupe R²R³C= forment un cycle carbocyclique non aromatique ou un cycle hétérocyclique comprenant 3 à 6 chaînons et 1 à 3 hétéroatomes cycliques pris dans le groupe comprenant N, 0 et S, qui est non substitué ou substitué par un ou plusieurs restes pris dans le groupe comprenant halogène, nitro, hydroxy, oxo, alkyle en C₁-C₄, haloalkyle en C₁-C₄, alkoxy en C₁-C₄, haloalkoxy en C₁-C₄ et (alkyl en C₁-C₄)-thio, ou
R⁵, R⁶ conjointement avec l'atome de carbone et les atomes d'oxygène adjacents du groupe C(OR⁵) (OR⁶) (OR⁷) forment un cycle hétérocyclique non aromatique saturé ou insaturé présentant 3 à 6 chaînons et 1 à 3 hétéroatomes cycliques pris dans le groupe comprenant N, O, P et S, qui est non substitué ou substitué par un ou plusieurs restes pris dans le groupe comprenant halogène, nitro, hydroxy, oxo, alkyle en C₁-C₄, haloalkyle en C₁-C₄, alkoxy en C₁-C₄, haloalkoxy en C₁-C₄ et (alkyl en C₁-C₄)thio, ou
R⁸, R⁹ ou R¹⁰, R¹¹ ou R¹³, R¹⁴ ou R¹⁴, R¹⁵ ou R¹⁶, R¹⁷, à chaque fois par paires avec les atomes du groupe à chaque fois défini forment un cycle hétérocyclique non aromatique saturé ou insaturé présentant 3 à 9 chaînons et 1 à 4 hétéroatomes cycliques pris dans le groupe comprenant N, O, P et S, qui est non substitué ou substitué par un ou plusieurs restes pris dans le groupe comprenant halogène, nitro, alkyle en C₁-C₄, haloalkyle en C₁-C₄, alkoxy en C₁-C₄, haloalkoxy en C₁-C₄ et (alkyl en C₁-C₄)-thio.

5. Utilisation selon l'une quelconque des revendications 1 à 4, **caractérisée en ce que**
G représente une liaison hydrocarbonée bivalente à chaîne droite, saturée ou insaturée présentant 1 à 8 atomes de carbone dans la chaîne, dans laquelle un ou plusieurs groupes -CH₂- à chaque fois indépendamment les uns des autres sont échangés contre O ou S,
ou
une liaison de formule -W¹-cycle-W²-, où
W¹, W² représentent indépendamment l'un de l'autre une liaison directe, un groupe CH₂, CH₂CH₂, OCH₂, SCH₂, CH₂CH₂CH₂, CH₂OCH₂, CH₂SCH₂, OCH₂CH₂ ou SCH₂CH₂ et
"cycle" représente 1,4-cyclohexylène, 1,2-phénylène, 1,3-phénylène, 1,4-phénylène, 1,2-naphtylène, 1,3-naphtylène, 1,4-naphtylène, 1,2-tétrahydro-naphtylène, 1,3-tétrahydronaphtylène, 1,4-tétrahydronaphtylène, 1,2-cyclopentylène, 1,3-cyclopentylène, 1,2-cyclohexylène, 1,3-cyclohexylène, 1,4-cyclohexylène, tétrahydro-furanne-2,5-diyle (oxolane), tétrahydro-thiophène-2,5-diyle, 2,5-dihydrofuran-2,5-diyle ou 2,5-dihydrothiophène-2,5-diyle, la liaison concernée est non substituée ou substituée par un ou plusieurs atomes d'halogène et de plus ou alternativement par un ou plusieurs restes identiques ou différents pris dans le groupe comprenant un atome d'hydrogène, différents restes de formule R¹, des restes de formule R²R³C= et des restes de formule L*, R¹, R², R³ et L* étant définis comme ci-dessus ou comme ci-dessous, ou de plus ou alternativement est lié à L de manière cyclique par une seconde liaison directe ou par un hétéroatome pris dans le groupe comprenant N, O et S,
R¹ à R¹⁷ indépendamment les uns des autres représentent chacun un atome d'hydrogène, un groupe alkyle en C₁-C₄, alcényle en C₂-C₉, alcynyle en C₂-C₄, cycloalkyle en C₃-C₆, cycloalcényle en C₅-C₆, phényle ou hétérocyclyle, chacun des derniers restes cités est non substitué ou substitué par un ou plusieurs restes pris dans le groupe comprenant amino, hydroxy, mercapto, cyano, halogène, azido, nitro, SF₅, aminosulfonyle, alcanoyle en C₁-C₄, (alcanoyl en C₁-C₄)amino, benzoylamino, (alcanoyl en C₁-C₄)oxy, (alcanoyl en C₁-C₄)thio, (alkoxy en C₁-C₄)carbonyle, mono(alkyl en C₁-C₄)-amino, di(alkyl en C₁-C₄)amino, (alkyl en C₁-C₄)-thio, (alcényl en C₃-C₄)thio, (alcynyl en C₃-C₄)-thio, (alkyl en C₁-C₄)sulfinyle, (alkyl en C₁-C₄)-sulfonyle, alkoxy en C₁-C₄, (alcényl en C₃-C₄)oxy, (alcynyl en C₃-C₄)oxy, cycloalkoxy en C₃-C₉, cycloalkyle en C₃-C₉, phényle, phényle substitué, hétéroaryle, hétéroaryle substitué et dans le cas de restes cycliques aussi alkyle en C₁-C₄, alcényle en C₂-C₄, alcynyle en C₂-C₄, haloalkyle en C₁-C₄, haloalcényle en C₂-C₄, haloalcynyle en C₂-C₄, hydroxyalkyle en C₁-C₄ et (alkoxy en C₁-C₄) - (alkyle en C₁-C₄), hétérocyclyle est un cycle hétérocyclique saturé ou insaturé présentant 3 à 6 chaînons ou un cycle hétéroaromatique présentant 5 ou 6 chaînons et à chaque fois 1 à 3 hétéroatomes sont pris dans le groupe comprenant N, O et S, et
hétéroryle est un cycle hétéroaromatique présentant 5 ou 6 chaînons et 1 à 3 hétéroatomes pris dans le groupe comprenant N, O et S, et
les substituants de phényle substitué ou d'hétéroaryle substitué sont un ou plusieurs pris dans le groupe comprenant halogène, nitro, alkyle en C₁-C₄, haloalkyle en C₁-C₄, alkoxy en C₁-C₄, haloalkoxy en C₁-C₄, (alkyl en C₁-C₄)thio, hydroxyalkyle en C₁-C₄ et (alkoxy en C₁-C₄)-(alkyle en C₁-C₄).

6. Utilisation selon l'une quelconque des revendications 1 à 5, **caractérisée en ce que**
L représente un groupe hydroxy, carboxy, (alkoxy en C₁-C₄)carbonyle, CONH₂, [(alkyl en C₁-C₄)-amino]-carbonyle, [(alkyl en C₁-C₄)-sulfonylamino]carbonyle, [(haloalkyl en C₁-C₄)-sulfonylamino] carbonyle, [cyano(alkyl en C₁-C₄)-sulfonylamino]carbonyle, (alkyl en C₁-C₄)-sulfonylamino, (haloalkyl en C₁-C₄)-sulfonylamino, cyano (alkyl en C₁-C₄)-sulfonylamino, (alcanoyl en C₁-C₅)oxy, benzoyloxy, (alkoxy en C₁-C₄)carbonyloxy, [(alkyl en C₁-C₄)amino]carbonyloxy, alkoxy en C₁-C₄, (alkyl en C₁-C₄)thio, hydroxyalkoxy en C₁-C₄, SO₂NHCONH₂, (alcanoyl en C₁-C₅)-aminosulfonyle, (haloalkyl en C₁-C₄)-carbonylaminosulfonyle, (alkoxy en C₁-C₄)-carbonylaminosulfonyle, (haloalkoxy en C₁-C₅)-carbonylaminosulfonyle, SO₂NH₂, di(alkyl en C₁-C₄)-aminosulfonyle, P(=O)(OH)₂, P(=S)(OH)₂, P(=O)(OR')₂ ou P(=O)(OH) (OR'), dans les deux dernières formules R' représente à chaque fois indépendamment des autres restes R' un groupe alkyle en C₁-C₄, haloalkyle en C₁-C₄, hydroxyalkyle en C₁-C₄ et (alcanoyl en C₁-C₄) - (alkyle en C₁-C₄), (alcanoyl en C₁-C₄)oxyalkyle en C₁-C₄) ou phényle.

7. Composés de formule (I), leurs tautomères, leurs sels et leurs produits d'addition d'eau tels que définis selon l'une quelconque des revendications 1 à 6, à l'exception du composé de formule (I) où A = CH, D = C, E = NH et G-L = β-D-ribofuranosyle.

8. Procédé pour la préparation des composés de formule générale (I) ou de leurs sels selon l'une des revendications 1 à 6, **caractérisé en ce que**
a) un composé de formule (II) 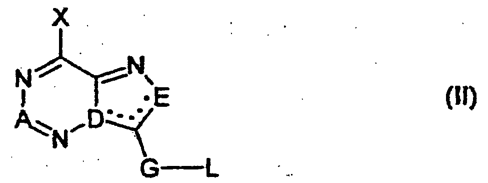 où X représente un groupe partant, est réduit en composé de formule (I), ou
b) un composé de formule (III) 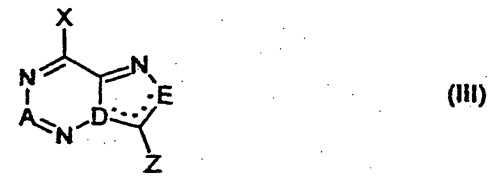 où X représente un groupe partant et Z est un précurseur du reste G-L, est réduit en composé de formule (III') 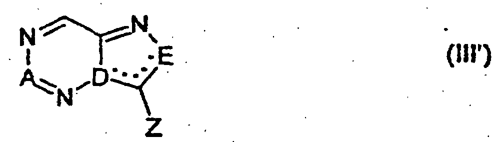 où Z est défini comme à la formule (III), et ensuite le composé (III) est modifié sur le groupe Z de façon à obtenir le composé (I),
c) un composé de formule (III'), où Z est un précurseur du reste G-L, est modifié sur le groupe Z, de façon à obtenir le composé (I), ou
d) dans le cas où A représente un groupe de formule C-R, un composé de formule (III") 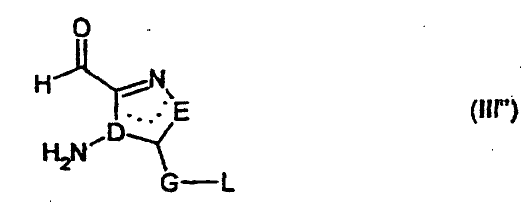 est cyclisé avec un composé de formule (III"')
**H**_{**2**}**N-A=NH (III''')**
,
où A représente un groupe C-R, en composé de formule (I),
dans les formules (II), (III), (III'), (III") et (III"'), les symboles A, D, E, G, L et R, sauf si autrement définis, sont définis comme à la formule (I) .

9. Procédé pour la préparation d'un composé de formule (V) 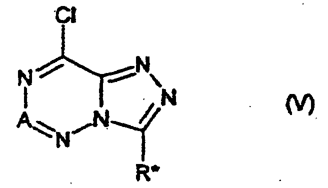 où R* = Z ou G-L et A, G et L sont définis comme à la formule (I) selon la revendication 1, et Z est un précurseur du reste G-L, **caractérisé en ce qu'**on fait réagir un composé de formule (IV) 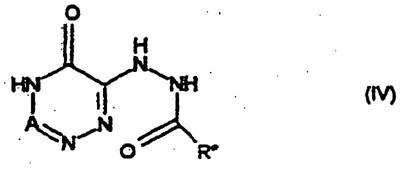 où A et R* sont définis comme à la formule (V),
sur un agent de chloration et on le cyclise en obtenant le composé de formule (V).

10. Composés de formule (V) selon la revendication 9.

11. Procédé pour la préparation d'un composé de formule (VI) 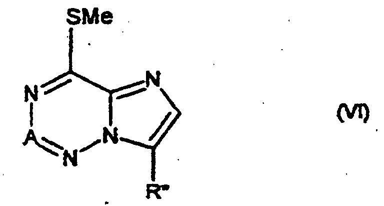 où R* = Z ou G-L et A, G et L sont définis comme à la formule (I) selon la revendication 1, et Z est un précurseur du reste G-L, **caractérisé en ce qu'**un composé de formule (VII) est condensé sur un composé de formule (VIII) 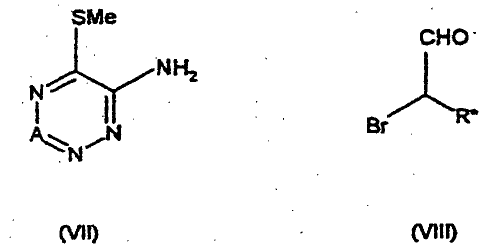 dans les formules (VII) et (VIII) A et R* sont définis comme à la formule (VI), et cyclisé.

12. Composés de formule (V) selon la revendication 11.

13. Agent herbicide ou régulateur, **caractérisé en ce qu'**il renferme un ou plusieurs composés de formule (I), leurs sels, leurs tautomères ou leurs produits d'addition d'eau, selon l'une quelconque des revendications 1 à 7, et des agents de formulation usuels dans le domaine phytosanitaire.

14. Procédé pour la lutte contre les mauvaises herbes ou pour la régulation de la croissance des plantes, **caractérisé en ce qu'**on applique une quantité efficace d'un ou plusieurs composés de formule (I), de leurs sels, de leurs tautomères ou de leurs produits d'addition d'eau, selon l'une quelconque des revendications 1 à 7, aux plantes, aux parties de plantes, à la semence des plantes ou à la surface cultivable.

15. Utilisation de composés de formule (I), de leurs sels, de leurs tautomères ou de leurs produits d'addition d'eau selon l'une quelconque des revendications 1 à 7, en tant qu'herbicides et agents régulateurs de plantes.

16. Utilisation selon la revendication 15, **caractérisée en ce qu'**on utilise les composés de formule (I), leurs sels, leurs tautomères ou leurs produits d'addition d'eau, pour la lutte contre les mauvaises herbes ou pour la régulation de la croissance dans des cultures de plantes utiles ou décoratives.

17. Utilisation selon la revendication 16, **caractérisée en ce que** les plantes de culture sont des plantes transgéniques.

18. Médicament **caractérisé par** une teneur en un composé de formule (I), ses sels, ses tautomères ou ses produits d'addition d'eau, selon la revendication 7.

19. Médicament selon la revendication 18, pour le traitement de maladies, que l'on peut traiter par inhibition des enzymes AMPDA ou ADA.

20. Médicament selon la revendication 19, pour le traitement de maladies du groupe comprenant les troubles d'irrigation sanguine ou l'anoxie.

21. Procédé pour la préparation d'un agent pharmaceutique pour le traitement de maladies, que l'on peut traiter par l'inhibition des enzymes AMPDA ou ADA, **caractérisé en ce qu'**on formule un composé de formule (I), ses sels, ses tautomères ou ses produits d'addition d'eau, selon la revendication 7, en tant qu'inhibiteur conjointement avec des agents de formulation en vue de l'obtention de l'agent.

22. Procédé pour la préparation d'un agent pharmaceutique pour le traitement de maladies appartenant au groupe comprenant
- les troubles d'irrigation,
- l'anoxie,
- l'infarctus du myocarde,
- l'angor de poitrine,
- les maladies cardiovasculaires,
- les maladies douloureuses et
- la maladie d'Alzheimer,
**caractérisé en ce qu'**on formule un composé de formule (I), où A = CH, D = C, E = NH et G-L = β-D-ribofuranosyle, ses sels, ses tautomères ou ses produits d'addition d'eau, conjointement avec des adjuvants de formulation.

23. Procédé selon la revendication 22, **caractérisé en ce que** les maladies appartiennent au groupe comprenant les troubles d'irrigation sanguine ou l'anoxie.
